# EUROPEAN PATENT SPECIFICATION

(11) **EP 3 519 612 B1**
(45) Date of publication and mention of the grant of the patent: **06.04.2022**
(21) Application number: 17857595.7
(22) Date of filing: 29.09.2017
(51) Int. Cl.: C40B 20/04, C40B 40/06, C40B 70/00, B01J 19/00, C12Q 1/6806, C12Q 1/6841

(54) **DNA BARCODE COMPOSITIONS AND METHODS OF IN SITU IDENTIFICATION IN A MICROFLUIDIC DEVICE**
DNA-BARCODE-ZUSAMMENSETZUNGEN UND VERFAHREN ZUR IN-SITU-IDENTIFIZIERUNG IN EINER MIKROFLUIDISCHEN VORRICHTUNG
COMPOSITIONS DE CODE-BARRES D'ADN ET PROCÉDÉS D'IDENTIFICATION IN SITU DANS UN DISPOSITIF MICROFLUIDIQUE

(30) Priority: 01.10.2016 US 201662403116 P; 01.10.2016 US 201662403111 P; 10.02.2017 US 201762457399 P; 10.02.2017 US 201762457582 P; 13.03.2017 US 201762470669 P
(43) Date of publication of application: 07.08.2019
(73) Proprietor: Berkeley Lights, Inc., Emeryville, CA 94608 (US)
(72) Inventor: SOUMILLON, Magali, Boston, MA 02127 (US); BENNETT, Hayley M., Emeryville, CA 94608 (US); MEJIA GONZALES, Yara X., Berkeley, CA 92703 (US); TOH, Mckenzi S., Oakland, CA 94607 (US); RAMENANI, Ravi K., Emeryville, CA 94608 (US)
(74) Representative: D Young & Co LLP
(86) International application number: PCT/US2017/054628
(87) International publication number: WO 2018/064640

(56) References cited:
- WO-A1-2014/047561
- WO-A1-2016/145409
- WO-A2-2005/080604
- US-A1- 2004 126 770
- US-A1- 2009 081 675
- US-A1- 2015 151 298
- US-A1- 2015 267 251
- US-A1- 2015 298 091
- US-A1- 2015 376 608
- H. CHRISTINA FAN ET AL: "Combinatorial labeling of single cells for gene expression cytometry", SCIENCE, vol. 347, no. 6222, 5 February 2015 (2015-02-05), page 1258367, XP055648411, US ISSN: 0036-8075, DOI: 10.1126/science.1258367

## Description

### BACKGROUND OF THE DISCLOSURE

The advent of single cell genome amplification techniques and next generation sequencing methods have led to breakthroughs in our ability to sequence the genome and transcriptome of individual biological cells. Despite these advances, it has remained extremely difficult - and often impossible - to link the genome and transcriptome sequence to the specific phenotype of the cell that was sequenced. As described further herein, the ability to decipher barcodes, such as DNA barcodes, within a microfluidic environment can enable linkage of genomic and transcriptomic data with the cell of origin and its phenotype.

WO 2014/047561 A1 provides methods for uniquely labeling populations of agents of interest using random combinations of oligonucleotides.

WO 2016/145409 A1 provides methods and compositions useful for labeling of target molecules with origin-specific nucleic acid identifiers (for example, barcodes), which can be used subsequently to identify, quantify, or otherwise characterize a feature or activity of target molecules originating from a particular discreet volume.

WO 2005/080604 A2 provides methods for sorting polynucleotides from a population based on predetermined sequence characteristics.

### SUMMARY OF THE DISCLOSURE

In a first aspect, the present invention provides a plurality of capture objects, wherein each capture object of said plurality is a capture object comprising a plurality of capture oligonucleotides, wherein each capture oligonucleotide of said plurality comprises:
a priming sequence;
a capture sequence; and
a barcode sequence comprising three or more cassetable oligonucleotide sequences, each cassetable oligonucleotide sequence being non-identical to the other cassetable oligonucleotide sequences of said barcode sequence, and,
wherein each capture oligonucleotide of said plurality comprises the same barcode sequence,
wherein said barcode sequence of said capture oligonucleotides of each capture object of said plurality is different from the barcode sequence of the capture oligonucleotides of every other capture object of said plurality.

In a second aspect, the present invention provides a set of spectrally distinguishable hybridization probes, each probe comprising:
a non-identical oligonucleotide sequence comprising a sequence of any one of SEQ ID NOs: 41 to 80 and a fluorescent label.

In third aspect, the present invention provides a reagent comprising a plurality of hybridization probes, wherein each hybridization probe of said plurality is a hybridization probe according to the second aspect, and wherein each hybridization probe of said plurality (i) comprises an oligonucleotide sequence which is non-identical to the oligonucleotide sequence of every other hybridization probe of the plurality and (ii) comprises a fluorescent label which is spectrally distinguishable from the fluorescent label of every other hybridization probe of said plurality.

In a fourth aspect, the present invention provides a method of in-situ identification of one or more capture objects from a plurality of capture objects according to the first aspect within a microfluidic device, said method comprising:
disposing a single capture object of said one or more capture objects within an isolation region of each of one or more sequestration pens located within an enclosure of said microfluidic device, wherein each capture object comprises a plurality of capture oligonucleotides, and wherein each capture oligonucleotide of said plurality comprises:
   a priming sequence;
   a capture sequence; and
   a barcode sequence, wherein said barcode sequence comprises three or more cassetable oligonucleotide sequences, each cassetable oligonucleotide sequence being non-identical to the other cassetable oligonucleotide sequences of said barcode sequence; and
   wherein each capture oligonucleotide of said plurality comprises said same barcode sequence;
flowing a first reagent solution comprising a first set of hybridization probes into a flow region within said enclosure of said microfluidic device, wherein said flow region is fluidically connected to each of said one or more sequestration pens, and wherein each hybridization probe of said first set comprises:
   an oligonucleotide sequence complementary to a cassetable oligonucleotide sequence comprised by any of said barcode sequences of any of said capture oligonucleotides of any of said one or more capture objects, wherein said complementary oligonucleotide sequence of each hybridization probe in said first set is non-identical to every other complementary oligonucleotide sequence of said hybridization probes in said first set; and
a fluorescent label selected from a set of spectrally distinguishable fluorescent labels, wherein said fluorescent label of each hybridization probe in said first set is different from the fluorescent label of every other hybridization probe in said first set of hybridization probes;
hybridizing said hybridization probes of said first set to corresponding cassetable oligonucleotide sequences in any of said barcode sequences of any of said capture oligonucleotides of any of said one or more capture objects;
detecting, for each hybridization probe of said first set of hybridization probes, a corresponding fluorescent signal associated with any of said one or more capture objects; and
generating a record, for each capture object disposed within one of said one or more sequestration pens, comprising (i) a location of said sequestration pen within said enclosure of said microfluidic device, and (ii) an association or non-association of said corresponding fluorescent signal of each hybridization probe of said first set of hybridization probes with said capture object, wherein said record of associations and non-associations constitute a barcode

In a fifth aspect, the present invention provides a method of correlating genomic data with a biological cell in a microfluidic device, comprising:
disposing a capture object from a plurality of capture objects according to the first aspect, into a sequestration pen of a microfluidic device, wherein said capture object comprises a plurality of capture oligonucleotides, wherein each capture oligonucleotide of said plurality comprises:
   a priming sequence;
   a capture sequence; and
   a barcode sequence, wherein said barcode sequence comprises three or more cassetable oligonucleotide sequences, each cassetable oligonucleotide sequence being non-identical to the other cassetable oligonucleotide sequences of said barcode sequence; and
wherein each capture oligonucleotide of said plurality comprises said same barcode sequence;
identifying said barcode sequence of said plurality of capture oligonucleotides in-situ and recording an association between said identified barcode sequence and said sequestration pen;
disposing said biological cell into said sequestration pen;
lysing said biological cell and allowing nucleic acids released from said lysed biological cell to be captured by said plurality of capture oligonucleotides comprised by said capture object;
transcribing said captured nucleic acids, thereby producing a plurality of barcoded cDNAs, each barcoded cDNA comprising a complementary captured nucleic acid sequence covalently linked to one of said capture oligonucleotides;
sequencing said transcribed nucleic acids and said barcode sequence, thereby obtaining read sequences of said plurality of transcribed nucleic acids associated with read sequences of said barcode sequence;
identifying said barcode sequence based upon said read sequences; and using said read sequence-identified barcode sequence and said in situ-identified barcode

Compositions, kits and methods are described herein relating to barcoded capture objects, which may be used to generate barcoded RNA-seq libraries and/or genomic DNA libraries from single cells, or small clonal populations of cells, and link sequence obtained from libraries back to the individual cells/clonal populations. The methods are performed in a microfluidic device having an enclosure containing one of more sequestration pens. One advantage of the methods is that cells may be selectively disposed within corresponding sequestration pens in the microfluidic device and their phenotypes may be observed prior to being processed for genome and/or transcriptome sequencing. A key feature of the barcoded capture objects and related methods is that the barcode can be "read" both *in situ* in the microfluidic device and in the sequence reads obtained from the genomic/transcriptomic libraries, thereby enabling linkage of the genomic/transcriptomic data with the observed phenotype of the source cell.

Capture objects in accordance with the first aspect of the invention are provided. The capture objects comprise at least two (e.g. a plurality of) capture oligonucleotides covalently linked to a solid support (e.g., a bead), each capture oligonucleotide having a barcode sequence, a priming sequence, and a capture sequence. The barcode sequences are designed using cassetable oligonucleotides sequences that form a set of non-identical oligonucleotide sequences (also termed "sub-barcode" sequences or "words"). Unique combinations of the cassetable oligonucleotide sequences are linked together to form unique barcode sequences (or "sentences"). Because the cassetable oligonucleotide sequences can be individually decoded using labeled (e.g., fluorescently labeled) probes, a set of hybridization probes complementary to the set of cassetable oligonucleotide sequences is sufficient to identify *in situ* all possible combinations of the cassetable oligonucleotide sequences, and thus all possible barcode sequences that can be generated from the set of cassetable oligonucleotide sequences.

Methods for *in situ* identification of capture objects within a microfluidic device, as well as for correlation of genomic/transcriptomic data with biological micro-objects, are provided. The methods involve disposing a capture object, which can be as described above or elsewhere herein, within a microfluidic device, and identifying/decoding the barcode sequence of the capture oligonucleotides of the capture object *in situ,* using a set of complementary hybridization probes. The microfluidic device in which the *in situ* identification is performed includes an enclosure comprising a flow region and a plurality of sequestration pens that are fluidically connected to the flow region, with the capture object being disposed within one of the sequestration pens. Each of the plurality of sequestration pens can hold at least one biological micro-object and at least one capture object.

One or more hybridization probes containing oligonucleotide sequences complementary to the cassetable oligonucleotide sequences of the barcode, may be introduced to the sequestration pen by flowing a solution including the probes into the flow region of the device. These hybridization probes, which may comprise a label, such as a fluorescent label, are annealed to their target complementary sequences (i.e., corresponding cassetable oligonucleotide sequences) within the barcode sequence of the capture oligonucleotides, thereby allowing the deciphering of the barcoded bead by label (e.g., fluorescence) observed due to the probe/cassetable oligonucleotide sequnce complementarity. The identification of the capture object barcode may be performed at various points during the process of capturing nucleic acids from the biological micro-object and the process of nucleic acid library preparation. The identification process may be performed either before or after nucleic acid from the one biological micro-object has been captured to the capture oligonucleotides or after transcription/reverse transcription. Alternatively, the identification of the capture object may be performed before the biological micro-object is disposed in the sequestration pen of the microfluidic device. The decoding process can be conducted with a system comprising an image acquisition unit.

In certain embodiments, a number of biological micro-objects (e.g., a single cell or a clonal population) may be disclosed in the sequestration pen, either before or after the capture object is disposed within the sequestration pen. The number of capture objects and biological micro-objects introduced into the sequestration pen can be deterministically set. For example, a single capture object and a single biological micro-object can be disposed in a single sequestration pen, a single capture object and a clonal population of biological cells can be disposed in a single sequestration pen, or multiple capture objects and one or more biological micro-objects can be disposed in a single sequestration pen. Prior to introduction into the sequestration pen, the source population of the biological micro-objects can be noted.

Upon lysis of the biological micro-object in the sequestration pen, the capture object can capture the nucleic acids released. The barcode becomes covalently bound to/incorporated within transcripts/genomic DNA fragments of the captured nucleic acids by different mechanisms such reverse-transcription, optionally coupled with PCR (RT-PCR). The barcoded transcripts may be further processed and subsequently sequenced. The genomic data and associated barcodes can be deciphered to permit a match between the specific source sequestration pen and thereby to a source biological micro-object and phenotype thereof.

The process of identification of the barcode of the capture oligonucleotide, and thereby the capture object at a particular location, may be an automated process. The image acquisition unit described herein can further comprise an imaging element configured to capture one or more images of the plurality of sequestration pens and the flow region of the microfluidic device. The system can further comprise an image processing unit communicatively connected to the image acquisition unit. The image processing unit can comprise an area of interest determination engine configured to receive each captured image and define an area of interest for each sequestration pen depicted in the image. The image processing unit can further comprise a scoring engine configured to analyze at least a portion of the image area within the area of interest of each sequestration pen, to determine scores that are indicative of the presence of a particular micro-object and any associated signal arising from a labeled hybridization probe associated therewith in each sequestration pen. The microfluidic device may further comprise at least one coated surface. In some embodiments of the methods, the enclosure of the microfluidic device may include at least one conditioned surface, which may comprise molecules covalently bound thereto, such as hydrophilic polymers and/or anionic polymers.

Described herein is a method for providing a barcoded cDNA library from a biological cell, including: disposing the biological cell within a sequestration pen located within an enclosure of a microfluidic device; disposing a capture object within the sequestration pen, wherein the capture object comprises a plurality of capture oligonucleotides, each capture oligonucleotide of the plurality including: a priming sequence that binds a primer; a capture sequence; and a barcode sequence, wherein the barcode sequence includes three or more cassetable oligonucleotide sequences, each cassetable oligonucleotide sequence being non-identical to every other cassetable oligonucleotide sequences of the barcode sequence; lysing the biological cell and allowing nucleic acids released from the lysed biological cell to be captured by the plurality of capture oligonucleotides comprised by the capture object; and transcribing the captured nucleic acids, thereby producing a plurality of barcoded cDNAs decorating the capture object, each barcoded cDNA including (i) an oligonucleotide sequence complementary to a corresponding one of the captured nucleic acids, covalently linked to (ii) one of the plurality of capture oligonucleotides.

The gene-specific primer sequence may target an mRNA sequence encoding a T cell receptor (TCR). The gene-specific primer sequence may target an mRNA sequence encoding a B-cell receptor (BCR).

The method may further include: identifying the barcode sequence of the plurality of capture oligonucleotides of the capture object in situ, while the capture object is located within the sequestration pen. The method may further include exporting said capture object or said plurality of said capture objects from said microfluidic device.

The enclosure of the microfluidic device may further include a dielectrophoretic (DEP) configuration, and wherein disposing the biological cell and/or disposing the capture object is performed by applying a dielectrophoretic (DEP) force on or proximal to the biological cell and/or the capture object.

Capture objects decorated with barcoded cDNAs may then be exported for further library preparation and sequencing. Barcodes and cDNA may be sequenced and genomic data can be matched to the source sequestration pen number and individual cells/colonies. This process may also be performed within the microfluidic device by an automated process as described herein.

Described herein is a method for providing a barcoded genomic DNA library from a biological micro-object, including disposing a biological micro-object including genomic DNA within a sequestration pen located within an enclosure of a microfluidic device; contacting the biological micro-object with a lysing reagent capable of disrupting a nuclear envelope of the biological micro-object, thereby releasing genomic DNA of the biological micro-object; tagmenting the released genomic DNA, thereby producing a plurality of tagmented genomic DNA fragments having a first end defined by a first tagmentation insert sequence and a second end defined by a second tagmentation insert sequence; disposing a capture object within the sequestration pen, wherein the capture object comprises a plurality of capture oligonucleotides, each capture oligonucleotide of the plurality including: a first priming sequence; a first tagmentation insert capture sequence; and a barcode sequence, wherein the barcode sequence includes three or more cassetable oligonucleotide sequences, each cassetable oligonucleotide sequence being non-identical to every other cassetable oligonucleotide sequence of the barcode sequence; contacting ones of the plurality of tagmented genomic DNA fragments with (i) the first tagmentation insert capture sequence of ones of the plurality of capture oligonucleotides of the capture object, (ii) an amplification oligonucleotide including a second priming sequence linked to a second tagmentation insert capture sequence, a randomized primer sequence, or a gene-specific primer sequence, and (iii) an enzymatic mixture including a strand displacement enzyme and a polymerase; incubating the contacted plurality of tagmented genomic DNA fragments for a period of time, thereby simultaneously amplifying the ones of the plurality of tagmented genomic DNA fragments and adding the capture oligonucleotide and the amplification oligonucleotide to the ends of the ones of the plurality of tagmented genomic DNA fragments to produce the barcoded genomic DNA library; and exporting the barcoded genomic DNA library from the microfluidic device.

The tagmenting may include contacting the released genomic DNA with a transposase loaded with (i) a first double-stranded DNA fragment including the first tagmentation insert sequence, and (ii) a second double-stranded DNA fragment including the second tagmentation insert sequence.

The first double-stranded DNA fragment may include a first mosaic end sequence linked to a third priming sequence, and wherein the second double-stranded DNA fragment may include a second mosaic end sequence linked to a fourth priming sequence.

The method may further include: identifying the barcode sequence of the plurality of capture oligonucleotides of the capture object in situ, while the capture object is located within the sequestration pen.

The enclosure of the microfluidic device may further comprise a dielectrophoretic (DEP) configuration, and wherein disposing the biological micro-object and/or disposing the capture object is performed by applying a dielectrophoretic (DEP) force on or proximal to the biological cell and/or the capture object.

Described herein is a method for providing a barcoded cDNA library and a barcoded genomic DNA library from a single biological cell, including: disposing the biological cell within a sequestration pen located within an enclosure of a microfluidic device; disposing a first capture object within the sequestration pen, where the first capture object comprises a plurality of capture oligonucleotides, each capture oligonucleotide of the plurality comprising: a first priming sequence; a first capture sequence; and a first barcode sequence, wherein the first barcode sequence comprises three or more cassetable oligonucleotide sequences, each cassetable oligonucleotide sequence being non-identical to every other cassetable oligonucleotide sequence of the first barcode sequence; obtaining the barcoded cDNA library by performing any method of obtaining a cDNA library as described herein, where lysing the biological cell is performed such that a plasma membrane of the biological cell is degraded, releasing cytoplasmic RNA from the biological cell, while leaving a nuclear envelope of the biological cell intact, thereby providing the first capture object decorated with the barcoded cDNA library from the RNA of the biological cell; exporting the cDNA library-decorated first capture object from the microfluidic device; disposing a second capture object within the sequestration pen, wherein the second capture object comprises a plurality of capture oligonucleotides, each including: a second priming sequence; a first tagmentation insert capture sequence; and a second barcode sequence, wherein the second barcode sequence comprises three or more cassetable oligonucleotide sequences, each cassetable oligonucleotide sequence being non-identical to every other cassetable oligonucleotide sequence of the second barcode sequence; obtaining the barcoded genomic DNA library by performing any method of obtaining a barcoded genomic DNA library as described herein, where a plurality of tagmented genomic DNA fragments from the biological cell are contacted with the first tagmentation insert capture sequence of ones of the plurality of capture oligonucleotides of the second capture object, thereby providing the barcoded genomic DNA library from the genomic DNA of the biological cell; and exporting the barcoded genomic DNA library from the microfluidic device.

The method may further include: identifying the barcode sequence of the plurality of capture oligonucleotides of the first capture object.

Identifying the barcode sequence of the plurality of capture oligonucleotides of the first capture object may be performed before disposing the biological cell in the sequestration pen; before obtaining the barcoded cDNA library from the RNA of the biological cell; or before exporting the barcoded cDNA library-decorated first capture object from the microfluidic device.

The method may further include: identifying the barcode sequence of the plurality of oligonucleotides of the second capture object.

Described herein is a method for providing a barcoded B cell receptor (BCR) sequencing library, including: generating a barcoded cDNA library from a B lymphocyte, where the generating is performed according to any method of generating a barcoded cDNA as described herein, where the barcoded cDNA library decorates a capture object including a plurality of capture oligonucleotides, each capture oligonucleotide of the plurality including a Not1 restriction site sequence; amplifying the barcoded cDNA library; selecting for barcoded BCR sequences from the barcoded cDNA library, thereby producing a library enriched for barcoded BCR sequences; circularizing sequences from the library enriched for barcoded BCR sequences, thereby producing a library of circularized barcoded BCR sequences; relinearizing the library of circularized barcoded BCR sequences to provide a library of rearranged barcoded BCR sequences, each presenting a constant (C) region of the BCR sequence 3' to a respective variable (V) sub-region and/or a respective diversity (D) sub-region; and, adding a sequencing adaptor and sub-selecting for the V sub-region and/or the D sub-region, thereby producing a barcoded BCR sequencing library.

The method may further include: identifying a barcode sequence of the plurality of capture oligonucleotides of the capture object using any method of identifying a barcode in-situ as described herein. Identifying may be performed before amplifying the barcoded cDNA library. Identifying may be performed while generating the barcoded cDNA library.

### BRIEF DESCRIPTION OF THE DRAWINGS

Figure 1A illustrates an example of a system for use with a microfluidic device and associated control equipment.
Figures 1B and 1C illustrate a microfluidic device.
Figures 2A and 2B illustrate isolation pens .
Figure 2C illustrates a detailed sequestration pen.
Figures 2D-F illustrate sequestration pens .
Figure 2G illustrates a microfluidic device .
Figure 2H illustrates a coated surface of the microfluidic device .
Figure 3A illustrates a specific example of a system for use with a microfluidic device and associated control equipment.
Figure 3B illustrates an imaging device .
Figure 4A illustrates the relationship between an in-situ detectable barcode sequence of a capture object and sequencing data for nucleic acid from a biological cell, where the nucleic acid is captured while within a microfluidic environment and sequenced after export.
Figure 4B is a schematic representation of a variety of nucleic acid workflows possible using an in-situ detectable barcode sequence of a capture object.
Figure 5 is a schematic representation of a capture oligonucleotide of a capture object of the disclosure.
Figure 6 is a schematic representation of capture oligonucleotides of a capture object of the disclosure, having barcode diversity of 10,000 arising from different combinations of cassetable sequences forming the barcode sequences.
Figure 7A is a schematic representation of a process for in-situ detection of a barcode of a capture object .
Figures 7B and 7C are photographic representations of a method of in-situ detection of a barcode sequence of a capture object .
Figures 8A-C are schematic representations of a method of in-situ detection of a barcode sequence of a capture object.
Figures 8D-8F are photographic representations of method of in-situ detection of two or more cassetable oligonucleotide sequences of a barcode sequence of a capture object.
Figure 9 illustrates schematic representations of a workflow for single cell RNA capture, library preparation, and sequencing.
Figures 10A-10D are photographic representations of a process for lysis of an outer cell membrane with subsequent RNA capture.
Figure 11A is a schematic representation of portions of a workflow providing a RNA library.
Figures 11B and 11C are graphical representations of analyses of sequencing library quality.
Figures 12 A-12F are pictorial representations of a workflow for single cell lysis, DNA library preparation, and sequencing.
Figure 12G is a schematic representation of single cell DNA library preparation.
Figures 13A and 13B are schematic representation of a workflow for single cell B cell receptor (BCR) capture, library preparation and sequencing.
Figure 14A is a photographic representation of a method of in-situ detection of a barcode sequence of a capture object.
Figure 14B is a photographic representation of export of a cDNA decorated capture object.
Figures 14C and 14D are graphical representations of the analysis of the quality of a sequencing library.
Figures 15A and 15B are graphical representations of sequencing reads from a library.
Figures 16A-16D are graphical representations of sequencing results obtained from a cDNA sequencing library.
Figure 17 is a graphical representation of the variance in sets of barcode sequences detected across experiments, testing randomization of capture object delivery.
Figure 18 is a graphical representation of the recovery of barcode sequence reads per experiment.
Figure 19 is a photographic representation of T-cells within a microfluidic device.
Figures 20A and 20B are photographic representations of a specific cell during culture, staining for antigen and in-situ barcode sequence detection.
Figures 21A and 21B are photographic representations of a specific cell during culture, staining for antigen and in-situ barcode sequence detection.
Figures 22A and 22B are photographic representations of a specific cell during culture, staining for antigen and in-situ barcode sequence detection.
Figure 23 is a graphical representation of sequencing results across activated, activated antigen-positive and activated antigen-negative cells.
Figure 24 is a photographic representation of substantially singly distributed cells.
Figure 25 is a photographic representation of a process for lysing and releasing nuclear DNA.
Figures 26A and 26B are photographic representations of stained cells prior to and subsequent to lysis.
Figure 27 is a graphical representation of the distribution of genomic DNA in a sequencing library.
Figure 28 is a graphical representation of expected length of chromosomes in sample genomic DNA and further including the experimental coverage observed for each chromosome.
Figures 29A-29D are graphical representations of the genomic DNA library quality.
Figures 30A-30F are photographic representations of a method of obtaining both RNA and genomic DNA sequencing libraries from a single cell.
Figures 31A and 31B are photographic representations of a method of detecting a barcode sequence on a capture object.
Figure 32 is a graphical representation of a correlation between an *in situ* determined barcode sequence, and sequencing results determining the barcode and genomic data.

### DETAILED DESCRIPTION OF THE INVENTION

This specification describes exemplary embodiments and applications of the disclosure.

The figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. Also, unless the context dictates otherwise, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements. Section divisions in the specification are for ease of review only and do not limit any combination of elements discussed.

Where dimensions of microfluidic features are described as having a width or an area, the dimension typically is described relative to an x-axial and/or y-axial dimension, both of which lie within a plane that is parallel to the substrate and/or cover of the microfluidic device. The height of a microfluidic feature may be described relative to a z-axial direction, which is perpendicular to a plane that is parallel to the substrate and/or cover of the microfluidic device. In some instances, a cross sectional area of a microfluidic feature, such as a channel or a passageway, may be in reference to a x-axial/z-axial, a y-axial/z-axial, or an x-axial/y-axial area.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "ones" means more than one.

As used herein, the term "plurality" can be 2, 3, 4, 5, 6, 7, 8, 9, 10, or more.

As used herein: µm means micrometer, µm³ means cubic micrometer, pL means picoliter, nL means nanoliter, and µL (or uL) means microliter.

As used herein, the term "disposed" encompasses within its meaning "located"; and the term "disposing" encompasses within its meaning "placing."

As used herein, a "microfluidic device" or "microfluidic apparatus" is a device that includes one or more discrete microfluidic circuits configured to hold a fluid, each microfluidic circuit comprised of fluidically interconnected circuit elements, including but not limited to region(s), flow path(s), channel(s), chamber(s), and/or pen(s), and at least one port configured to allow the fluid (and, optionally, micro-objects suspended in the fluid) to flow into and/or out of the microfluidic device. Typically, a microfluidic circuit of a microfluidic device will include a flow region, which may include a microfluidic channel, and at least one chamber, and will hold a volume of fluid of less than about 1 mL, e.g., less than about 750, 500, 250, 200, 150, 100, 75, 50, 25, 20, 15, 10, 9, 8, 7, 6, 5, 4, 3, or 2 µL. In certain embodiments, the microfluidic circuit holds about 1-2, 1-3, 1-4, 1-5, 2-5, 2-8, 2-10, 2-12, 2-15, 2-20, 5-20, 5-30, 5-40, 5-50, 10-50, 10-75, 10-100, 20-100, 20-150, 20-200, 50-200, 50-250, or 50-300 µL. The microfluidic circuit may be configured to have a first end fluidically connected with a first port (e.g., an inlet) in the microfluidic device and a second end fluidically connected with a second port (e.g., an outlet) in the microfluidic device.

As used herein, a "nanofluidic device" or "nanofluidic apparatus" is a type of microfluidic device having a microfluidic circuit that contains at least one circuit element configured to hold a volume of fluid of less than about 1 µL, e.g., less than about 750, 500, 250, 200, 150, 100, 75, 50, 25, 20, 15, 10, 9, 8, 7, 6, 5, 4, 3, 2, 1 nL or less. A nanofluidic device may comprise a plurality of circuit elements (e.g., at least 2, 3, 4, 5, 6, 7, 8, 9, 10, 15, 20, 25, 50, 75, 100, 150, 200, 250, 300, 400, 500, 600, 700, 800, 900, 1000, 1500, 2000, 2500, 3000, 3500, 4000, 4500, 5000, 6000, 7000, 8000, 9000, 10,000, or more). In certain embodiments, one or more (e.g., all) of the at least one circuit elements is configured to hold a volume of fluid of about 100 pL to 1 nL, 100 pL to 2 nL, 100 pL to 5 nL, 250 pL to 2 nL, 250 pL to 5 nL, 250 pL to 10 nL, 500 pL to 5 nL, 500 pL to 10 nL, 500 pL to 15 nL, 750 pL to 10 nL, 750 pL to 15 nL, 750 pL to 20 nL, 1 to 10 nL, 1 to 15 nL, 1 to 20 nL, 1 to 25 nL, or 1 to 50 nL. In other embodiments, one or more (e.g., all) of the at least one circuit elements are configured to hold a volume of fluid of about 20 nL to 200nL, 100 to 200 nL, 100 to 300 nL, 100 to 400 nL, 100 to 500 nL, 200 to 300 nL, 200 to 400 nL, 200 to 500 nL, 200 to 600 nL, 200 to 700 nL, 250 to 400 nL, 250 to 500 nL, 250 to 600 nL, or 250 to 750 nL.

A microfluidic device or a nanofluidic device may be referred to herein as a "microfluidic chip" or a "chip"; or "nanofluidic chip" or "chip".

A "microfluidic channel" or "flow channel" as used herein refers to flow region of a microfluidic device having a length that is significantly longer than both the horizontal and vertical dimensions. For example, the flow channel can be at least 5 times the length of either the horizontal or vertical dimension, e.g., at least 10 times the length, at least 25 times the length, at least 100 times the length, at least 200 times the length, at least 500 times the length, at least 1,000 times the length, at least 5,000 times the length, or longer. In some embodiments, the length of a flow channel is about 100,000 microns to about 500,000 microns, including any value therebetween. In some embodiments, the horizontal dimension is about 100 microns to about 1000 microns (e.g., about 150 to about 500 microns) and the vertical dimension is about 25 microns to about 200 microns, (e.g., from about 40 to about 150 microns). It is noted that a flow channel may have a variety of different spatial configurations in a microfluidic device, and thus is not restricted to a perfectly linear element. For example, a flow channel may be, or include one or more sections having, the following configurations: curve, bend, spiral, incline, decline, fork (e.g., multiple different flow paths), and any combination thereof. In addition, a flow channel may have different cross-sectional areas along its path, widening and constricting to provide a desired fluid flow therein. The flow channel may include valves, and the valves may be of any type known in the art of microfluidics. Examples of microfluidic channels that include valves are disclosed in U.S. Patents 6,408,878 and 9,227,200.

As used herein, the term "obstruction" refers generally to a bump or similar type of structure that is sufficiently large so as to partially (but not completely) impede movement of target micro-objects between two different regions or circuit elements in a microfluidic device. The two different regions/circuit elements can be, for example, the connection region and the isolation region of a microfluidic sequestration pen.

As used herein, the term "constriction" refers generally to a narrowing of a width of a circuit element (or an interface between two circuit elements) in a microfluidic device. The constriction can be located, for example, at the interface between the isolation region and the connection region of a microfluidic sequestration pen of the instant disclosure.

As used herein, the term "transparent" refers to a material which allows visible light to pass through without substantially altering the light as is passes through.

As used herein, the term "micro-object" refers generally to any microscopic object that may be isolated and/or manipulated in accordance with the present disclosure. Non-limiting examples of micro-objects include: inanimate micro-objects such as microparticles; microbeads (e.g., polystyrene beads, Luminex^{™} beads, or the like); magnetic beads; microrods; microwires; quantum dots, and the like; biological micro-objects such as cells; biological organelles; vesicles, or complexes; synthetic vesicles; liposomes (e.g., synthetic or derived from membrane preparations); lipid nanorafts, and the like; or a combination of inanimate micro-objects and biological micro-objects (e.g., microbeads attached to cells, liposome-coated micro-beads, liposome-coated magnetic beads, or the like). Beads may include moieties/molecules covalently or non-covalently attached, such as detectable labels, proteins, carbohydrates, antigens, small molecule signaling moieties, or other chemical/biological species capable of use in an assay. Lipid nanorafts have been described, for example, in Ritchie et al. (2009) "Reconstitution of Membrane Proteins in Phospholipid Bilayer Nanodiscs," Methods Enzymol., 464:211-231.

As used herein, the term "cell" is used interchangeably with the term "biological cell." Non-limiting examples of biological cells include eukaryotic cells, plant cells, animal cells, such as mammalian cells, reptilian cells, avian cells, fish cells, or the like, prokaryotic cells, bacterial cells, fungal cells, protozoan cells, or the like, cells dissociated from a tissue, such as muscle, cartilage, fat, skin, liver, lung, neural tissue, and the like, immunological cells, such as T cells, B cells, natural killer cells, macrophages, and the like, embryos (e.g., zygotes), oocytes, ova, sperm cells, hybridomas, cultured cells, cells from a cell line, cancer cells, infected cells, transfected and/or transformed cells, reporter cells, and the like. A mammalian cell can be, for example, from a human, a mouse, a rat, a horse, a goat, a sheep, a cow, a primate, or the like.

A colony of biological cells is "clonal" if all of the living cells in the colony that are capable of reproducing are daughter cells derived from a single parent cell. In certain embodiments, all the daughter cells in a clonal colony are derived from the single parent cell by no more than 10 divisions. In other embodiments, all the daughter cells in a clonal colony are derived from the single parent cell by no more than 14 divisions. In other embodiments, all the daughter cells in a clonal colony are derived from the single parent cell by no more than 17 divisions. In other embodiments, all the daughter cells in a clonal colony are derived from the single parent cell by no more than 20 divisions. The term "clonal cells" refers to cells of the same clonal colony.

As used herein, a "colony" of biological cells refers to 2 or more cells (e.g. about 2 to about 20, about 4 to about 40, about 6 to about 60, about 8 to about 80, about 10 to about 100, about 20 to about 200, about 40 to about 400, about 60 to about 600, about 80 to about 800, about 100 to about 1000, or greater than 1000 cells).

As used herein, the term "maintaining (a) cell(s)" refers to providing an environment comprising both fluidic and gaseous components and, optionally a surface, that provides the conditions necessary to keep the cells viable and/or expanding.

As used herein, the term "expanding" when referring to cells, refers to increasing in cell number.

A "component" of a fluidic medium is any chemical or biochemical molecule present in the medium, including solvent molecules, ions, small molecules, antibiotics, nucleotides and nucleosides, nucleic acids, amino acids, peptides, proteins, sugars, carbohydrates, lipids, fatty acids, cholesterol, metabolites, or the like.

As used herein, "capture moiety" is a chemical or biological species, functionality, or motif that provides a recognition site for a micro-object. A selected class of micro-objects may recognize the in situ-generated capture moiety and may bind or have an affinity for the in situ-generated capture moiety. Non-limiting examples include antigens, antibodies, and cell surface binding motifs.

As used herein, "B" used to denote a single nucleotide, is a nucleotide selected from G (guanosine), C (cytidine) and T (thymidine) nucleotides but does not include A (adenine).

As used herein, "H" used to denote a single nucleotide, is a nucleotide selected from A, C and T, but does not include G.

As used herein, "D" used to denote a single nucleotide, is a nucleotide selected from A, G, and T, but does not include C.

As used herein, "V" used to denote a single nucleotide, is a nucleotide selected from A, G, and C, and does not include T.

As used herein, "N" used to denote a single nucleotide, is a nucleotide selected from A, C, G, and T.

As used herein, "S" used to denote a single nucleotide, is a nucleotide selected from G and C.

As used herein, "Y" used to denote a single nucleotide, is a nucleotide selected from C and T.

As used herein, A, C, T, G followed by "^{∗}" indicates phosophorothioate substitution in the phosphate linkage of that nucleotide.

As used herein, IsoG is isoguanosine; IsoC is isocytidine; IsodG is a isoguanosine deoxyribonucleotide and IsodC is a isocytidine deoxyribonucleotide. Each of the isoguanosine and isocytidine ribo- or deoxyribo- nuleotides contain a nucleobase that is isomeric to guanine nucleobase or cytosine nucleobase, respectively, usually incorporated within RNA or DNA.

As used herein, rG denotes a ribonucleotide included within a nucleic acid otherwise containing deoxyribonucleotides. A nucleic acid containing all ribonucleotides may not include labeling to indicated that each nucleotide is a ribonucleotide, but is made clear by context.

As used herein, a "priming sequence" is an oligonucleotide sequence which is part of a larger oligonucleotide and, when separated from the larger oligonucleotide such that the priming sequence includes a free 3' end, can function as a primer in a DNA (or RNA) polymerization reaction.

As used herein, "antibody" refers to an immunoglobulin (Ig) and includes both polyclonal and monoclonal antibodies; primatized (e.g., humanized); murine; mouse-human; mouse-primate; and chimeric; and may be an intact molecule, a fragment thereof (such as scFv, Fv, Fd, Fab, Fab' and F(ab)'2 fragments), or multimers or aggregates of intact molecules and/or fragments; and may occur in nature or be produced, e.g., by immunization, synthesis or genetic engineering. An "antibody fragment," as used herein, refers to fragments, derived from or related to an antibody, which bind antigen and which in some embodiments may be derivatized to exhibit structural features that facilitate clearance and uptake, e.g., by the incorporation of galactose residues. This includes, e.g., F(ab), F(ab)'2, scFv, light chain variable region (VL), heavy chain variable region (VH), and combinations thereof.

An antigen, as referred to herein, is a molecule or portion thereof that can bind with specificity to another molecule, such as an Ag-specific receptor. Antigens may be capable of inducing an immune response within an organism, such as a mammal (e.g., a human, mouse, rat, rabbit, etc.), although the antigen may be insufficient to induce such an immune response by itself. An antigen may be any portion of a molecule, such as a conformational epitope or a linear molecular fragment, and often can be recognized by highly variable antigen receptors (B-cell receptor or T-cell receptor) of the adaptive immune system. An antigen may include a peptide, polysaccharide, or lipid. An antigen may be characterized by its ability to bind to an antibody's variable Fab region. Different antibodies have the potential to discriminate among different epitopes present on the antigen surface, the structure of which may be modulated by the presence of a hapten, which may be a small molecule.

In some embodiments, an antigen is a cancer cell- associated antigen. The cancer cell-associated antigen can be simple or complex; the antigen can be an epitope on a protein, a carbohydrate group or chain, a biological or chemical agent other than a protein or carbohydrate, or any combination thereof; the epitope may be linear or conformational.

The cancer cell-associated antigen can be an antigen that uniquely identifies cancer cells (e.g., one or more particular types of cancer cells) or is upregulated on cancer cells as compared to its expression on normal cells. Typically, the cancer cell-associated antigen is present on the surface of the cancer cell, thus ensuring that it can be recognized by an antibody. The antigen can be associated with any type of cancer cell, including any type of cancer cell that can be found in a tumor known in the art or described herein. In particular, the antigen can be associated with lung cancer, breast cancer, melanoma, and the like. As used herein, the term "associated with a cancer cells," when used in reference to an antigen, means that the antigen is produced directly by the cancer cell or results from an interaction between the cancer cell and normal cells.

As used herein in reference to a fluidic medium, "diffuse" and "diffusion" refer to thermodynamic movement of a component of the fluidic medium down a concentration gradient.

The phrase "flow of a medium" means bulk movement of a fluidic medium primarily due to any mechanism other than diffusion. For example, flow of a medium can involve movement of the fluidic medium from one point to another point due to a pressure differential between the points. Such flow can include a continuous, pulsed, periodic, random, intermittent, or reciprocating flow of the liquid, or any combination thereof. When one fluidic medium flows into another fluidic medium, turbulence and mixing of the media can result.

The phrase "substantially no flow" refers to a rate of flow of a fluidic medium that, averaged over time, is less than the rate of diffusion of components of a material (e.g., an analyte of interest) into or within the fluidic medium. The rate of diffusion of components of such a material can depend on, for example, temperature, the size of the components, and the strength of interactions between the components and the fluidic medium.

As used herein in reference to different regions within a microfluidic device, the phrase "fluidically connected" means that, when the different regions are substantially filled with fluid, such as fluidic media, the fluid in each of the regions is connected so as to form a single body of fluid. This does not mean that the fluids (or fluidic media) in the different regions are necessarily identical in composition. Rather, the fluids in different fluidically connected regions of a microfluidic device can have different compositions (e.g., different concentrations of solutes, such as proteins, carbohydrates, ions, or other molecules) which are in flux as solutes move down their respective concentration gradients and/or fluids flow through the microfluidic device.

As used herein, a "flow path" refers to one or more fluidically connected circuit elements (e.g. channel(s), region(s), chamber(s) and the like) that define, and are subject to, the trajectory of a flow of medium. A flow path is thus an example of a swept region of a microfluidic device. Other circuit elements (e.g., unswept regions) may be fluidically connected with the circuit elements that comprise the flow path without being subject to the flow of medium in the flow path.

As used herein, "isolating a micro-object" confines a micro-object to a defined area within the microfluidic device.

A microfluidic (or nanofluidic) device can comprise "swept" regions and "unswept" regions. As used herein, a "swept" region is comprised of one or more fluidically interconnected circuit elements of a microfluidic circuit, each of which experiences a flow of medium when fluid is flowing through the microfluidic circuit. The circuit elements of a swept region can include, for example, regions, channels, and all or parts of chambers. As used herein, an "unswept" region is comprised of one or more fluidically interconnected circuit element of a microfluidic circuit, each of which experiences substantially no flux of fluid when fluid is flowing through the microfluidic circuit. An unswept region can be fluidically connected to a swept region, provided the fluidic connections are structured to enable diffusion but substantially no flow of media between the swept region and the unswept region. The microfluidic device can thus be structured to substantially isolate an unswept region from a flow of medium in a swept region, while enabling substantially only diffusive fluidic communication between the swept region and the unswept region. For example, a flow channel of a microfluidic device is an example of a swept region while an isolation region (described in further detail below) of a microfluidic device is an example of an unswept region.

**Generating gDNA sequencing libraries from one or more cells within a microfluidic environment.** Generation of DNA sequencing data with a cross-reference to the physical location of cells cultured, observed or phenotyped within a microfluidic device is a highly desirable improvement to currently available sequencing strategies. Reasons for sequencing gDNA include characterization of variation or mutations within the DNA of cells, assessment of gene editing events, and process validation for clonality. The ability to correlate sequencing data to the specific cell(s) from which the DNA was isolated has not been previously available.

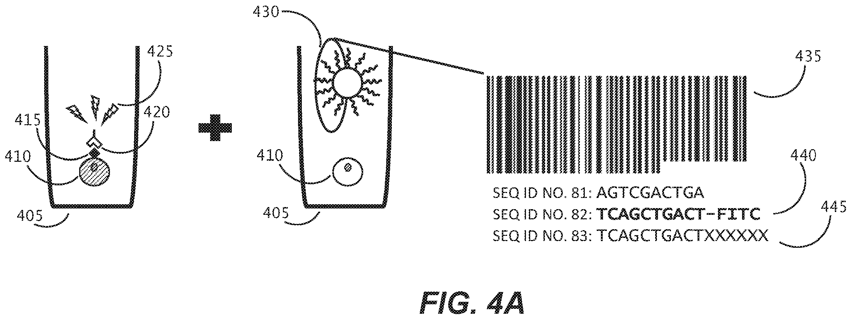

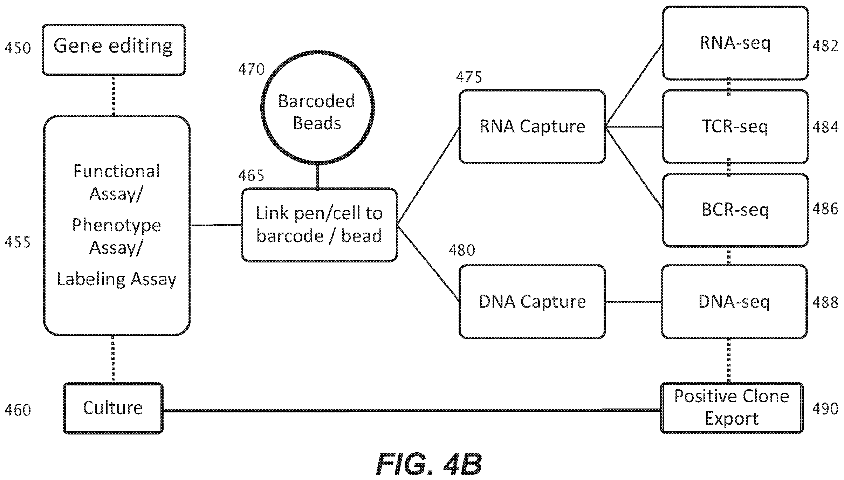

A workflow for generating DNA sequencing libraries from cells within a microfluidic device which introduces a barcode sequence that can be read both in-situ within the microfluidic device and from the resultant sequencing data is described herein. The ability to decipher barcodes within the microfluidic environment permits linkage of genomic data to cellular phenotype. As shown in Figure 4, a biological cell 410 may be disposed within a sequestration pen 405 within the enclosure of a microfluidic device, and maintained and assayed there. During the assay, which may be, but is not limited to an assay detecting a cell surface marker 415, a reagent 420 (e.g., which may be an antibody) may bind to the cell surface marked 415 and permit detection of a detectable signal 425 upon so binding. This phenotype can be connected to genomic data from that specific biological cell 410, by using the methods described herein to capture nucleic acid released from cell 410 with capture object 430, which includes a barcode sequence 435 comprising three or more cassetable oligonucleotide sequences. The barcode 435 can be detected in-situ by fluorescent probe 440 in detection methods described herein. The released nucleic acid captured to the capture object 430 can be used to generate a sequencing library, which upon being sequenced, provides sequencing data 445 that includes both the genomic information from the released nucleic acid of biological cell 405 and the sequence of the associated barcode 435. A correlation between phenotype and genomic data is thus provided. This ability provides entry into a generalized workflow as shown in Figure 4B. For instance, cells coming through a pathway, which may include gene editing 450, functional /phenotypic assay or a labeling assay 455, either before, after or during cell culture 460, can enter a linking process 465 using barcoded capture beads 470, to then capture RNA (475) and/or DNA (480), and provide RNA-seq 482, T cell Receptor (TCR)-seq 484, B cell Receptor (BCR)-seq 486; or DNA seq (488) data that is correlated back to the source cell. If the cell was part of a clonal population, positive clone export 490 can result.

Further, using the protocols described herein for RNA capture/library prep, and DNA capture/library prep, sequencing results for both RNA and DNA may be obtained from the same single cell, and may be correlated to the location within the microfluidic device of the specific single cell source of the sequenced RNA and DNA.

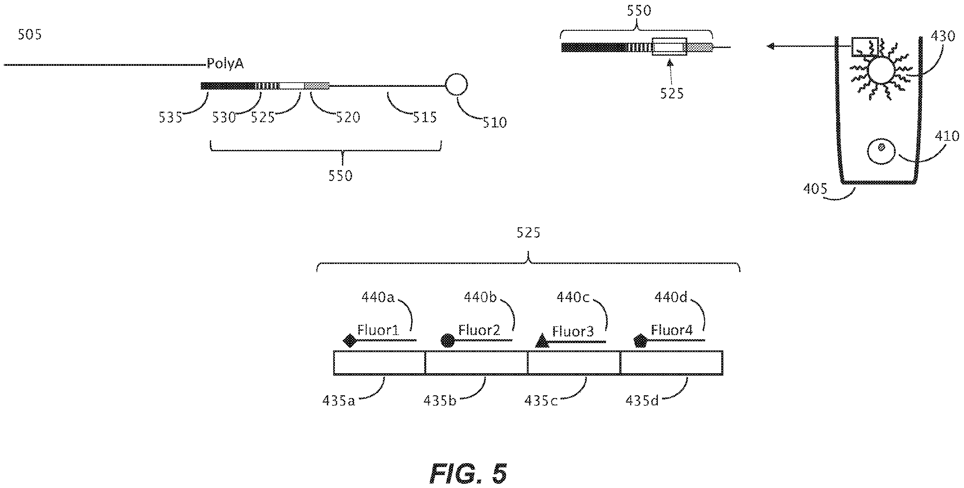

DNA barcodes 525 are described herein, which are designed using cassetable (e.g., changeable sub-units 435a, 435b, 435c, 435d, that in some embodiments, may be completely interchangeable) sub-barcodes or "words" that are individually decoded using fluorescence, as shown schematically in Figure 5. Detection of the barcode may be performed in-situ, by detecting each of the four cassetable oligonucleotide sequences using complementary fluorescently labeled hybridization probes. As shown here, cassetable oligonucleotide sequence 435a is detected in-situ by hybridization with hybridization probe 440a, which includes fluorophore Fluor 1. Respectively, the second cassetable oligonucleotide sequence 435b may be similarly detected by hybridization probe 440b (including Fluor 2); the third cassetable oligonucleotide sequence 435c may be detected by hybridization probe 440c having Fluor 3, and the four cassetable oligonucleotide sequence 435d may be detected by hybridization probe 440c, having Fluor 4. Each of the fluorophores Fluor 1, Fluor 2, Fluor 3, and Fluor 4 are spectrally distinguishable, permitting unequivocal identification of each respective cassetable oligonucleotide sequence.

In the method illustrated in Figure 5, capture objects 430, which comprise beads 510 carrying a plurality of capture oligonucleotides (a single capture oligonucleotide 550 of the plurality is shown) which each include the DNA barcode 525 along with a priming sequence 520 may be introduced to each sequestration pen 405 as one capture object430 /one barcode 525 for one cell 410 /one cell colony (not shown). More than one capture object may be placed into a sequestration pen to capture a greater quantity of nucleic acid from the biological cells under examination.

A schematic representation is presented in Figure 5 of the capture of nucleic acid released from the biological cell 410 upon lysis (the released nucleic acid may be RNA 505), by the capture object 430 comprising a bead 510 linked via linker 515 to the capture oligonucleotide 550 including a priming sequence 520, barcode 525, optional Unique Molecular Identifier (UMI) 525, and capture sequence 535. In this example, barcode 525 includes four cassetable oligonucleotide sequences 435a, 435b, 435c, and 435d. In this example, capture sequence 535 of the capture oligonucleotide captures the released nucleic acid 505 by hybridizing with the Poly A segment of the released nucleic acid 505.

Cells to be lysed may either be imported into the microfluidic device specifically for library preparation and sequencing or may be present within the microfluidic device, being maintained for any desirable period of time.

**Capture object.** A capture object includes a plurality of capture oligonucleotides, wherein each of said plurality includes: a priming sequence which is a primer binding sequence; a capture sequence; and a barcode sequence comprising three or more cassetable oligonucleotide sequences, each cassetable oligonucleotide sequence being non-identical to the other cassetable oligonucleotide sequences of said barcode sequence.

Each capture oligonucleotide comprises a 5'-most nucleotide and a 3'-most nucleotide. In various embodiments, the priming sequence may be adjacent to or comprises said 5'-most nucleotide. In various embodiments, the capture sequence may be adjacent to or comprises said 3'-most nucleotide. Typically, the barcode sequence may be located 3' to the priming sequence and 5' to the capture sequence.

**Capture object composition.** Typically, the capture object has a composition such that it is amenable to movement using a dielectrophoretic (DEP) force, such as a negative DEP force. For example, the capture object can be a bead (or similar object) having a core that includes a paramagnetic material, a polymeric material and/or glass. The polymeric material may be polystyrene or any other plastic material which may be functionalized to link the capture oligonucleotide. The core material of the capture object may be coated to provide a suitable material to attach linkers to the capture oligonucleotide, which may include functionalized polymers, although other arrangements are possible. The linkers used to link the capture oligonucleotides to the capture object may be any suitable linker as is known in the art. The linker may include hydrocarbon chains, which may be unsubstituted or substituted, or interrupted or non-interrupted with functional groups such as amide, ether or keto- groups, which may provide desirable physicochemical properties. The linker may have sufficient length to permit access by processing enzymes to priming sites near the end of the capture oligonucleotide linked to the linker. The capture oligonucleotides may be linked to the linker covalently or non-covalently, as is known in the art. A nonlimiting example of a non-covalent linkage to the linker may be via a biotin/streptavidin pair.

The capture object may be of any suitable size, as long as it is small enough to passage through the flow channel(s) of the flow region and into/out of a sequestration pen of any microfluidic device as described herein. Further, the capture object may be selected to have a sufficiently large number of capture oligonucleotides linked thereto, such that nucleic acid may be captured in sufficient quantity to generate a nucleic acid library useful for sequencing. In some embodiments, the capture object may be a spherical or partially spherical bead and have a diameter greater than about 5 microns and less than about 40 microns. In some embodiments, the spherical or partially spherical bead may have a diameter of about 5, about 7, about 8, about 10, about 12, about 14, about 16, about 18, about 20, about 22, about 24, or about 26 microns.

Each capture oligonucleotide attached to a capture object has the same barcode sequence, and each capture object has a unique barcode sequence. Using capture beads having unique barcodes on each capture bead permits unique identification of the sequestration pen into which the capture object is placed. In experiments where a plurality of cells is placed within sequestration pens, often singly, a plurality of capture objects are also delivered and placed into the occupied sequestration pens, one capture bead per sequestration. Each of the plurality of capture beads has a unique barcode, and the barcode is non-identical to any other barcode of any other capture present within the microfluidic device. As a result, the cell (or, in some embodiments, cells) within the sequestration pen, will have a unique barcode identifier incorporated within its sequencing library.

**Barcode Sequence.** The barcode sequence includes three or more (e.g., 3, 4, 5, or more) cassetable oligonucleotide sequences, each of which is non-identical to the other cassetable oligonucleotide sequences of the barcode sequence. A barcode sequence is "non-identical" to other barcode sequences in a set when the n (e.g., three or more) cassetable oligonucleotide sequences of any one barcode sequence in the set of barcode sequences do not completely overlap with the n' (e.g., three or more) cassetable oligonucleotide sequences of any other barcode sequence in the set of barcode sequences; partial overlap (e.g., up to n-1) is permissible, so long as each barcode sequence in the set is different from every other barcode sequence in the set by a minimum of 1 cassetable oligonucleotide sequence. In certain embodiments, the barcode sequence consists of (or consists essentially of) three or more (e.g., 3, 4, 5, or more) cassetable oligonucleotide sequences. As used herein, a "cassetable oligonucleotide sequence" is an oligonucleotide sequence that is one of a defined set of oligonucleotide sequences (e.g., a set of 12 or more oligonucleotide sequences) wherein, for each oligonucleotide sequence in the defined set, the complementary oligonucleotide sequence (which can be part of a hybridization probe, as described elsewhere herein) does not substantially hybridize to any of the other oligonucleotide sequences in the defined set of oligonucleotide sequences. In certain embodiments, all (or substantially all) of the oligonucleotide sequences in the defined set will have the same length (or number of nucleotides). For example, the oligonucleotides sequences in the defined set can all have a length of 10 nucleotides. However, other lengths are also suitable for use in the present invention, ranging from about 6 nucleotides to about 15 nucleotides. Thus, for example, each oligonucleotide sequence in the defined set, for substantially all oligonucleotide sequences in the defined set, can have a length of 6 nucleotides, 7 nucleotides, 8 nucleotides, 9 nucleotides, 10 nucleotides, 11 nucleotides, 12 nucleotides, 13 nucleotides, 14 nucleotides, or 15 nucleotides. Alternatively, each or substantially all oligonucleotide sequences in the defined set may have length of 6-8, 7-9, 8-10, 9-11, 10-12, 11-12, 12-14, or 13-16 nucleotides.

Each oligonucleotide sequence selected from the defined set of oligonucleotide sequences (and, thus, in a barcode sequence) can be said to be "non-identical" to the other oligonucleotide sequences in the defined set (and thus, the barcode sequence) because each oligonucleotide sequence can be specifically identified as being present in a barcode sequence based on its unique nucleotide sequence, which can be detected both by (i) sequencing the barcode sequence, and (ii) performing a hybridization reaction with a probe (e.g., hybridization probe) that contains an oligonucleotide sequence that is complementary to the cassetable oligonucleotide sequence.

In some embodiments, the three or more cassetable oligonucleotide sequences of the barcode sequence are linked in tandem without any intervening oligonucleotide sequences. In other embodiments, the three or more cassetable oligonucleotide sequences may have one or more linkage between one of the cassetable oligonucleotides and its neighboring cassetable oligonucleotide that is not a direct linkage. Such linkages between any of the three or more cassetable oligonucleotide sequences may be present to facilitate synthesis by ligation rather than by total synthesis. In various embodiments, however, the oligonucleotide sequences of the cassetable oligonucleotides are not interrupted by any other of the other oligonucleotide sequences forming one or more priming sequences, optional index sequences, optional Unique Molecular Identifier sequences or optional restriction sites, including but not limited to Not1 restriction site sequences.

As used herein in connection with cassetable oligonucleotide sequences and their complementary oligonucleotide sequences (including hybridization probes that contain all or part of such complementary oligonucleotide sequences), the term "substantially hybridize" means that the level of hybridization between a cassetable oligonucleotide sequence and its complementary oligonucleotide sequence is above a threshold level, wherein the threshold level is greater than and experimentally distinguishable from a level of cross-hybridization between the complementary oligonucleotide sequence and any other cassetable oligonucleotide sequence in the defined set of oligonucleotide sequences. As persons skilled in the art will readily understand, the threshold for determining whether a complementary oligonucleotide sequence does or does not substantially hybridize to a particular cassetable oligonucleotide sequence depends upon a number of factors, including the length of the cassetable oligonucleotide sequences, the components of the solution in which the hybridization reaction is taking place, the temperature at which the hybridization reaction is taking place, and the chemical properties of the label (which may be attached to the complementary oligonucleotide sequence) used to detect hybridization. Applicants have provided exemplary conditions that can be used to defined sets of oligonucleotide sequences that are non-identical, but persons skilled in the art can readily identify additional conditions that are suitable.

Each of the three or more cassetable oligonucleotide sequences may be selected from a set of at least 12 cassetable oligonucleotide sequences. For example, the set can include at least 12, 15, 16, 18, 20, 21, 24, 25, 27, 28, 30, 32, 33, 35, 36, 39, 40, 42, 44, 45, 48, 50, 51, 52, 54, 55, 56, 57, 60, 63, 64, 65, 66, 68, 69, 70, 72, 75, 76, 78, 80, 81, 84, 85, 87, 88, 90, 92, 93, 95, 96, 99, 100, or more, including any number in between any of the foregoing.

A set of forty cassetable oligonucleotide sequences SEQ ID. Nos. 1-40 as shown in Table 1 has been designed for use in the in-situ detection methods, using 10-mer oligonucleotides, which optimally permits fluorophore probe hybridization during detection. At least 6 bases of the 10mer are differentiated to prevent mis-annealing in the detection methods. The set was designed using the barcode generator python script from the Comai lab: (http://comailab.genomecenter.ucdavis.edu/index.php/Barcode_generator), and further selection to the sequences shown, was based on selecting for sequences having a Tm (Melting Temperature) of equal to or greater than 28°C. The Tm calculation was performed using the IDT OligoAnalyzer 3.1(https://www.idtdna.com/calc/analyzer).

**Table 1. Cassetable oligonucleotide sequences for incorporation within a barcode, and hybridization probe sequences for in-situ detection thereof.**

| **Barcode name** | **SEQ ID No.** | **Barcode sequence** | **Probe name** | **SEQ ID No.** | **Probe sequence** | **Fluorescent channel** |
|---|---|---|---|---|---|---|
| BC1_C1 | 1 | CAGCCTTCTG | probe_C1 | 41 | CAGAAGGCTG/3AlexF647N/ | Cy5 |
| BC1_C2 | 2 | TGTGAGTTCC | probe_C2 | 42 | GGAACTCACA/3AlexF647N/ | Cy5 |
| BC1_C3 | 3 | GAATACGGGG | probe_C3 | 43 | CCCCGTATTC/3AlexF647N/ | Cy5 |
| BC1_ C4 | 4 | CTTTGGACCC | probe_C4 | 44 | GGGTCCAAAG/3AlexF647N/ | Cy5 |
| BC1_C5 | 5 | GCCATACACG | probe_C5 | 45 | CGTGTATGGC/3AlexF647N/ | Cy5 |
| BC1_C6 | 6 | AAGCTGAAGC | probe C6 | 46 | GCTTCAGCTT/3AlexF647N/ | Cy5 |
| BC1_C7 | 7 | TGTGGCCATT | probe C7 | 47 | AATGGCCACA/3AlexF647N/ | Cy5 |
| BC1_C8 | 8 | CGCAATCTCA | probe_C8 | 48 | TGAGATTGCG/3AlexF647N/ | Cy5 |
| BC1_C9 | 9 | TGCGTTGTTG | probe_C9 | 49 | CAACAACGCA/3AlexF647N/ | Cy5 |
| BC1_C10 | 10 | TACAGTTGGC | probe C10 | 50 | GCCAACTGTA/3AlexF647N/ | Cy5 |
| BC2_D11 | 11 | TTCCTCTCGT | probe D11 | 51 | /5AlexF405N/ ACGAGAGGAA | Dapi |
| BC2_D12 | 12 | GACGTTACGA | probe_D12 | 52 | /5AlexF405N/TCGTAACGTC | Dapi |
| BC2_D13 | 13 | ACTGACGCGT | probe_D13 | 53 | /5AlexF405N/ACGCGTCAGT | Dapi |
| BC2_D14 | 14 | AGGAGCAGCA | probe_D14 | 54 | /5AlexF405N/TGCTGCTCCT | Dapi |
| BC2_D15 | 15 | TGACGCGCAA | probe D15 | 55 | /5AlexF405N/TTGCGCGTCA | Dapi |
| BC2_D16 | 16 | TCCTCGCCAT | probe_D16 | 156 | /5AlexF405N/ATGGCGAGGA | Dapi |
| BC2_D17 | 17 | TAGCAGCCCA | probe_D17 | 57 | /5AlexF405NATGGGCTGCTA | Dapi |
| BC2_D18 | 18 | CAGACGCTGT | probe_D18 | 58 | /5AlexF405N/ACAGCGTCTG | Dapi |
| BC2_D19 | 19 | TGGAAAGCGG | probe_D19 | _{'}59 | /5AlexF405N/CCGCTTTCCA | Dapi |
| BC2_D20 | 20 | GCGACAAGAC | probe D20 | 60 | /5AlexF405N/GTCTTGTCGC | Dapi |
| BC3_F21 | 21 | TGTCCGAAAG | probe_F21 | 61 | CTTTCGGACA/3AlexF488N/ | FITC |
| BC3_F22 | 22 | AACATCCCTC | probe_F22 | 62 | GAGGGATGTT/3AlexF488N/ | FITC |
| BC3_F23 | 23 | AAATGTCCCG | probe_F23 | 63 | CGGGACATTT/3AlexF488N/ | FITC |
| BC3_F24 | 24 | TTAGCGCGTC | probe F24i | 64 | GACGCGCTAA/3AlexF488N/ | FITC |
| BC3_F25 | 25 | AGTTCAGGCG | probe F25 | 65 | CGCCTGAACT/3AlexF488N/ | FITC |
| BC3_F26 | 26 | ACAGGGGAAC | probe F26 | 66 | GTTCCCCTGT/3AlexF488N/ | FITC |
| BC3_F27 | 27 | ACCGGATTGG | probe_F27 | 67 | CCAATCCGGT/3AlexF488N/ | FITC |
| BC3_F28 | 28 | TCGTGTGTGA | probe_F28 | 68 | TCACACACGA/3AlexF488N/ | FITC |
| BC3_F29 | 29 | TAGGTCTGCG | probe F29 | 69 | CGCAGACCTA/3AlexF488N/ | FITC |
| BC3_F30 | 30 | ACCCATACCC | probe_F30 | 70 | GGGTATGGGT/3AlexF488N/ | FITC |
| BC4_T31 | 31 | CCGCACTTCT | probe_T31 | 71 | AGAAGTGCGG/3AlexF594N/ | Texas Red |
| BC4_T32 | 32 | TTGGGTACAG | probe_T32 | 72 | CTGTACCCAA/3AlexF594N/ | Texas Red |
| BC4_T33 | 33 | ATTCGTCGGA | probe_T33 | 73 | TCCGACGAAT/3AlexF594N/ | Texas Red |
| BC4_T34 | 34 | GCCAGCGTAT | probe_T34 | 74 | ATACGCTGGC/3AlexF594N/ | Texas Red |
| BC4_T35 | 35 | GTTGAGCAGG | probe_T35 | 75 | CCTGCTCAAC/3AlexF594N/ | Texas Red |
| BC4_T36 | 36 | GGTACCTGGT | probe_T36 | 76 | ACCAGGTACC/3AlexF594N/ | Texas Red |
| BC4_T37 | 37 | GCATGAACGT | probe_T37 | 77 | ACGTTCATGC/3AlexF594N/ | Texas Red |
| BC4_T38 | 38 | TGGCTACGAT | probe_T38 | 78 | ATCGTAGCCA/3AlexF594N/ | Texas Red |
| BC4_T39 | 39 | CGAAGGTAGG | probe T39 | 79 | CCTACCTTCG/3AlexF594N/ | Texas Red |
| BC4_T40- | 40 | TTCAACCGAG | probe_T40 | 80 | CTCGGTTGAA/3AlexF594N/ | Texas Red |

In various embodiments, each of the three or more cassetable oligonucleotides sequences of a barcode sequence has a sequence of any one of SEQ ID NOs: 1-40, wherein none of the three or more cassetable oligonucleotides are identical. The cassetable sequences may be presented within the capture oligonucleotide in any order; the order does not change the in-situ detection and the sequences of each of the cassetable oligonucleotide sequences can be deconvoluted from the sequencing reads. In some embodiments, the barcode sequence may have four cassetable oligonucleotide sequences.

In some embodiments, a first cassetable oligonucleotide sequence of a barcode has a sequence selected from a first sub-set of SEQ ID Nos. 1-40; a second cassetable sequence of a barcode has a sequence selected from a second sub-set of SEQ ID Nos. 1-40; a third cassetable sequence of a barcode has a sequence selected from a third sub-set of SEQ ID Nos. 1-40; and a fourth cassetable sequence of a barcode has a sequence selected from a foruth sub-set of SEQ ID Nos. 1-40;

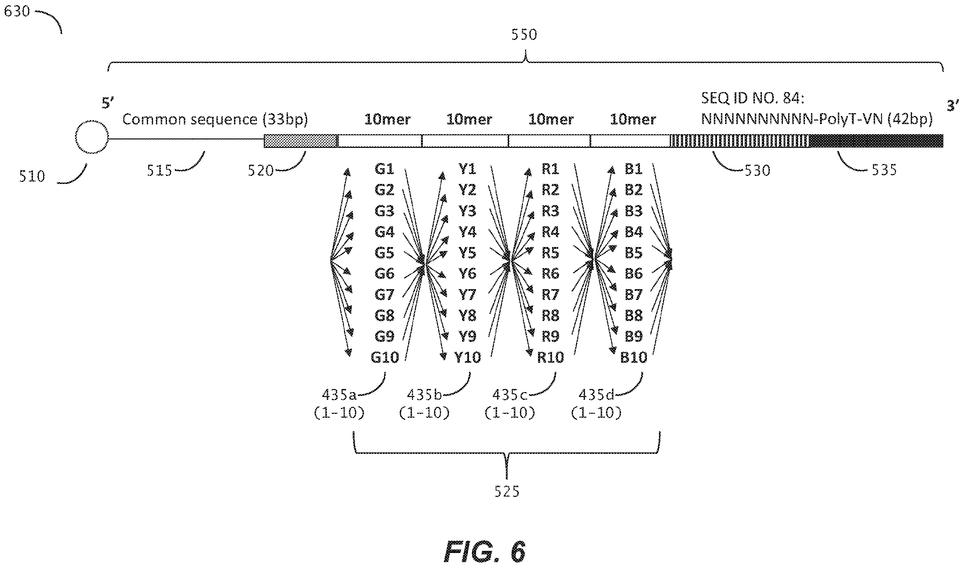

In some embodiments, a first cassetable oligonucleotide sequence of a barcode has a sequence of any one of SEQ ID NOs: 1-10; a second cassetable oligonucleotide sequence of the barcode has a sequence of any one of SEQ ID NOs: 11- 20; a third cassetable oligonucleotide sequence of the barcode has a sequence of any one of SEQ ID NOs: 21-30; and a fourth cassetable oligonucleotide sequence of the barcode has a sequence of any one of SEQ ID NOs: 31-40. In some embodiments, when a first cassetable oligonucleotide sequence of a barcode has a sequence of any one of SEQ ID NOs: 1-10; a second cassetable oligonucleotide sequence of the barcode has a sequence of any one of SEQ ID NOs: 11- 20; a third cassetable oligonucleotide sequence of the barcode has a sequence of any one of SEQ ID NOs: 21-30; and a fourth cassetable oligonucleotide sequence of the barcode has a sequence of any one of SEQ ID NOs: 31-40, each of the first, second, third and fourth cassetable oligonucleotide sequences are located along the length of the capture oligonucleotide in order, 5'to 3' of the barcode sequence, That is, the first cassetable oligonucleotide will be 5' to the second cassetable oligonucleotide sequence, which is in turn located 5' to the third cassetable oligonucleotide sequence, which is located 5' to the fourth cassetable oligonucleotide sequence. This is shown schematically in Figure 6, where one of cassetable oligonucleotide sequences G1-G10 is located in the first cassetable oligonucleotide sequence position; one of Y1-Y10 sequences is placed in the second cassetable oligonucleotide sequence position, one of R1-R10 sequences is placed in the third cassetable oligonucleotide sequence position, and one of B1-B10 sequences is placed in the fourth cassetable oligonucleotide sequence position of the barcode. However, the order does not matter and the in-situ detection and the sequencing read determining the presence or absence does not rely upon the order of presentation.

**Capture sequence.** The capture object includes a capture sequence configured to capture nucleic acid. The capture sequence is an oligonucleotide sequence having from about 6 to about 50 nucleotides. In some embodiments, the capture oligonucleotide sequence captures a nucleic acid by hybridizing to a nucleic acid released from a cell of interest. One non-limiting example includes polyT sequences, (having about 30 to about 40 nucleotides) which can capture and hybridize to RNA fragments having Poly A at their 3' ends. The polyT sequence may further contain two nucleotides VN at its 3' end. Other examples of capture oligonucleotides include random hexamers ("randomers") which may be used in a mixture to hybridize to and thus capture complementary nucleic acids. Alternatively, complements to gene specific sequences may be used for targeted capture of nucleic acids, such as B cell receptor or T cell receptor sequences.

In another embodiment, a capture oligonucleotide sequence may be used to capture nucleic acid released from a cell, by shepherding recognizable end sequences through recombinase/polymerase directed strand extension to effectively "capture" appropriately tagged released nucleic acid, to thereby add sequencing adaptors, barcodes, and indices. Examples of this type of capture oligonucleotide sequence includes a mosaic end (ME) sequence or other tagmentation insert sequence, as is known in the art. A mosaic end insert sequence is a short oligonucleotide that is easily recognized by transposons and can be used to provide priming/tagging to nucleic acid fragments. A suitable oligonucleotide sequence for this purpose may contain about 8 nucleotides to about 50 nucleotides. In some embodiments, ME plus additional insert sequence may be about 33 nucleotides long, and may be inserted by use of commercially available tagmentation kits, such as Nextera DNA Library Prep Kit, Illumina, Cat. # 15028212, and the like. In this embodiment, the capture performed by the capture sequence is not a hybridization event but a shepherding and directing interaction between the capture oligonucleotide, the tagged DNA, and the recombinase/ polymerase machinery to the priming sequence(s), barcode sequence and any other indices, adaptors, or functional sites such as, but not limited to a Notl restriction site sequence (as discussed below), onto the tagmented DNA fragment. The shepherding and directing interaction provides an equivalent product to the cDNA product described above using a hybridization interaction to capture released nucleic acids. In both cases, an augmented nucleic acid fragment is provided from the released nucleic acid, which now includes at least sequencing adaptor(s) and the barcode, permitting amplification, further sequencing library adaptation, and the ability to obtain sequenced barcode and sample nucleic acid reads.

**Priming sequence.** The capture oligonucleotide of the capture object has a priming sequence, and the priming sequence may be adjacent to or comprises the 5'-most nucleotide of the capture oligonucleotide(s). The priming sequence may be a generic or a sequence-specific priming sequence. The priming sequence may bind to a primer that, upon binding, primes a reverse transcriptase or a polymerase. In some embodiments, the generic priming sequence may bind to a P7 (5'-CAAGCAGAAGACGGCATACGAGAT-3' (SEQ ID. NO 107)) or a P5 (5'-AATGATACGGCGACCACCGA -3' (SEQ ID NO. 108)) primer.

In other embodiments, the generic priming sequence may bind to a primer having a sequence of one of the following:
5'-Me-isodC//Me-isodG//Me-isodC/ACACTCTTTCCCTACACGACGCrGrGrG-3' (SEQ ID. NO. 103);
5'- ACACTCTTTCCCTACACGACGCTCTTCCGATCT-3' (SEQ ID. NO. 104);
5'-/5Biosg/ACACTCTTTCCCT ACACGACGC-3' (SEQ ID. NO. 105); 
5'- AATGATACGGCGACCACCGAGATCTACACTCTTTCCCTACACGACGCTCTTC
C*G*A*T*C*T-3' (SEQ ID NO. 106);
 
5'-/5BiotinTEG/CAAGCAGAAGACGGCATACGAGATTCGCCTTAGTCTCGTGG-GCTCG*G-
3' (SEQ ID No. 109);
5' /5BiotinTEG/CAAGCAGAAGACGGCATACGAGATCTAGTACGGTCTCGTG-GGCTCG^{∗}G-3' (SEQ ID NO. 110; and

5'-/5BiotinTEG/AATGATACGGCGACCACCGAGATCTACACACTGCATATCGTCGG-
CAGCGT*C
(SEQ ID NO. 111).

Additional priming and/or adaptor sequences. The capture oligonucleotide(s) may optionally have one or more additional priming/adaptor sequences, which either provide a landing site for primer extension or a site for immobilization to complementary hybridizing anchor sites within a massively parallel sequencing array or flow cell.

**Optional oligonucleotide sequences.** Each capture oligonucleotide of the plurality of capture oligonucleotides may optionally further include a unique molecule identifier (UMI) sequence. Each capture oligonucleotide of the plurality may have a different UMI from the other capture oligonucleotides of a capture object, permitting identification of unique captures as opposed to numbers of amplified sequences. In some embodiments, the UMI may be located 3' to the priming sequence and 5' to the capture sequence. The UMI sequence may be an oligonucleotide having about 5 to about 20 nucleotides. In some embodiments, the oligonucleotide sequence of the UMI sequence may have about 10 nucleotides.

In some embodiments, each capture oligonucleotide of the plurality of capture oligonucleotides may also include a Notl restriction site sequence (GCGGCCGC, SEQ ID NO. 160). The Notl restriction site sequence may be located 5' to the capture sequence of the capture oligonucleotide. In some embodiments, the Notl restriction site sequence may be located 3' to the barcode sequence of the capture oligonucleotide.

In other embodiments, each capture oligonucleotide of the plurality of capture oligonucleotides may also include additional indicia such as a pool Index sequence. The Index sequence is a sequence of 4 to 10 oligonucleotides which uniquely identify a set of capture objects belonging to one experiment, permitting multiplex sequencing combining sequencing libraries from several different experiments to save on sequencing run cost and time, while still permitting deconvolution of the sequencing data, and correlation back to the correct experiment and capture objects associated therein.

Some exemplary, but not limiting capture objects are illustrated in Table 2, where only one capture oligonucleotide is shown, for clarity. Capture objects including a priming sequence, a barcode, a UMI and a capture sequence for capturing RNA may be a capture object having SEQ ID No. 97, SEQ ID No. 98, SEQ ID No. 99, or SEQ ID No. 100. A capture object including a priming sequence, a barcode, a UMI, a Not1 sequence, and a capture sequence for capturing RNA may be a capture object having SEQ ID NO. 101 or SEQ ID NO. 102.

**Table 2. Exemplary capture objects.**

| SEQ ID NO | Sequence |
|---|---|
| 97 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNTTTTTTTTTTTTTTTTTTTTVN- 3' |
| 98 | Bead-5'-Linker- ACACTCTTTCCCTACACGACGCTCTTCCGATCTCGCAATCTCACAGACGCTGT TCGTGTGTGATGGCTACGATNNNNNNNNNNTTTTTTTTTTTTTTTTTTTTVN- 3' |
| 99 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNNNTTTTTTTTTTTTTTTTTTTTVN- 3' |
| 100 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNNTTTTTTTTTTTTTTTTTTTTVN- 3' |
| 101 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNATCTCGTATGCCGTCTTCTGCTT GGCGGCCGCTTTTTTTTTTTTTTTTTTTTVN |
| 102 | Bead-5'-Linker- ACACTCTTTCCCTACACGACGCTCTTCCGATCTTGCGTTGTTGTGGAAAGCGG TAGGTCTGCGCGAAGGTAGGNNNNNNNNNNATCTCGTATGCCGTCTTCTGCT TGGCGGCCGCTTTTTTTTTTTTTTTTTTTTVN- 3' |

**A plurality of capture objects.** A plurality of capture objects is provided in accordance with the first aspect of the invention for use in multiplex nucleic acid capture.

For each capture object of the plurality, each capture oligonucleotide of that capture object has the same barcode sequence, and the barcode sequence of the capture oligonucleotides of each capture object of the plurality is different from the barcode sequence of the capture oligonucleotides of every other capture object of the plurality. In some embodiments, the plurality of capture objects may include at least 256 capture objects. In other embodiments, the plurality of capture objects may include at least 10,000 capture objects. A schematic showing the construction of a plurality of capture objects is shown in Figure 6. The capture object 630 has a bead 510 to which capture oligonucleotide 550 is attached via linker 515. Linker 515 attaches to the 5' end of the capture oligonucleotide 550, and in particular to the 5' end of the priming sequence 520. Linker 515 and priming sequence 520 (shown here as 33 bp in length) are common to all capture oligonucleotides of all capture objects in this example, but in other embodiments, the linker and/or the priming sequence may be different for different capture oligonucleotides on a capture object or alternatively the linker and/or the priming sequence may be different for different capture objects in the plurality. Capture sequence 535 of the capture oligonucleotide 550 is located at or proximal to the 3' end of the capture oligonucleotide 550. In this non-limiting example, the capture sequence 535 is shown as a PolyT-VN sequence, which generically captures released RNA. In some embodiments, the capture sequence 535 is common to all capture oligonucleotides 550 of all of the capture objects 630 of the plurality of capture objects. However, in other pluralities of capture objects, the capture sequence 535 on each capture oligonucleotide of the plurality of capture oligonucleotides 550 of the capture object 630 may not necessarily be the same. In this example, an optional Unique Molecular Identifier (UMI) 530 is present, and is located 5' to the capture sequence 535 but 3' to the priming sequence 520. In this particular example, the UMI 530 is located along the capture oligonucleotide 3' to the barcode sequence 525. However, in other embodiments, a UMI 530 may be located 5' to the barcode. However, a UMI 530 is located 3' to the priming sequence 520, in order to be incorporated within the amplified nucleic acid product. In this example, the UMI 530 is 10bp in length. Here the UMI 530 is shown having a sequence of NNNNNNNNNN (SEQ. ID NO 84). Generally, the UMI 530 may be composed of a random combination of any nucleotides, with the proviso that it is not identical to any of the cassetable oligonucleotides sequences 435a, 435b, 435c, 435d of the barcode 525 nor is it identical to the priming sequence 520. In many embodiments, the UMI is designed to not include a sequence of ten T, which would overlap with the capture sequence 535, as shown in for this case. The UMI 530 is unique for each capture oligonucleotide 550 of each capture object 630. In some embodiments, the unique UMI 530 of each capture oligonucleotide 550 of a capture object 630 may be used within a capture oligonucleotide 550 of a different capture object 630 of the plurality, as the barcode 525 of the different capture object 630 can permit deconvolution of the sequencing reads.

Barcode 525 of the capture oligonucleotide is 3' to the priming sequence, and contains 4 cassetable sequences 435a, 435b, 435c, and 435d, which each are 10 bp in length. Each capture oligonucleotide of the plurality of capture oligonucleotides 550 of on a single capture object 630 has an identical barcode 525, and the barcode 525 for the plurality of capture objects are different for each of the capture object 630 of the plurality. The diversity of the barcodes 525 for each of the capture objects may be obtained by making the selection for the cassetable oligonucleotides from defined sets of oligonucleotides as described below. In this example, 10, 000 different barcodes can be made by choosing one of each of the four defined sets of oligonucleotides, each of which contain 10 different possible choices.

**Cassetable oligonucleotide sequence.** A cassetable oligonucleotide sequence is described herein for use within a barcode as described herein and may have an oligonucleotides sequence of any one of SEQ ID Nos. 1 to 40.

**Barcode sequence.** A barcode sequence is described herein for use within the capture oligonucleotide of the capture object and methods described herein, where the barcode sequence may include three or more cassetable oligonucleotide sequences, wherein each of the three or more cassetable oligonucleotides sequences of the barcode sequence has a sequence of any one of SEQ ID NOs: 1-40, and wherein each cassetable oligonucleotide sequence of the barcode sequence is non-identical to the other cassetable oligonucleotide sequences of the barcode sequence. The barcode sequence comprises three or more (e.g., 3, 4, 5, or more) cassetable oligonucleotide sequences, each of which is non-identical to the other cassetable oligonucleotide sequences of the barcode sequence. In certain embodiments, the barcode sequence consists of (or consists essentially of) three or more (e.g., 3, 4, 5, or more) cassetable oligonucleotide sequences. The cassetable oligonucleotide sequences of the barcode sequence can be as described elsewhere herein. For example, each of the three or more cassetable oligonucleotide sequences can be one from a defined set of oligonucleotide sequences (e.g., a set of 12 or more oligonucleotide sequences) wherein, for each oligonucleotide sequence in the defined set, the complementary oligonucleotide sequence does not substantially hybridize to any of the other oligonucleotide sequences in the defined set. In certain embodiments, each of the three or more cassetable oligonucleotide sequences in the barcode sequence can comprise 6 to 15 nucleotides (e.g., a length of 6, 7, 8, 9, 10, 11, 12, 13, 14, or 15 nucleotides). In other embodiments, each of the three or more cassetable oligonucleotide sequences can consist of (or consist essentially of) 6 to 15 nucleotides (e.g., a length of 6, 7, 8, 9, 10, 11, 12, 13, 14, or 15 nucleotides). In still other embodiments, each of the three or more cassetable sequences in the barcode sequence can comprise, consist or consist essentially of 6-8, 7-9, 8-10, 9-11, 10-12, 11-13, 12-14 or 13-15 nucleotides. In a specific embodiment, the cassetable oligonucleotides sequences can be any one of the set defined by SEQ ID NOs: 1-40. In some embodiments, the barcode may include three or four cassetable oligonucleotide sequences. In various embodiments, the three or four cassetable oligonucleotide sequences of the barcode are linked in tandem without any intervening oligonucleotide sequences. In other embodiments, one or more of the three or four cassetable oligonucleotide sequences may be linked together using intervening one, two or three nucleotides between the cassetable oligonucleotide sequences. This is useful when the cassetable oligonucleotides sequences are linked using ligation chemistry. In some embodiments, no other nucleotide sequences having other functions within the capture oligonucleotide interrupt the linkage of the three or four cassetable oligonucleotide sequences.

**Set of barcode sequences.** A set of barcode sequences is described herein, which includes at least 64 non-identical barcode sequences, each barcode sequence of the set having a structure according to any barcode as described herein. As used herein, a barcode sequence is "non-identical" to other barcode sequences in a set when the n (e.g., three or more) cassetable oligonucleotide sequences of any one barcode sequence in the set of barcode sequences do not completely overlap with the n' (e.g., three or more) cassetable oligonucleotide sequences of any other barcode sequence in the set of barcode sequences; partial overlap (e.g., up to n-1) is permissible, so long as each barcode sequence in the set is different from every other barcode sequence in the set by a minimum of 1 cassetable oligonucleotide sequence. In some embodiments, the set of barcode sequences may consist essentially of 64, 81, 100, 125, 216, 256, 343, 512, 625, 729, 1000, 1296, 2401, 4096, 6561, or 10,000 barcode sequences.

**Hybridization probes.** Also disclosed are hybridization probes which have an oligonucleotide sequence which is complementary to a cassetable oligonucleotide sequence; and a detectable label. The detectable label can be, for example, a fluorescent label, such as, but not limited to a fluorescein, a cyanine, a rhodamine, a phenyl indole, a coumarin, or an acridine dye. Some non-limiting examples include Alexa Fluor dyes such as Alexa Fluor^{®} 647, Alexa Fluor^{®} 405, Alexa Fluor^{®} 488; Cyanine dyes such as Cy^{®} 5 or Cy^{®} 7, or any suitable fluorescent label as known in the art. Any set of distinguishable fluorophores may be selected to be present on hybridization probes flowed into the microfluidic environment for detection of the barcode, as long as each dye's fluorescent signal is detectable distinguishable. Alternatively, the detectable label can be luminescent agent such as a luciferase reporter, lanthanide tag or an inorganic phosphor, a Quantum Dot, which may be tunable and may include semiconductor materials. Other types of detectable labels may be incorporated such as FRET labels which can include quencher molecules along with fluorophore molecules. FRET labels can include dark quenchers such as Black Hole Quencher^{®} (Biosearch); Iowa Black^{™} or dabsyl. The FRET labels may be any of TaqMan^{®} probes, hairpin probes, Scorpion^{®} probes, Molecular Beacon probes and the like.

Further details of the hybridization conditions are described below, and one of skill may determine other variations of such conditions suitable to gain binding specificity for a range of barcodes and their hybridization probe pairs.

**Hybridization probe.** A hybridization probe is described herein including an oligonucleotide sequence having a sequence of any one of SEQ ID NOs: 41 to 80 (See Table 1); and a detectable label. The detectable label may be a rhodamine, cyanine or fluorescein fluorescent dye label. In various embodiments, the oligonucleotide sequence of the hybridization probe consists essentially of one sequence of any one of SEQ ID Nos. 41-80, and has no other nucleotides forming part of the hybridization probe.

**Hybridization Reagent.** A hybridization reagent is provided in accordance with the third aspect of the invention, including a plurality of hybridization probes, where each hybridization probe of the plurality is a hybridization probe as described herein, and where each hybridization probe of the plurality (i) comprises an oligonucleotide sequence which is non-identical to the oligonucleotide sequence of every other hybridization probe of the plurality and (ii) comprises a detectable label which is spectrally distinguishable from the detectable label of every other hybridization probe of the plurality.

Also disclosed are reagents that comprise a plurality of (e.g., 2, 3, 4, 5, or more) hybridization probes. The hybridization probes can be any of the hybridization probes disclosed herein. The reagent can be a liquid, such as a solution. Alternatively, the reagent can be a solid, such as a lyophilized powder. When provided as a solid, the addition of an appropriate volume of water (or a suitable solution) can be added to generate a liquid reagent suitable for introduction into a microfluidic device.

In some embodiments, the plurality of hybridization probes consists of two to four hybridization probes. In some embodiments of the plurality of hybridization probes, a first hybridization probe of the plurality includes a sequence selected from a first subset of SEQ ID NOs: 41-80, and a first detectable label; and a second hybridization probe of the plurality includes a sequence selected from a second subset of SEQ ID NOs: 41-80, and a second detectable label which is spectrally distinguishable from the first detectable label, and where the first and second subsets of SEQ ID NOs: 41-80 are non-overlapping subsets.

The first hybridization probe can include a sequence that comprises one of the sequences set forth in SEQ ID NOs: 41-80, or a subset of sequences thereof. In certain embodiments, the first hybridization probe can include a sequence that consists of (or consists essentially of) one of the sequences set forth in SEQ ID NOs: 41-80, or a subset of sequences thereof. The second hybridization probe can include a sequence that comprises one of the sequences set forth in SEQ ID NOs: 41-80, or a subset of sequences thereof (e.g., a subset that does not include the sequence present in the first hybridization probe, or a subset that is non-overlapping with the subset from which the sequence present in the first hybridization probe is selected). In certain embodiments, the second hybridization probe can include a sequence that consists of (or consists essentially of) one of the sequences set forth in SEQ ID NOs: 41-80, or a subset of sequences thereof (e.g., a subset that does not include the sequence present in the first hybridization probe, or a subset that is non-overlapping with the subset from which the sequence present in the first hybridization probe is selected). The third hybridization probe (if present) can include a sequence that comprises one of the sequences set forth in SEQ ID NOs: 41-80, or a subset of sequences thereof (e.g., a subset that does not include the sequences present in the first and second hybridization probes, or a subset that is non-overlapping with the subsets from which the sequences present in the first and second hybridization probes are selected). In certain embodiments, the third hybridization probe (if present) can include a sequence that consists of (or consists essentially of) one of the sequences set forth in SEQ ID NOs: 41-80, or a subset of sequences thereof (e.g., a subset that does not include the sequences present in the first and second hybridization probes, or a subset that is non-overlapping with the subsets from which the sequences present in the first and second hybridization probes are selected). The fourth hybridization probe (if present) can include a sequence that comprises one of the sequences set forth in SEQ ID NOs: 41-80, or a subset of sequences thereof (e.g., a subset that does not include the sequences present in the first, second, and third hybridization probes, or a subset that is non-overlapping with the subsets from which the sequences present in the first, second, and third hybridization probes are selected). In certain embodiments, the fourth hybridization probe (if present) can include a sequence that consists of (or consists essentially of) one of the sequences set forth in SEQ ID NOs: 41-80, or a subset of sequences thereof (e.g., a subset that does not include the sequences present in the first, second, and third hybridization probes, or a subset that is non-overlapping with the subsets from which the sequences present in the first, second, and third hybridization probes are selected). As will be evident to persons skilled in the art, the reagent could include fifth, sixth, etc. hybridization probes, which can have properties analogous to the first, second, third, and fourth hybridization probes.

In some embodiments, the third hybridization probe of the plurality may include a sequence selected from a third subset of SEQ ID NOs: 41-80, and a third detectable label which is spectrally distinguishable from each of the first and second detectable labels, wherein the first, second, and third subsets of SEQ ID NOs: 41-80 are non-overlapping subsets.

In yet other embodiments, the reagent may further include a fourth hybridization probe of the plurality, wherein the fourth hybridization probe may include a sequence selected from a fourth subset of SEQ ID NOs: 41-80, and a fourth detectable label which is spectrally distinguishable from each of the first, second, and third detectable labels, wherein the first, second, third, and fourth subsets of SEQ ID NOs: 41-80 are non-overlapping subsets.

In various embodiments of the hybridization reagent, each subset of SEQ ID NOs: 41-80 may include at least 10 sequences. In various embodiments of the hybridization reagent, the first subset contains SEQ ID NOs: 41-50, the second subset contains SEQ ID NOs: 51-60, the third subset contains SEQ ID NOs: 61-70, and the fourth subset contains SEQ ID NOs: 71-80.

**Kit.** A kit for detecting the cassetable oligonucleotide sequences of the barcode of a capture object is described herein, where the kit includes a plurality of reagents as described herein, wherein the plurality of hybridization probes of each reagent forms a set that is non-overlapping with the set of hybridization probes of every other reagent in the plurality.

The kit may include 3, 4, 5, 6, 7, 8, 9, or 10 of the reagents.

**Method for in-situ identification of capture object(s)**. Also provided in accordance with the fourth aspect of the invention is a method of in-situ identification of one or more capture objects within a microfluidic device, where the method includes:
disposing a single capture object of the one or more capture objects into each of one or more sequestration pens located within an enclosure of the microfluidic device, wherein each capture object has a plurality of capture oligonucleotides, and where each capture oligonucleotide of the plurality includes: a priming sequence; a capture sequence; and a barcode sequence, where the barcode sequence includes three or more cassetable oligonucleotide sequences, each cassetable oligonucleotide sequence being non-identical to the other cassetable oligonucleotide sequences of the barcode sequence;
flowing a first reagent solution including a first set of hybridization probes into a flow region within the enclosure of the microfluidic device, where the flow region is fluidically connected to each of the one or more sequestration pens, and where each hybridization probe of the first set has an oligonucleotide sequence complementary to a cassetable oligonucleotide sequence comprised by any of the barcode sequences of any of the capture oligonucleotides of any of the one or more capture objects, where the complementary oligonucleotide sequence of each hybridization probe in the first set is non-identical to every other complementary oligonucleotide sequence of the hybridization probes in the first set; and a detectable label selected from a set of spectrally distinguishable detectable labels, where the detectable label of each hybridization probe in the first set is different from the detectable label of every other hybridization probe in the first set of hybridization probes;
hybridizing the hybridization probes of the first set to corresponding cassetable oligonucleotide sequences in any of the barcode sequences of any of the capture oligonucleotides of any of the one or more capture objects;
detecting, for each hybridization probe of the first set of hybridization probes, a corresponding detectable signal associated with any of the one or more capture objects; and
generating a record, for each capture object disposed within one of the one or more sequestration pens, including (i) a location of the sequestration pen within the enclosure of the microfluidic device, and (ii) an association or non-association of the corresponding fluorescent signal of each hybridization probe of the first set of hybridization probes with the capture object, where the record of associations and non-associations constitute a barcode which links the capture object with the sequestration pen.

The one or more capture objects, as used in this method, may each be any capture object as described herein. Generally, all of the barcode sequences will have the same number of cassetable oligonucleotide sequences. Each capture oligonucleotide of the plurality of capture oligonucleotide that are comprised by a particular capture object will have the same barcode sequence. As discussed above, the three or more cassetable oligonucleotide sequences of each barcode sequence are selected from a set of non-identical cassetable oligonucleotide sequences. The set of cassetable oligonucleotides sequences can, for example, include 12 to 100 (or more) non-identical oligonucleotide sequences. Thus, the set of cassetable oligonucleotide sequences can comprise a number of cassetable oligonucleotide sequences greater than the number of spectrally distinguishable labels in the set of spectrally distinguishable labels, which can include 2 or more (e.g., 2 to 5) spectrally distinguishable labels.

The number of hybridization probes in the first (or subsequent) set can be identical to the number of cassetable oligonucleotides in each barcode sequence. However, these numbers do not have to be the same. For example, the number of hybridization probes in the first (or any subsequent) set can be greater than the number of cassetable oligonucleotides in each barcode sequence.

Detecting each hybridization probe (or class of label) comprises identifying distinguishing spectral characteristics of each hybridization probe (or label) of the first set of hybridization probes. Furthermore, detecting a given hybridization probe generally requires detection of a level of the distinguishing spectral characteristic(s) that exceeds a background or threshold level associated with the system (e.g., optical train) used to detect the distinguishing spectral characteristic(s). Following such identification, any detected label can be correlated with the presence of a cassetable oligonucleotide sequence which is complementary to the oligonucleotide sequence of the hybridization probe. The detectable label can be, for example, a fluorescent label, such as, but not limited to a fluorescein, a cyanine, a rhodamine, a phenyl indole, a coumarin, or an acridine dye. Some non-limiting examples include Alexa Fluor dyes such as Alexa Fluor^{®} 647, Alexa Fluor^{®} 405, Alexa Fluor^{®} 488; Cyanine dyes such as Cy^{®} 5 or Cy^{®} 7, or any suitable fluorescent label as known in the art. Any set of distinguishable fluorophores may be selected to be present on hybridization probes flowed into the microfluidic environment for detection of the barcode, as long as each dye's fluorescent signal is detectable distinguishable. Alternatively, the detectable label can be luminescent agent such as a luciferase reporter, lanthanide tag or an inorganic phosphor, a Quantum Dot, which may be tunable and may include semiconductor materials. Other types of detectable labels may be incorporated such as FRET labels which can include quencher molecules along with fluorophore molecules. FRET labels can include dark quenchers such as Black Hole Quencher^{®} (Biosearch); Iowa Black^{™} or dabsyl. The FRET labels may be any of TaqMan^{®} probes, hairpin probes, Scorpion^{®} probes, Molecular Beacon probes and the like.

Detecting and/or generating a record can be automated, for example, by means of a controller.

The method of in-situ identification may further include flowing an n^{th} reagent solution comprising an n^{th} set of hybridization probes into the flow region of the microfluidic device, where each hybridization probe of the n^{th} set may include: an oligonucleotide sequence complementary to a cassetable oligonucleotide sequence comprised by any of the barcode sequences of any of the capture oligonucleotides of any of the one or more capture objects, wherein the complementary oligonucleotide sequence of each hybridization probe in the n^{th} set is non-identical to every other complementary oligonucleotide sequence of the hybridization probes in the n^{th} set and any other set of hybridization probes flowed into the flow region of the microfluidic device; and a detectable label selected from a set of spectrally distinguishable detectable labels, wherein the detectable label of each hybridization probe in the n^{th} set is different from the detectable label of every other hybridization probe in the n^{th} set of hybridization probes;
hybridizing the hybridization probes of the n^{th} set to corresponding cassetable oligonucleotide sequences in any of the barcode sequences of any of the capture oligonucleotides of any of the one or more capture objects;
detecting, for each hybridization probe of the n^{th} set of hybridization probes, a corresponding detectable signal associated with any of the one or more capture objects; and
supplementing the record, for each capture object disposed within one of the one or more sequestration pens, with an association or non-association of the corresponding detectable signal of each hybridization probe of the n^{th} set of hybridization probes with the capture object, where n is a set of positive integers having values of {2,..., m}, where m is a positive integer having a value of 2 or greater, and where the foregoing steps of flowing the n^{th} reagent, hybridizing the n^{th} set of hybridization probes, detecting the corresponding detectable signals, and supplementing the records are repeated for each value of n in the set of positive integers {2,....,m}.

In various embodiments, m may have a value greater than or equal to 3 and less than or equal to 20 (e.g., greater than or equal to 5 and less than or equal to 15). In some embodiments, m may have a value greater than or equal to 8 and less than or equal to 12 (e.g., 10).

In various embodiments, flowing the first reagent solution and/or the nth reagent solution into the flow region may further include permitting the first reagent solution and/or the n^{th} reagent solution to equilibrate by diffusion into the one or more sequestration pens.

Detecting the corresponding fluorescent signal associated with any of the one or more capture objects may further include: flowing a rinsing solution having no hybridization probes through the flow region of the microfluidic device; and equilibrating by diffusion the rinsing solution into the one or more sequestration pens, thereby allowing unhybridized hybridization probes of the first set or any of the n^{th} sets to diffuse out of the one or more sequestration pens. In some embodiments, the flowing of the rinsing solution may be performed before detecting the fluorescent signal.

Each barcode sequence of each capture oligonucleotide of each capture object includes three cassetable oligonucleotide sequences. In some embodiments, the first set of hybridization probes and each of the n^{th} sets of hybridization probes may include three hybridization probes.

In various embodiments of the method of in-situ detection, each barcode sequence of each capture oligonucleotide of each capture object may include four cassetable oligonucleotide sequences. In some embodiments, the first set of hybridization probes and each of the n^{th} sets of hybridization probes comprise four hybridization probes.

Disposing each of the one or more capture objects includes disposing each of the one or more capture objects within an isolation region of the one or more sequestration pens within the microfluidic device.

In some embodiments, the method may further include disposing one or more biological cells within the one or more sequestration pens of the microfluidic device. In some embodiments, each one of the one or more biological cells may be disposed in a different one of the one or more sequestration pens. The one or more biological cells may be disposed within the isolation regions of the one or more sequestration pens of the microfluidic device. In some embodiments of the method, at least one of the one or more biological cells may be disposed within a sequestration pen having one of the one or more capture objects disposed therein. In some embodiments, the one or more biological cells may be a plurality of biological cells from a clonal population. In various embodiments of the method, disposing the one or more biological cells may be performed before disposing the one or more capture objects.

In various embodiments of the method of in-situ detection, the enclosure of the microfluidic device may further include a dielectrophoretic (DEP) configuration, and disposing the one or more capture objects into one or more sequestration pens may be performed using dielectrophoretic (DEP) force. In various embodiments of the method of in-situ detection, the enclosure of the microfluidic device may further include a dielectrophoretic (DEP) configuration, and disposing the one or more biological cells within the one or more sequestration pens may be performed using dielectrophoretic (DEP) forces. The microfluidic device can be any microfluidic device disclosed herein. For example, the microfluidic device can comprise at least one coated surface (e.g., a covalently bound surface). The at least one coated surface can comprise a hydrophilic or a negatively charged coated surface.

In various embodiments of the method of in-situ identification, at least one of the plurality of capture oligonucleotides of each capture object may further include a target nucleic acid captured thereto by the capture sequence.

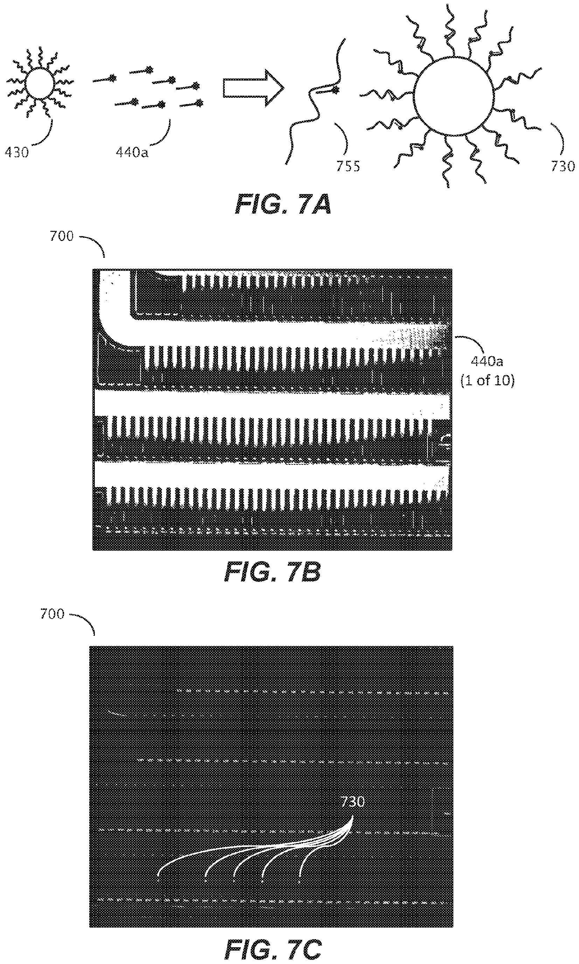

Turning to Figure 7A for better understanding of the method of in-situ identification of capture object(s) within a microfluidic device, a schematic is shown of capture object 430, having capture oligonucleotides including barcodes as described herein, being exposed to a flow of hybridization probes 440a, which include a detectable label as described herein. Upon associating of the probe 440a with its target cassetable oligonucleotide of the capture oligonucleotide, a hybridized probe: cassetable oligonucleotide sequence is formed upon the capture oligonucleotide length 755. This gives rise to a capture object having multiple hybridized probe: cassetable oligonucleotide pairs along at least a portion of the capture oligonucleotides of the capture object 730. Figure 7B shows a photograph of the microfluidic channel within the microfluidic device having sequestration pens opening off of the channel where capture objects (not seen in this photograph) have been disposed within the sequestration pens. Additionally, while there were capture objects within the pens opening to all three of the channel lengths visible, only capture objects placed within the sequestration pens at the bottom most channel length had a barcode that included the target cassetable oligonucleotide of probe 440a. The capture objects in the sequestration pens opening to the uppermost channel or the middle channel had no cassetable oligonucleotides on their respective capture oligonucleotides that were hybridization targets for probe 440a. The photograph shows a timepoint when reagent flow including hybridization probe 440a was being flowed through the flow channel and was diffusing into the sequestration pens. The fluorescence of the detectable label of probe was visible throughout the flow channel and within the sequestration pens. After permitting reagent flow for about 20 min, a rinsing flow, having no hybridization probe 440a, was performed as described herein. Figure 7B shows the same field of view, under fluorescent excitation appropriate to excite the detectable label of probe 440a, after the rinsing flow was completed. What was seen was capture objects 730 in the sequestration pens opening off the bottommost channel, providing a detectable signal from the hybridization probes 440a hybridized there. What was also seen was that the other classes of capture objects, within the sequestration pens opening off the uppermost and middle channel lengths, were not visible under fluorescent illumination. This illustrated the specific and selective identification of only the target cassetable oligonucleotide sequence within the microfluidic device using hybridization probes to perform the identification.

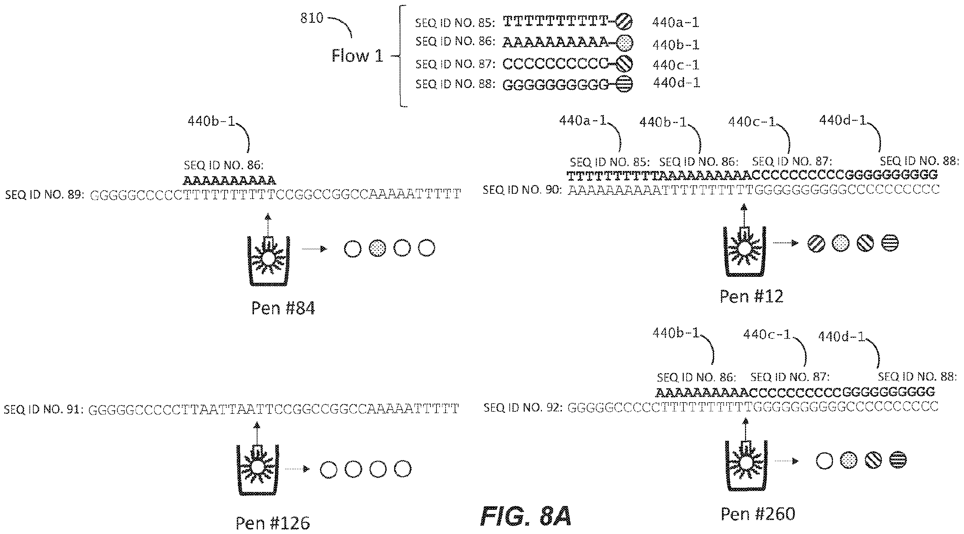

Figures 8A-8C show how the multiplexed and multiple flows of reagent having, in this example, four different hybridization probes may be used to identify each barcode of each capture object within a sequestration pen of a microfluidic device. Figure 8A shows a schematic representation of detection of the barcode for each of four sequestration pens illustrated, Pen# 84, Pen # 12, Pen #126, and Pen # 260, each pen having a capture object present within it. Each capture object has a unique barcode which includes four cassetable oligonucleotide sequences. The capture object in Pen # 84 has a barcode having a sequence: GGGGGCCCCCTTTTTTTTTTCCGGCCGGCCAAAAATTTTT (SEQ ID NO. 89). The capture object in Pen # 12 has a barcode having a sequence of: AAAAAAAAAATTTTTTTTTTGGGGGGGGGGCCCCCCCCCC (SEQ ID NO. 90). The capture object in Pen # 126 has a barcode having a sequence of: GGGGGCCCCCTTAATTAATTCCGGCCGGCCAAAAATTTTT (SEQ ID 91). The capture object in Pen #260 has a barcode having a sequence of: GGGGGCCCCCTTTTTTTTTTGGGGGGGGGGCCCCCCCCCC (SEQ ID No. 92).

The first reagent flow 820 includes four hybridization probes having sequences and detectable labels as follows; a first probe 440a-1 having a sequence of TTTTTTTTTT (SEQ ID 85)(for this illustration, the choice of sequence is only for explication, and does not represent a probe sequence used in combination with a capture sequence of PolyT) having a first detectable label selected from a set of four distinguishable labels (represented as a circle having pattern 1; a second probe 440b-1 having a sequence of AAAAAAAAAA (SEQ ID NO. 86), having a second detectable label selected from the set of distinguishable labels (represented as the circle having pattern 2); a third probe 440c-1 having a sequence of CCCCCCCCCC (SEQ ID No. 87)and a third detectable label selected from the set of distinguishable labels ( represented as the circle having pattern 3); and a fourth probe 440d-1, having a sequence of GGGGGGGGGG (SEQ ID NO. 88, and a fourth detectable label selected from the set of distinguishable labels (represented as the circle having pattern 4).

After the first flow 810 has been permitted to diffuse into the sequestration pens, and the probes have hybridized to any target cassetable oligonucleotide sequences present in any of the barcodes, flushing with probe-free medium is performed to remove unhybridized probes, while retaining hybridized probes in place. This is accomplished by use of medium that does not dissociate hybridized pairs of probes from their target, such as use of DPBS or Duplex buffer, as described below in the Experimental section. After the excess, unhybridized probe containing medium has been flushed, excitation with the appropriate excitation wavelengths permit detection of the detectable labels on the probes still hybridized to their targets. In this example, it is observed that for Pen #84, a signal is observed for the wavelength of the second distinguishable detection, and no other. This is notated with the patterned circle next to Pen #84 indicating pattern 2 was observed in Flow 1 (810). For Pen # 12, signals in all four distinguishable detection wavelengths is observed, and notated with the corresponding patterns 1-4. For Pen # 126, no detectable signal observed, and the circles along the figure so notate. Last, Pen #260, three of the probes, 440b-1, 440c-1 and 440d-1 bind, and notation of the detectable signals observed is made showing pattern 2, 3, and 4.

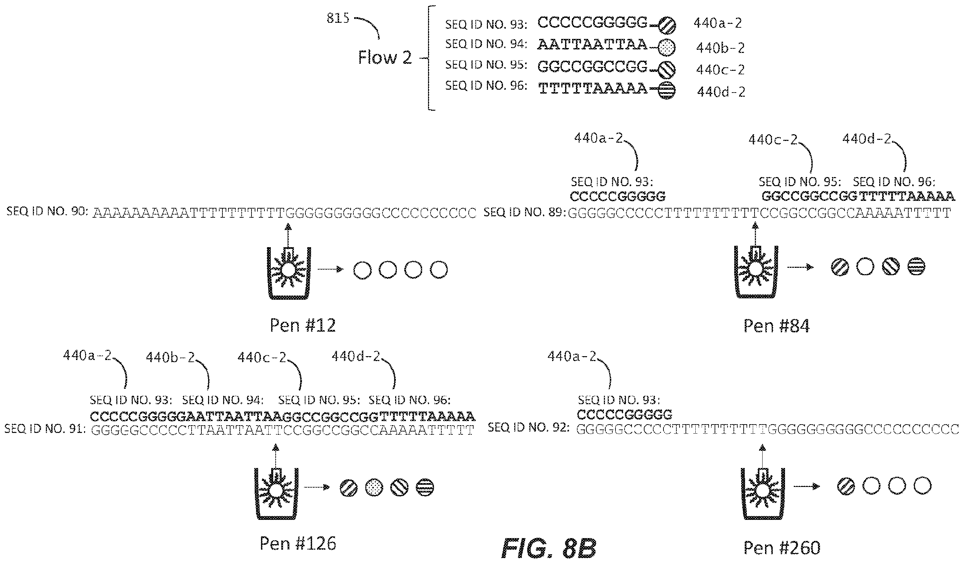

It can be seen that not all cassetable oligonucleotide sequences have been detected, so a second flow 815 is then performed as shown in Figure 8B. The second flow contains four non-identical probes, probe 440a-2 having a sequence of CCCCCGGGGG (SEQ ID NO. 93) with detectable label 1 of the set of distinguishable labels (represented as pattern 1); a second probe 440b-2 having a sequence of AATTAATTAA (SSEQ ID No. 94) having detectable label 2 of the set (represented as pattern 2); a third probe 440c-2, having a sequence of GGCCGGCCGG (SEQ ID No. 95) having the third detectable label of the set (represented as pattern 3); and a fourth probe 440d-2, having a sequence of TTTTTAAAAA (SSEQ ID No. 96) with the fourth detectable label of the set (represented as pattern 4).

The same process of flowing the reagent flow 2 (815) in, permitting diffusion and binding, flushing unhybridized probes and then detecting in each of the four distinguishable wavelengths is performed. As shown in Figure 8B, Pen #12 has no detectable signals as none of the probes of the second flow are configured to hybridize with any of the cassetable sequences therein. Further, all of the cassetable sequences of the barcode of the capture object in Pen #12 were already detected. Additionally, in these methods, it is noted when a signal in one of the detectable label wavelength channels has been detected as the cassetable sequences are selected to have only one of each detectable signal and will have no repeats. Detectable signals in that channel in later flows may be disregarded as the probe that binds that cassetable oligonucleotide sequence of the barcode has already been detected. In some instances, signal may be seen in later flows, but that is a result of probes from an earlier flow still remaining hybridized to the barcode sequence, not of the new flow reagents binding to the cassetable oligonucleotide sequence.

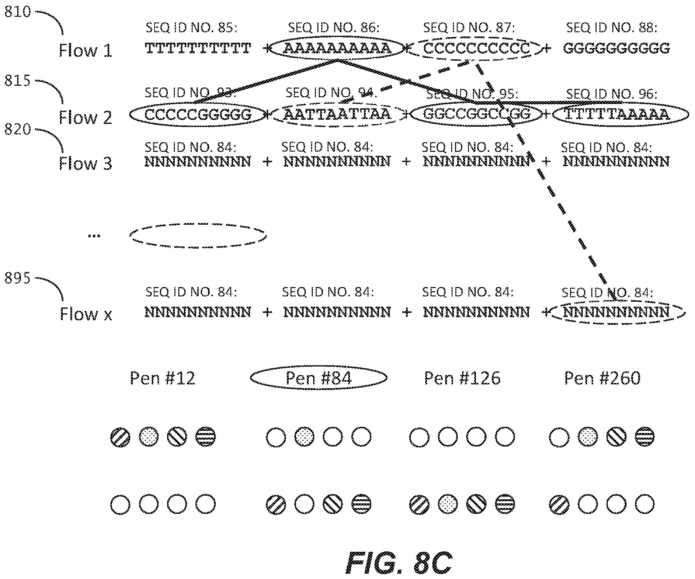

Returning to the analysis from detection of the second flow 815, the capture object in Pen #84 is noted to having signal in the first, third and fourth detectable signal wavelength channel, and notated with the first, third and fourth pattern. The capture object in Pen #126 has all four probes binding, so is notated with the first, second, third and fourth pattern. The capture object in Pen #260 is notated as having signal in the first detectable label signal wavelength channel, and notated with the first pattern. The results can be tabulated as in Figure 8C, for the first flow 810, second flow 815, a third flow 820 and so one to the x^{th} Flow 895, until the entire reference set of cassetable sequences has been tested with corresponding hybridization probes.

The sequence of each barcode on a capture object in a specific sequestration pen can be derived as shown, matching the detected signal pattern to the complementary sequence of each cassetable oligonucleotide as the sequence of the hybridization probe is known. The sequence of the capture object can then be assigned as shown, where the barcode of the capture object in Pen #12 is determined by the in-situ method of detection to have a sequence of SEQ ID NO. 90; the barcode of the capture object in Pen #84 to have a sequence of SEQ ID NO. 89; the barcode of the capture object in Pen #126 to have a sequence of SEQ ID No. 91, and the barcode of the capture object in Pen # 260 to have a sequence of SEQ ID NO. 92.

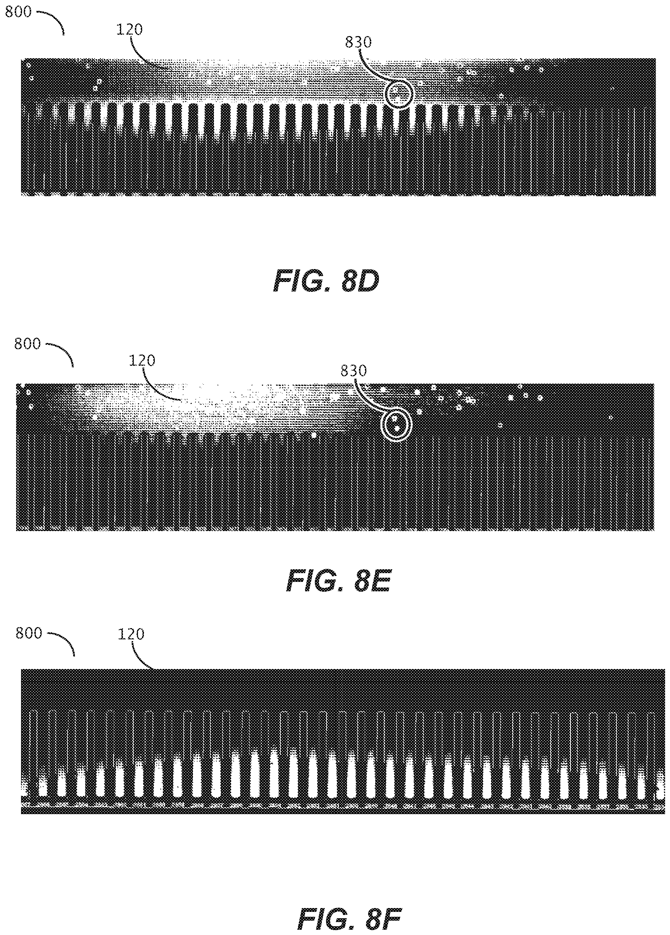

Figures 8D-F illustrate another experiment showing the ability to hybridize and detect multiple probes along the barcode sequence at the same time. In this experiment, the dyes that were utilized on the hybridization probes used were Alexa Fluor^{®} 647 (detectable in a Cy^{®}5 channel (e.g. detection filters that will detect a Cy^{®}5dye but can also detect an Alexa Fluor^{®} 647 dye) and Alexa Fluor^{®} 594 (detectable in a Texas Red channel (Detection filter that can also detect Alexa Fluor^{®} 594). In this experiment, a plurality of capture objects all having the same two cassetable oligonucleotide sequences, which were situated adjacent to each other within the barcode sequence, were flowed into the microfluidic channel 120 within the microfluidic device 800, and no attempt to dispose them into sequestration pens was made. A flow was then made including a first hybridization probe having a sequence that binds the first cassetable oligonucleotide of the barcode of the capture objects and an Alexa Fluor^{®} 594 dye. The flow also contained a second hybridization probe having a sequence that binds the second cassetable oligonucleotide of the barcode of the capture objects and an Alexa Fluor 647^{®} dye. After permitting diffusion, hybridization and flushing to remove unhybridized probes,

Figures 8D, 8E and 8F each showed the detection channel (filter) for different wavelength regions. Figure 8D shows a Texas Red detection channel, with a 200 ms exposure, and capture objects 830 that have been excited and were detected. This confirmed that the Alexa Fluor^{®} 594 label of the first hybridization probe was present (e.g., was bound to the cassetable oligonucleotide sequence of the barcode). Figure 8E shows the same view within the microfluidic device channel 120, and is the Cy^{®}5detection channel, 800ms exposure, which detected Alexa Fluor 647 labels that are bound to a capture object. Capture objects 830 also were detected able in this channel, confirming that the second hybridization probe was bound to the capture objects 830 at the same time as the first hybridization probe, and that both signals are detectable. Figure 8F is the same view in a FITC detection (filter) channel, 2000 ms exposure, where no signal from capture objects in the channel were seen. This experiment demonstrated the ability to hybridize side-by-side fluorescent probes, with no loss of detection specificity.

In various embodiments, the detectable labels used may include Alexa Fluor^{®} 647, which is detected in the Cy^{®}5fluorescent channel of the optical system that used to excite, observe and record events within the microfluidic device; Alexa Fluor^{®} 405, which is detectable in the Dapi fluorescent channel of the optical system; Alexa Fluor^{®} 488 which is detectable in the FITC fluorescent channel of the optical system; and Alexa Fluor^{®} 594, which is detectable in the Texas Red fluorescent channel of the optical system. The fluorophores may be attached to the hybridization probe as is suitable for synthesis and can be at the 5' or the 3' end of the probe. Hybridization of two probes, one labeled at the 5' end and one labeled at the 3' end, was found to be unaffected by the presence of adjacent labels (data not shown).

**Method of correlating genomic data with a cell in a microfluidic device.** A method is provided in accordance with the fifth aspect of the invention for correlating genomic data with a biological cell in a microfluidic device, including:
disposing a capture object (which may be a single capture object) into a sequestration pen of a microfluidic device, where the capture object includes a plurality of capture oligonucleotides, where each capture oligonucleotide of the plurality includes: a priming sequence; a capture sequence; and a barcode sequence, where the barcode sequence includes three or more cassetable oligonucleotide sequences, each cassetable oligonucleotide sequence being non-identical to the other cassetable oligonucleotide sequences of the barcode sequence; and where each capture oligonucleotide of the plurality includes the same barcode sequence;
identifying the barcode sequence of the plurality of capture oligonucleotides in-situ and recording an association between the identified barcode sequence and the sequestration pen (i.e., identifying a location of the capture object within the microfluidic device);
disposing the biological cell into the sequestration pen;
lysing the biological cell and allowing nucleic acids released from the lysed biological cell to be captured by the plurality of capture oligonucleotides comprised by the capture object;
transcribing (e.g., reverse transcribing) the captured nucleic acids, thereby producing a plurality (which could be a library) of barcoded cDNAs, each barcoded cDNA including a complementary captured nucleic acid sequence covalently linked to one of the capture oligonucleotides;
sequencing the transcribed nucleic acids and the barcode sequence, thereby obtaining read sequences of the plurality of transcribed nucleic acids associated with read sequences of the barcode sequence;
identifying the barcode sequence based upon the read sequences; and
using the read sequence-identified barcode sequence and the in situ-identified barcode sequence to link the read sequences of the plurality of transcribed nucleic acids with the sequestration pen and thereby correlate the read sequences of the plurality of transcribed nucleic acids with the biological cell placed into the sequestration pen.

In some embodiments, a single biological cell may be disposed in the sequestration pen and subjected to the above method. Alternatively, more than one biological cell (e.g., a group of two or more biological cells that are from the same clonal population of cells) may be disposed within the sequestration pen and subjected to the above method.

The disposing of the capture object, identifying of the barcode of the capture object, disposing the biological cell, lysing/transcribing/ sequencing, and identifying the barcode sequence based upon the read sequence of the foregoing method can be performed in the order in which they are written or in other orders, with the limitation that the rearrangement of the order of these activities does not violate logical order (e.g., transcribing before lysing, and so on). As an example, in situ identification of the barcode sequence can be performed after introducing the biological cell into the sequestration pen, after lysing the biological cell, or after transcribing the captured nucleic acids. Likewise, the step of introducing the capture object into the sequestration pen can be performed after introducing the at least one biological cell into the sequestration pen.

In various embodiments, the method of correlating genomic data with a biological cell, may further include observing a phenotype of the biological cell; and correlating the read sequences of the plurality of transcribed nucleic acids with the phenotype of the biological cell. The method may additionally include observing a phenotype of the biological cell, where the biological cell is a representative of a clonal population; and correlating the read sequences of the plurality of transcribed nucleic acids with the phenotype of the biological cell and the clonal population. In some embodiments, observing the phenotype of the biological cell may include observing at least one physical characteristic of the at least one biological cell. In other embodiments, observing the phenotype of the biological cell may include performing an assay on the biological cell and observing a detectable signal generated during the assay. In some embodiments, the assay may be a protein expression assay.

For example, observing the phenotype of the biological cell can include observing a detectable signal generated when the biological cell interacts with an assay reagent. The detectable signal can be a fluorescent signal. Alternatively, the assay can be based upon the lack of a detectable signal. Further examples of assays that may be performed that provide a detectable signal identifying observation about the phenotype of the biological cell may be found within the disclosures of WO2015/061497 (Hobbs et al.); US2015/0165436 (Chapman et al.); and, International Application Serial No. PCT/US2017/027795 (Lionberger, et al.).

In various embodiments, identifying the barcode sequence of the plurality of capture oligonucleotides in-situ and recording an association between the identified barcode sequence and the sequestration pen may be performed before disposing the biological cell into the sequestration pen. In some other embodiments, identifying the barcode sequence of the plurality of capture oligonucleotides in-situ and recording an association between the identified barcode sequence and the sequestration pen may be performed after introducing the biological cell into the sequestration pen.

In yet other embodiments, disposing the capture object and, optionally, identifying the barcode sequence of the plurality of capture oligonucleotides in-situ and recording an association between the identified barcode sequence and the sequestration pen may be performed after observing a phenotype of the biological cell. In some embodiments, identifying the barcode sequence of the plurality of capture oligonucleotides in-situ and recording an association between the identified barcode sequence and the sequestration pen may be performed after lysing the biological cell and allowing the nucleic acids released from the lysed biological cell to be captured by the plurality of capture oligonucleotides comprised by the capture object. In various embodiments, identifying the barcode sequence of the plurality of capture oligonucleotide in-situ may include performing any variation of the method as described herein. In various embodiments of the method of correlating genomic data with a biological cell in a microfluidic device, the capture object may be any capture object as described herein.

In various embodiments of the method, the enclosure of the microfluidic device may include a dielectrophoretic (DEP) configuration, and disposing the capture object into the sequestration pen may include using dielectrophoretic (DEP) forces to move the capture object. In some other embodiments of the method, the enclosure of the microfluidic device may further include a dielectrophoretic (DEP) configuration, and disposing the biological cell within the sequestration pen may include using dielectrophoretic (DEP) forces to move the biological cell.

In various embodiments of the method of correlating genomic data with a biological cell in a microfluidic device, the method may further include: disposing a plurality of capture objects into a corresponding plurality of sequestration pens of the microfluidic device (e.g., this may include disposing a single capture object per sequestration pen}; disposing a plurality of biological cells into the corresponding plurality of sequestration pens, and processing each of the plurality of capture objects and plurality of biological cells according to the additional steps of the method.

A kit for producing a nucleic acid library. A kit is also described herein for producing a nucleic acid library, including: a microfluidic device comprising an enclosure, where the enclosure includes a flow region and a plurality of sequestration pens opening off of the flow region; and a plurality of capture objects, where each capture object of the plurality includes a plurality of capture oligonucleotides, each capture oligonucleotide of the plurality including: a capture sequence; and a barcode sequence comprising at least three cassetable oligonucleotide sequences, where each cassetable oligonucleotide sequence of the barcode sequence is non-identical to the other cassetable oligonucleotide sequences of the barcode sequence, and where each capture oligonucleotide of the plurality comprises the same barcode sequence.

Each capture oligonucleotide of the plurality may include at least three cassetable oligonucleotide sequences (e.g., three, four, five, or more cassetable oligonucleotide sequences). The cassetable oligonucleotide sequences can be as described elsewhere herein. For example, the cassetable oligonucleotide sequences can be selected from a set of non-identical cassetable oligonucleotide sequences. The set can include 12 or more (e.g., 12 to 100) non-identical cassetable oligonucleotide sequences.

The microfluidic device can be any microfluidic device as described herein.

The enclosure of the microfluidic device may further include a dielectrophoretic (DEP) configuration.

The plurality of capture objects may be any plurality of capture objects as described herein.

Each of the plurality of capture objects may be disposed singly into corresponding sequestration pens of plurality.

The kit may further include an identification table, wherein the identification table correlates the barcode sequence of the plurality of capture oligonucleotides of each of the plurality of capture objects with the corresponding sequestration pens of the plurality.

The kit may further include: a plurality of hybridization probes, where each hybridization probe includes: an oligonucleotide sequence complementary to any one of the cassetable oligonucleotide sequences of the plurality of capture oligonucleotides of any one of the plurality of capture objects; and a label, where the complementary sequence of each hybridization probe of the plurality is complementary to a different cassetable oligonucleotide sequence; and where the label of each hybridization probe of the plurality is selected from a set of spectrally distinguishable labels.

Each complementary sequence of a hybridization probe of the plurality may include an oligonucleotide sequence comprising a sequence of any one of SEQ ID NOs: 41 to 80. The label may be a fluorescent label.

**Method for producing a capture object.** A method is also described herein for producing a capture object having a plurality of capture oligonucleotides, including: chemically linking each of the plurality of capture oligonucleotides to the capture object, wherein each capture oligonucleotide of the plurality includes: a priming sequence which binds to a primer; a capture sequence (e.g., configured to hybridize with a target nucleic acid); and a barcode sequence, wherein the barcode sequence includes three or more cassetable oligonucleotide sequences, each cassetable oligonucleotide sequence being non-identical to the other cassetable oligonucleotide sequences of the barcode sequence; and wherein each capture oligonucleotide of the plurality comprises the same barcode sequence.

The capture object may be a bead. For example, the capture object can be a bead (or similar object) having a core that includes a paramagnetic material, a polymeric material and/or glass. The polymeric material may be polystyrene or any other plastic material which may be functionalized to link the capture oligonucleotide. The core material of the capture object may be coated to provide a suitable material to attach linkers to the capture oligonucleotide, which may include functionalized polymers, although other arrangements are possible.

Linking may include covalently linking each of the plurality of capture oligonucleotides to the capture object. Alternatively, each of the plurality of capture oligonucleotides may be non-covalently linked to the bead, which may be via a streptavidin/biotin linkage. The barcoded beads may be synthesized in any suitable manner as is known in the art. The priming sequence/Unique molecular identifier tag/Cell Barcode/primer sequence may be synthesized by total oligonucleotide synthesis, split and pool synthesis, ligation of oligonucleotide segments of any length, or any combination thereof.

Each capture oligonucleotide of the plurality may include a 5'-most nucleotide and a 3'-most nucleotide, where the priming sequence may be adjacent to or comprises the 5'-most nucleotide, where the capture sequence may be adjacent to or comprises the 3'-most nucleotide, and where the barcode sequence may be located 3' to the priming sequence and 5' to the capture sequence.

The three or more cassetable oligonucleotide sequences of each barcode sequence may be linked in tandem without any intervening oligonucleotide sequences. The one or more of the cassetable oligonucleotides may be linked to another cassetable oligonucleotide sequence via intervening one or two nucleotides to permit linking via ligation chemistry.

The method may further include: introducing each of the three or more cassetable oligonucleotide sequences into the capture oligonucleotides of the plurality via a split and pool synthesis.

Each cassetable oligonucleotide sequence may include about 6 to 15 nucleotides, and may include about 10 nucleotides.

The method may further include: selecting each of the three or more cassetable oligonucleotide sequences of each barcode sequence from a set of 12 to 100 non-identical cassetable oligonucleotide sequences. The method may include selecting each of the three or more cassetable oligonucleotides sequences of each barcode sequence from SEQ ID NOs: 1-40.

The cell-associated barcode sequence may include four cassetable oligonucleotide sequences. The method may include selecting: a first cassetable oligonucleotide sequence from any one of SEQ ID NOs: 1-10; selecting a second cassetable oligonucleotide sequence from any one of SEQ ID NOs: 11- 20; selecting a third cassetable oligonucleotide sequence from any one of SEQ ID NOs: 21-30; and selecting a fourth cassetable oligonucleotide sequence from any one of SEQ ID NOs: 31-40.

The priming sequence, when separated from said capture oligonucleotide, may prime a DNA polymerase.

The DNA polymerase may be a reverse transcriptase. The priming sequence may comprise a sequence of a P7 or P5 primer.

The method may further include: introducing a unique molecule identifier (UMI) sequence into each capture oligonucleotide of the plurality, such that each capture oligonucleotide of the plurality includes a different UMI. The UMI may be an oligonucleotide sequence comprising 5 to 20 nucleotides (e.g., 8 to 15 nucleotides).

The capture sequence may include a poly-dT sequence, a random hexamer, or a mosaic end sequence.

The method may further include: introducing the primer sequence into each capture oligonucleotide of the plurality near a 5' end of the capture oligonucleotide; and, introducing the capture sequence into each capture oligonucleotide of the plurality near a 3' end of the capture oligonucleotide.

The method may further include: introducing the barcode sequence into each capture oligonucleotide of the plurality after introducing the priming sequence and before introducing the capture sequence.

The method may further include: introducing the UMI into each capture oligonucleotide of the plurality after introducing the priming sequence and before introducing the capture sequence. The method may further include: introducing a sequence comprising a Not1 restriction site into each capture oligonucleotide of the plurality. The method may further include: introducing the sequence comprising the Not1 restriction site after introducing the barcode sequence and before introducing the capture sequence.

The method may further include: introducing one or more adapter sequences into each capture oligonucleotide of the plurality.

**Methods of Generating Sequencing Libraries.** Based on the workflows described herein, a variety of sequencing libraries may be prepared that will permit correlation of genomic data with the location of the source cell as well as phenotype information observed for that cell. The approaches shown here are adapted for eventual use with Illumina^{®} sequencing by
synthesis chemistries, but are not so limited. Any sort of sequencing chemistries may be suitable for use within these methods and may include emulsion PCR, sequencing by synthesis, pyrosequencing and semiconductor detection. One of skill can adapt the methods and construction of the capture oligonucleotides and associated adaptors, primers and the like to use these methods within other massively parallel sequencing platforms and chemistries such as PacBio long read systems (SMRT, Pacific Biosystems), Ion Torrent (ThermoFisher Scientific), Roche 454, Oxford Nanopore, and the like.

**RNA capture and library preparation.** Also, a method is described herein for providing a barcoded cDNA library from a biological cell, including: disposing the biological cell within a sequestration pen located within an enclosure of a microfluidic device; disposing a capture object within the sequestration pen, wherein the capture object comprises a plurality of capture oligonucleotides, each capture oligonucleotide of the plurality including: a priming sequence; a capture sequence; and a barcode sequence, wherein the barcode sequence comprises three or more cassetable oligonucleotide sequences, each cassetable oligonucleotide sequence being non-identical to every other cassetable oligonucleotide sequence of the barcode sequence; lysing the biological cell and allowing nucleic acids released from the lysed biological cell to be captured by the plurality of capture oligonucleotides comprised by the capture object; and

transcribing the captured nucleic acids, thereby producing a plurality of barcoded cDNAs decorating the capture object, each barcoded cDNA comprising (i) an oligonucleotide sequence complementary to a corresponding one of the captured nucleic acids, covalently linked to (ii) one of the plurality of capture oligonucleotides. The capture object may be a single capture object. The nucleic acids released from the lysed biological cell may be captured by the capture sequence of each of the plurality of capture oligonucleotides of the capture object.

Transcribing may include reverse transcribing. The capture object and/or biological cell can be, for example, disposed within an isolation region of the sequestration pen.

The biological cell may be an immune cell, for example a T cell, B cell, NK cell, macrophage, and the like. The biological cell may be a cancer cell, such as a melanoma cancer cell, breast cancer cell, neurological cancer cell, etc.

The biological cell may be a stem cell (e.g., embryonic stem cell, induced pluripotent (iPS) stem cell, etc.) or a progenitor cell. The biological cell may be an embryo (e.g., a zygote, a 2 to 200 cell embryo, a blastula, etc.).

The biological cell may be a single biological cell. Alternatively, the biological cell can be a plurality of biological cells, such as a clonal population.

Disposing the biological cell may further include marking the biological cell (e.g., with a marker for nucleic acids, such as Dapi or Hoechst stain.

The capture object may be any capture object as described herein.

The capture sequence of one or more (which can be each) of the plurality of capture oligonucleotides may include an oligo-dT primer sequence.

The capture sequence of one or more (e.g., each) of the plurality of capture oligonucleotides may include a gene-specific primer sequence. The gene-specific primer sequence may target (or may bind to) an mRNA sequence encoding a T cell receptor (TCR) (e.g., a TCR alpha chain or TCR beta chain, particularly a region of the mRNA encoding a variable region or a region of the mRNA located 3' but proximal to the variable region). The gene-specific primer sequence may target (or may bind to) an mRNA sequence encoding a B-cell receptor (BCR) (e.g., a BCR light chain or BCR heavy chain, particularly a region of the mRNA encoding a variable region or a region of the mRNA located 3' but proximal to the variable region).

The capture sequence of one or more (e.g., all or substantially all) of the plurality of capture oligonucleotides may bind to one of the released nucleic acids and primes the released nucleic acid, thereby allowing a polymerase (e.g., reverse transcriptase) to transcribe the captured nucleic acids.

The capture object may include a magnetic component (e.g., a magnetic bead). Alternatively, the capture object can be non-magnetic.

Disposing the biological cell within the sequestration pen may be performed before disposing the capture object within the sequestration pen.

Disposing the capture object within the sequestration pen may be performed before disposing the biological cell within the sequestration pen.

The method may further include: identifying the barcode sequence of the plurality of capture oligonucleotides of the capture object in situ, while the capture object is located within the sequestration pen. Identifying the barcode may be performed using any method of identifying the barcode as described herein.

Identifying the barcode sequence may be performed before lysing the biological cell.

The enclosure of the microfluidic device may include at least one coated surface. The coated surface can be coated with Tris and/or a polymer, such as a PEG-PPG block co-polymer. The enclosure of the microfluidic device may include at least one conditioned surface.

The at least one conditioned surface may include a covalently bound hydrophilic moiety or a negatively charged moiety. A covalently bound hydrophilic moiety or negatively charged moiety can be a hydrophilic or negatively charged polymer.

The enclosure of the microfluidic device may further include a dielectrophoretic (DEP) configuration. Disposing the biological cell and/or disposing the capture object may be performed by applying a dielectrophoretic (DEP) force on or proximal to the biological cell and/or the capture object.

The microfluidic device may further include a plurality of sequestration pens.

The method may further include disposing a plurality of the biological cells within the plurality of sequestration pens. The plurality of the biological cells may be a clonal population. Disposing the plurality of the biological cells within the plurality of sequestration pens may include disposing substantially only one biological cell of the plurality in corresponding sequestration pens of the plurality. Thus, each sequestration pen of the plurality having a biological cell disposed therein will generally contain a single biological cell. For example, less than 10%, 7%, 5%, 3% or 1% of occupied sequestration pens may contain more than one biological cell.

The method may further include: disposing a plurality of the capture objects within the plurality of sequestration pens. Disposing the plurality of the capture objects within the plurality of sequestration pens may include disposing substantially only one capture object within corresponding ones of sequestration pens of the plurality. Disposing the plurality of capture objects within the plurality of sequestration pens may be performed before the lysing the biological cell or the plurality of the biological cells. The plurality of the capture objects may be any plurality of capture objects as described herein.

The method may further include: exporting the capture object or the plurality of the capture objects from the microfluidic device. The capture object or capture objects may be cDNA decorated capture objects. Exporting the plurality of the capture objects may include exporting each of the plurality of the capture objects individually, (i.e., one at a time). The method may further include: delivering each the capture object of the plurality to a separate destination container outside of the microfluidic device.

One or more of the disposing the biological cell or plurality of the biological cells; the disposing the capture object or the plurality of the capture objects; the lysing the biological cell or the plurality of the biological cells and the allowing nucleic acids released from the lysed biological cell or the plurality of the biological cells to be captured; the transcribing; and the identifying the barcode sequence of the capture object or each the capture object of the plurality in-situ (if performed), may be performed in an automated manner.

Also, a method is described herein for providing a barcoded sequencing library, including: amplifying a cDNA library of a capture object or a cDNA library of each of a plurality of the capture objects obtained by any method described herein; and tagmenting the amplified DNA library or the plurality of cDNA libraries, thereby producing one or a plurality of barcoded sequencing libraries. Amplifying the cDNA library or the plurality of cDNA libraries may include introducing a pool index sequence, wherein the pool index sequence comprises 4 to 10 nucleotides. The method may further include combining a plurality of the barcoded sequencing libraries, wherein each barcoded sequencing library of the plurality comprises a different barcode sequence and/or a different pool index sequence.

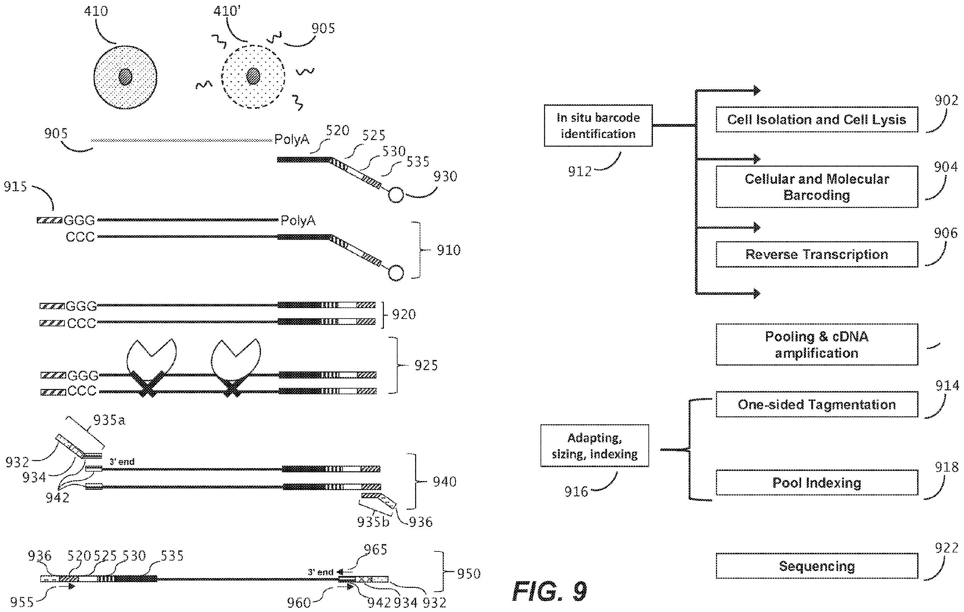

The method of obtaining cDNA from released nucleic acid, such as RNA, may be more fully understood by turning to Figure 9, which is a schematic representation of the process. For Cell Isolation and Cell Lysis Box 902, a biological cell 410 may be placed within a sequestration pen within a microfluidic device. A capture object 930, which may be configured as any capture object described herein, may be disposed into the same sequestration pen, which may be performed before or after disposing the cell 410 into the sequestration pen. The cell 410 may be lysed using a lysis reagent which lyses the outer cell membrane of cell 410 but not the nuclear membrane, as is described in the Examples below. A lysed cell 410' results from this process and releases nucleic acid 905, e.g., RNA. The capture oligo nucleotide of capture object 930 includes a priming sequence 520, which has a sequence of 5'-ACACTCTTTCCCTACACGACGCTCTTCCGATCT (SEQ ID NO. 104), and a barcode sequence 525, which may be configured like any barcode described herein. The capture oligonucleotide of capture object 930 may optionally include a UMI 530. The capture oligonucleotide of capture object 930 includes a capture sequence, which in this case includes a PolyT sequence which can capture the released nucleic acid 905 having a PolyA sequence at its 3' end. The capture sequence 535 captures the released nucleic acid 905. In the Cellular and Molecular barcoding box 904 and Reverse Transcription box 906 the capture oligonucleotide is, and reversed transcribed from the released nucleic acid 905 while in the presence of template switching oligonucleotide 915, which has a sequence of /5Me-isodC//isodG//iMe-isodC/ACACTCTTTCCCTACACGACGCrGrGrG (SEQ ID NO. 103). Identification of the barcode 912 may be performed, using any of the methods described herein either before RNA capture to the barcoded beads; before reverse transcription of the RNA captured to the beads, or after reverse transcription of the RNA on the bead. Identification of the cell specific barcode may be performed after reverse transcription of RNA captured to the bead. After both reverse transcription and in-situ identification of the barcode of the capture object has been achieved, the cDNA decorated capture object is exported out of the microfluidic device. A plurality of cDNA capture objects may be exported at the same time and the Pooling and cDNA amplification box 912(creating DNA amplicons 92) is performed, using an amplification primer having a sequence of 5'-/5Biosg/ACACTCTTTCCCT ACACGACGC-3' (SEQ ID NO. 105). Adapting, sizing and indexing box 916 is then performed on the amplified DNA 920. This includes the One Sided Tagmentation box 914 which fragments DNA to size the DNA 925 and insert tagmentation adaptors 942. While tagmentation is illustrated herein, this process can also be performed by enzymatic fragmentation, such as using fragmentase (NEB, Kapa), followed by end repair.

Also included in box 0916 is Pool Indexing box 918 where tagmented DNA 940 is acted upon by primers 935a and 935b. A first primer 935a, directed against the tagmentation adaptor 942 introduce a P7 sequencing adaptor 932, having a sequence of: 5'-CAAGCAGAAGACGGCATACGAGAT-3 (SESQ ID NO. 107); and also introduces optional Pool Index 934. A second primer 935b, having a sequence of: (5'-AATGATACGGCGACCACCGAGATCTACACTCTTTCCCTACACGACGCTCTTC C^{∗}G^{∗}A^{∗}T^{∗}C^{∗}T-3 (SEQ ID No. 106) has a portion directed against priming sequence 520 and introduces a P5 sequencing adaptor sequence 936. The sized, indexed and adapted sequencing library 950 may be sequenced in the Sequencing box 922, where a first sequencing read 955 (point of sequence read initiation reads the barcode 525 and optional UMI 539. A second sequencing read 960 reads Pool Index 934. A third sequencing read 965 reads a desired number of bp within the DNA library itself, to generate genomic reads.

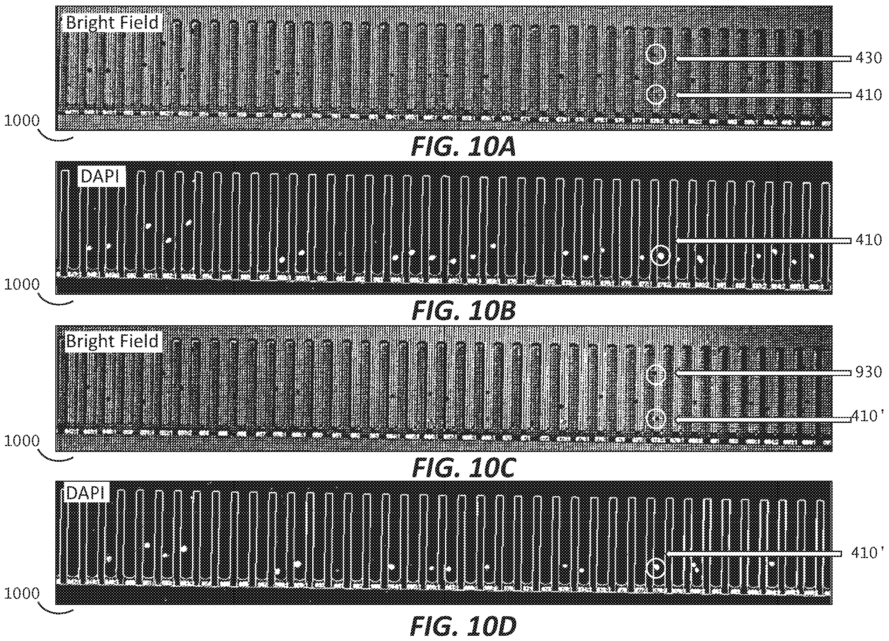

Figures 10A-10D are photographic representations of a process for lysis of an outer cell membrane with subsequent RNA capture.

Figure 10A shows a brightfield image showing the capture object 430 and cell 430 prior to lysis, each disposed within a sequestration pen within microfluidic device 1000. Figure 10B shows fluorescence from DAPI stained nucleic of the intact cells 410 at the same timepoint, before lysis. Figure 10C shows brightfield image of the capture object 930 and the remaining, unlysed nuclei 410' after lysis has been completed. Figure 10D shows a fluorescent image at the same timepoint as Figure 10C after lysis, showing DAPI fluorescence from the unbreached nuclei 410', showing that the nucleus is intact.

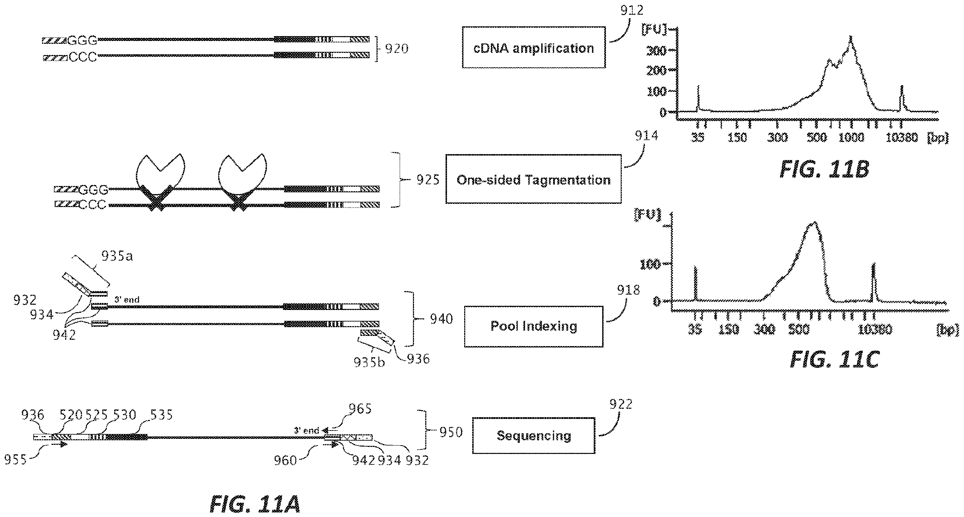

Figure 11A is a schematic representation of the processing of the cDNA resulting from the capture of RNA as shown in Figure 9, that is performed outside of the microfluidic environment, including cDNA amplification box 912, One-sided Tagmentation box 914, Pool Indexing box 918 and Sequencing box 922, along with some quality analysis. The QC after cDNA amplification box 912 is shown for amplified DNA 920 in Figure 11B, showing a size distribution having a large amount of product having a size of 700 to well over 1000bp. After completion of the tagmentation step, the size distribution of the resultant fragments in the barcoded library is shown in Figure 11C, and is within 300-800 bp, which is optimal for sequencing by synthesis protocols. Quantitation measured by Qubit shows that about 1.160 ng/ microliter of barcoded DNA sample was obtained from a single cell. For a sequencing run, and individually barcoded material from about 100 single cells was pooled to perform a sequencing run, providing sequencing data for each of the about 100 single cells.

This workflow may also be adapted to PacBio library preparation (SMRT system, Pacific Biosystems) by processing the barcoded cDNA obtained above, and SMRTbell adaptors may be directly ligated to the full length barcoded transcripts.

**DNA capture and generation of sequencing libraries.** Also, a method is described herein for providing a barcoded genomic DNA library from a biological micro-object, including disposing a biological micro-object comprising genomic DNA within a sequestration pen located within an enclosure of a microfluidic device; contacting the biological micro-object with a lysing reagent capable of disrupting a nuclear envelope of the biological micro-object, thereby releasing genomic DNA of the biological micro-object; tagmenting the released genomic DNA, thereby producing a plurality of tagmented genomic DNA fragments having a first end defined by a first tagmentation insert sequence and a second end defined by a second tagmentation insert sequence; disposing a capture object within the sequestration pen, wherein the capture object comprises a plurality of capture oligonucleotides, each capture oligonucleotide of the plurality comprising: a first priming sequence; a first tagmentation insert capture sequence; and a barcode sequence, wherein the barcode sequence comprises three or more cassetable oligonucleotide sequences, each cassetable oligonucleotide sequence being non-identical to every other cassetable oligonucleotide sequence of the barcode sequence; contacting ones of the plurality of tagmented genomic DNA fragments with (i) the first tagmentation insert capture sequence of ones of the plurality of capture oligonucleotides of the capture object, (ii) an amplification oligonucleotide comprising a second priming sequence linked to a second tagmentation insert capture sequence, a randomized primer sequence, or a gene-specific primer sequence, and (iii) an enzymatic mixture comprising a strand displacement enzyme and a polymerase; incubating the contacted plurality of tagmented genomic DNA fragments for a period of time, thereby simultaneously amplifying the ones of the plurality of tagmented genomic DNA fragments and adding the capture oligonucleotide and the amplification oligonucleotide to the ends of the ones of the plurality of tagmented genomic DNA fragments to produce the barcoded genomic DNA library; and exporting the barcoded genomic DNA library from the microfluidic device.

The genomic DNA can include mitochondrial DNA.

The capture object can be placed in sequestration pen before or after the tagmenting step. Incubation can be performed under isothermal conditions (e.g., about 30°C to about 45°C, typically about 37°C).

Exporting can include allowing the amplified genomic DNA to diffuse out of the sequestration pen into a flow region (e.g., a channel) to which the sequestration pen is connected, and then flowing medium (e.g., amplification buffer, export buffer, or the like) through the flow region, out of the microfluidic device, and into an appropriate receptacle (e.g., a well of a well-plate, a tube, such as a microcentrifuge tube, or the like).

Disposing the biological micro-object within the sequestration pen may be performed before disposing the capture object within the sequestration pen.

The biological micro-object may be a biological cell.

The biological micro-object may be a nucleus of a biological cell (e.g., a eukaryotic cell).

The biological cell may be an immune cell (e.g., T cell, B cell, NK cell, macrophage, etc.). The biological cell may be a cancer cell (e.g., melanoma cancer cell, breast cancer cell, neurological cancer cell, etc.).

The lysing reagent may include at least one ribonuclease inhibitor.

The tagmenting may include contacting the released genomic DNA with a transposase loaded with (i) a first double-stranded DNA fragment comprising the first tagmentation insert sequence, and (ii) a second double-stranded DNA fragment comprising the second tagmentation insert sequence. The first double-stranded DNA fragment may include a first mosaic end sequence linked to a third priming sequence, and the second double-stranded DNA fragment may include a second mosaic end sequence linked to a fourth priming sequence.

The first tagmentation insert capture sequence of each capture oligonucleotide of the capture object may include a sequence which is at least partially (or it may be fully) complementary to the first tagmentation insert sequence. The second tagmentation insert capture sequence of the amplification oligonucleotide comprises a sequence which is at least partially (or it may be fully) complementary to the second tagmentation insert sequence. For example, the first tagmentation insert capture sequence of each capture oligonucleotide can be at least partially (e.g., fully) complementary to the first mosaic end sequence and/or the third priming sequence of the first tagmentation insert sequence. In other examples, the second tagmentation insert capture sequence of the amplification oligonucleotide can be at least partially (e.g., fully) complementary to the second mosaic end sequence and/or the fourth priming sequence of the second tagmentation insert sequence.

The capture object may be any capture object as described herein. The capture object may include a magnetic component (e.g., a magnetic bead). Alternatively, the capture object can be non-magnetic.

The method may further inlcude: identifying the barcode sequence of the plurality of capture oligonucleotides of the capture object in situ, while the capture object is located within the sequestration pen. Identifying the barcode sequence may be performed using any method as described herein. Identifying the barcode sequence may be performed before lysing the biological cell. Alternatively, identifying the barcode sequence can be performed before tagmenting the released genomic DNA, or after exporting the barcoded genomic DNA library.

The enclosure of the microfluidic device may include at least one coated surface. The coated surface can be coated with Tris and/or a polymer, such as a PEG-PPG block co-polymer. The enclosure of the microfluidic device may comprise at least one conditioned surface.

The at least one conditioned surface may comprise a covalently bound hydrophilic moiety or a negatively charged moiety. The covalently bound hydrophilic or negatively charged moiety can be a hydrophilic or negatively charged polymer.

The enclosure of the microfluidic device may further include a dielectrophoretic (DEP) configuration, and disposing the biological micro-object and/or disposing the capture object may be performed by applying a dielectrophoretic (DEP) force on or proximal to the biological cell and/or the capture object.

The microfluidic device may further include a plurality of sequestration pens. The method may further include disposing a plurality of the biological micro-objects within the plurality of sequestration pens.

Disposing the plurality of the biological micro-objects within the plurality of sequestration pens may include disposing substantially only one biological micro-object of the plurality in corresponding sequestration pens of the plurality.

Thus, each sequestration pen of the plurality having a biological micro-object disposed therein will generally contain a single biological micro-object. For example, less than 10%, 7%, 5%, 3% or 1% of occupied sequestration pens may contain more than one biological micro-object. The plurality of the biological micro-objects may be a clonal population of biological cells.

The method may further include: disposing a plurality of the capture objects within the plurality of sequestration pens. Disposing the plurality of the capture objects within the plurality of sequestration pens may include disposing substantially only one capture object within corresponding ones of sequestration pens of the plurality. Disposing the plurality of capture objects within the plurality of sequestration pens may be performed before the lysing the biological micro-object or the plurality of the biological micro-objects.

The plurality of the capture objects may be any plurality of capture objects as described herein.

The steps of tagmenting, contacting, and incubating may be performed at substantially the same time for each of the sequestration pens containing one of the plurality of biological micro-objects.

One or more of the disposing the biological micro-object or the plurality of the biological micro-objects; the disposing the capture object or the plurality of the capture objects; the lysing the biological micro-object or the plurality of the biological micro-objects and the allowing nucleic acids released from the lysed biological cell or the plurality of the biological cells to be captured; the tagmenting the released genomic DNA; the contacting ones of the plurality of tagmented genomic DNA fragments; the incubating the contacted plurality of tagmented genomic DNA fragments; the exporting the barcoded genomic DNA library or the plurality of DNA libraries; and the identifying the barcode sequence of the capture object or each the capture object of the plurality in-situ may be performed in an automated manner.

The method may further include: exporting the capture object or the plurality of the capture objects from the microfluidic device. Exporting the plurality of the capture objects may include exporting each of the plurality of the capture objects individually.

The method may further include: delivering each the capture object of the plurality to a separate destination container outside of the microfluidic device.

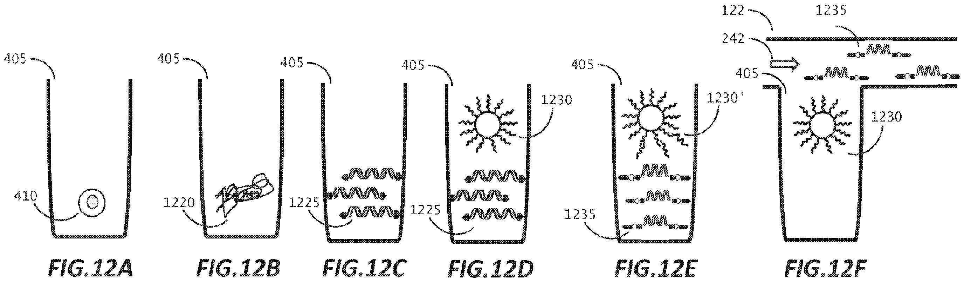

The methods may be better understood by turning to Figures 12A-G and Examples 3 and 4 below. Figures 12A-12F illustrate a workflow for obtaining a sequencing library having an in-situ detectable barcode as described herein. A biological cell 410 is disposed within a sequestration pen 405 which opens to a microfluidic channel (not shown) within a microfluidic device (Figure 12A). The cell is lysed to breach both the cell membrane and also the nuclear membrane, and release genomic DNA 1220, as in Figure 12B. Figure 12C illustrates the next process, tagmentation which is employed to create properly sized fragments and to insert tags providing tagmented DNA 1225 that permits capture and amplification. In Figure 12D, the capture object 1230 having a plurality of capture oligonucleotides is introduced to the pen 405 containing the tagmented DNA 1225. Each of the plurality of capture oligonucleotides includes an in-situ detectable barcode, priming sequence, and a capture sequence. In Figure 12E, tagmented DNA 1225 is subjected to an isothermal amplification using a recombinase/polymerase amplification, where the capture sequence of the capture oligonucleotides shepherd and direct (capture object 1230') the tagmented DNA, in the presence of the recombinase/polymerase machinery, to provided amplified DNA 1235, which includes sequencing adaptors, the barcode, and optional indices such as UMI or pool Index. Throughout amplification and thereafter, the amplified adapted barcoded DNA 1235 diffuses out of the sequestration pen 405 and into the microfluidic channel 122 to which the sequestration pens open, and are exported out of the microfluidic device using fluidic medium flow 242 (Figure 12F). Once exported, the amplified adapted barcoded DNA 1235 is quantified for use as a sequencing library, and may be pooled with other libraries for the sequencing run. After export of the DNA 1235 is complete, the in-situ determination of the barcode of the capture oligonucleotides of the capture object 1230 is performed using any of the methods described herein (Figure 12F).

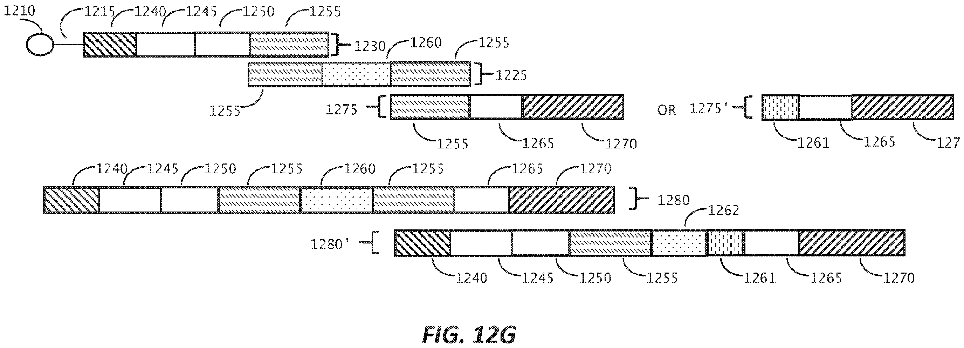

Figure 12G shows schematic representations of the capture oligonucleotide and DNA processing in the method of generating a sequencing library from DNA of a cell. Each of the capture oligonucleotides of the capture object 1230 is linked, covalently or non-covalently via linker 1215, and includes, from the 5' end of the capture oligonucleotide: priming sequence/adaptor 1240; barcode 1245, optional UMI 1250 and capture sequence 1255, which has a tagmentation insert capture sequence and may capture (e.g., shepherd and direct) a Mosaic End insert sequence. The barcode sequence 1245 may be any barcode sequence containing at least three cassetable oligonucleotide sequences as described herein. Tagmented DNA 1225 has tagmentation insert sequences 1255, which may be Mosaic End sequence insert, and DNA fragment 1260. It is primed during the isothermal recombinase polymerase driven amplification by either a generic primer 1275 having a P5 adaptor/priming sequence 1270, an optional Pool Index 1265 and the tagmentation insert capture sequence 1255. Alternatively, a gene specific primer 1275' may be used, where a portion of the primer 1261 is directed to select for a sub-set of DNA, e.g. a gene specific sequence.

The product of the isothermal amplification, which forms the sequencing library is amplified and adapted DNA 1280 or 1280'. Amplified and adapted DNA 1280 is the product of generic primer 1275, and includes generic library of DNA fragments 1260, while amplified DNA 1280', has a DNA fragment region 1262 (remainder of gene specific DNA primed by the gene specific priming sequence) plus 1261(gene specific priming sequence) which include gene specific amplification products.

**Generation of a barcoded cDNA library and a barcoded genomic DNA library from the same cell.** Also, a method is described herein for providing a barcoded cDNA library and a barcoded genomic DNA library from a single biological cell, including: disposing the biological cell within a sequestration pen located within an enclosure of a microfluidic device; disposing a first capture object within the sequestration pen, where the first capture object comprises a plurality of capture oligonucleotides, each capture oligonucleotide of the plurality comprising: a first priming sequence; a first capture sequence (e.g., configured to capture a released nucleic acid); and a first barcode sequence, wherein the first barcode sequence comprises three or more cassetable oligonucleotide sequences, each cassetable oligonucleotide sequence being non-identical to every other cassetable oligonucleotide sequence of the first barcode sequence; obtaining the barcoded cDNA library by performing any method of obtaining a cDNA library as described herein, where lysing the biological cell is performed such that a plasma membrane of the biological cell is degraded, releasing cytoplasmic RNA from the biological cell, while leaving a nuclear envelope of the biological cell intact, thereby providing the first capture object decorated with the barcoded cDNA library from the RNA of the biological cell; exporting the cDNA library-decorated first capture object from the microfluidic device; disposing a second capture object within the sequestration pen, wherein the second capture object comprises a plurality of capture oligonucleotides, each including: a second priming sequence; a first tagmentation insert capture sequence; and a second barcode sequence, wherein the second barcode sequence comprises three or more cassetable oligonucleotide sequences, each cassetable oligonucleotide sequence being non-identical to every other cassetable oligonucleotide sequence of the second barcode sequence; obtaining the barcoded genomic DNA library by performing any method of obtaining a barcoded genomic DNA library as described herein, where a plurality of tagmented genomic DNA fragments from the biological cell are contacted with the first tagmentation insert capture sequence of ones of the plurality of capture oligonucleotides of the second capture object, thereby providing the barcoded genomic DNA library from the genomic DNA of the biological cell; and exporting the barcoded genomic DNA library from the microfluidic device.

The method may further include: identifying the barcode sequence of the plurality of capture oligonucleotides of the first capture object.

Identifying the barcode sequence of the plurality of capture oligonucleotides of the first capture object may be performed before disposing the biological cell in the sequestration pen; before obtaining the barcoded cDNA library from the RNA of the biological cell; or before exporting the barcoded cDNA library-decorated first capture object from the microfluidic device.

The method may further include: identifying the barcode sequence of the plurality of oligonucleotides of the second capture object.

Identifying the barcode sequence of the plurality of capture oligonucleotides of the second capture may be performed before obtaining the barcoded genomic DNA library or after exporting the barcoded genomic DNA library from the microfluidic device.

Identifying the barcode sequence of the plurality of capture oligonucleotides of the first or the second capture object may be performed using any method of identifying a barcode in-situ as described herein.

The first capture object and the second capture object may each be any capture object as described herein.

The first priming sequence of the plurality of capture oligonucleotides of the first capture object may be different from the second priming sequence of the plurality of capture oligonucleotides of the second capture object. The first capture sequence of the plurality of capture oligonucleotides of the first capture object may be different from the first tagmentation insert capture sequence of the plurality of capture oligonucleotides of the second capture object.

The barcode sequence of the plurality of capture oligonucleotides of the first capture object may be the same as the barcode sequence of the plurality of capture oligonucleotides of the second capture object. The barcode sequence of the plurality of capture oligonucleotides of the first capture object may different from the barcode sequence of the plurality of capture oligonucleotides of the second capture object.

### Generation of B cell Receptor sequencing libraries.

A method is described herein for providing a barcoded B cell receptor (BCR) sequencing library, including: generating a barcoded cDNA library from a B lymphocyte, where the generating is performed according to any method of generation a barcoded cDNA library as described herein, where the barcoded cDNA library decorates a capture object including a plurality of capture oligonucleotides, each capture oligonucleotide of the plurality including a Not1 restriction site sequence; amplifying the barcoded cDNA library; selecting for barcoded BCR sequences from the barcoded cDNA library, thereby producing a library enriched for barcoded BCR sequences; circularizing sequences from the library enriched for barcoded BCR sequences, thereby producing a library of circularized barcoded BCR sequences; relinearizing the library of circularized barcoded BCR sequences to provide a library of rearranged barcoded BCR sequences, each presenting a constant (C) region of the BCR sequence 3' to a respective variable (V) sub-region and/or a respective diversity (D) sub-region; and, adding a sequencing adaptor and sub-selecting for the V sub-region and/or the D sub-region, thereby producing a barcoded BCR sequencing library.

The method may further include amplifying the BCR sequencing library to provide an amplified library of barcoded BCR sub-region sequences.

Amplifying the barcoded cDNA library may be performed using a universal primer.

Selecting for a BCR sequence region my include performing a polymerase chain reaction (PCR) selective for BCR sequences, thereby producing the library of barcoded BCR region selective amplified DNA. Selecting for barcoded BCR sequences may further include adding at least one sequencing primer sequence and/or at least one index sequence. Circularizing sequences from the library enriched for barcoded BCR sequences may include ligating a 5' end of each barcoded BCR sequence to its respective 3' end. Relinearizing the library of circularized barcoded BCR sequences may include digesting each of the library of circularized barcoded BCR sequences at the Not1 restriction site.

Adding the sequencing adaptor and sub-selecting for V and/or D sub-regions may include performing PCR, thereby adding a sequencing adaptor and sub-selecting for the V and/or D sub-regions.

The capture object may be any capture object as described herein.

The method may further include: identifying a barcode sequence of the plurality of capture oligonucleotides of the capture object using any method of identifying a barcode in-situ as described herein. Identifying may be performed before amplifying the barcoded cDNA library. Identifying may be performed while generating the barcoded cDNA library.

Any of amplifying the barcoded cDNA library; performing the polymerase chain reaction (PCR) selective for barcoded BCR sequences; circularizing sequences; relinearizing the library of circularized barcoded BCR sequences at the Not1 restriction site; and adding the sequencing adaptor and sub-selecting for V and/or D sub-regions may be performed within a sequestration pen located within an enclosure of a microfluidic device.

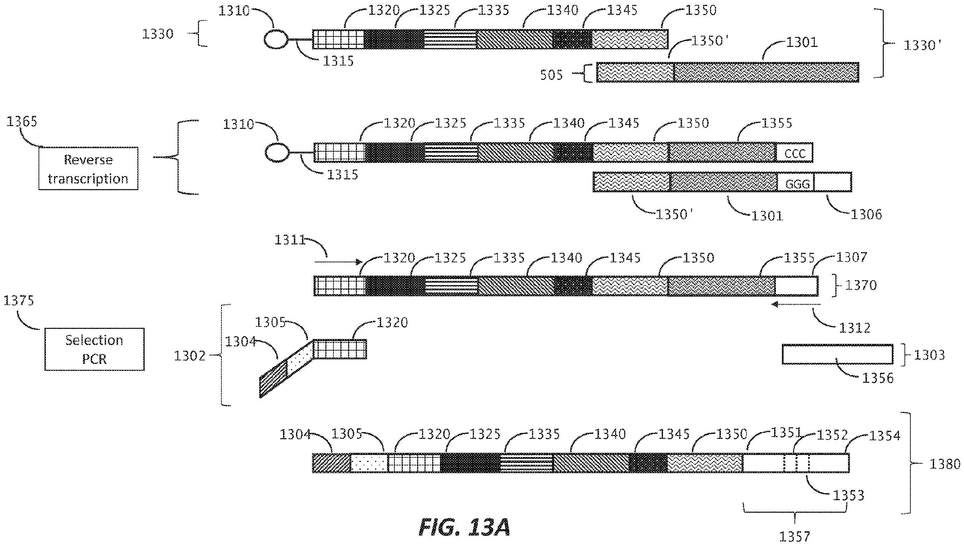

The methods for generating B cell Receptor (BCR) sequencing libraries may be better understood by turning to Figures 13A-B. Figures 13A-B are schematic representations of process of generating a BCR sequencing library as described here and in Example 7. Capture object 1330 includes a bead 1310 with only one capture oligonucleotide of the plurality of capture oligonucleotides shown for clarity. The capture oligonucleotide of capture object 1330 is linked to the bead 1310 via a linker 1315, which may be covalent or non-covalent. The linker 1315 is linked to the 5' end of the capture oligonucleotide where priming sequence 1 (1320) is located along the length of the capture oligonucleotide. The capture oligonucleotide also includes barcode 1325, which may be any in-situ detectable barcode as described herein; an optional UMI 1335; a sequencing adaptor sequence 1340; a Not1 restriction site sequence and a capture sequence 1350, which in this example is a generic capture sequence for RNA, Poly T (which may have two additional nucleotides at the 3'end, VN). The Not1 sequence 1345 is to the 5' of the capture sequence 1350 and is 3' to the barcode 1325, priming sequence 1320, sequencing adaptor 1340 and any UMI 1335. The capture oligonucleotide is configured to capture RNA 505, having RNA sequences of interest 1301 (other than PolyA sequence 1350') which is released upon lysis of the cell membrane of the source cell. The RNA is captured to the capture object by hybridizing its PolyA sequence 1350' to the PolyT capture sequence 1350 of the capture object, thus forming modified capture object 1330'. Reverse transcription box 1365, in the presence of template switching oligonucleotide 1306, provides cDNA decorated capture objects, where the capture oligonucleotide now contains a region of reverse transcribed nucleic acid 1355. Amplification of the cDNA via PCR, using generic primers 1311, 1312 (See Table 6, SEQ ID NO. 113) directed to the priming sequence 1 (1320) and to the portion of the Template switching oligonucleotide 1307 incorporated into the cDNA, provides an amplified DNA library 1370. Selection PCR 1375 using primer 1302 (which has a sequence 1320 directed against priming sequence 1(1320) of DNA 1370; optional Pool Index 1305 and Sequencing priming sequence 2 (1304) and BCR selective primer 1303, which selects only for BCR sequences, and species dependent. BCR selective primer 1303 may be a mixture of primers, which can target heavy or light chain regions (1356). See Table 6, SEQ ID Nos. 114-150). The product selected DNA 1380 has the priming sequence, UMI, barcode sequences as listed above for the product of the amplification 1370, but the DNA fragment now contains BCR region 1357 only, where the 5'most region of the BCR region 1357 is the full-length constant (C) sub-region 1351 of the BCR, with the Join (J) region 1352 (if present in the species under study); Diversity (D) sub-region 1353, and finally, to the 3' end Variable (V) region 1354.

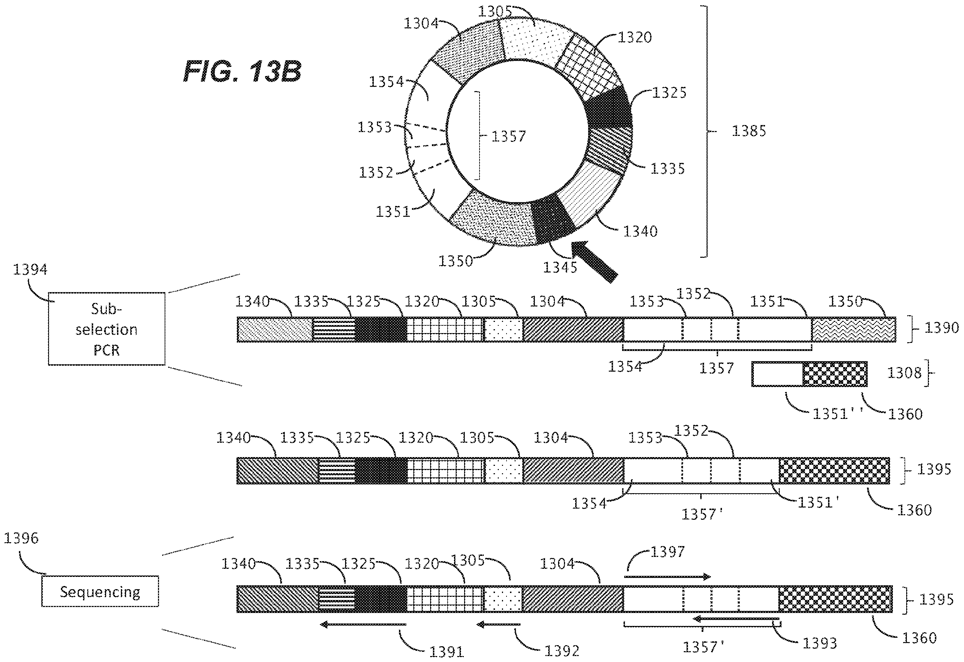

To make the BCR sub-regions of greater interest (V, D, J) more amenable to sequencing analysis, a rearrangement is performed. Selected DNA 1380 is circularized via ligation, to yield circularized DNA library 1385 in Figure 13B. Circularized DNA library 1385 is then digested at the Not1 restriction site 1345 (black arrow) to yield a re-linearize DNA library 1390. The effect of the circularization and relinearization is to bring the BCR sub-regions of greater interest (V, D, J) to better proximity of sequencing priming sites so that higher quality reads can be achieved. In the relinearized DNA library 1390, the order of the BCR sub-region sequences 5' to 3' have been reversed, and Variable (V) region 1354 is now disposed towards the 5' end of all of the BCR sub-regions, followed in order in the 3' direction by Diversity (D) sub-region 1353; Join (J) region 1352 (if present in the species under study); and finally, full length constant (C) sub-region 1351 of the BCR at the 3' most section of the BCR region sequence 1357.

Sub-selection PCR 1394 is performed next, where a primer 1308, including primer sequence 1360 and selection region 1351", is directed towards a sequence 1351' of the constant (C) sub-region. The sequence 135' is selected to be close to the 5' end of the C sub-region) to excise much of the C sub-region. This yields sub-selected DNA library 1395, which has had priming sequence 1360 added as well. The sub-selected BCR region now permits higher quality reads and length of read into the V, D, and J regions by placing it in better juxtaposition with sequencing priming sites and by 1) removing the polyT sequence 1350 entirely and 2) removing a substantial region of BCR C sub-region. Sequencing 1396 is performed upon the sub-selected DNA library 1395, and yields a first read of barcode 1325 and optional UMI 1335. Sequencing 1392 reads the optional pool index. Sequencing 1393 and 1397 reads the sub-selected BCR 1357'.

Any of the methods for generating a sequencing library may also be performed by introducing two or more capture objects to the sequestration pen. Each of the two or more capture objects may include two or more capture oligonucleotides having a cell-associated barcode including one or more cassetable sub-units as described above, as well as a priming sequence.

Each of the two or more capture objects may have the same barcode.

When two or more capture objects are introduced to the same pen, each capture object may have a different cell-associated barcode. Each of the two or more capture objects may have the same cell-associated barcode. Using more than one capture object may permit more nucleic acid capture capacity. The methods of in-situ identification described herein may easily be extended to identify two or more capture objects within one sequestration pen.

### Microfluidic devices and systems for operating and observing such devices.

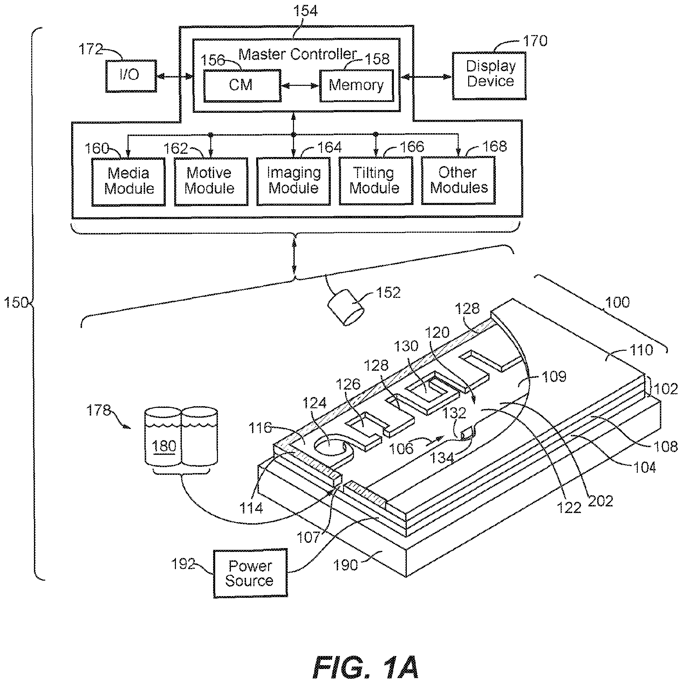

Figure 1A illustrates an example of a microfluidic device 100 and a system 150 which can be used for maintaining, isolating, assaying or culturing biological micro-objects. A perspective view of the microfluidic device 100 is shown having a partial cut-away of its cover 110 to provide a partial view into the microfluidic device 100. The microfluidic device 100 generally comprises a microfluidic circuit 120 comprising a flow path 106 through which a fluidic medium 180 can flow, optionally carrying one or more micro-objects (not shown) into and/or through the microfluidic circuit 120. Although a single microfluidic circuit 120 is illustrated in Figure 1A, suitable microfluidic devices can include a plurality (e.g., 2 or 3) of such microfluidic circuits. Regardless, the microfluidic device 100 can be configured to be a nanofluidic device. As illustrated in Figure 1A, the microfluidic circuit 120 may include a plurality of microfluidic sequestration pens 124, 126, 128, and 130, where each sequestration pens may have one or more openings in fluidic communication with flow path 106. In some embodiments of the device of Figure 1A, the sequestration pens may have only a single opening in fluidic communication with the flow path 106. As discussed further below, the microfluidic sequestration pens comprise various features and structures that have been optimized for retaining micro-objects in the microfluidic device, such as microfluidic device 100, even when a medium 180 is flowing through the flow path 106. Before turning to the foregoing, however, a brief description of microfluidic device 100 and system 150 is provided.

As generally illustrated in Figure 1A, the microfluidic circuit 120 is defined by an enclosure 102. Although the enclosure 102 can be physically structured in different configurations, in the example shown in Figure 1A the enclosure 102 is depicted as comprising a support structure 104 (e.g., a base), a microfluidic circuit structure 108, and a cover 110. The support structure 104, microfluidic circuit structure 108, and cover 110 can be attached to each other. For example, the microfluidic circuit structure 108 can be disposed on an inner surface 109 of the support structure 104, and the cover 110 can be disposed over the microfluidic circuit structure 108. Together with the support structure 104 and cover 110, the microfluidic circuit structure 108 can define the elements of the microfluidic circuit 120.

The support structure 104 can be at the bottom and the cover 110 at the top of the microfluidic circuit 120 as illustrated in Figure 1A. Alternatively, the support structure 104 and the cover 110 can be configured in other orientations. For example, the support structure 104 can be at the top and the cover 110 at the bottom of the microfluidic circuit 120. Regardless, there can be one or more ports 107 each comprising a passage into or out of the enclosure 102. Examples of a passage include a valve, a gate, a pass-through hole, or the like. As illustrated, port 107 is a pass-through hole created by a gap in the microfluidic circuit structure 108. However, the port 107 can be situated in other components of the enclosure 102, such as the cover 110. Only one port 107 is illustrated in Figure 1A but the microfluidic circuit 120 can have two or more ports 107. For example, there can be a first port 107 that functions as an inlet for fluid entering the microfluidic circuit 120, and there can be a second port 107 that functions as an outlet for fluid exiting the microfluidic circuit 120. Whether a port 107 function as an inlet or an outlet can depend upon the direction that fluid flows through flow path 106.

The support structure 104 can comprise one or more electrodes (not shown) and a substrate or a plurality of interconnected substrates. For example, the support structure 104 can comprise one or more semiconductor substrates, each of which is electrically connected to an electrode (e.g., all or a subset of the semiconductor substrates can be electrically connected to a single electrode). The support structure 104 can further comprise a printed circuit board assembly ("PCBA"). For example, the semiconductor substrate(s) can be mounted on a PCBA.

The microfluidic circuit structure 108 can define circuit elements of the microfluidic circuit 120. Such circuit elements can comprise spaces or regions that can be fluidly interconnected when microfluidic circuit 120 is filled with fluid, such as flow regions (which may include or be one or more flow channels), chambers, pens, traps, and the like. In the microfluidic circuit 120 illustrated in Figure 1A, the microfluidic circuit structure 108 comprises a frame 114 and a microfluidic circuit material 116. The frame 114 can partially or completely enclose the microfluidic circuit material 116. The frame 114 can be, for example, a relatively rigid structure substantially surrounding the microfluidic circuit material 116. For example, the frame 114 can comprise a metal material.

The microfluidic circuit material 116 can be patterned with cavities or the like to define circuit elements and interconnections of the microfluidic circuit 120. The microfluidic circuit material 116 can comprise a flexible material, such as a flexible polymer (e.g. rubber, plastic, elastomer, silicone, polydimethylsiloxane ("PDMS"), or the like), which can be gas permeable. Other examples of materials that can compose microfluidic circuit material 116 include molded glass, an etchable material such as silicone (e.g. photo-patternable silicone or "PPS"), photo-resist (e.g., SU8), or the like. In some embodiments, such materials-and thus the microfluidic circuit material 116-can be rigid and/or substantially impermeable to gas. Regardless, microfluidic circuit material 116 can be disposed on the support structure 104 and inside the frame 114.

The cover 110 can be an integral part of the frame 114 and/or the microfluidic circuit material 116. Alternatively, the cover 110 can be a structurally distinct element, as illustrated in Figure 1A. The cover 110 can comprise the same or different materials than the frame 114 and/or the microfluidic circuit material 116. Similarly, the support structure 104 can be a separate structure from the frame 114 or microfluidic circuit material 116 as illustrated, or an integral part of the frame 114 or microfluidic circuit material 116. Likewise, the frame 114 and microfluidic circuit material 116 can be separate structures as shown in Figure 1A or integral portions of the same structure.

In some embodiments, the cover 110 can comprise a rigid material. The rigid material may be glass or a material with similar properties. In some embodiments, the cover 110 can comprise a deformable material. The deformable material can be a polymer, such as PDMS. In some embodiments, the cover 110 can comprise both rigid and deformable materials. For example, one or more portions of cover 110 (e.g., one or more portions positioned over sequestration pens 124, 126, 128, 130) can comprise a deformable material that interfaces with rigid materials of the cover 110. In some embodiments, the cover 110 can further include one or more electrodes. The one or more electrodes can comprise a conductive oxide, such as indium-tin-oxide (ITO), which may be coated on glass or a similarly insulating material. Alternatively, the one or more electrodes can be flexible electrodes, such as single-walled nanotubes, multi-walled nanotubes, nanowires, clusters of electrically conductive nanoparticles, or combinations thereof, embedded in a deformable material, such as a polymer (e.g., PDMS). Flexible electrodes that can be used in microfluidic devices have been described, for example, in U.S. 2012/0325665 (Chiou et al.). In some embodiments, the cover 110 can be modified (e.g., by conditioning all or part of a surface that faces inward toward the microfluidic circuit 120) to support cell adhesion, viability and/or growth. The modification may include a coating of a synthetic or natural polymer. In some embodiments, the cover 110 and/or the support structure 104 can be transparent to light. The cover 110 may also include at least one material that is gas permeable (e.g., PDMS or PPS).

Figure 1A also shows a system 150 for operating and controlling microfluidic devices, such as microfluidic device 100. System 150 includes an electrical power source 192, an imaging device, and a tilting device 190 (part of tilting module 166).

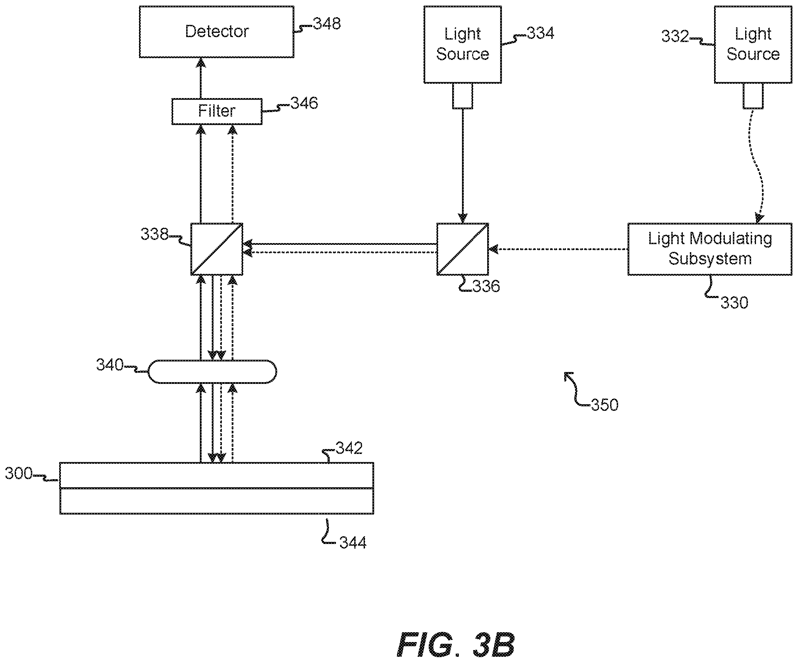

The electrical power source 192 can provide electric power to the microfluidic device 100 and/or tilting device 190, providing biasing voltages or currents as needed. The electrical power source 192 can, for example, comprise one or more alternating current (AC) and/or direct current (DC) voltage or current sources. The imaging device (part of imaging module 164, discussed below) can comprise a device, such as a digital camera, for capturing images inside microfluidic circuit 120. In some instances, the imaging device further comprises a detector having a fast frame rate and/or high sensitivity (e.g. for low light applications). The imaging device can also include a mechanism for directing stimulating radiation and/or light beams into the microfluidic circuit 120 and collecting radiation and/or light beams reflected or emitted from the microfluidic circuit 120 (or micro-objects contained therein). The emitted light beams may be in the visible spectrum and may, e.g., include fluorescent emissions. The reflected light beams may include reflected emissions originating from an LED or a wide spectrum lamp, such as a mercury lamp (e.g. a high pressure mercury lamp) or a Xenon arc lamp. As discussed with respect to Figure 3B, the imaging device may further include a microscope (or an optical train), which may or may not include an eyepiece.

System 150 further comprises a tilting device 190 (part of tilting module 166, discussed below) configured to rotate a microfluidic device 100 about one or more axes of rotation. In some embodiments, the tilting device 190 is configured to support and/or hold the enclosure 102 comprising the microfluidic circuit 120 about at least one axis such that the microfluidic device 100 (and thus the microfluidic circuit 120) can be held in a level orientation (i.e. at 0° relative to x- and y-axes), a vertical orientation (i.e. at 90° relative to the x-axis and/or the y-axis), or any orientation therebetween. The orientation of the microfluidic device 100 (and the microfluidic circuit 120) relative to an axis is referred to herein as the "tilt" of the microfluidic device 100 (and the microfluidic circuit 120). For example, the tilting device 190 can tilt the microfluidic device 100 at 0.1°, 0.2°, 0.3°, 0.4°, 0.5°, 0.6°, 0.7°, 0.8°, 0.9°, 1°, 2°, 3°, 4°, 5°, 10°, 15°, 20°, 25°, 30°, 35°, 40°, 45°, 50°, 55°, 60°, 65°, 70°, 75°, 80°, 90° relative to the x-axis or any degree therebetween. The level orientation (and thus the x- and y-axes) is defined as normal to a vertical axis defined by the force of gravity. The tilting device can also tilt the microfluidic device 100 (and the microfluidic circuit 120) to any degree greater than 90° relative to the x-axis and/or y-axis, or tilt the microfluidic device 100 (and the microfluidic circuit 120) 180° relative to the x-axis or the y-axis in order to fully invert the microfluidic device 100 (and the microfluidic circuit 120). Similarly, in some embodiments, the tilting device 190 tilts the microfluidic device 100 (and the microfluidic circuit 120) about an axis of rotation defined by flow path 106 or some other portion of microfluidic circuit 120.

In some instances, the microfluidic device 100 is tilted into a vertical orientation such that the flow path 106 is positioned above or below one or more sequestration pens. The term "above" as used herein denotes that the flow path 106 is positioned higher than the one or more sequestration pens on a vertical axis defined by the force of gravity (i.e. an object in a sequestration pen above a flow path 106 would have a higher gravitational potential energy than an object in the flow path). The term "below" as used herein denotes that the flow path 106 is positioned lower than the one or more sequestration pens on a vertical axis defined by the force of gravity (i.e. an object in a sequestration pen below a flow path 106 would have a lower gravitational potential energy than an object in the flow path).

In some instances, the tilting device 190 tilts the microfluidic device 100 about an axis that is parallel to the flow path 106. Moreover, the microfluidic device 100 can be tilted to an angle of less than 90° such that the flow path 106 is located above or below one or more sequestration pens without being located directly above or below the sequestration pens. In other instances, the tilting device 190 tilts the microfluidic device 100 about an axis perpendicular to the flow path 106. In still other instances, the tilting device 190 tilts the microfluidic device 100 about an axis that is neither parallel nor perpendicular to the flow path 106.

System 150 can further include a media source 178. The media source 178 (e.g., a container, reservoir, or the like) can comprise multiple sections or containers, each for holding a different fluidic medium 180. Thus, the media source 178 can be a device that is outside of and separate from the microfluidic device 100, as illustrated in Figure 1A. Alternatively, the media source 178 can be located in whole or in part inside the enclosure 102 of the microfluidic device 100. For example, the media source 178 can comprise reservoirs that are part of the microfluidic device 100.

Figure 1A also illustrates simplified block diagram depictions of examples of control and monitoring equipment 152 that constitute part of system 150 and can be utilized in conjunction with a microfluidic device 100. As shown, examples of such control and monitoring equipment 152 include a master controller 154 comprising a media module 160 for controlling the media source 178, a motive module 162 for controlling movement and/or selection of micro-objects (not shown) and/or medium (e.g., droplets of medium) in the microfluidic circuit 120, an imaging module 164 for controlling an imaging device (e.g., a camera, microscope, light source or any combination thereof) for capturing images (e.g., digital images), and a tilting module 166 for controlling a tilting device 190. The control equipment 152 can also include other modules 168 for controlling, monitoring, or performing other functions with respect to the microfluidic device 100. As shown, the equipment 152 can further include a display device 170 and an input/output device 172.

The master controller 154 can comprise a control module 156 and a digital memory 158. The control module 156 can comprise, for example, a digital processor configured to operate in accordance with machine executable instructions (e.g., software, firmware, source code, or the like) stored as non-transitory data or signals in the memory 158. Alternatively, or in addition, the control module 156 can comprise hardwired digital circuitry and/or analog circuitry. The media module 160, motive module 162, imaging module 164, tilting module 166, and/or other modules 168 can be similarly configured. Thus, functions, processes acts, actions, or steps of a process discussed herein as being performed with respect to the microfluidic device 100 or any other microfluidic apparatus can be performed by any one or more of the master controller 154, media module 160, motive module 162, imaging module 164, tilting module 166, and/or other modules 168 configured as discussed above. Similarly, the master controller 154, media module 160, motive module 162, imaging module 164, tilting module 166, and/or other modules 168 may be communicatively coupled to transmit and receive data used in any function, process, act, action or step discussed herein.

The media module 160 controls the media source 178. For example, the media module 160 can control the media source 178 to input a selected fluidic medium 180 into the enclosure 102 (e.g., through an inlet port 107). The media module 160 can also control removal of media from the enclosure 102 (e.g., through an outlet port (not shown)). One or more media can thus be selectively input into and removed from the microfluidic circuit 120. The media module 160 can also control the flow of fluidic medium 180 in the flow path 106 inside the microfluidic circuit 120. For example, in some embodiments media module 160 stops the flow of media 180 in the flow path 106 and through the enclosure 102 prior to the tilting module 166 causing the tilting device 190 to tilt the microfluidic device 100 to a desired angle of incline.

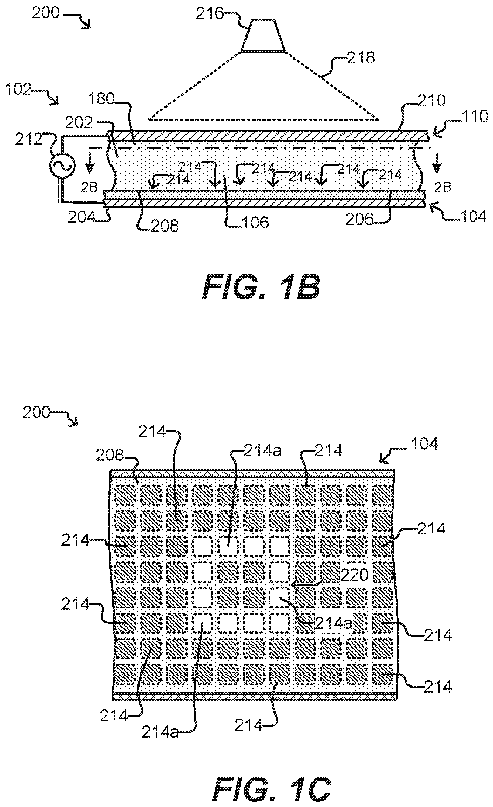

The motive module 162 can be configured to control selection, trapping, and movement of micro-objects (not shown) in the microfluidic circuit 120. As discussed below with respect to Figures 1B and 1C, the enclosure 102 can comprise a dielectrophoresis (DEP), optoelectronic tweezers (OET) and/or opto-electrowetting (OEW) configuration (not shown in Figure 1A), and the motive module 162 can control the activation of electrodes and/or transistors (e.g., phototransistors) to select and move micro-objects (not shown) and/or droplets of medium (not shown) in the flow path 106 and/or sequestration pens 124, 126, 128, 130.

The imaging module 164 can control the imaging device. For example, the imaging module 164 can receive and process image data from the imaging device. Image data from the imaging device can comprise any type of information captured by the imaging device (e.g., the presence or absence of micro-objects, droplets of medium, accumulation of detectable label, such as fluorescent label, etc.). Using the information captured by the imaging device, the imaging module 164 can further calculate the position of objects (e.g., micro-objects, droplets of medium) and/or the rate of motion of such objects within the microfluidic device 100.

The tilting module 166 can control the tilting motions of tilting device 190. Alternatively, or in addition, the tilting module 166 can control the tilting rate and timing to optimize transfer of micro-objects to the one or more sequestration pens via gravitational forces. The tilting module 166 is communicatively coupled with the imaging module 164 to receive data describing the motion of micro-objects and/or droplets of medium in the microfluidic circuit 120. Using this data, the tilting module 166 may adjust the tilt of the microfluidic circuit 120 in order to adjust the rate at which micro-objects and/or droplets of medium move in the microfluidic circuit 120. The tilting module 166 may also use this data to iteratively adjust the position of a micro-object and/or droplet of medium in the microfluidic circuit 120.

In the example shown in Figure 1A, the microfluidic circuit 120 is illustrated as comprising a microfluidic channel 122 and sequestration pens 124, 126, 128, 130. Each pen comprises an opening to channel 122, but otherwise is enclosed such that the pens can substantially isolate micro-objects inside the pen from fluidic medium 180 and/or micro-objects in the flow path 106 of channel 122 or in other pens. The walls of the sequestration pen extend from the inner surface 109 of the base to the inside surface of the cover 110 to provide enclosure. The opening of the pen to the microfluidic channel 122 is oriented at an angle to the flow 106 of fluidic medium 180 such that flow 106 is not directed into the pens. The flow may be tangential or orthogonal to the plane of the opening of the pen. In some instances, pens 124, 126, 128, 130 are configured to physically corral one or more micro-objects within the microfluidic circuit 120. Sequestration pens in accordance with the present disclosure can comprise various shapes, surfaces and features that are optimized for use with DEP, OET, OEW, fluid flow, and/or gravitational forces, as will be discussed and shown in detail below.

The microfluidic circuit 120 may comprise any number of microfluidic sequestration pens. Although five sequestration pens are shown, microfluidic circuit 120 may have fewer or more sequestration pens. As shown, microfluidic sequestration pens 124, 126, 128, and 130 of microfluidic circuit 120 each comprise differing features and shapes which may provide one or more benefits useful for maintaining, isolating, assaying or culturing biological micro-objects. In some embodiments, the microfluidic circuit 120 comprises a plurality of identical microfluidic sequestration pens.

In the embodiment illustrated in Figure 1A, a single channel 122 and flow path 106 is shown. However, other embodiments may contain multiple channels 122, each configured to comprise a flow path 106. The microfluidic circuit 120 further comprises an inlet valve or port 107 in fluid communication with the flow path 106 and fluidic medium 180, whereby fluidic medium 180 can access channel 122 via the inlet port 107. In some instances, the flow path 106 comprises a single path. In some instances, the single path is arranged in a zigzag pattern whereby the flow path 106 travels across the microfluidic device 100 two or more times in alternating directions.

In some instances, microfluidic circuit 120 comprises a plurality of parallel channels 122 and flow paths 106, wherein the fluidic medium 180 within each flow path 106 flows in the same direction. In some instances, the fluidic medium within each flow path 106 flows in at least one of a forward or reverse direction. In some instances, a plurality of sequestration pens is configured (e.g., relative to a channel 122) such that the sequestration pens can be loaded with target micro-objects in parallel.

In some embodiments, microfluidic circuit 120 further comprises one or more micro-object traps 132. The traps 132 are generally formed in a wall forming the boundary of a channel 122, and may be positioned opposite an opening of one or more of the microfluidic sequestration pens 124, 126, 128, 130. In some embodiments, the traps 132 are configured to receive or capture a single micro-object from the flow path 106. In some embodiments, the traps 132 are configured to receive or capture a plurality of micro-objects from the flow path 106. In some instances, the traps 132 comprise a volume approximately equal to the volume of a single target micro-object.

The traps 132 may further comprise an opening which is configured to assist the flow of targeted micro-objects into the traps 132. In some instances, the traps 132 comprise an opening having a height and width that is approximately equal to the dimensions of a single target micro-object, whereby larger micro-objects are prevented from entering into the micro-object trap. The traps 132 may further comprise other features configured to assist in retention of targeted micro-objects within the trap 132. In some instances, the trap 132 is aligned with and situated on the opposite side of a channel 122 relative to the opening of a microfluidic sequestration pen, such that upon tilting the microfluidic device 100 about an axis parallel to the microfluidic channel 122, the trapped micro-object exits the trap 132 at a trajectory that causes the micro-object to fall into the opening of the sequestration pen. In some instances, the trap 132 comprises a side passage 134 that is smaller than the target micro-object in order to facilitate flow through the trap 132 and thereby increase the likelihood of capturing a micro-object in the trap 132.

In some embodiments, dielectrophoretic (DEP) forces are applied across the fluidic medium 180 (e.g., in the flow path and/or in the sequestration pens) via one or more electrodes (not shown) to manipulate, transport, separate and sort micro-objects located therein. For example, in some embodiments, DEP forces are applied to one or more portions of microfluidic circuit 120 in order to transfer a single micro-object from the flow path 106 into a desired microfluidic sequestration pen. In some embodiments, DEP forces are used to prevent a micro-object within a sequestration pen (e.g., sequestration pen 124, 126, 128, or 130) from being displaced therefrom. Further, in some embodiments, DEP forces are used to selectively remove a micro-object from a sequestration pen that was previously collected in accordance with the embodiments of the current disclosure. In some embodiments, the DEP forces comprise optoelectronic tweezer (OET) forces.

In other embodiments, optoelectrowetting (OEW) forces are applied to one or more positions in the support structure 104 (and/or the cover 110) of the microfluidic device 100 (e.g., positions helping to define the flow path and/or the sequestration pens) via one or more electrodes (not shown) to manipulate, transport, separate and sort droplets located in the microfluidic circuit 120. For example, in some embodiments, OEW forces are applied to one or more positions in the support structure 104 (and/or the cover 110) in order to transfer a single droplet from the flow path 106 into a desired microfluidic sequestration pen. In some embodiments, OEW forces are used to prevent a droplet within a sequestration pen (e.g., sequestration pen 124, 126, 128, or 130) from being displaced therefrom. Further, in some embodiments, OEW forces are used to selectively remove a droplet from a sequestration pen that was previously collected in accordance with the embodiments of the current disclosure.

In some embodiments, DEP and/or OEW forces are combined with other forces, such as flow and/or gravitational force, so as to manipulate, transport, separate and sort micro-objects and/or droplets within the microfluidic circuit 120. For example, the enclosure 102 can be tilted (e.g., by tilting device 190) to position the flow path 106 and micro-objects located therein above the microfluidic sequestration pens, and the force of gravity can transport the micro-objects and/or droplets into the pens. In some embodiments, the DEP and/or OEW forces can be applied prior to the other forces. In other embodiments, the DEP and/or OEW forces can be applied after the other forces. In still other instances, the DEP and/or OEW forces can be applied at the same time as the other forces or in an alternating manner with the other forces.

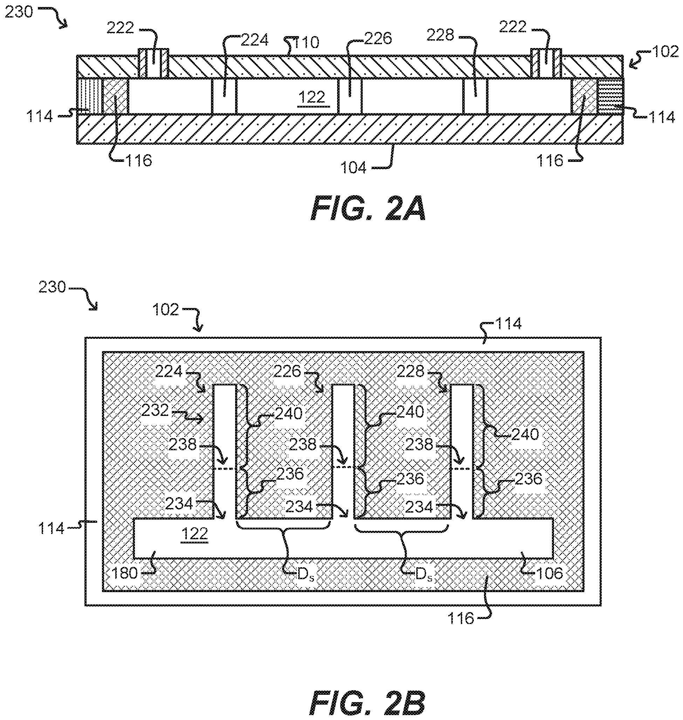

Figures 1B, 1C, and 2A-2H illustrates various embodiments of microfluidic devices that can be used in the practice of the embodiments of the present disclosure. Figure 1B depicts an embodiment in which the microfluidic device 200 is configured as an optically-actuated electrokinetic device. A variety of optically-actuated electrokinetic devices are known in the art, including devices having an optoelectronic tweezer (OET) configuration and devices having an opto-electrowetting (OEW) configuration. Examples of suitable OET configurations are illustrated in the following U.S. patent documents:
U.S. Patent No. RE 44,711 (Wu et al.) (originally issued as U.S. Patent No. 7,612,355); and U.S. Patent No. 7,956,339 (Ohta et al.). Examples of OEW configurations are illustrated in U.S. Patent No. 6,958,132 (Chiou et al.) and U.S. Patent Application Publication No. 2012/0024708 (Chiou et al.).

Yet another example of an optically-actuated electrokinetic device includes a combined OET/OEW configuration, examples of which are shown in U.S. Patent Publication Nos. 20150306598 (Khandros et al.) and 20150306599 (Khandros et al.) and their corresponding PCT Publications WO2015/164846 and WO2015/164847.

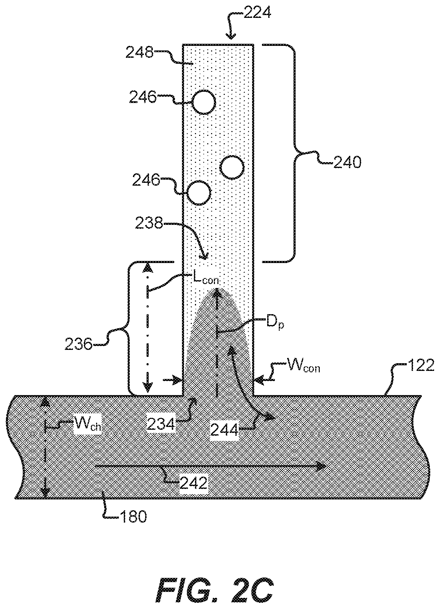

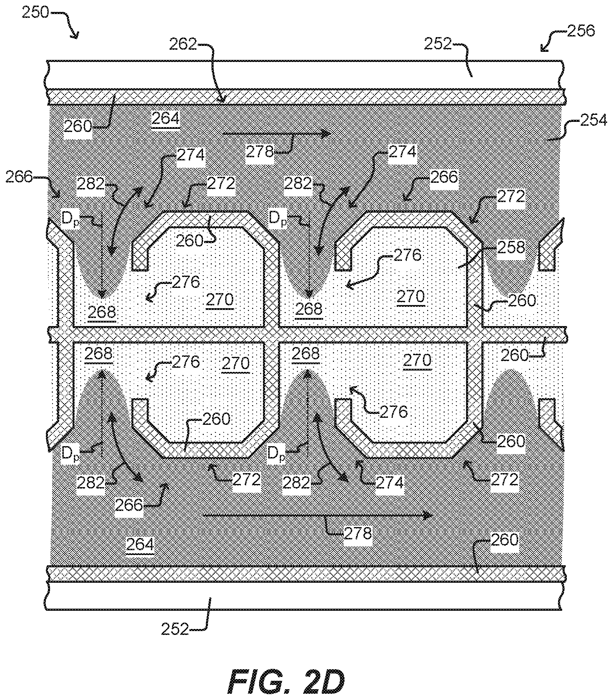

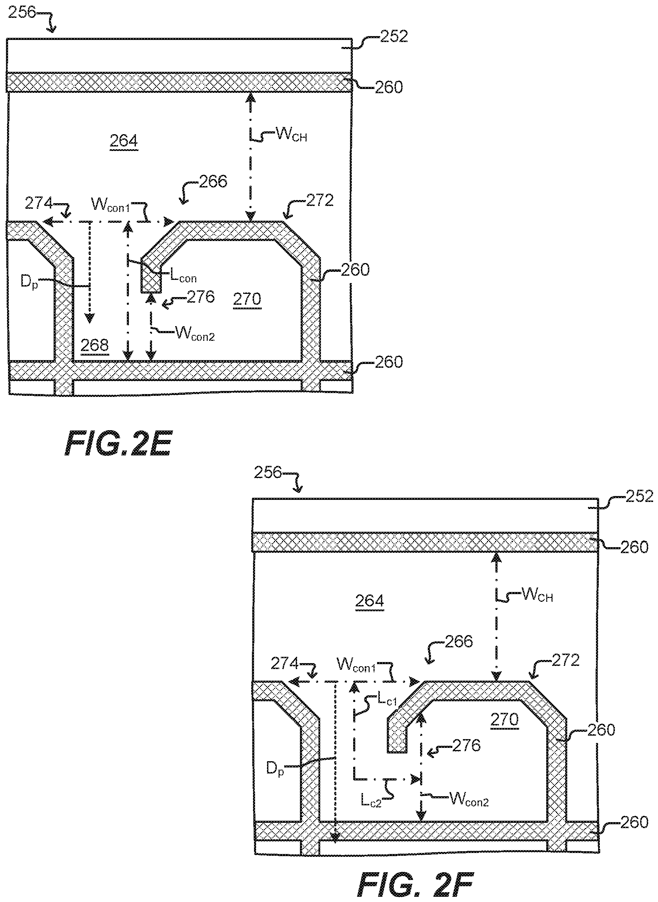

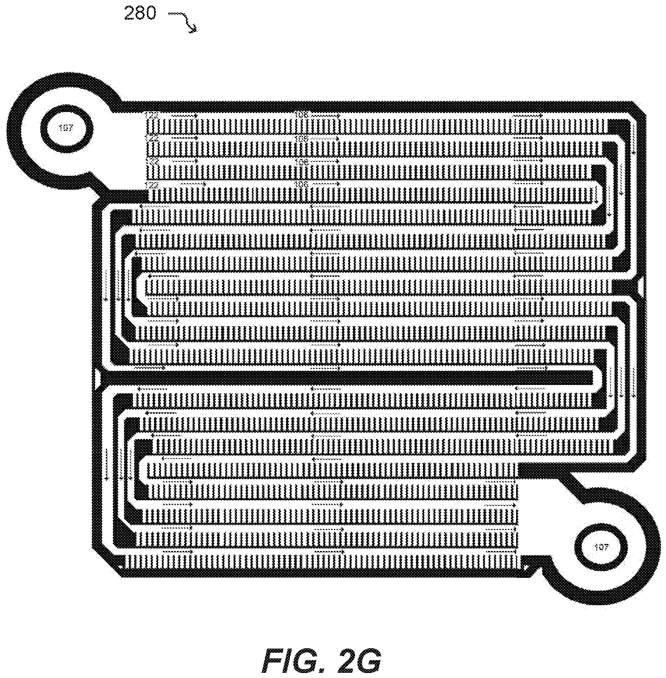

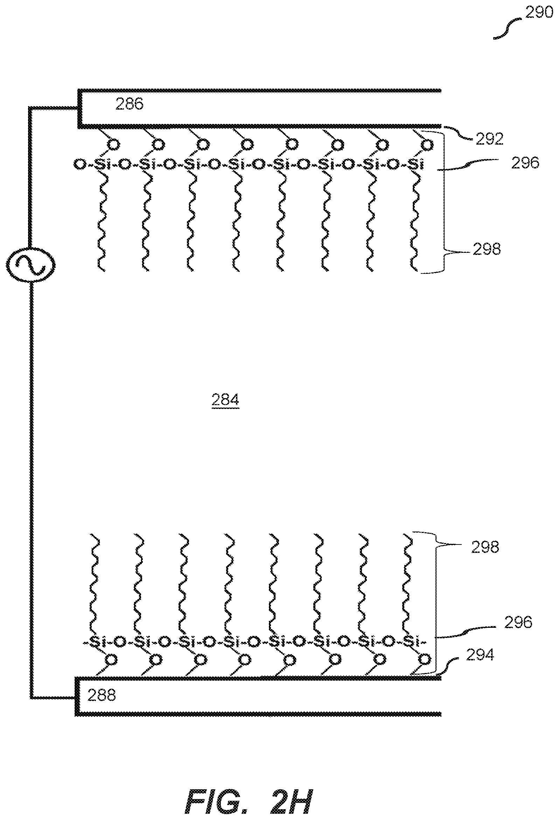

Examples of microfluidic devices having pens in which biological micro-objects can be placed, cultured, and/or monitored have been described, for example, in US 2014/0116881 (application no. 14/060,117, filed October 22, 2013), US 2015/0151298 (application no. 14/520,568, filed October 22, 2014), and US 2015/0165436 (application no. 14/521,447, filed October 22, 2014). US application nos. 14/520,568 and 14/521,447 also describe exemplary methods of analyzing secretions of cells cultured in a microfluidic device. Each of the foregoing applications further describes microfluidic devices configured to produce dielectrophoretic (DEP) forces, such as optoelectronic tweezers (OET) or configured to provide opto-electro wetting (OEW). For example, the optoelectronic tweezers device illustrated in Figure 2 of US 2014/0116881 is an example of a device that can be utilized in embodiments of the present disclosure to select and move an individual biological micro-object or a group of biological micro-objects.

**Microfluidic device motive configurations.** As described above, the control and monitoring equipment of the system can comprise a motive module for selecting and moving objects, such as micro-objects or droplets, in the microfluidic circuit of a microfluidic device. The microfluidic device can have a variety of motive configurations, depending upon the type of object being moved and other considerations. For example, a dielectrophoresis (DEP) configuration can be utilized to select and move micro-objects in the microfluidic circuit. Thus, the support structure 104 and/or cover 110 of the microfluidic device 100 can comprise a DEP configuration for selectively inducing DEP forces on micro-objects in a fluidic medium 180 in the microfluidic circuit 120 and thereby select, capture, and/or move individual micro-objects or groups of micro-objects. Alternatively, the support structure 104 and/or cover 110 of the microfluidic device 100 can comprise an electrowetting (EW) configuration for selectively inducing EW forces on droplets in a fluidic medium 180 in the microfluidic circuit 120 and thereby select, capture, and/or move individual droplets or groups of droplets.

One example of a microfluidic device 200 comprising a DEP configuration is illustrated in Figures 1B and 1C. While for purposes of simplicity Figures 1B and 1C show a side cross-sectional view and a top cross-sectional view, respectively, of a portion of an enclosure 102 of the microfluidic device 200 having a region/chamber 202, it should be understood that the region/chamber 202 may be part of a fluidic circuit element having a more detailed structure, such as a growth chamber, a sequestration pen, a flow region, or a flow channel. Furthermore, the microfluidic device 200 may include other fluidic circuit elements. For example, the microfluidic device 200 can include a plurality of growth chambers or sequestration pens and/or one or more flow regions or flow channels, such as those described herein with respect to microfluidic device 100. A DEP configuration may be incorporated into any such fluidic circuit elements of the microfluidic device 200, or select portions thereof. It should be further appreciated that any of the above or below described microfluidic device components and system components may be incorporated in and/or used in combination with the microfluidic device 200. For example, system 150 including control and monitoring equipment 152, described above, may be used with microfluidic device 200, including one or more of the media module 160, motive module 162, imaging module 164, tilting module 166, and other modules 168.

As seen in Figure 1B, the microfluidic device 200 includes a support structure 104 having a bottom electrode 204 and an electrode activation substrate 206 overlying the bottom electrode 204, and a cover 110 having a top electrode 210, with the top electrode 210 spaced apart from the bottom electrode 204. The top electrode 210 and the electrode activation substrate 206 define opposing surfaces of the region/chamber 202. A medium 180 contained in the region/chamber 202 thus provides a resistive connection between the top electrode 210 and the electrode activation substrate 206. A power source 212 configured to be connected to the bottom electrode 204 and the top electrode 210 and create a biasing voltage between the electrodes, as required for the generation of DEP forces in the region/chamber 202, is also shown. The power source 212 can be, for example, an alternating current (AC) power source.

In certain embodiments, the microfluidic device 200 illustrated in Figures 1B and 1C can have an optically-actuated DEP configuration. Accordingly, changing patterns of light 218 from the light source 216, which may be controlled by the motive module 162, can selectively activate and deactivate changing patterns of DEP electrodes at regions 214 of the inner surface 208 of the electrode activation substrate 206. (Hereinafter the regions 214 of a microfluidic device having a DEP configuration are referred to as "DEP electrode regions.") As illustrated in Figure 1C, a light pattern 218 directed onto the inner surface 208 of the electrode activation substrate 206 can illuminate select DEP electrode regions 214a (shown in white) in a pattern, such as a square. The non-illuminated DEP electrode regions 214 (cross-hatched) are hereinafter referred to as "dark" DEP electrode regions 214. The relative electrical impedance through the DEP electrode activation substrate 206 (i.e., from the bottom electrode 204 up to the inner surface 208 of the electrode activation substrate 206 which interfaces with the medium 180 in the flow region 106) is greater than the relative electrical impedance through the medium 180 in the region/chamber 202 (i.e., from the inner surface 208 of the electrode activation substrate 206 to the top electrode 210 of the cover 110) at each dark DEP electrode region 214. An illuminated DEP electrode region 214a, however, exhibits a reduced relative impedance through the electrode activation substrate 206 that is less than the relative impedance through the medium 180 in the region/chamber 202 at each illuminated DEP electrode region 214a.

With the power source 212 activated, the foregoing DEP configuration creates an electric field gradient in the fluidic medium 180 between illuminated DEP electrode regions 214a and adjacent dark DEP electrode regions 214, which in turn creates local DEP forces that attract or repel nearby micro-objects (not shown) in the fluidic medium 180. DEP electrodes that attract or repel micro-objects in the fluidic medium 180 can thus be selectively activated and deactivated at many different such DEP electrode regions 214 at the inner surface 208 of the region/chamber 202 by changing light patterns 218 projected from a light source 216 into the microfluidic device 200. Whether the DEP forces attract or repel nearby micro-objects can depend on such parameters as the frequency of the power source 212 and the dielectric properties of the medium 180 and/or micro-objects (not shown).

The square pattern 220 of illuminated DEP electrode regions 214a illustrated in Figure 1C is an example only. Any pattern of the DEP electrode regions 214 can be illuminated (and thereby activated) by the pattern of light 218 projected into the microfluidic device 200, and the pattern of illuminated/activated DEP electrode regions 214 can be repeatedly changed by changing or moving the light pattern 218.

In some embodiments, the electrode activation substrate 206 can comprise or consist of a photoconductive material. In such embodiments, the inner surface 208 of the electrode activation substrate 206 can be featureless. For example, the electrode activation substrate 206 can comprise or consist of a layer of hydrogenated amorphous silicon (a-Si:H). The a-Si:H can comprise, for example, about 8% to 40% hydrogen (calculated as 100 ^{∗} the number of hydrogen atoms / the total number of hydrogen and silicon atoms). The layer of a-Si:H can have a thickness of about 500 nm to about 2.0 µm. In such embodiments, the DEP electrode regions 214 can be created anywhere and in any pattern on the inner surface 208 of the electrode activation substrate 206, in accordance with the light pattern 218. The number and pattern of the DEP electrode regions 214 thus need not be fixed, but can correspond to the light pattern 218. Examples of microfluidic devices having a DEP configuration comprising a photoconductive layer such as discussed above have been described, for example, in U.S. Patent No. RE 44,711 (Wu et al.) (originally issued as U.S. Patent No. 7,612,355).

In other embodiments, the electrode activation substrate 206 can comprise a substrate comprising a plurality of doped layers, electrically insulating layers (or regions), and electrically conductive layers that form semiconductor integrated circuits, such as is known in semiconductor fields. For example, the electrode activation substrate 206 can comprise a plurality of phototransistors, including, for example, lateral bipolar phototransistors, each phototransistor corresponding to a DEP electrode region 214. Alternatively, the electrode activation substrate 206 can comprise electrodes (e.g., conductive metal electrodes) controlled by phototransistor switches, with each such electrode corresponding to a DEP electrode region 214. The electrode activation substrate 206 can include a pattern of such phototransistors or phototransistor-controlled electrodes. The pattern, for example, can be an array of substantially square phototransistors or phototransistor-controlled electrodes arranged in rows and columns, such as shown in Fig. 2B. Alternatively, the pattern can be an array of substantially hexagonal phototransistors or phototransistor-controlled electrodes that form a hexagonal lattice. Regardless of the pattern, electric circuit elements can form electrical connections between the DEP electrode regions 214 at the inner surface 208 of the electrode activation substrate 206 and the bottom electrode 210, and those electrical connections (i.e., phototransistors or electrodes) can be selectively activated and deactivated by the light pattern 218. When not activated, each electrical connection can have high impedance such that the relative impedance through the electrode activation substrate 206 (i.e., from the bottom electrode 204 to the inner surface 208 of the electrode activation substrate 206 which interfaces with the medium 180 in the region/chamber 202) is greater than the relative impedance through the medium 180 (i.e., from the inner surface 208 of the electrode activation substrate 206 to the top electrode 210 of the cover 110) at the corresponding DEP electrode region 214. When activated by light in the light pattern 218, however, the relative impedance through the electrode activation substrate 206 is less than the relative impedance through the medium 180 at each illuminated DEP electrode region 214, thereby activating the DEP electrode at the corresponding DEP electrode region 214 as discussed above. DEP electrodes that attract or repel micro-objects (not shown) in the medium 180 can thus be selectively activated and deactivated at many different DEP electrode regions 214 at the inner surface 208 of the electrode activation substrate 206 in the region/chamber 202 in a manner determined by the light pattern 218.

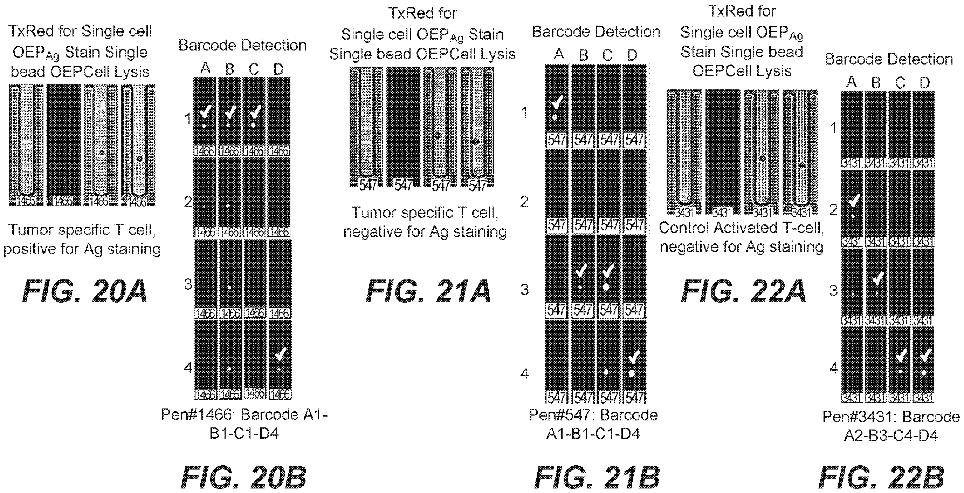

Examples of microfluidic devices having electrode activation substrates that comprise phototransistors have been described, for example, in U.S. Patent No. 7,956,339 (Ohta et al.) (see, e.g., device 300 illustrated in Figures 21 and 22, and descriptions thereof).

Examples of microfluidic devices having electrode activation substrates that comprise electrodes controlled by phototransistor switches have been described, for example, in U.S. Patent Publication No. 2014/0124370 (Short et al.) (see, e.g., devices 200, 400, 500, 600, and 900 illustrated throughout the drawings, and descriptions thereof).

In some embodiments of a DEP configured microfluidic device, the top electrode 210 is part of a first wall (or cover 110) of the enclosure 102, and the electrode activation substrate 206 and bottom electrode 204 are part of a second wall (or support structure 104) of the enclosure 102. The region/chamber 202 can be between the first wall and the second wall. In other embodiments, the electrode 210 is part of the second wall (or support structure 104) and one or both of the electrode activation substrate 206 and/or the electrode 210 are part of the first wall (or cover 110). Moreover, the light source 216 can alternatively be used to illuminate the enclosure 102 from below.

With the microfluidic device 200 of Figures 1B-1C having a DEP configuration, the motive module 162 can select a micro-object (not shown) in the medium 180 in the region/chamber 202 by projecting a light pattern 218 into the microfluidic device 200 to activate a first set of one or more DEP electrodes at DEP electrode regions 214a of the inner surface 208 of the electrode activation substrate 206 in a pattern (e.g., square pattern 220) that surrounds and captures the micro-object. The motive module 162 can then move the in situ-generated captured micro-object by moving the light pattern 218 relative to the microfluidic device 200 to activate a second set of one or more DEP electrodes at DEP electrode regions 214. Alternatively, the microfluidic device 200 can be moved relative to the light pattern 218.

In other embodiments, the microfluidic device 200 can have a DEP configuration that does not rely upon light activation of DEP electrodes at the inner surface 208 of the electrode activation substrate 206. For example, the electrode activation substrate 206 can comprise selectively addressable and energizable electrodes positioned opposite to a surface including at least one electrode (e.g., cover 110). Switches (e.g., transistor switches in a semiconductor substrate) may be selectively opened and closed to activate or inactivate DEP electrodes at DEP electrode regions 214, thereby creating a net DEP force on a micro-object (not shown) in region/chamber 202 in the vicinity of the activated DEP electrodes. Depending on such characteristics as the frequency of the power source 212 and the dielectric properties of the medium (not shown) and/or micro-objects in the region/chamber 202, the DEP force can attract or repel a nearby micro-object. By selectively activating and deactivating a set of DEP electrodes (e.g., at a set of DEP electrodes regions 214 that forms a square pattern 220), one or more micro-objects in region/chamber 202 can be trapped and moved within the region/chamber 202. The motive module 162 in Figure 1A can control such switches and thus activate and deactivate individual ones of the DEP electrodes to select, trap, and move particular micro-objects (not shown) around the region/chamber 202. Microfluidic devices having a DEP configuration that includes selectively addressable and energizable electrodes are known in the art and have been described, for example, in U.S. Patent Nos. 6,294,063 (Becker et al.) and 6,942,776 (Medoro).

As yet another example, the microfluidic device 200 can have an electrowetting (EW) configuration, which can be in place of the DEP configuration or can be located in a portion of the microfluidic device 200 that is separate from the portion which has the DEP configuration. The EW configuration can be an opto-electrowetting configuration or an electrowetting on dielectric (EWOD) configuration, both of which are known in the art. In some EW configurations, the support structure 104 has an electrode activation substrate 206 sandwiched between a dielectric layer (not shown) and the bottom electrode 204. The dielectric layer can comprise a hydrophobic material and/or can be coated with a hydrophobic material, as described below. For microfluidic devices 200 that have an EW configuration, the inner surface 208 of the support structure 104 is the inner surface of the dielectric layer or its hydrophobic coating.

The dielectric layer (not shown) can comprise one or more oxide layers, and can have a thickness of about 50 nm to about 250 nm (e.g., about 125 nm to about 175 nm). In certain embodiments, the dielectric layer may comprise a layer of oxide, such as a metal oxide (e.g., aluminum oxide or hafnium oxide). In certain embodiments, the dielectric layer can comprise a dielectric material other than a metal oxide, such as silicon oxide or a nitride. Regardless of the exact composition and thickness, the dielectric layer can have an impedance of about 10 kOhms to about 50 kOhms.

In some embodiments, the surface of the dielectric layer that faces inward toward region/chamber 202 is coated with a hydrophobic material. The hydrophobic material can comprise, for example, fluorinated carbon molecules. Examples of fluorinated carbon molecules include perfluoro-polymers such as polytetrafluoroethylene (e.g., TEFLON^{®}) or poly(2,3-difluoromethylenyl-perfluorotetrahydrofuran) (e.g., CYTOP^{™}). Molecules that make up the hydrophobic material can be covalently bonded to the surface of the dielectric layer. For example, molecules of the hydrophobic material can be covalently bound to the surface of the dielectric layer by means of a linker such as a siloxane group, a phosphonic acid group, or a thiol group. Thus, in some embodiments, the hydrophobic material can comprise alkyl-terminated siloxane, alkyl-termination phosphonic acid, or alkyl-terminated thiol. The alkyl group can be long-chain hydrocarbons (e.g., having a chain of at least 10 carbons, or at least 16, 18, 20, 22, or more carbons). Alternatively, fluorinated (or perfluorinated) carbon chains can be used in place of the alkyl groups. Thus, for example, the hydrophobic material can comprise fluoroalkyl-terminated siloxane, fluoroalkyl-terminated phosphonic acid, or fluoroalkyl-terminated thiol. In some embodiments, the hydrophobic coating has a thickness of about 10 nm to about 50 nm. In other embodiments, the hydrophobic coating has a thickness of less than 10 nm (e.g., less than 5 nm, or about 1.5 to 3.0 nm).

In some embodiments, the cover 110 of a microfluidic device 200 having an electrowetting configuration is coated with a hydrophobic material (not shown) as well. The hydrophobic material can be the same hydrophobic material used to coat the dielectric layer of the support structure 104, and the hydrophobic coating can have a thickness that is substantially the same as the thickness of the hydrophobic coating on the dielectric layer of the support structure 104. Moreover, the cover 110 can comprise an electrode activation substrate 206 sandwiched between a dielectric layer and the top electrode 210, in the manner of the support structure 104. The electrode activation substrate 206 and the dielectric layer of the cover 110 can have the same composition and/or dimensions as the electrode activation substrate 206 and the dielectric layer of the support structure 104. Thus, the microfluidic device 200 can have two electrowetting surfaces.

In some embodiments, the electrode activation substrate 206 can comprise a photoconductive material, such as described above. Accordingly, in certain embodiments, the electrode activation substrate 206 can comprise or consist of a layer of hydrogenated amorphous silicon (a-Si:H). The a-Si:H can comprise, for example, about 8% to 40% hydrogen (calculated as 100 ^{∗} the number of hydrogen atoms / the total number of hydrogen and silicon atoms). The layer of a-Si:H can have a thickness of about 500 nm to about 2.0 µm. Alternatively, the electrode activation substrate 206 can comprise electrodes (e.g., conductive metal electrodes) controlled by phototransistor switches, as described above. Microfluidic devices having an opto-electrowetting configuration are known in the art and/or can be constructed with electrode activation substrates known in the art. For example, U.S. Patent No. 6,958,132 (Chiou et al.).
discloses opto-electrowetting configurations having a photoconductive material such as a-Si:H, while U.S. Patent Publication No. 2014/0124370 (Short et al.), referenced above, discloses electrode activation substrates having electrodes controlled by phototransistor switches.

The microfluidic device 200 thus can have an opto-electrowetting configuration, and light patterns 218 can be used to activate photoconductive EW regions or photoresponsive EW electrodes in the electrode activation substrate 206. Such activated EW regions or EW electrodes of the electrode activation substrate 206 can generate an electrowetting force at the inner surface 208 of the support structure 104 (i.e., the inner surface of the overlaying dielectric layer or its hydrophobic coating). By changing the light patterns 218 (or moving microfluidic device 200 relative to the light source 216) incident on the electrode activation substrate 206, droplets (e.g., containing an aqueous medium, solution, or solvent) contacting the inner surface 208 of the support structure 104 can be moved through an immiscible fluid (e.g., an oil medium) present in the region/chamber 202.

In other embodiments, microfluidic devices 200 can have an EWOD configuration, and the electrode activation substrate 206 can comprise selectively addressable and energizable electrodes that do not rely upon light for activation. The electrode activation substrate 206 thus can include a pattern of such electrowetting (EW) electrodes. The pattern, for example, can be an array of substantially square EW electrodes arranged in rows and columns, such as shown in Fig. 2B. Alternatively, the pattern can be an array of substantially hexagonal EW electrodes that form a hexagonal lattice. Regardless of the pattern, the EW electrodes can be selectively activated (or deactivated) by electrical switches (e.g., transistor switches in a semiconductor substrate). By selectively activating and deactivating EW electrodes in the electrode activation substrate 206, droplets (not shown) contacting the inner surface 208 of the overlaying dielectric layer or its hydrophobic coating can be moved within the region/chamber 202. The motive module 162 in Figure 1A can control such switches and thus activate and deactivate individual EW electrodes to select and move particular droplets around region/chamber 202. Microfluidic devices having a EWOD configuration with selectively addressable and energizable electrodes are known in the art and have been described, for example, in U.S. Patent No. 8,685,344 (Sundarsan et al.).

Regardless of the configuration of the microfluidic device 200, a power source 212 can be used to provide a potential (e.g., an AC voltage potential) that powers the electrical circuits of the microfluidic device 200. The power source 212 can be the same as, or a component of, the power source 192 referenced in Fig. 1. Power source 212 can be configured to provide an AC voltage and/or current to the top electrode 210 and the bottom electrode 204. For an AC voltage, the power source 212 can provide a frequency range and an average or peak power (e.g., voltage or current) range sufficient to generate net DEP forces (or electrowetting forces) strong enough to trap and move individual micro-objects (not shown) in the region/chamber 202, as discussed above, and/or to change the wetting properties of the inner surface 208 of the support structure 104 (i.e., the dielectric layer and/or the hydrophobic coating on the dielectric layer) in the region/chamber 202, as also discussed above. Such frequency ranges and average or peak power ranges are known in the art. See, e.g., US Patent No. 6,958,132 (Chiou et al.), US Patent No. RE44,711 (Wu et al.) (originally issued as US Patent No. 7,612,355), and US Patent Application Publication Nos. US2014/0124370 (Short et al.), US2015/0306598 (Khandros et al.), and US2015/0306599 (Khandros et al.).

**Sequestration pens.** Non-limiting examples of generic sequestration pens 224, 226, and 228 are shown within the microfluidic device 230 depicted in Figures 2A-2C. Each sequestration pen 224, 226, and 228 can comprise an isolation structure 232 defining an isolation region 240 and a connection region 236 fluidically connecting the isolation region 240 to a channel 122. The connection region 236 can comprise a proximal opening 234 to the microfluidic channel 122 and a distal opening 238 to the isolation region 240. The connection region 236 can be configured so that the maximum penetration depth of a flow of a fluidic medium (not shown) flowing from the microfluidic channel 122 into the sequestration pen 224, 226, 228 does not extend into the isolation region 240. Thus, due to the connection region 236, a micro-object (not shown) or other material (not shown) disposed in an isolation region 240 of a sequestration pen 224, 226, 228 can thus be isolated from, and not substantially affected by, a flow of medium 180 in the microfluidic channel 122.

The sequestration pens 224, 226, and 228 of Figures 2A-2C each have a single opening which opens directly to the microfluidic channel 122. The opening of the sequestration pen opens laterally from the microfluidic channel 122. The electrode activation substrate 206 underlays both the microfluidic channel 122 and the sequestration pens 224, 226, and 228. The upper surface of the electrode activation substrate 206 within the enclosure of a sequestration pen, forming the floor of the sequestration pen, is disposed at the same level or substantially the same level of the upper surface the of electrode activation substrate 206 within the microfluidic channel 122 (or flow region if a channel is not present), forming the floor of the flow channel (or flow region, respectively) of the microfluidic device. The electrode activation substrate 206 may be featureless or may have an irregular or patterned surface that varies from its highest elevation to its lowest depression by less than about 3 microns, 2.5 microns, 2 microns, 1.5 microns, 1 micron, 0.9 microns, 0.5 microns, 0.4 microns, 0.2 microns, 0.1 microns or less. The variation of elevation in the upper surface of the substrate across both the microfluidic channel 122 (or flow region) and sequestration pens may be less than about 3%, 2%, 1%. 0.9%, 0.8%, 0.5%, 0.3% or 0.1% of the height of the walls of the sequestration pen or walls of the microfluidic device. While described in detail for the microfluidic device 200, this also applies to any of the microfluidic devices 100, 230, 250, 280, 290, 300, 700, 800, 1000 described herein.

The microfluidic channel 122 can thus be an example of a swept region, and the isolation regions 240 of the sequestration pens 224, 226, 228 can be examples of unswept regions. As noted, the microfluidic channel 122 and sequestration pens 224, 226, 228 can be configured to contain one or more fluidic media 180. In the example shown in Figures 2A-2B, the ports 222 are connected to the microfluidic channel 122 and allow a fluidic medium 180 to be introduced into or removed from the microfluidic device 230. Prior to introduction of the fluidic medium 180, the microfluidic device may be primed with a gas such as carbon dioxide gas. Once the microfluidic device 230 contains the fluidic medium 180, the flow 242 of fluidic medium 180 in the microfluidic channel 122 can be selectively generated and stopped. For example, as shown, the ports 222 can be disposed at different locations (e.g., opposite ends) of the microfluidic channel 122, and a flow 242 of medium can be created from one port 222 functioning as an inlet to another port 222 functioning as an outlet.

Figure 2C illustrates a detailed view of an example of a sequestration pen 224 according to the present disclosure. Examples of micro-objects 246 are also shown.

As is known, a flow 242 of fluidic medium 180 in a microfluidic channel 122 past a proximal opening 234 of sequestration pen 224 can cause a secondary flow 244 of the medium 180 into and/or out of the sequestration pen 224. To isolate micro-objects 246 in the isolation region 240 of a sequestration pen 224 from the secondary flow 244, the length L_{con} of the connection region 236 of the sequestration pen 224 (i.e., from the proximal opening 234 to the distal opening 238) should be greater than the penetration depth Dₚ of the secondary flow 244 into the connection region 236. The penetration depth Dₚ of the secondary flow 244 depends upon the velocity of the fluidic medium 180 flowing in the microfluidic channel 122 and various parameters relating to the configuration of the microfluidic channel 122 and the proximal opening 234 of the connection region 236 to the microfluidic channel 122. For a given microfluidic device, the configurations of the microfluidic channel 122 and the opening 234 will be fixed, whereas the rate of flow 242 of fluidic medium 180 in the microfluidic channel 122 will be variable. Accordingly, for each sequestration pen 224, a maximal velocity Vₘₐₓ for the flow 242 of fluidic medium 180 in channel 122 can be identified that ensures that the penetration depth Dₚ of the secondary flow 244 does not exceed the length L_{con} of the connection region 236. As long as the rate of the flow 242 of fluidic medium 180 in the microfluidic channel 122 does not exceed the maximum velocity Vₘₐₓ, the resulting secondary flow 244 can be limited to the microfluidic channel 122 and the connection region 236 and kept out of the isolation region 240. The flow 242 of medium 180 in the microfluidic channel 122 will thus not draw micro-objects 246 out of the isolation region 240. Rather, micro-objects 246 located in the isolation region 240 will stay in the isolation region 240 regardless of the flow 242 of fluidic medium 180 in the microfluidic channel 122.

Moreover, as long as the rate of flow 242 of medium 180 in the microfluidic channel 122 does not exceed Vₘₐₓ, the flow 242 of fluidic medium 180 in the microfluidic channel 122 will not move miscellaneous particles (e.g., microparticles and/or nanoparticles) from the microfluidic channel 122 into the isolation region 240 of a sequestration pen 224. Having the length L_{con} of the connection region 236 be greater than the maximum penetration depth Dₚ of the secondary flow 244 can thus prevent contamination of one sequestration pen 224 with miscellaneous particles from the microfluidic channel 122 or another sequestration pen (e.g., sequestration pens 226, 228 in Fig. 2D).

Because the microfluidic channel 122 and the connection regions 236 of the sequestration pens 224, 226, 228 can be affected by the flow 242 of medium 180 in the microfluidic channel 122, the microfluidic channel 122 and connection regions 236 can be deemed swept (or flow) regions of the microfluidic device 230. The isolation regions 240 of the sequestration pens 224, 226, 228, on the other hand, can be deemed unswept (or non-flow) regions. For example, components (not shown) in a first fluidic medium 180 in the microfluidic channel 122 can mix with a second fluidic medium 248 in the isolation region 240 substantially only by diffusion of components of the first medium 180 from the microfluidic channel 122 through the connection region 236 and into the second fluidic medium 248 in the isolation region 240. Similarly, components (not shown) of the second medium 248 in the isolation region 240 can mix with the first medium 180 in the microfluidic channel 122 substantially only by diffusion of components of the second medium 248 from the isolation region 240 through the connection region 236 and into the first medium 180 in the microfluidic channel 122. In some embodiments, the extent of fluidic medium exchange between the isolation region of a sequestration pen and the flow region by diffusion is greater than about 90%, 91%, 92%, 93%, 94% 95%, 96%, 97%, 98%, or greater than about 99% of fluidic exchange. The first medium 180 can be the same medium or a different medium than the second medium 248. Moreover, the first medium 180 and the second medium 248 can start out being the same, then become different (e.g., through conditioning of the second medium 248 by one or more cells in the isolation region 240, or by changing the medium 180 flowing through the microfluidic channel 122).

The maximum penetration depth Dₚ of the secondary flow 244 caused by the flow 242 of fluidic medium 180 in the microfluidic channel 122 can depend on a number of parameters, as mentioned above. Examples of such parameters include: the shape of the microfluidic channel 122 (e.g., the microfluidic channel can direct medium into the connection region 236, divert medium away from the connection region 236, or direct medium in a direction substantially perpendicular to the proximal opening 234 of the connection region 236 to the microfluidic channel 122); a width W_{ch} (or cross-sectional area) of the microfluidic channel 122 at the proximal opening 234; and a width W_{con} (or cross-sectional area) of the connection region 236 at the proximal opening 234; the velocity V of the flow 242 of fluidic medium 180 in the microfluidic channel 122; the viscosity of the first medium 180 and/or the second medium 248, or the like.

In some embodiments, the dimensions of the microfluidic channel 122 and sequestration pens 224, 226, 228 can be oriented as follows with respect to the vector of the flow 242 of fluidic medium 180 in the microfluidic channel 122: the microfluidic channel width W_{ch} (or cross-sectional area of the microfluidic channel 122) can be substantially perpendicular to the flow 242 of medium 180; the width W_{con} (or cross-sectional area) of the connection region 236 at opening 234 can be substantially parallel to the flow 242 of medium 180 in the microfluidic channel 122; and/or the length L_{con} of the connection region can be substantially perpendicular to the flow 242 of medium 180 in the microfluidic channel 122. The foregoing are examples only, and the relative position of the microfluidic channel 122 and sequestration pens 224, 226, 228 can be in other orientations with respect to each other.

As illustrated in Figure 2C, the width W_{con} of the connection region 236 can be uniform from the proximal opening 234 to the distal opening 238. The width W_{con} of the connection region 236 at the distal opening 238 can thus be any of the values identified herein for the width W_{con} of the connection region 236 at the proximal opening 234. Alternatively, the width W_{con} of the connection region 236 at the distal opening 238 can be larger than the width W_{con} of the connection region 236 at the proximal opening 234.

As illustrated in Figure 2C, the width of the isolation region 240 at the distal opening 238 can be substantially the same as the width W_{con} of the connection region 236 at the proximal opening 234. The width of the isolation region 240 at the distal opening 238 can thus be any of the values identified herein for the width W_{con} of the connection region 236 at the proximal opening 234. Alternatively, the width of the isolation region 240 at the distal opening 238 can be larger or smaller than the width W_{con} of the connection region 236 at the proximal opening 234. Moreover, the distal opening 238 may be smaller than the proximal opening 234 and the width W_{con} of the connection region 236 may be narrowed between the proximal opening 234 and distal opening 238. For example, the connection region 236 may be narrowed between the proximal opening and the distal opening, using a variety of different geometries (e.g. chamfering the connection region, beveling the connection region). Further, any part or subpart of the connection region 236 may be narrowed (e.g. a portion of the connection region adjacent to the proximal opening 234).

Figures 2D-2F depict another exemplary embodiment of a microfluidic device 250 containing a microfluidic circuit 262 and flow channels 264, which are variations of the respective microfluidic device 100, circuit 132 and channel 134 of Figure 1A. The microfluidic device 250 also has a plurality of sequestration pens 266 that are additional variations of the above-described sequestration pens 124, 126, 128, 130, 224, 226 or 228. In particular, it should be appreciated that the sequestration pens 266 of device 250 shown in Figures 2D-2F can replace any of the above-described sequestration pens 124, 126, 128, 130, 224, 226 or 228 in devices 100, 200, 230, 280, 290, 300, 700, 800, 1000. Likewise, the microfluidic device 250 is another variant of the microfluidic device 100, and may also have the same or a different DEP configuration as the above-described microfluidic device 100, 200, 230, 280, 290, 300, 700, 800, 1000 as well as any of the other microfluidic system components described herein.

The microfluidic device 250 of Figures 2D-2Fcomprises a support structure (not visible in Figures 2D-2F, but can be the same or generally similar to the support structure 104 of device 100 depicted in Figure 1A), a microfluidic circuit structure 256, and a cover (not visible in Figures 2D-2F, but can be the same or generally similar to the cover 122 of device 100 depicted in Figure 1A). The microfluidic circuit structure 256 includes a frame 252 and microfluidic circuit material 260, which can be the same as or generally similar to the frame 114 and microfluidic circuit material 116 of device 100 shown in Figure 1A. As shown in Figure 2D, the microfluidic circuit 262 defined by the microfluidic circuit material 260 can comprise multiple channels 264 (two are shown but there can be more) to which multiple sequestration pens 266 are fluidically connected.

Each sequestration pen 266 can comprise an isolation structure 272, an isolation region 270 within the isolation structure 272, and a connection region 268. From a proximal opening 274 at the microfluidic channel 264 to a distal opening 276 at the isolation structure 272, the connection region 268 fluidically connects the microfluidic channel 264 to the isolation region 270. Generally, in accordance with the above discussion of Figures 2B and 2C, a flow 278 of a first fluidic medium 254 in a channel 264 can create secondary flows 282 of the first medium 254 from the microfluidic channel 264 into and/or out of the respective connection regions 268 of the sequestration pens 266.

As illustrated in Figure 2E, the connection region 268 of each sequestration pen 266 generally includes the area extending between the proximal opening 274 to a channel 264 and the distal opening 276 to an isolation structure 272. The length L_{con} of the connection region 268 can be greater than the maximum penetration depth Dₚ of secondary flow 282, in which case the secondary flow 282 will extend into the connection region 268 without being redirected toward the isolation region 270 (as shown in Figure 2D). Alternatively, at illustrated in Figure 2F, the connection region 268 can have a length L_{con} that is less than the maximum penetration depth Dₚ, in which case the secondary flow 282 will extend through the connection region 268 and be redirected toward the isolation region 270. In this latter situation, the sum of lengths L_{c1} and L_{c2} of connection region 268 is greater than the maximum penetration depth Dₚ, so that secondary flow 282 will not extend into isolation region 270. Whether length L_{con} of connection region 268 is greater than the penetration depth Dₚ, or the sum of lengths L_{c1} and L_{c2} of connection region 268 is greater than the penetration depth Dₚ, a flow 278 of a first medium 254 in channel 264 that does not exceed a maximum velocity Vₘₐₓ will produce a secondary flow having a penetration depth Dₚ, and micro-objects (not shown but can be the same or generally similar to the micro-objects 246 shown in Figure 2C) in the isolation region 270 of a sequestration pen 266 will not be drawn out of the isolation region 270 by a flow 278 of first medium 254 in channel 264. Nor will the flow 278 in channel 264 draw miscellaneous materials (not shown) from channel 264 into the isolation region 270 of a sequestration pen 266. As such, diffusion is the only mechanism by which components in a first medium 254 in the microfluidic channel 264 can move from the microfluidic channel 264 into a second medium 258 in an isolation region 270 of a sequestration pen 266. Likewise, diffusion is the only mechanism by which components in a second medium 258 in an isolation region 270 of a sequestration pen 266 can move from the isolation region 270 to a first medium 254 in the microfluidic channel 264. The first medium 254 can be the same medium as the second medium 258, or the first medium 254 can be a different medium than the second medium 258. Alternatively, the first medium 254 and the second medium 258 can start out being the same, then become different, e.g., through conditioning of the second medium by one or more cells in the isolation region 270, or by changing the medium flowing through the microfluidic channel 264.

As illustrated in Figure 2E, the width W_{ch} of the microfluidic channels 264 (i.e., taken transverse to the direction of a fluid medium flow through the microfluidic channel indicated by arrows 278 in Figure 2D) in the microfluidic channel 264 can be substantially perpendicular to a width W_{con1} of the proximal opening 274 and thus substantially parallel to a width W_{con2} of the distal opening 276. The width W_{con1} of the proximal opening 274 and the width W_{con2} of the distal opening 276, however, need not be substantially perpendicular to each other. For example, an angle between an axis (not shown) on which the width W_{con1} of the proximal opening 274 is oriented and another axis on which the width W_{con2} of the distal opening 276 is oriented can be other than perpendicular and thus other than 90°. Examples of alternatively oriented angles include angles of: about 30° to about 90°, about 45° to about 90°, about 60° to about 90°, or the like.

In various embodiments of sequestration pens (e.g. 124, 126, 128, 130, 224, 226, 228, or 266), the isolation region (e.g. 240 or 270) is configured to contain a plurality of micro-objects. In other embodiments, the isolation region can be configured to contain only one, two, three, four, five, or a similar relatively small number of micro-objects. Accordingly, the volume of an isolation region can be, for example, at least 1×10⁶, 2×10⁶, 4×10⁶, 6×10⁶ cubic microns, or more.

In various embodiments of sequestration pens, the width W_{ch} of the microfluidic channel (e.g., 122) at a proximal opening (e.g. 234) can be about 50-1000 microns, 50-500 microns, 50-400 microns, 50-300 microns, 50-250 microns, 50-200 microns, 50-150 microns, 50-100 microns, 70-500 microns, 70-400 microns, 70-300 microns, 70-250 microns, 70-200 microns, 70-150 microns, 90-400 microns, 90-300 microns, 90-250 microns, 90-200 microns, 90-150 microns, 100-300 microns, 100-250 microns, 100-200 microns, 100-150 microns, or 100-120 microns. In some other embodiments, the width W_{ch} of the microfluidic channel (e.g., 122) at a proximal opening (e.g. 234) can be about 200-800 microns, 200-700 microns, or 200-600 microns. The foregoing are examples only, and the width W_{ch} of the microfluidic channel 122 can be any width within any of the endpoints listed above. Moreover, the W_{ch} of the microfluidic channel 122 can be selected to be in any of these widths in regions of the microfluidic channel other than at a proximal opening of a sequestration pen.

In some embodiments, a sequestration pen has a height of about 30 to about 200 microns, or about 50 to about 150 microns. In some embodiments, the sequestration pen has a cross-sectional area of about 1 ×10⁴ - 3 ×10⁶ square microns, 2 ×10⁴ - 2 ×10⁶ square microns, 4 ×10⁴ - 1 ×10⁶ square microns, 2 ×10⁴- 5 ×10⁵ square microns, 2 ×10⁴- 1 ×10⁵ square microns or about 2 ×10⁵ - 2×10⁶ square microns.

In various embodiments of sequestration pens, the height H_{ch} of the microfluidic channel (e.g.,122) at a proximal opening (e.g., 234) can be a height within any of the following heights: 20-100 microns, 20-90 microns, 20-80 microns, 20-70 microns, 20-60 microns, 20-50 microns, 30-100 microns, 30-90 microns, 30-80 microns, 30-70 microns, 30-60 microns, 30-50 microns, 40-100 microns, 40-90 microns, 40-80 microns, 40-70 microns, 40-60 microns, or 40-50 microns. The foregoing are examples only, and the height H_{ch} of the microfluidic channel (e.g.,122) can be a height within any of the endpoints listed above. The height H_{ch} of the microfluidic channel 122 can be selected to be in any of these heights in regions of the microfluidic channel other than at a proximal opening of a sequestration pen.

In various embodiments of sequestration pens a cross-sectional area of the microfluidic channel ( e.g., 122) at a proximal opening (e.g., 234) can be about 500-50,000 square microns, 500-40,000 square microns, 500-30,000 square microns, 500-25,000 square microns, 500-20,000 square microns, 500-15,000 square microns, 500-10,000 square microns, 500-7,500 square microns, 500-5,000 square microns, 1,000-25,000 square microns, 1,000-20,000 square microns, 1,000-15,000 square microns, 1,000-10,000 square microns, 1,000-7,500 square microns, 1,000-5,000 square microns, 2,000-20,000 square microns, 2,000-15,000 square microns, 2,000-10,000 square microns, 2,000-7,500 square microns, 2,000-6,000 square microns, 3,000-20,000 square microns, 3,000-15,000 square microns, 3,000-10,000 square microns, 3,000-7,500 square microns, or 3,000 to 6,000 square microns. The foregoing are examples only, and the cross-sectional area of the microfluidic channel (e.g., 122) at a proximal opening (e.g., 234) can be any area within any of the endpoints listed above.

In various embodiments of sequestration pens, the length L_{con} of the connection region (e.g., 236) can be about 1-600 microns, 5-550 microns, 10-500 microns, 15-400 microns, 20-300 microns, 20-500 microns, 40-400 microns, 60-300 microns, 80-200 microns, or about 100-150 microns. The foregoing are examples only, and length L_{con} of a connection region (e.g., 236) can be in any length within any of the endpoints listed above.

In various embodiments of sequestration pens the width W_{con} of a connection region (e.g., 236) at a proximal opening (e.g., 234) can be about 20-500 microns, 20-400 microns, 20-300 microns, 20-200 microns, 20-150 microns, 20-100 microns, 20-80 microns, 20-60 microns, 30-400 microns, 30-300 microns, 30-200 microns, 30-150 microns, 30-100 microns, 30-80 microns, 30-60 microns, 40-300 microns, 40-200 microns, 40-150 microns, 40-100 microns, 40-80 microns, 40-60 microns, 50-250 microns, 50-200 microns, 50-150 microns, 50-100 microns, 50-80 microns, 60-200 microns, 60-150 microns, 60-100 microns, 60-80 microns, 70-150 microns, 70-100 microns, or 80-100 microns. The foregoing are examples only, and the width W_{con} of a connection region (e.g., 236) at a proximal opening (e.g., 234) can be different than the foregoing examples (e.g., any value within any of the endpoints listed above).

In various embodiments of sequestration pens, the width W_{con} of a connection region (e.g., 236) at a proximal opening (e.g., 234) can be at least as large as the largest dimension of a micro-object (e.g.,biological cell which may be a T cell, B cell, or an ovum or embryo) that the sequestration pen is intended for. The foregoing are examples only, and the width W_{con} of a connection region (e.g., 236) at a proximal opening (e.g., 234) can be different than the foregoing examples (e.g., a width within any of the endpoints listed above).

In various embodiments of sequestration pens, the width Wₚᵣ of a proximal opening of a connection region may be at least as large as the largest dimension of a micro-object (e.g., a biological micro-object such as a cell) that the sequestration pen is intended for. For example, the width Wₚᵣ may be about 50 microns, about 60 microns, about 100 microns, about 200 microns, about 300 microns or may be about 50-300 microns, about 50-200 microns, about 50 -100 microns, about 75- 150 microns, about 75-100 microns, or about 200- 300 microns.

In various embodiments of sequestration pens, a ratio of the length L_{con} of a connection region (e.g., 236) to a width W_{con} of the connection region (e.g., 236) at the proximal opening 234 can be greater than or equal to any of the following ratios: 0.5, 1.0, 1.5, 2.0, 2.5, 3.0, 3.5, 4.0, 4.5, 5.0, 6.0, 7.0, 8.0, 9.0, 10.0, or more. The foregoing are examples only, and the ratio of the length L_{con} of a connection region 236 to a width W_{con} of the connection region 236 at the proximal opening 234 can be different than the foregoing examples.

In various embodiments of microfluidic devices 100, 200, 23, 250, 280, 290, 300, 700, 800, 1000, Vₘₐₓ can be set around 0.2, 0.5, 0.7, 1.0, 1.3, 1.5, 2.0, 2.5, 3.0, 3.5, 4.0, 4.5, 5.0, 5.5, 6.0, 6.7, 7.0, 7.5, 8.0, 8.5, 9.0, 10, 11, 12, 13, 14, or 15 microliters/sec.

In various embodiments of microfluidic devices having sequestration pens, the volume of an isolation region (e.g., 240) of a sequestration pen can be, for example, at least 5×10⁵, 8×10⁵, 1×10⁶, 2×10⁶, 4×10⁶, 6×10⁶, 8×10⁶, 1×10⁷, 5×10⁷, 1×10⁸, 5×10⁸, or 8×10⁸ cubic microns, or more. In various embodiments of microfluidic devices having sequestration pens, the volume of a sequestration pen may be about 5×10⁵, 6×10⁵, 8×10⁵, 1×10⁶, 2×10⁶, 4×10⁶, 8×10⁶, 1×10⁷, 3×10⁷, 5×10⁷, or about 8×10⁷ cubic microns, or more. In some other embodiments, the volume of a sequestration pen may be about 1 nanoliter to about 50 nanoliters, 2 nanoliters to about 25 nanoliters, 2 nanoliters to about 20 nanoliters, about 2 nanoliters to about 15 nanoliters, or about 2 nanoliters to about 10 nanoliters.

In various embodiment, the microfluidic device has sequestration pens configured as in any of the embodiments discussed herein where the microfluidic device has about 5 to about 10 sequestration pens, about 10 to about 50 sequestration pens, about 100 to about 500 sequestration pens; about 200 to about 1000 sequestration pens, about 500 to about 1500 sequestration pens, about 1000 to about 2000 sequestration pens, about 1000 to about 3500 sequestration pens, about 3000 to about 7000 sequestration pens, about 5000 to about 10,000 sequestration pens, about 9,000 to about 15,000 sequestration pens, or about 12, 000 to about 20,000 sequestration pens. The sequestration pens need not all be the same size and may include a variety of configurations (e.g., different widths, different features within the sequestration pen).

Figure 2G illustrates a microfluidic device 280 according to one embodiment. The microfluidic device 280 illustrated in Figure 2G is a stylized diagram of a microfluidic device 100. In practice the microfluidic device 280 and its constituent circuit elements (e.g. channels 122 and sequestration pens 128) would have the dimensions discussed herein. The microfluidic circuit 120 illustrated in Figure 2G has two ports 107, four distinct channels 122 and four distinct flow paths 106. The microfluidic device 280 further comprises a plurality of sequestration pens opening off of each channel 122. In the microfluidic device illustrated in Figure 2G, the sequestration pens have a geometry similar to the pens illustrated in Figure 2C and thus, have both connection regions and isolation regions. Accordingly, the microfluidic circuit 120 includes both swept regions (e.g. channels 122 and portions of the connection regions 236 within the maximum penetration depth Dₚ of the secondary flow 244) and non-swept regions (e.g. isolation regions 240 and portions of the connection regions 236 not within the maximum penetration depth Dₚ of the secondary flow 244).

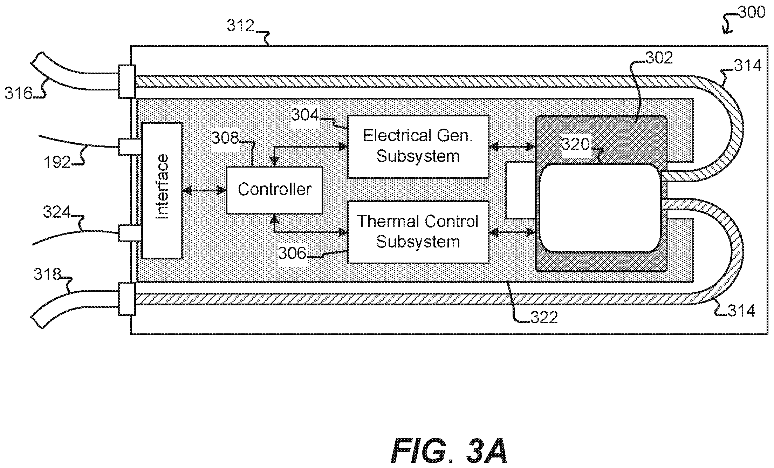

Figures 3A through 3B shows various embodiments of system 150 which can be used to operate and observe microfluidic devices (e.g. 100, 200, 230, 250, 280, 290, 300, 700, 800, 1000) according to the present disclosure. As illustrated in Figure 3A, the system 150 can include a structure ("nest") 300 configured to hold a microfluidic device 100 (not shown), or any other microfluidic device described herein. The nest 300 can include a socket 302 capable of interfacing with the microfluidic device 320 (e.g., an optically-actuated electrokinetic device 100) and providing electrical connections from power source 192 to microfluidic device 320. The nest 300 can further include an integrated electrical signal generation subsystem 304. The electrical signal generation subsystem 304 can be configured to supply a biasing voltage to socket 302 such that the biasing voltage is applied across a pair of electrodes in the microfluidic device 320 when it is being held by socket 302. Thus, the electrical signal generation subsystem 304 can be part of power source 192. The ability to apply a biasing voltage to microfluidic device 320 does not mean that a biasing voltage will be applied at all times when the microfluidic device 320 is held by the socket 302. Rather, in most cases, the biasing voltage will be applied intermittently, e.g., only as needed to facilitate the generation of electrokinetic forces, such as dielectrophoresis or electrowetting, in the microfluidic device 320.

As illustrated in Figure 3A, the nest 300 can include a printed circuit board assembly (PCBA) 322. The electrical signal generation subsystem 304 can be mounted on and electrically integrated into the PCBA 322. The exemplary support includes socket 302 mounted on PCBA 322, as well.

Typically, the electrical signal generation subsystem 304 will include a waveform generator (not shown). The electrical signal generation subsystem 304 can further include an oscilloscope (not shown) and/or a waveform amplification circuit (not shown) configured to amplify a waveform received from the waveform generator. The oscilloscope, if present, can be configured to measure the waveform supplied to the microfluidic device 320 held by the socket 302. In certain embodiments, the oscilloscope measures the waveform at a location proximal to the microfluidic device 320 (and distal to the waveform generator), thus ensuring greater accuracy in measuring the waveform actually applied to the device. Data obtained from the oscilloscope measurement can be, for example, provided as feedback to the waveform generator, and the waveform generator can be configured to adjust its output based on such feedback. An example of a suitable combined waveform generator and oscilloscope is the Red Pitaya^{™}.

In certain embodiments, the nest 300 further comprises a controller 308, such as a microprocessor used to sense and/or control the electrical signal generation subsystem 304. Examples of suitable microprocessors include the Arduino^{™} microprocessors, such as the Arduino Nano^{™}. The controller 308 may be used to perform functions and analysis or may communicate with an external master controller 154 (shown in Figure 1A) to perform functions and analysis. In the embodiment illustrated in Figure 3A the controller 308 communicates with a master controller 154 through an interface 310 (e.g., a plug or connector).

In some embodiments, the nest 300 can comprise an electrical signal generation subsystem 304 comprising a Red Pitaya^{™} waveform generator/oscilloscope unit ("Red Pitaya unit") and a waveform amplification circuit that amplifies the waveform generated by the Red Pitaya unit and passes the amplified voltage to the microfluidic device 100. In some embodiments, the Red Pitaya unit is configured to measure the amplified voltage at the microfluidic device 320 and then adjust its own output voltage as needed such that the measured voltage at the microfluidic device 320 is the desired value. In some embodiments, the waveform amplification circuit can have a +6.5V to -6.5V power supply generated by a pair of DC-DC converters mounted on the PCBA 322, resulting in a signal of up to 13 Vpp at the microfluidic device 100.

As illustrated in Figure 3A, the support structure 300 (e.g., nest) can further include a thermal control subsystem 306. The thermal control subsystem 306 can be configured to regulate the temperature of microfluidic device 320 held by the support structure 300. For example, the thermal control subsystem 306 can include a Peltier thermoelectric device (not shown) and a cooling unit (not shown). The Peltier thermoelectric device can have a first surface configured to interface with at least one surface of the microfluidic device 320. The cooling unit can be, for example, a cooling block (not shown), such as a liquid-cooled aluminum block. A second surface of the Peltier thermoelectric device (e.g., a surface opposite the first surface) can be configured to interface with a surface of such a cooling block. The cooling block can be connected to a fluidic path 314 configured to circulate cooled fluid through the cooling block. In the embodiment illustrated in Figure 3A, the support structure 300 comprises an inlet 316 and an outlet 318 to receive cooled fluid from an external reservoir (not shown), introduce the cooled fluid into the fluidic path 314 and through the cooling block, and then return the cooled fluid to the external reservoir. In some embodiments, the Peltier thermoelectric device, the cooling unit, and/or the fluidic path 314 can be mounted on a casing 312of the support structure 300. In some embodiments, the thermal control subsystem 306 is configured to regulate the temperature of the Peltier thermoelectric device so as to achieve a target temperature for the microfluidic device 320. Temperature regulation of the Peltier thermoelectric device can be achieved, for example, by a thermoelectric power supply, such as a Pololu^{™} thermoelectric power supply (Pololu Robotics and Electronics Corp.). The thermal control subsystem 306 can include a feedback circuit, such as a temperature value provided by an analog circuit. Alternatively, the feedback circuit can be provided by a digital circuit.

In some embodiments, the nest 300 can include a thermal control subsystem 306 with a feedback circuit that is an analog voltage divider circuit (not shown) which includes a resistor (e.g., with resistance 1 kOhm+/-0.1 %, temperature coefficient +/-0.02 ppm/C0) and a NTC thermistor (e.g., with nominal resistance 1 kOhm+/-0.01 %). In some instances, the thermal control subsystem 306 measures the voltage from the feedback circuit and then uses the calculated temperature value as input to an on-board PID control loop algorithm. Output from the PID control loop algorithm can drive, for example, both a directional and a pulse-width-modulated signal pin on a Pololu^{™} motor drive (not shown) to actuate the thermoelectric power supply, thereby controlling the Peltier thermoelectric device.

The nest 300 can include a serial port 324 which allows the microprocessor of the controller 308 to communicate with an external master controller 154 via the interface 310 (not shown). In addition, the microprocessor of the controller 308 can communicate (e.g., via a Plink tool (not shown)) with the electrical signal generation subsystem 304 and thermal control subsystem 306. Thus, via the combination of the controller 308, the interface 310, and the serial port 324, the electrical signal generation subsystem 304 and the thermal control subsystem 306 can communicate with the external master controller 154. In this manner, the master controller 154 can, among other things, assist the electrical signal generation subsystem 304 by performing scaling calculations for output voltage adjustments. A Graphical User Interface (GUI) (not shown) provided via a display device 170 coupled to the external master controller 154, can be configured to plot temperature and waveform data obtained from the thermal control subsystem 306 and the electrical signal generation subsystem 304, respectively. Alternatively, or in addition, the GUI can allow for updates to the controller 308, the thermal control subsystem 306, and the electrical signal generation subsystem 304.

As discussed above, system 150 can include an imaging device. In some embodiments, the imaging device comprises a light modulating subsystem 330 (See Figure 3B). The light modulating subsystem 330 can include a digital mirror device (DMD) or a microshutter array system (MSA), either of which can be configured to receive light from a light source 332 and transmits a subset of the received light into an optical train of microscope 350. Alternatively, the light modulating subsystem 330 can include a device that produces its own light (and thus dispenses with the need for a light source 332), such as an organic light emitting diode display (OLED), a liquid crystal on silicon (LCOS) device, a ferroelectric liquid crystal on silicon device (FLCOS), or a transmissive liquid crystal display (LCD). The light modulating subsystem 330 can be, for example, a projector. Thus, the light modulating subsystem 330 can be capable of emitting both structured and unstructured light. In certain embodiments, imaging module 164 and/or motive module 162 of system 150 can control the light modulating subsystem 330.

In certain embodiments, the imaging device further comprises a microscope 350. In such embodiments, the nest 300 and light modulating subsystem 330 can be individually configured to be mounted on the microscope 350. The microscope 350 can be, for example, a standard research-grade light microscope or fluorescence microscope. Thus, the nest 300 can be configured to be mounted on the stage 344 of the microscope 350 and/or the light modulating subsystem 330 can be configured to mount on a port of microscope 350. In other embodiments, the nest 300 and the light modulating subsystem 330 described herein can be integral components of microscope 350.

In certain embodiments, the microscope 350 can further include one or more detectors 348. In some embodiments, the detector 348 is controlled by the imaging module 164. The detector 348 can include an eye piece, a charge-coupled device (CCD), a camera (e.g., a digital camera), or any combination thereof. If at least two detectors 348 are present, one detector can be, for example, a fast-frame-rate camera while the other detector can be a high sensitivity camera. Furthermore, the microscope 350 can include an optical train configured to receive reflected and/or emitted light from the microfluidic device 320 and focus at least a portion of the reflected and/or emitted light on the one or more detectors 348. The optical train of the microscope can also include different tube lenses (not shown) for the different detectors, such that the final magnification on each detector can be different.

In certain embodiments, the imaging device is configured to use at least two light sources. For example, a first light source 332 can be used to produce structured light (e.g., via the light modulating subsystem 330) and a second light source 334 can be used to provide unstructured light. The first light source 332 can produce structured light for optically-actuated electrokinesis and/or fluorescent excitation, and the second light source 334 can be used to provide bright field illumination. In these embodiments, the motive module 164 can be used to control the first light source 332 and the imaging module 164 can be used to control the second light source 334. The optical train of the microscope 350 can be configured to (1) receive structured light from the light modulating subsystem 330 and focus the structured light on at least a first region in a microfluidic device, such as an optically-actuated electrokinetic device, when the device is being held by the nest 300, and (2) receive reflected and/or emitted light from the microfluidic device and focus at least a portion of such reflected and/or emitted light onto detector 348. The optical train can be further configured to receive unstructured light from a second light source and focus the unstructured light on at least a second region of the microfluidic device, when the device is held by the nest 300. In certain embodiments, the first and second regions of the microfluidic device can be overlapping regions. For example, the first region can be a subset of the second region. In other embodiments, the second light source 334 may additionally or alternatively include a laser, which may have any suitable wavelength of light. The representation of the optical system shown in Figure 3B is a schematic representation only, and the optical system may include additional filters, notch filters, lenses and the like. When the second light source 334 includes one or more light source(s) for brightfield and/or fluorescent excitation, as well as laser illumination the physical arrangement of the light source(s) may vary from that shown in Figure 3B, and the laser illumination may be introduced at any suitable physical location within the optical system. The schematic locations of light source 334 and light source 332/light modulating subsystem 330 may be interchanged as well.

In Figure 3B, the first light source 332 is shown supplying light to a light modulating subsystem 330, which provides structured light to the optical train of the microscope 350 of system 355 (not shown). The second light source 334 is shown providing unstructured light to the optical train via a beam splitter 336. Structured light from the light modulating subsystem 330 and unstructured light from the second light source 334 travel from the beam splitter 336 through the optical train together to reach a second beam splitter (or dichroic filter 338, depending on the light provided by the light modulating subsystem 330), where the light gets reflected down through the objective 336 to the sample plane 342. Reflected and/or emitted light from the sample plane 342 then travels back up through the objective 340, through the beam splitter and/or dichroic filter 338, and to a dichroic filter 346. Only a fraction of the light reaching dichroic filter 346 passes through and reaches the detector 348.

In some embodiments, the second light source 334 emits blue light. With an appropriate dichroic filter 346, blue light reflected from the sample plane 342 is able to pass through dichroic filter 346 and reach the detector 348. In contrast, structured light coming from the light modulating subsystem 330 gets reflected from the sample plane 342, but does not pass through the dichroic filter 346. In this example, the dichroic filter 346 is filtering out visible light having a wavelength longer than 495 nm. Such filtering out of the light from the light modulating subsystem 330 would only be complete (as shown) if the light emitted from the light modulating subsystem did not include any wavelengths shorter than 495 nm. In practice, if the light coming from the light modulating subsystem 330 includes wavelengths shorter than 495 nm (e.g., blue wavelengths), then some of the light from the light modulating subsystem would pass through filter 346 to reach the detector 348. In such an embodiment, the filter 346 acts to change the balance between the amount of light that reaches the detector 348 from the first light source 332 and the second light source 334. This can be beneficial if the first light source 332 is significantly stronger than the second light source 334. In other embodiments, the second light source 334 can emit red light, and the dichroic filter 346 can filter out visible light other than red light (e.g., visible light having a wavelength shorter than 650 nm).

Coating solutions **and** coating agents. Without intending to be limited by theory, maintenance of a biological micro-object (e.g., a biological cell) within a microfluidic device (e.g., a DEP-configured and/or EW-configured microfluidic device) may be facilitated (i.e., the biological micro-object exhibits increased viability, greater expansion and/or greater portability within the microfluidic device) when at least one or more inner surfaces of the microfluidic device have been conditioned or coated so as to present a layer of organic and/or hydrophilic molecules that provides the primary interface between the microfluidic device and biological micro-object(s) maintained therein. In some embodiments, one or more of the inner surfaces of the microfluidic device (e.g. the inner surface of the electrode activation substrate of a DEP-configured microfluidic device, the cover of the microfluidic device, and/or the surfaces of the circuit material) may be treated with or modified by a coating solution and/or coating agent to generate the desired layer of organic and/or hydrophilic molecules.

The coating may be applied before or after introduction of biological micro-object(s), or may be introduced concurrently with the biological micro-object(s). In some embodiments, the biological micro-object(s) may be imported into the microfluidic device in a fluidic medium that includes one or more coating agents. In other embodiments, the inner surface(s) of the microfluidic device (e.g., a DEP-configured microfluidic device) are treated or "primed" with a coating solution comprising a coating agent prior to introduction of the biological micro-object(s) into the microfluidic device.

In some embodiments, at least one surface of the microfluidic device includes a coating material that provides a layer of organic and/or hydrophilic molecules suitable for maintenance and/or expansion of biological micro-object(s) (e.g. provides a conditioned surface as described below). In some embodiments, substantially all the inner surfaces of the microfluidic device include the coating material. The coated inner surface(s) may include the surface of a flow region (e.g., channel), chamber, or sequestration pen, or a combination thereof. In some embodiments, each of a plurality of sequestration pens has at least one inner surface coated with coating materials. In other embodiments, each of a plurality of flow regions or channels has at least one inner surface coated with coating materials. In some embodiments, at least one inner surface of each of a plurality of sequestration pens and each of a plurality of channels is coated with coating materials.

Coating agent/Solution. Any convenient coating agent/coating solution can be used, including but not limited to: serum or serum factors, bovine serum albumin (BSA), polymers, detergents, enzymes, and any combination thereof.

Polymer-based coating materials. The at least one inner surface may include a coating material that comprises a polymer. The polymer may be covalently or non-covalently bound (or may be non-specifically adhered) to the at least one surface. The polymer may have a variety of structural motifs, such as found in block polymers (and copolymers), star polymers (star copolymers), and graft or comb polymers (graft copolymers), all of which may be suitable for the methods disclosed herein.

The polymer may include a polymer including alkylene ether moieties. A wide variety of alkylene ether containing polymers may be suitable for use in the microfluidic devices described herein. One non-limiting exemplary class of alkylene ether containing polymers are amphiphilic nonionic block copolymers which include blocks of polyethylene oxide (PEO) and polypropylene oxide (PPO) subunits in differing ratios and locations within the polymer chain. Pluronic^{®} polymers (BASF) are block copolymers of this type and are known in the art to be suitable for use when in contact with living cells. The polymers may range in average molecular mass M_{w} from about 2000Da to about 20KDa. In some embodiments, the PEO-PPO block copolymer can have a hydrophilic-lipophilic balance (HLB) greater than about 10 (e.g. 12-18). Specific Pluronic^{®} polymers useful for yielding a coated surface include Pluronic^{®} L44, L64, P85, and F127 (including F127NF). Another class of alkylene ether containing polymers is polyethylene glycol (PEG M_{w} <100,000Da) or alternatively polyethylene oxide (PEO, M_{w}>100,000). In some embodiments, a PEG may have an M_{w} of about 1000Da, 5000Da, 10,000Da or 20,000Da.

In other embodiments, the coating material may include a polymer containing carboxylic acid moieties. The carboxylic acid subunit may be an alkyl, alkenyl or aromatic moiety containing subunit. One non-limiting example is polylactic acid (PLA). In other embodiments, the coating material may include a polymer containing phosphate moieties, either at a terminus of the polymer backbone or pendant from the backbone of the polymer. In yet other embodiments, the coating material may include a polymer containing sulfonic acid moieties. The sulfonic acid subunit may be an alkyl, alkenyl or aromatic moiety containing subunit. One non-limiting example is polystyrene sulfonic acid (PSSA) or polyanethole sulfonic acid. In further embodiments, the coating material may include a polymer including amine moieties. The polyamino polymer may include a natural polyamine polymer or a synthetic polyamine polymer. Examples of natural polyamines include spermine, spermidine, and putrescine.

In other embodiments, the coating material may include a polymer containing saccharide moieties. In a non-limiting example, polysaccharides such as xanthan gum or dextran may be suitable to form a material which may reduce or prevent cell sticking in the microfluidic device. For example, a dextran polymer having a size about 3kDa may be used to provide a coating material for a surface within a microfluidic device.

In other embodiments, the coating material may include a polymer containing nucleotide moieties, i.e. a nucleic acid, which may have ribonucleotide moieties or deoxyribonucleotide moieties, providing a polyelectrolyte surface. The nucleic acid may contain only natural nucleotide moieties or may contain unnatural nucleotide moieties which comprise nucleobase, ribose or phosphate moiety analogs such as 7-deazaadenine, pentose, methyl phosphonate or phosphorothioate moieties without limitation.

In yet other embodiments, the coating material may include a polymer containing amino acid moieties. The polymer containing amino acid moieties may include a natural amino acid containing polymer or an unnatural amino acid containing polymer, either of which may include a peptide, a polypeptide or a protein. In one non-limiting example, the protein may be bovine serum albumin (BSA) and/or serum (or a combination of multiple different sera) comprising albumin and/or one or more other similar proteins as coating agents. The serum can be from any convenient source, including but not limited to fetal calf serum, sheep serum, goat serum, horse serum, and the like. In certain embodiments, BSA in a coating solution is present in a concentration from about 1 mg/mL to about 100 mg/mL, including 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, or more or anywhere in between. In certain embodiments, serum in a coating solution may be present in a concentration of about 20% (v/v) to about 50% v/v, including 25%, 30%, 35%, 40%, 45%, or more or anywhere in between. In some embodiments, BSA may be present as a coating agent in a coating solution at 5 mg/mL, whereas in other embodiments, BSA may be present as a coating agent in a coating solution at 70 mg/mL. In certain embodiments, serum is present as a coating agent in a coating solution at 30%. In some embodiments, an extracellular matrix (ECM) protein may be provided within the coating material for optimized cell adhesion to foster cell growth. A cell matrix protein, which may be included in a coating material, can include, but is not limited to, a collagen, an elastin, an RGD-containing peptide (e.g. a fibronectin), or a laminin. In yet other embodiments, growth factors, cytokines, hormones or other cell signaling species may be provided within the coating material of the microfluidic device.

In some embodiments, the coating material may include a polymer containing more than one of alkylene oxide moieties, carboxylic acid moieties, sulfonic acid moieties, phosphate moieties, saccharide moieties, nucleotide moieties, or amino acid moieties. In other embodiments, the polymer conditioned surface may include a mixture of more than one polymer each having alkylene oxide moieties, carboxylic acid moieties, sulfonic acid moieties, phosphate moieties, saccharide moieties, nucleotide moieties, and/or amino acid moieties, which may be independently or simultaneously incorporated into the coating material.

**Covalently linked coating materials.** In some embodiments, the at least one inner surface includes covalently linked molecules that provide a layer of organic and/or hydrophilic molecules suitable for maintenance/expansion of biological micro-object(s) within the microfluidic device, providing a conditioned surface for such cells.

The covalently linked molecules include a linking group, wherein the linking group is covalently linked to one or more surfaces of the microfluidic device, as described below. The linking group is also covalently linked to a moiety configured to provide a layer of organic and/or hydrophilic molecules suitable for maintenance/expansion of biological micro-object(s).

In some embodiments, the covalently linked moiety configured to provide a layer of organic and/or hydrophilic molecules suitable for maintenance/expansion of biological micro-object(s) may include alkyl or fluoroalkyl (which includes perfluoroalkyl) moieties; mono- or polysaccharides (which may include but is not limited to dextran); alcohols (including but not limited to propargyl alcohol); polyalcohols, including but not limited to polyvinyl alcohol; alkylene ethers, including but not limited to polyethylene glycol; polyelectrolytes ( including but not limited to polyacrylic acid or polyvinyl phosphonic acid); amino groups (including derivatives thereof, such as, but not limited to alkylated amines, hydroxyalkylated amino group, guanidinium, and heterocylic groups containing an unaromatized nitrogen ring atom, such as, but not limited to morpholinyl or piperazinyl); carboxylic acids including but not limited to propiolic acid (which may provide a carboxylate anionic surface); phosphonic acids, including but not limited to ethynyl phosphonic acid (which may provide a phosphonate anionic surface); sulfonate anions; carboxybetaines; sulfobetaines; sulfamic acids; or amino acids.

In various embodiments, the covalently linked moiety configured to provide a layer of organic and/or hydrophilic molecules suitable for maintenance/expansion of biological micro-object(s) in the microfluidic device may include non-polymeric moieties such as an alkyl moiety, a substituted alkyl moiety, such as a fluoroalkyl moiety (including but not limited to a perfluoroalkyl moiety), amino acid moiety, alcohol moiety, amino moiety, carboxylic acid moiety, phosphonic acid moiety, sulfonic acid moiety, sulfamic acid moiety, or saccharide moiety. Alternatively, the covalently linked moiety may include polymeric moieties, which may be any of the moieties described above.

In some embodiments, the covalently linked alkyl moiety may comprise carbon atoms forming a linear chain (e.g., a linear chain of at least 10 carbons, or at least 14, 16, 18, 20, 22, or more carbons) and may be an unbranched alkyl moiety. In some embodiments, the alkyl group may include a substituted alkyl group (e.g., some of the carbons in the alkyl group can be fluorinated or perfluorinated). In some embodiments, the alkyl group may include a first segment, which may include a perfluoroalkyl group, joined to a second segment, which may include a non-substituted alkyl group, where the first and second segments may be joined directly or indirectly (e.g., by means of an ether linkage). The first segment of the alkyl group may be located distal to the linking group, and the second segment of the alkyl group may be located proximal to the linking group.

In other embodiments, the covalently linked moiety may include at least one amino acid, which may include more than one type of amino acid. Thus, the covalently linked moiety may include a peptide or a protein. In some embodiments, the covalently linked moiety may include an amino acid which may provide a zwitterionic surface to support cell growth, viability, portability, or any combination thereof.

In other embodiments, the covalently linked moiety may include at least one alkylene oxide moiety, and may include any alkylene oxide polymer as described above. One useful class of alkylene ether containing polymers is polyethylene glycol (PEG M_{w} <100,000Da) or alternatively polyethylene oxide (PEO, MW>100,000). In some embodiments, a PEG may have an M_{w} of about 1000Da, 5000Da, 10,000Da or 20,000Da.

The covalently linked moiety may include one or more saccharides. The covalently linked saccharides may be mono-, di-, or polysaccharides. The covalently linked saccharides may be modified to introduce a reactive pairing moiety which permits coupling or elaboration for attachment to the surface. Exemplary reactive pairing moieties may include aldehyde, alkyne or halo moieties. A polysaccharide may be modified in a random fashion, wherein each of the saccharide monomers may be modified or only a portion of the saccharide monomers within the polysaccharide are modified to provide a reactive pairing moiety that may be coupled directly or indirectly to a surface. One exemplar may include a dextran polysaccharide, which may be coupled indirectly to a surface via an unbranched linker.

The covalently linked moiety may include one or more amino groups. The amino group may be a substituted amine moiety, guanidine moiety, nitrogen-containing heterocyclic moiety or heteroaryl moiety. The amino containing moieties may have structures permitting pH modification of the environment within the microfluidic device, and optionally, within the sequestration pens and/or flow regions (e.g., channels).

The coating material providing a conditioned surface may comprise only one kind of covalently linked moiety or may include more than one different kind of covalently linked moiety. For example, the fluoroalkyl conditioned surfaces (including perfluoroalkyl) may have a plurality of covalently linked moieties which are all the same, e.g., having the same linking group and covalent attachment to the surface, the same overall length, and the same number of fluoromethylene units comprising the fluoroalkyl moiety. Alternatively, the coating material may have more than one kind of covalently linked moiety attached to the surface. For example, the coating material may include molecules having covalently linked alkyl or fluoroalkyl moieties having a specified number of methylene or fluoromethylene units and may further include a further set of molecules having charged moieties covalently attached to an alkyl or fluoroalkyl chain having a greater number of methylene or fluoromethylene units, which may provide capacity to present bulkier moieties at the coated surface. In this instance, the first set of molecules having different, less sterically demanding termini and fewer backbone atoms can help to functionalize the entire substrate surface and thereby prevent undesired adhesion or contact with the silicon/silicon oxide, hafnium oxide or alumina making up the substrate itself. In another example, the covalently linked moieties may provide a zwitterionic surface presenting alternating charges in a random fashion on the surface.

**Conditioned surface properties**. Aside from the composition of the conditioned surface, other factors such as physical thickness of the hydrophobic material can impact DEP force. Various factors can alter the physical thickness of the conditioned surface, such as the manner in which the conditioned surface is formed on the substrate (e.g. vapor deposition, liquid phase deposition, spin coating, flooding, and electrostatic coating). In some embodiments, the conditioned surface has a thickness of about 1nm to about 10nm; about 1 nm to about 7 nm; about 1nm to about 5nm; or any individual value therebetween. In other embodiments, the conditioned surface formed by the covalently linked moieties may have a thickness of about 10 nm to about 50 nm. In various embodiments, the conditioned surface prepared as described herein has a thickness of less than 10nm. In some embodiments, the covalently linked moieties of the conditioned surface may form a monolayer when covalently linked to the surface of the microfluidic device (e.g., a DEP configured substrate surface) and may have a thickness of less than 10 nm (e.g., less than 5 nm, or about 1.5 to 3.0 nm). These values are in contrast to that of a surface prepared by spin coating, for example, which may typically have a thickness of about 30nm. In some embodiments, the conditioned surface does not require a perfectly formed monolayer to be suitably functional for operation within a DEP-configured microfluidic device.

In various embodiments, the coating material providing a conditioned surface of the microfluidic device may provide desirable electrical properties. Without intending to be limited by theory, one factor that impacts robustness of a surface coated with a particular coating material is intrinsic charge trapping. Different coating materials may trap electrons, which can lead to breakdown of the coating material. Defects in the coating material may increase charge trapping and lead to further breakdown of the coating material. Similarly, different coating materials have different dielectric strengths (i.e. the minimum applied electric field that results in dielectric breakdown), which may impact charge trapping. In certain embodiments, the coating material can have an overall structure (e.g., a densely-packed monolayer structure) that reduces or limits that amount of charge trapping.

In addition to its electrical properties, the conditioned surface may also have properties that are beneficial in use with biological molecules. For example, a conditioned surface that contains fluorinated (or perfluorinated) carbon chains may provide a benefit relative to alkyl-terminated chains in reducing the amount of surface fouling. Surface fouling, as used herein, refers to the amount of indiscriminate material deposition on the surface of the microfluidic device, which may include permanent or semi-permanent deposition of biomaterials such as protein and its degradation products, nucleic acids and respective degradation products and the like.

**Unitary or Multi-part conditioned surface.** The covalently linked coating material may be formed by reaction of a molecule which already contains the moiety configured to provide a layer of organic and/or hydrophilic molecules suitable for maintenance/expansion of biological micro-object(s) in the microfluidic device, as is described below. Alternatively, the covalently linked coating material may be formed in a two-part sequence by coupling the moiety configured to provide a layer of organic and/or hydrophilic molecules suitable for maintenance/expansion of biological micro-object(s) to a surface modifying ligand that itself has been covalently linked to the surface.

**Methods of preparing a covalently linked coating material.** In some embodiments, a coating material that is covalently linked to the surface of a microfluidic device (e.g., including at least one surface of the sequestration pens and/or flow regions) has a structure of Formula 1 or Formula 2. When the coating material is introduced to the surface in one step, it has a structure of Formula 1, while when the coating material is introduced in a multiple step process, it has a structure of Formula 2.

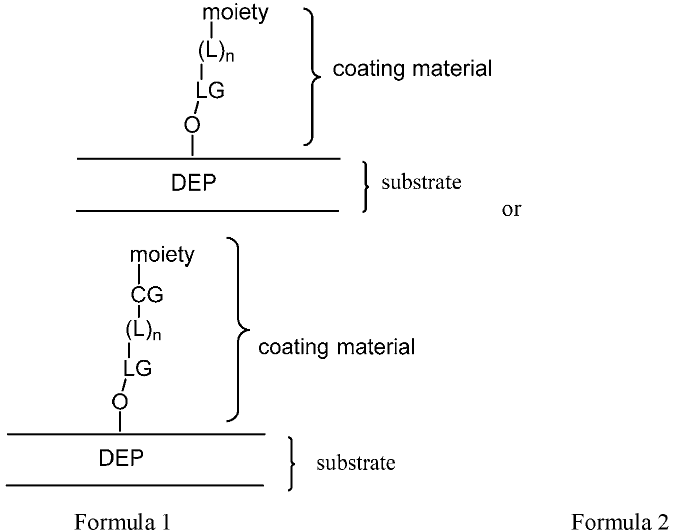

The coating material may be linked covalently to oxides of the surface of a DEP-configured or EW- configured substrate. The DEP- or EW- configured substrate may comprise silicon, silicon oxide, alumina, or hafnium oxide. Oxides may be present as part of the native chemical structure of the substrate or may be introduced as discussed below.

The coating material may be attached to the oxides via a linking group ("LG"), which may be a siloxy or phosphonate ester group formed from the reaction of a siloxane or phosphonic acid group with the oxides. The moiety configured to provide a layer of organic and/or hydrophilic molecules suitable for maintenance/expansion of biological micro-object(s) in the microfluidic device can be any of the moieties described herein. The linking group LG may be directly or indirectly connected to the moiety configured to provide a layer of organic and/or hydrophilic molecules suitable for maintenance/expansion of biological micro-object(s) in the microfluidic device. When the linking group LG is directly connected to the moiety, optional linker ("L") is not present and n is 0. When the linking group LG is indirectly connected to the moiety, linker L is present and n is 1. The linker L may have a linear portion where a backbone of the linear portion may include 1 to 200 non-hydrogen atoms selected from any combination of silicon, carbon, nitrogen, oxygen, sulfur and/or phosphorus atoms, subject to chemical bonding limitations as is known in the art. It may be interrupted with any combination of one or more moieties, which may be chosen from ether, amino, carbonyl, amido, and/or phosphonate groups, arylene, heteroarylene, or heterocyclic groups. In some embodiments, the backbone of the linker L may include 10 to 20 atoms. In other embodiments, the backbone of the linker L may include about 5 atoms to about 200 atoms; about 10 atoms to about 80 atoms; about 10 atoms to about 50 atoms; or about 10 atoms to about 40 atoms. In some embodiments, the backbone atoms are all carbon atoms.

In some embodiments, the moiety configured to provide a layer of organic and/or hydrophilic molecules suitable for maintenance/expansion of biological micro-object(s) may be added to the surface of the substrate in a multi-step process, and has a structure of Formula 2, as shown above. The moiety may be any of the moieties described above.

In some embodiments, the coupling group CG represents the resultant group from reaction of a reactive moiety Rₓ and a reactive pairing moiety Rₚₓ (i.e., a moiety configured to react with the reactive moiety Rₓ). For example, one typical coupling group CG may include a carboxamidyl group, which is the result of the reaction of an amino group with a derivative of a carboxylic acid, such as an activated ester, an acid chloride or the like. Other CG may include a triazolylene group, a carboxamidyl, thioamidyl, an oxime, a mercaptyl, a disulfide, an ether, or alkenyl group, or any other suitable group that may be formed upon reaction of a reactive moiety with its respective reactive pairing moiety. The coupling group CG may be located at the second end (i.e., the end proximal to the moiety configured to provide a layer of organic and/or hydrophilic molecules suitable for maintenance/expansion of biological micro-object(s) in the microfluidic device) of linker L, which may include any combination of elements as described above. In some other embodiments, the coupling group CG may interrupt the backbone of the linker L. When the coupling group CG is triazolylene, it may be the product resulting from a Click coupling reaction and may be further substituted (e.g., a dibenzocylcooctenyl fused triazolylene group).

In some embodiments, the coating material (or surface modifying ligand) is deposited on the inner surfaces of the microfluidic device using chemical vapor deposition. The vapor deposition process can be optionally improved, for example, by pre-cleaning the cover 110, the microfluidic circuit material 116, and/or the substrate (e.g., the inner surface 208 of the electrode activation substrate 206 of a DEP-configured substrate, or a dielectric layer of the support structure 104 of an EW-configured substrate), by exposure to a solvent bath, sonication or a combination thereof. Alternatively, or in addition, such pre-cleaning can include treating the cover 110, the microfluidic circuit material 116, and/or the substrate in an oxygen plasma cleaner, which can remove various impurities, while at the same time introducing an oxidized surface (e.g. oxides at the surface, which may be covalently modified as described herein). Alternatively, liquid-phase treatments, such as a mixture of hydrochloric acid and hydrogen peroxide or a mixture of sulfuric acid and hydrogen peroxide (e.g., piranha solution, which may have a ratio of sulfuric acid to hydrogen peroxide from about 3:1 to about 7:1) may be used in place of an oxygen plasma cleaner.

In some embodiments, vapor deposition is used to coat the inner surfaces of the microfluidic device 200 after the microfluidic device 200 has been assembled to form an enclosure 102 defining a microfluidic circuit 120. Without intending to be limited by theory, depositing such a coating material on a fully-assembled microfluidic circuit 120 may be beneficial in preventing delamination caused by a weakened bond between the microfluidic circuit material 116 and the electrode activation substrate 206 dielectric layer and/or the cover 110. In embodiments where a two-step process is employed the surface modifying ligand may be introduced via vapor deposition as described above, with subsequent introduction of the moiety configured provide a layer of organic and/or hydrophilic molecules suitable for maintenance/expansion of biological micro-object(s). The subsequent reaction may be performed by exposing the surface modified microfluidic device to a suitable coupling reagent in solution.

Figure 2H depicts a cross-sectional view of a microfluidic device 290 having an exemplary covalently linked coating material providing a conditioned surface. As illustrated, the coating materials 298 (shown schematically) can comprise a monolayer of densely-packed molecules covalently bound to both the inner surface 294 of a base 286, which may be a DEP substrate, and the inner surface 292 of a cover 288 of the microfluidic device 290. The coating material 298 can be disposed on substantially all inner surfaces 294, 292 proximal to, and facing inwards towards, the enclosure 284 of the microfluidic device 290, including, in some embodiments and as discussed above, the surfaces of microfluidic circuit material (not shown) used to define circuit elements and/or structures within the microfluidic device 290. In alternate embodiments, the coating material 298 can be disposed on only one or some of the inner surfaces of the microfluidic device 290.

In the embodiment shown in Figure 2H, the coating material 298 can include a monolayer of organosiloxane molecules, each molecule covalently bonded to the inner surfaces 292, 294 of the microfluidic device 290 via a siloxy linker 296. Any of the above-discussed coating materials 298 can be used (e.g. an alkyl-terminated, a fluoroalkyl terminated moiety, a PEG- terminated moiety, a dextran terminated moiety, or a terminal moiety containing positive or negative charges for the organosiloxy moieties), where the terminal moiety is disposed at its enclosure-facing terminus (i.e. the portion of the monolayer of the coating material 298 that is not bound to the inner surfaces 292, 294 and is proximal to the enclosure 284).

In other embodiments, the coating material 298 used to coat the inner surface(s) 292, 294 of the microfluidic device 290 can include anionic, cationic, or zwitterionic moieties, or any combination thereof. Without intending to be limited by theory, by presenting cationic moieties, anionic moieties, and/or zwitterionic moieties at the inner surfaces of the enclosure 284 of the microfluidic circuit 120, the coating material 298 can form strong hydrogen bonds with water molecules such that the resulting water of hydration acts as a layer (or "shield") that separates the biological micro-objects from interactions with non-biological molecules (e.g., the silicon and/or silicon oxide of the substrate). In addition, in embodiments in which the coating material 298 is used in conjunction with coating agents, the anions, cations, and/or zwitterions of the coating material 298 can form ionic bonds with the charged portions of non-covalent coating agents (e.g. proteins in solution) that are present in a medium 180 (e.g. a coating solution) in the enclosure 284.

In still other embodiments, the coating material may comprise or be chemically modified to present a hydrophilic coating agent at its enclosure-facing terminus. In some embodiments, the coating material may include an alkylene ether containing polymer, such as PEG. In some embodiments, the coating material may include a polysaccharide, such as dextran. Like the charged moieties discussed above (e.g., anionic, cationic, and zwitterionic moieties), the hydrophilic coating agent can form strong hydrogen bonds with water molecules such that the resulting water of hydration acts as a layer (or "shield") that separates the biological micro-objects from interactions with non-biological molecules (e.g., the silicon and/or silicon oxide of the substrate).

Further details of appropriate coating treatments and modifications may be found at U.S. Application Serial No. 15/135,707, filed on April 22, 2016.

**Additional system components for maintenance of viability of cells within the sequestration pens of the microfluidic device**. In order to promote growth and/or expansion of cell populations, environmental conditions conducive to maintaining functional cells may be provided by additional components of the system. For example, such additional components can provide nutrients, cell growth signaling species, pH modulation, gas exchange, temperature control, and removal of waste products from cells.

**Additional system components for maintenance of viability of cells within the sequestration pens of the microfluidic device**. In order to promote growth and/or expansion of cell populations, environmental conditions conducive to maintaining functional cells may be provided by additional components of the system. For example, such additional components can provide nutrients, cell growth signaling species, pH modulation, gas exchange, temperature control, and removal of waste products from cells.

**Methods of loading**. Loading of biological micro-objects or micro-objects such as, but not limited to, beads, can involve the use of fluid flow, gravity, a dielectrophoresis (DEP) force, electrowetting, a magnetic force, or any combination thereof as described herein. The DEP force can be generated optically, such as by an optoelectronic tweezers (OET) configuration and/or electrically, such as by activation of electrodes/electrode regions in a temporal/spatial pattern. Similarly, electrowetting force may be provided optically, such as by an opto-electro wetting (OEW) configuration and/or electrically, such as by activation of electrodes/electrode regions in a temporal spatial pattern.

### Experimental

System and Microfluidic device. System and Microfluidic device: Manufactured by Berkeley Lights, Inc. The system included at least a flow controller, temperature controller, fluidic medium conditioning and pump component, light source for light activated DEP configurations, mounting stage for the microfluidic device, and a camera. T The microfluidic device was an OptoSelect^{™} device (Berkeley Lights, Inc.), configured with OptoElectroPositioning (OEP^{™}) technology. The microfluidic device included a microfluidic channel and a plurality of NanoPen^{™} chambers fluidically connected thereto, with the chambers having a volume of about 7X10⁵ cubic microns.

Priming regime. 250 microliters of 100% carbon dioxide was flowed in at a rate of 12 microliters/sec. This was followed by 250 microliters of a priming medium composed as follows: 1000 ml Iscove's Modified Dulbecco's Medium (ATCC^{®} Catalog No. 30-2005), 200 ml Fetal Bovine Serum (ATCC^{®} Cat. #30-2020), 10 ml penicillin- streptomycin (Life Technologies^{®} Cat. # 15140-122), and 10 mL Pluronic F-127 (Life Tech Catalog No. 50-310-494). The final step of priming included 250 microliters of the priming medium, flowed in at 12 microliters/sec. Introduction of the culture medium follows.

Perfusion regime. The perfusion method was either of the following two methods:
1. Perfuse at 0.01 microliters/sec for 2h; perfuse at 2 microliters/sec for 64 sec; and repeat.
2. Perfuse at 0.02 microliters/sec for 100 sec; stop flow 500 sec; perfuse at 2 microliters/sec for 64 sec; and repeat.

Barcoded nucleic acid capture beads: Beads were either polystyrene (16 micron) or magnetic (22 micron), Spherotech #SVP-150-4 or #SVM-200-4. Beads were modified to include oligonucleotides having a barcode as described herein. The barcoded beads may be synthesized in any suitable manner as is known in the art.

**Table 3. Primers used in this experiment.**

| SEQ ID No. | |
|---|---|
| 103 | /5Me-isodC//isodG//iMe-isodC/ACACTCTTTCCCTACACGACGCrGrGrG |
| 104 | 5'- ACACTCTTTCCCTACACGACGCTCTTCCGATCT |
| 105 | 5'-/5Biosg/ACACTCTTTCCCT ACACGACGC-3' |
| 106 | (5'- AATGATACGGCGACCACCGAGATCTACACTCTTTCCCTACACGACG CTCTTC C*G*A*T*C*T-3' |
| 107 | 5' -CAAGCAGAAGACGGCA T ACGAGA T -3' |
| 108 | 5'-AATGATACGGCGACCACCGA-3' |

**RNA sequencing:** The beads were modified to display an oligo(dT) capture sequence/Unique Molecular Identifier sequence/barcode/priming sequence. The barcode was selected to be unique for each bead. The oligo(dT) primer/Unique molecular identifier tag/Cell Barcode/primer sequence may be synthesized by total oligonucleotide synthesis, split and pool synthesis, ligation of oligonucleotide segments of any length, or any combination thereof. The oligo(dT) primer/Unique molecular identifier tag/Cell Barcode/primer sequence may be covalently attached directly or indirectly to the bead or may be attached non-covalently, e.g., via a streptavidin/biotin linker or the like. In this experiment, a fully synthesized oligonucleotide including the capture sequence, UMI, barcode and priming sequence was attached to the bead via a non-covalent biotin/streptavidin linkage.

**Example 1.** RNA capture, sequencing library preparation and sequencing results as demonstrated for OKT3 cells.

Cells: OKT3 cells, a murine myeloma hybridoma cell line, were obtained from the ATCC (ATCC^{®} Cat. # CRL-S001^{™}). The cells were provided as a suspension cell line. Cultures were maintained by seeding about 1x10⁵ to about 2x10⁵ viable cells/mL and incubating at 37°C, using 5% carbon dioxide in air as the gaseous environment. Cells were split every 2-3 days. OKT3 cell number and viability were counted and cell density is adjusted to 5x10⁵/ml for loading to the microfluidic device.

Culture medium: 1000 ml Iscove's Modified Dulbecco's Medium (ATCC^{®} Catalog No. 30-2005), 200 ml Fetal Bovine Serum (ATCC^{®} Cat. #30-2020) and 10 ml penicillin-streptomycin (Life Technologies^{®} Cat. # 15140-122) were combined to make the culture medium. The complete medium was filtered through a 0.22µm filter and stored away from light at 4°C until use.

When perfusing during incubation periods, the culture medium was conditioned continuously with 5% carbon dioxide in air before introduction into the OptoSelect device.

**Experiment:** A sample of OKT3 cells were introduced into the OptoSelect device at a density of 2E6 in 200 microliters. 250 of the cells were moved by optically actuated dielectrophoretic force to load one cell per NanoPen chamber. Each cell was positioned within the section of the chamber furthest from the opening to the microfluidic channel (e.g., isolation region). A single uniquely barcoded bead was subsequently loaded into each of the occupied chambers. The total number of beads loaded to the NanoPen chambers having single biological cells was 223, and each bead was also positioned within the portion of each chamber that was not subjected to penetrating fluidic flow. In this experiment, 256 uniquely barcoded beads were created, each having a total of 4 cassetable sequences. Diversity was created by selecting by selecting one of four possible sequences in a first position; one of four of a second, different set of four possible sequences in a second position, one of four of a third different set of four possible sequences in a third position and one of four of a fourth different set of four possible sequences in a four position within the barcode.

Lysis reagent (Single Cell Lysis Kit, Ambion Catalog No. 4458235) was flowed into the microfluidic channel and permitted to diffuse into the NanoPen chambers. The individually penned OKT3 cells were exposed to the lysis buffer for 10 minutes. Lysis was stopped by flowing in stop lysis buffer (Single Cell Lysis Kit, Ambion Catalog No. 4458235) and incubating for 2 minutes at room temperature while there was no flow in the microfluidic channel. (Similar results can be obtained using other lysis buffers, including but not limited to Clontech lysis buffer, Cat #635013, which does not require a stop lysis treatment step. Under the conditions used, the nuclear membrane was not disrupted. The released mRNA was captured onto the barcoded bead present within the same NanoPen chamber.

The captured RNA was reverse transcribed to cDNA by flowing in a RT reagent mixture (Thermo Scientific^{™} Maxima^{™} H Minus RT (Thermofisher, Catalog No. EP0751: 4 mciroliters of RT buffer; 2 microliters of 10 millimolar each of dNTPs (New England Biolabs Cat #NO447L; 2 microliters of 10 micromolar E5V6 primer (5Me-isodC//iisodG//iMeisodC/ACACTCTTTCCCTACACGACGCrGrGrG; SEQ ID No. 103); 1 microliter H Minus RT enzyme; 11 microliters of water). Alternatively, a Clontech SMARTscribeTM reverse transcriptase kit (Cat. # 639536), including enzyme, buffer and DTT can be used to obtain cDNA from the captured nucleic acid. Diffusion of the reagent mixture into the NanoPen Chamber was permitted during a 20 minute period at 16°C, followed by a reaction period of 90 minutes at 42°C.

After reverse transcription, a blank export of 12 microliters at 3microliters/sec was performed as negative control. This control was then processed separately but similarly to handling of the export group of beads as described below.

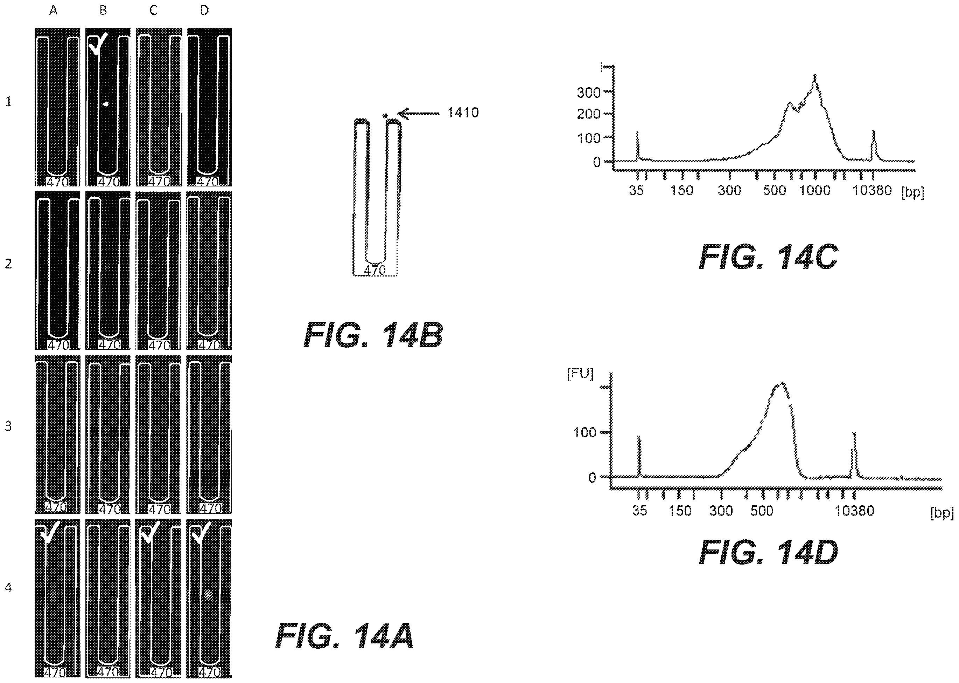

The unique Cell Barcode was then identified for each bead by multiplexed flows of fluorescently labeled hybridization probes as described above. Fluorescently labeled probes (provided in sets of four probes per reagent flow, each probe containing a different fluorophore and a non-identical oligonucleotide sequence from any of the other probes in the flow) were flowed, each group of four probes having distinguishable fluorescent labels, into the microfluidic channel of the microfluidic device at 1 micromolar diluted in 1 x DPBS from a 100 mM stock, and permitted to diffuse into the NanoPen chambers at 16°C over a period of 20 min, and then permitted to hybridize for 90 minutes at 42C.. (Alternatively, a different buffer solution, IDT Duplex buffer Cat. # 11-05-01-12 was also used successfully. Use of this buffer, which is nuclease free, and contains 30 mM Hepes, and 100 mM potassium acetate at pH7.5, also facilitated excellent duplex formation under these conditions.) After completion of the hybridization period, fresh medium (DPBS or Duplex buffer) was flowed through the microfluidic device for 20 min (300 microliters, at 0.25 microliter/sec) to flush unassociated hybridization probes out of the flow region of the microfluidic device. The flush period was selected to be long enough for unhybridized hybridization probes to diffuse out of each NanoPen chamber. Each distinguishable fluorescent wavelength (Cy5, FITC, DAPI, and Texas Red channels) was subsequently excited, and identification of which, if any of the NanoPen chambers demonstrated a fluorescent signal. The location and color of the fluorescent label of each probe localized to a NanoPen chamber was noted, and correlated to the known sequence and fluorescent label of the hybridization probes of the first reagent flow, and the identity of the corresponding cassetable sequence of the barcode on the bead was assigned. Successive additional reagent flows of further sets of fluorescently labeled hybridization probes, each having non-identical oligonucleotide sequences to each other and different from the sequences of the first and any other preceding reagent flows were flowed in as above and detection continued. Between each round of reagent flow and detection, flushing was performed using a first flush of 100 microliters of IX DPBS (Dulbecco's PBS), followed by a second 50 microliter flush of the same medium, both performed at 0.5 microliters/sec. To minimize misidentification of a cassetable sequence in a second or further reagent flow, only the first identified fluorescent signal of each distinguishable fluorophore was used to assign cassetable sequence identity for the barcode. Upon completing reagent flows totaling all of the cassetable sequences used in the barcodes of all the beads within the microfluidic device, the barcodes for all beads in the NanoPen chambers were assigned to each respective single NanoPen chamber. The assigned location of a specific barcode sequence assigned by this method was used to identify from which specific cell the RNA was captured to the bead, e.g., the location of the source nucleic acid within the Nanopen chambers of the microfluidic device. Figure 14A shows successive points in the process for one NanoPen chamber, #470. Each of the distinguishable fluorescent signal regions as shown at the top of each column labeled A-D. Each flow is shown vertically, labeled 1-4. After the probes of flow 1 have been allowed to hybridize, and flushing completed, the bead in NanoPen chamber 470 had a fluorescent signal only in color channel B. Detecting after the second reagent flow has been introduced, hybridization permitted, and flushing, no additional labels were detected. Note that, while NanoPen chamber #470 shows a signal during the second flow in the "B" fluorescence channel, each barcode and each probe was designed so that each barcode had only one cassetable sequence having each of the distinguishable fluorescent labels. This second signaling is not recorded as it represented first flow probe remaining bound to the bead. No additional cassetable sequences were identified by the probes of reagent flow 2, nor by reagent flow 3. However, fluorescent signal was identified in the fourth flow for each of the other three fluorescent channels. As a result, the barcode for the bead in NanoPen chamber 470, the barcode was identified as having the sequence correlated with A4B1C4D4 cassetable sequences. After detection, remaining hybridization probes were removed by flushing the flow region of the microfluidic device twice with 10mM Tris-HCl (200 microliters at 0.5microliters/sec), prior to further manipulation.

Optically actuated dielectrophoretic force was then used to export the barcoded beads from the NanoPen chambers into the flow region (e.g., flow channel) in a displacement buffer, 10 micromolar Tris, as shown in Figure 14B. The beads that were exported from the NanoPen chambers were exported out of the microfluidic device using flow and pooled. Positive control beads were present in the export group. After reverse transcriptase inactivation by incubation for 10 minutes at 80°C and treatment with Exonuclease I (NEB, catalog number M0293L) in Exo 1 buffer (17 microliters of exported beads, 1 microliter of exonuclease solution and 2 microliters of Exo I buffer), the export group of beads (20 microliter volume) was added to 5microliters of 10X Advantage 2 PCR buffer, dNTPs, 10 micromolar SNGV6 primer (5'- /5Biosg/ACACTCTTTCCCT ACACGACGC-3'; SEQ ID No. 105),1 microliter Advantage 2 polymerase mix; and 22 microliters water. This sequence was present both on the E5V6 primer and is present within the oligo on the beads and was used to amplify the cDNA, via single primer PCR to enrich for full length cDNA over shorter fragments. The cDNA was subjected to 18 cycles of DNA amplification (Advantage^{®} 2 PCR kit, Clontech, Catalog no. 639206).

Initial purification of the crude amplification mixture for the export group was performed using 0.6x SPRI (Solid Phase Reversible Immobilization) beads (Agencourt AMPure XP beads (Beckman Coulter, catalog no. A63881) according to supplier instructions. Quantification was performed (Bioanalyzer 2100, Agilent, Inc.) electrophoretically and/or fluorescently (Qubit^{™}, ThermoFisher Scientific) (Figure 14C) and showed acceptable recovery of amplified DNA. for use in before further library preparation performing one-sided tagmentation (Nextera XT DNA Library Preparation Kit, Illumina^{®}, Inc.), according to supplier instructions. After a second 0.6x SPRI purification, size selection was performed (Pre-Cast Agarose Gel Electrophoresis System. Ladder: 50 bp ladder (ThermoFisher, catalog no. 10488-099). E-gel^{®}: 2% Agarose (Thermofisher, catalog no. G501802). Gel Extraction Kit: QIAquick Gel Extraction Kit (Qiagen, #28704). Quantification was performed as above, providing a library having the appropriate 300- 800 bp size for sequencing. (Figure 14D)

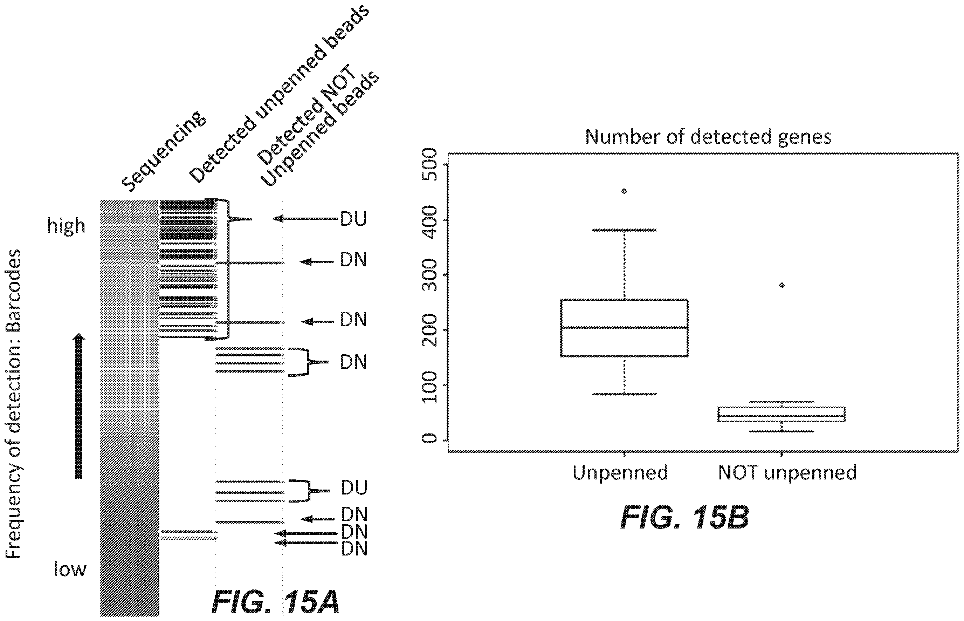

Sequencing was performed using a MiSeq Sequencer (Illumina^{®}, Inc.). Initial analysis of sequencing results indicated that data obtained from the blank control export looks different from the export group of DNA bearing beads, and the sequencing reads appear to be related with positive control sequences. (data not shown). Analyzing barcode identity within the blank control export, it was seen that most highly represented barcodes were derived from the positive control beads. (data not shown). Because the barcodes were linkable to a specific NanoPen chamber, comparison of cell barcodes showed that the Cell Barcodes from detected and exported beads ("unpenned") were far more represented than the Cell Barcodes that were detected but had not been exported from its specific NanoPen chamber location ("not unpenned"). As shown in Figure 15A, the heatmap representation showed a large group of detected barcodes from beads known to be exported from the NanoPen chamber ("unpenned"), labeled as "DU" Most of the detected DU barcodes were at higher y-axis locations of the heatmap designating more frequently identified sequences. The smaller set of detected barcodes that were known to be associated with beads that were not exported are shown in the column labeled "DN" (e.g., detected but not unpenned). Again, the vertical position of each DN barcode indicated its relative frequency of barcode sequence identification. Sequencing was performed using a MiSeq Sequencer (Illumina^{®}, Inc.) 55 cycles of sequencing was performed on read 1 to sequence 40bp of barcode and 10bp of UMIs. 4 additional cycles were required in between the first two "words" of the full-length barcode and the following two as 4 bp were used for barcode ligation in that specific experiment. The last cycle was used for base-calling purposes. An additional 8bp was sequenced, which represents the pool index added during the Nextera library preparation and allowed for multiplexing of several chip/experiments on the same sequencing run. Finally, an additional 46 cycles of sequencing were performed on read 2 (paired-end run) that provided the sequences of the cDNA (transcript/gene). Additional cycles are possible to be performed, depending on the sequencing kit used and the information desired. Figure 15B showed a boxplot depiction of the same data. Without being bound by theory, these cell barcodes from detected but not unpenned locations may have arisen as an artifact of primers and/or bead synthesis. Comparison of the representation of barcodes found in the sequencing data shows that the bead export sample looks significantly different from the barcodes retrieved from the blank export.

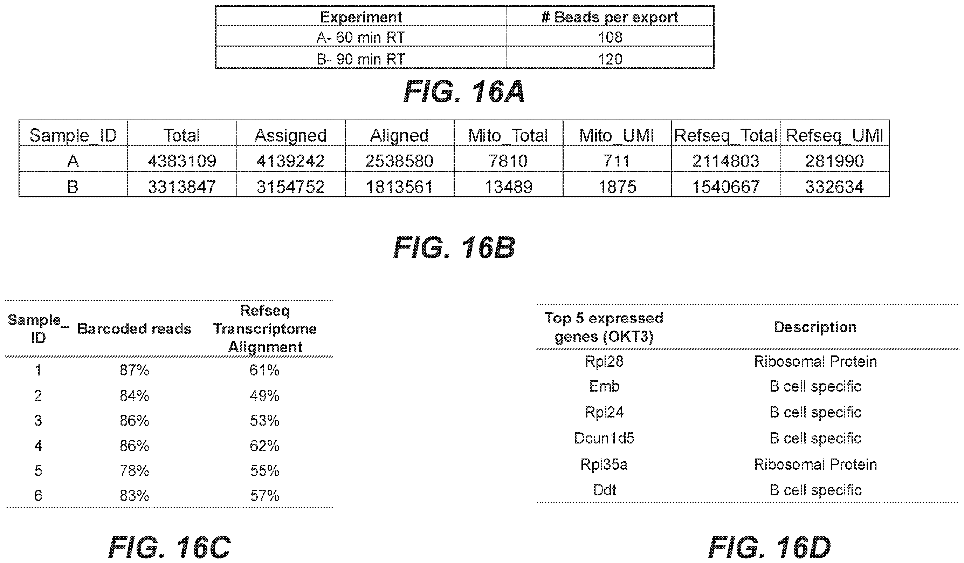

Figures 16A and B illustrate additional quality evaluations of the sequencing data and library preparation, using these methods. Figure 16A lists two different experiments, A and B, performed as above, differing in the length of time (60 min, 90 min) the reverse transcription step was performed. Experiment A included data from a DNA library resulting from export of 108 beads (capturing RNA from 108 cells). Experiment B included data from a DNA library resulting from export of 120 beads (capturing RNA from 120 cells). In Figure 16B, the Total column showed the total number of reads obtained from the sequencing data for each experiment. The Assigned column represented the number of reads which 1) map to a barcode and 2) have a sequencing quality above a preselected quality threshold. The Aligned column showed the number of reads that map to the genome of interest. Assigned reads that mapped to pseudogenes, mis-annotated genes, and intergenic regions which were not in the reference were removed to obtain this total. The Mito Total column included the number of reads mapping to a mitochondrial reference, which relate to cells in poor physiological condition, which usually express increased numbers of mitochondrial genes. The Mito UMI column represented the number of reads with distinct Unique Molecule Identifiers which mapped to the Mitochondrial reference. The Refseq Total column represented the number of reads aligned to the mRNA Refseq reference which the Refseq UMI column represented the number of reads with distinct UMIs aligned to the mRNA Refseq reference, and represented the original number of molecules captured by the capture beads upon lysis of the cell. All of these numbers indicate that the DNA libraries provided by these methods yield good quality sequencing data, representative of the repertoire of the cell.

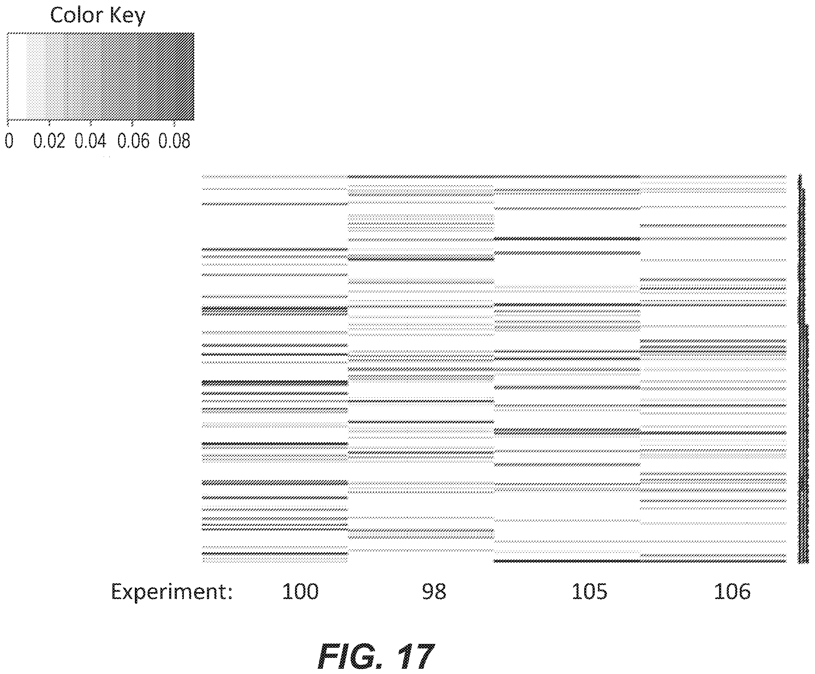

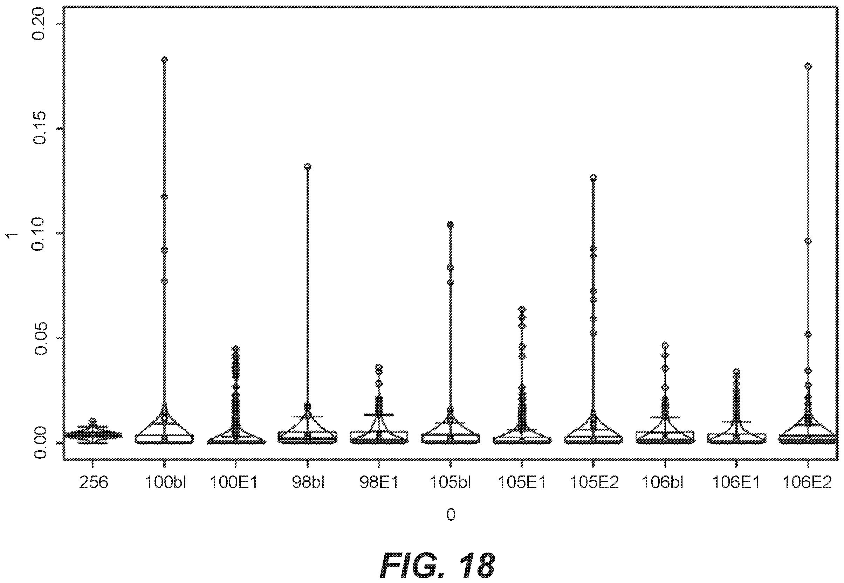

Some other analyses were used to evaluate the quality of the sequencing sample library. An off-chip experiment was conducted using 1ng of extracted total RNA from a pool of the same cells. cDNA was prepared using a mix of beads containing all 256 barcode combinations. The downstream processing was performed as described above, providing a bulk control, requiring no identification of barcodes. Equal amounts of input DNA were sequenced from each of these inputs. Comparison of the sequencing data obtained from these samples is shown in Figures 16C and D. The percentage of barcode reads that were identifiable within the sequencing data ranges from about 78% to about 87% of the total read number and the sequences covered by the sequencing reads ranged from about 49% to about 61% when aligned to the reference transcriptome. (Figure 16C). Finally, the top 5 expressed genes included RP128 (ribosomal protein); Emb (B cell specific); Rpl24 (B cell specific); Dcunld5 (B cell specific), Rp35a (ribosomal protein) and Ddt (B cell specific), which were consistent with the cell type and origin. (Figure 16D). Figure 17 showed that across experiments 100, 98, 105, 106, using 90 minute reverse transcription reaction periods, the sets of barcodes detected between each of the experiments varied, indicating good randomization of bead delivery to NanoPen chambers. The comparison of the off- chip experiment (labeled 256), blank (XXX-bl) and the exported bead data for each of four experiments (experiments 100, 98, 105, 106) is shown in Figure 18. Figure 18 showed retrieval of sequenced reads for a number of NanoPen chambers. For each experiment, XXX-E1 was a first export of cDNA decorated beads, and XXX-E2 was a subsequent second export from the same pens. The y axis of the violin plot of Figure 18 was the amount of barcode reads from each sample. The off-microfluidic device control 256 had all barcode represented equivalently. The exported bead data (XXX-E1 or XXX-E2) showed less than all barcodes represented and the amount of barcode reads also was less equivalently represented. Unsurprisingly, samples XXX-E2 showed even fewer reads, but with more variable numbers of those reads. Finally, blank reads showed, as discussed before, a very low number of barcode reads, but with one or two of the reads having a reasonable frequency of occurrence.

### Example 2. T cell phenotyping, culturing, assaying and RNA sequencing. Linkage of phenotype to genomic information.

The microfluidic system, materials and methods were the same as in Experiment 1, except for the following:
Cells: Control cells were human peripheral blood T cells. Sample cells were human T cells derived from a human tumor sample.

Culture medium: RPMI 1640 medium (Gibco, #12633-012), 10% Fetal Bovine Serum (FBS), (Seradigm, #1500-500); 2% Human AB Serum (Zen-bio, #HSER-ABP100ml), IL-2 (R&D Systems, 202-IL-010) 2U/ml; IL-7 (PeproTech, #200-07) 10ng/ml; IL-15 {PeproTech, #200-15) 10ng/ml, IX Pluronic F-127 (Life Tech Catalog No. 50-310-494).

Human T cells derived from a human tumor sample were stained with an antigen off-chip then introduced to the microfluidic channel of the OptoSelect device at a density of at a density of 5xE6 cells/ml. Both antigen positive T cells (P-Ag) and antigen negative cells (NAg) were moved by optically actuated dielectrophoretic force to isolate a single T cell into an individual NanoPen chamber, forming a plurality of populated NanoPen chambers.

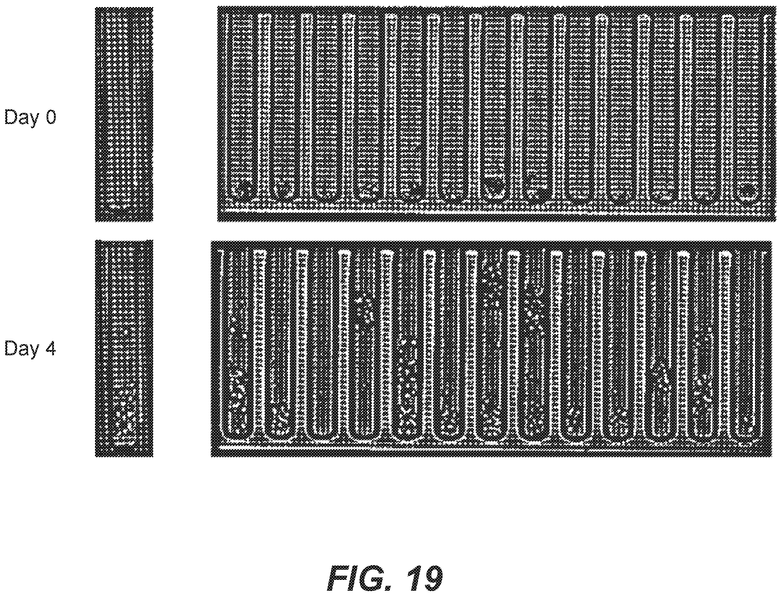

Human peripheral blood T cells were activated in the presence of CD3/28 beads (Dynabeads^{®} Human T-Activator CD2/CD28, ThermoFisher No. Gibco^{™} #11131D), during a four day culture period (Figure 19), forming an activated but not antigen specific population. Treatment with a labeled antigen did not result in labeled control- activated T cells. A population of these control activated T cells were introduced into the microfluidic channel at a density of 5xE6 cells/ml and a selected plurality of the control activated T cells were moved by optically actuated dielectrophoretic force to place a single control activated T cell into each of a plurality of Nanopen chambers, which were different from the set of NanoPen chambers containing the set of T cells derived from the tumor sample.

To each occupied chamber, were added a single barcoded bead which were synthesized via ligation (in this specific experiment). Each bead included a priming sequence, a barcode sequence, a UMI sequence, and a capture sequence as described above. Lysis and capture of RNA followed, as described above. Under the conditions used, the nuclear membrane is not disrupted. The released mRNA was captured onto the barcoded bead present within the same NanoPen chamber.

In Figures 20A, 21A, and 22A, a set of four photographic images illustrates representative occupied Nanopen chambers. Each set of the photographs, from left to right, showed: 1) brightfield illumination of a T cell after placement into the NanoPen chamber using optically actuated dielectrophoretic force; 2) fluorescent detection (Texas Red channel) probing for antigen- specific staining; 3) brightfield illumination of the Nanopen chamber after one barcoded capture bead was imported using optically actuated dielectrophoretic force; and 4) brightfield illumination after lysis. As above, the lysis conditions ruptured the cell membrane but did not disturb the nuclear membrane.

In Figure 20A, a NanoPen chamber having a location identifier of 1446, was shown to be occupied by one cell. This cell was an antigen positive stained cell (P-Ag), as shown by the second photograph of the set of Figure 20A, having a fluorescent signal (shown within the white circle within the NanoPen chamber). The third photograph of the set of Figure 20A showed that a single bead was placed within the NanoPen chamber, and the fourth photograph of the set of Figure 20A shows that the bead and the remaining nucleus was still located within the NanoPen chamber. In Figure 21A, similar placement of a cell (first photograph of the second set) and a bead (third photograph of the second set) into the NanoPen chamber No. 547 was shown. However, this cell did not stain with the antigen; and t no fluorescent signal was detected in the second photograph of the set of photographs of Figure 21A. Therefore, this cell was identified as an antigen negative T cell (N-Ag). In Figure 22A, an equivalent set of photographs was shown for NanoPen chamber, 3431, containing a control activated T cell. As expected, there was no fluorescent signal in the second photograph of the set, corroborating that this cell is not antigen positive.

**RNA release, capture, library prep and sequencing.** The protocol described in Example 1 for reverse transcription and barcode reading within the microfluidic environment was performed and identification of the barcode for each NanoPen chamber was recorded. In Figures 20B, 21B and 22B, images of the barcode detection process of the respective NanoPen chambers are shown. NanoPen chamber 1446 was determined to have a bead containing the barcode A1B1C1D4; NanoPen chamber 547 had a bead having the barcode A1B3C3D4; and NanoPen chamber 3431 had a bead containing the barcode A2B3C4D4. Bead export, and off chip amplification, tagmentation, purification, and size selection of the cDNA from the exported decorated beads was performed as described in Example 1.

Sequencing was performed using a MiSeq Sequencer (Illumina^{®}, Inc.) A first sequencing read sequenced 55 cycles on read 1 to sequence 40bp of barcode and 10bp of UMIs. 4 additional cycles were required in between the first two cassetable sequences and the last two cassetable sequences of the full-length barcode as an additional 4 bp were used for barcode ligation in this specific experiment. The last cycle was used for base-calling purposes. A second sequencing read sequenced 8bp representing the pool index added during the Nextera library preparation, allowing for multiplexing of several experiments on the same sequencing run. Finally, an additional 46 cycles was sequenced on read 2 (paired-end run) that provides sequencing of the cDNA (transcript/gene). Longer reads may be obtained, if desired, but was not used in this experiment.

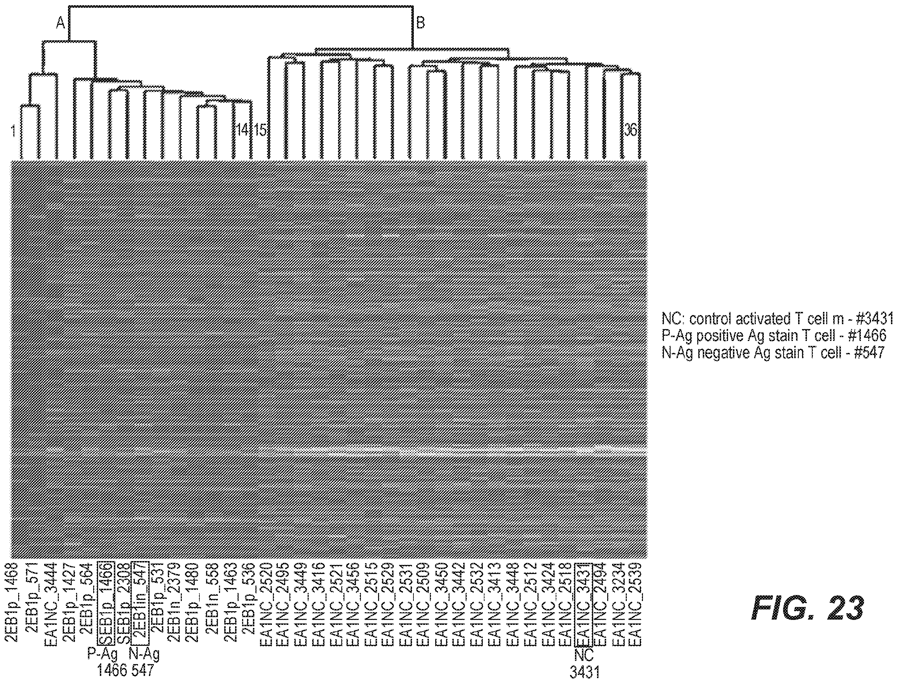

Figure 23 shows the heat map of the sequencing results of this experiment, having columns of sequencing reads, each column representing RNA captured to a single bead from the one cell in the NanoPen chamber, which was 1) tumor antigen exposed, positive for Antigen; 2) tumor antigen exposed, negative for antigen; or 3) negative control, activated T cell but not antigen exposed. The columns are arranged according to their similarity in sequencing reads, which is correlated with gene expression information. The color (dark vs light bands) represented the level of expression. Columns 1-14 were more closely related to each other than to Columns 15-36. Since the readable barcodes were identifiable for each column (each bead, from one cell), the location from which the bead was retrieved was determined, and, the phenotype of the cell from which the RNA was sourced. For example, the three beads identified above, from NanoPen chambers 1446 (labelled 2EB1p_1466 (P-Ag), found at column #6 within group A), 547 (2EB1n_547 (N-Ag), found at column #8 within Group A), and 3431 (labelled EA1NC_3431 (NC) found at column #33 in Group B), provided gene expression profiles shown at the respective highlighted and labeled columns. The difference between the gene expression for columns 15-36 (clustered in group B in the relationship bracket at the top of the heat map, and that of Columns 1-14 (group A) was seen to be substantially dependent on exposure to the tumor antigen. The source cells for substantially all the columns 1-14 of group A had been exposed to tumor antigen, whether positive or negative for antigen staining. In contrast, all of the source cells of Columns 15-36, were negative control cells, and had not been exposed to tumor antigen. Each column represents sequencing reads for one experiment and the color represents the level of expression. The sequencing reads of each of the bead-activated, antigen nonspecific control T cell (NC) were clearly differentiable from either of the sequencing reads of an antigen-positive tumor derived T cell (P-Ag) or an antigen- negative tumor derived (N-Ag) T cell. Specific and differentiable single cell RNA sequencing was demonstrated. Further, it was shown that phenotypic information was linkable to the gene expression profile for a single cell

**Example 3. DNA capture, sequencing library preparation and sequencing results as demonstrated for OKT3 cells.** Apparatus, priming and perfusion regimes, cell source and preparation were used/performed as in the general methods above, unless specifically noted in this example. The media and OptoSelect device were maintained at 37°C, unless otherwise specified.

**Table 4. Primers for use in this experiment.**

| SEQ ID No. | Sequence/s |
|---|---|
| 109 | BiotinTEG_N701 /5BiotinTEG/CAAGCAGAAGACGGCATACGAGATTCGCCTTAGTCTCG TGGGCTCG*G |
| 110 | BiotinTEG_N702 /5BiotinTEG/CAAGCAGAAGACGGCATACGAGATCTAGTACGGTCTCG TGGGCTCG*G |
| 111 | BiotinTEG_S506 /5BiotinTEG/AATGATACGGCGACCACCGAGATCTACACACTGCATAT CGTCGGCAGCGT*C |

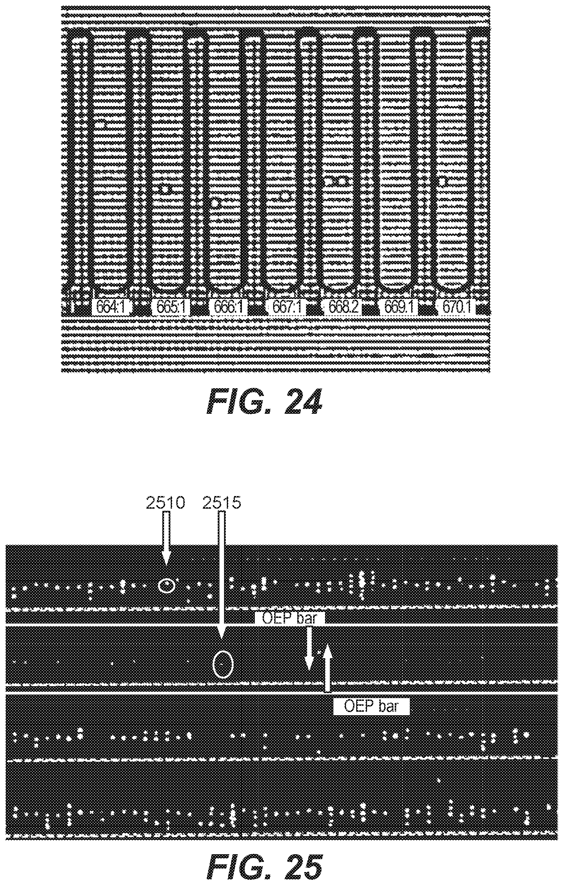

This experiment demonstrated that Nextera sequencing libraries (Illumina) can be generated with isothermal PCR using one biotinylated priming sequence (carrying a barcode) attached to a bead and one primer free in solution. OKT3 cells (150) were imported into the OptoSelect device, and loaded using optically actuated dielectrophoretic forces into NanoPen chambers. Figure 24 showed the cells after delivery to the NanoPen chambers. The optically actuated dielectrophoretic forces delivered one cell per NanoPen chamber for 7 NanoPen chambers, missed delivering a cell to one NanoPen chamber, and delivered 2 cells to one NanoPen chamber, thereby delivering substantially only one cell per NanoPen chamber.

**Lysis**. The lysis procedure was performed using an automated sequence, but may be suitably performed via manual control of each step. Lysis buffer was flowed into the OptoSelect device (Buffer TCL (Qiagen, Catalog # 1031576) and flow was then stopped for 2 mins to permit buffer diffusion into the pens. Lysis of both the cell membrane and the nuclear membrane was effected. The OptoSelect device was then flushed three times with 50 microliters of culture media, including a 30 second pause after each 50 microliter flush. Proteinase K (Ambion Catalog # AM2546, 20 mg/ml) at a concentration of 800 micrograms/milliliter was introduced to the OptoSelect device and maintained without perfusion for 20 min. Proteinase K diffused into the NanoPen chamber and proteolyzed undesired proteins and disrupted chromatin to permit gDNA extraction. After completion, the OptoSelect device was flushed with three cycles of 50 microliters of PBS including 10 min hold periods after each flow.

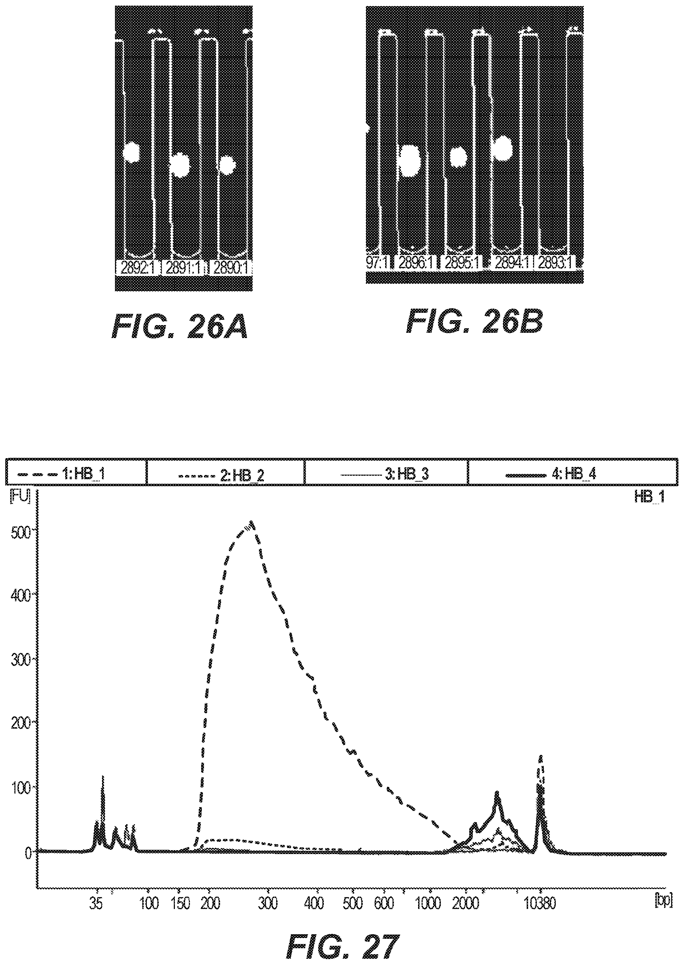

Staining with SYBR^{®} Green I stain (ThermoFisher Scientific, Catalog # S7585), at 1:1000 in 1xPBS, was performed to demonstrate that compacted DNA 2510 of the nucleus was present, as shown in Figure 25. Additionally, a sweep using optically actuated dielectrophoresis forces scanning vertically in both directions (up and down, crossing over) through the NanoPen chambers was performed. In figure 25, the two light patterns ("OEP bars") are shown that were used to create the vertical "crossover" sweep. This resulted in a blurred and enlarged area of fluorescent signal from released DNA 2515 of the nucleus, demonstrating the ability to drag the compacted DNA from the compacted form to a larger, more dispersed area, indicating lysis of the nuclear membrane. Figure 26A shows a photograph of a set of specific NanoPen chambers each containing a stained OKT3 cell before lysis, and Figure 26B shows a photograph of the same NanoPen chambers after the OEP sweep, demonstrating dispersion of the stain (e.g., DNA) to a larger area within the chamber.

**Tagmentation.** Tagmentation of DNA with transposase. A protocol for tagmentation was followed by introducing a 15 microliter volume of transposome reagents (Nextera DNA Library Prep Kit, Illumina, Cat. # 15028212) including 3.3 microliters of Tagment DNA Buffer (TD); 16 microliters of Tagment DNA enzyme mix (TDE1 Buffer); and 14 microliters of nuclease free H₂O (Ambion Cat. # AM9937) into the OptoSelect device. The tagmentation reagents diffused into the Nanopen chambers over a 15 minute period. The OptoSelect device was then flushed extensively, including clearing the inlet and outlet lines with 100 microliters of PBS, and flushing the device itself with 50 microliters of PBS. Figure 27 shows graphical distribution (Bioanalyzer, Agilent) of the size of tagmented products obtained via this protocol, with a maximum of the distribution just under 300bp and little of the tagmented products having a size greater than about 600 bp, which demonstrated suitability for massively parallel sequencing methods.

**DNA capture to beads.** Biotinylated 16 micron polystyrene capture beads (Spherotech) were modified by streptavidin labelled oligonucleotides. The oligonucleotides included a priming sequence, a barcode sequence, and a capture sequence (e.g., mosaic sequence), in 5' to 3' order. The barcode sequence contained at least one sub-barcode module, permitting identification of the source cell within a specific NanoPen chamber of the OptoSelect device. The priming sequence incorporated within the oligonucleotide was P7 (P7 adaptor sequence), in this experiment. (However, other priming sequences may be utilized such as P5 or a priming sequence specifically designed for compatibility with the recombinase and polymerase of the RPA process. After binding with an excess of SA- oligonucleotide for 15 min in a binding buffer includinglM NaCl, 20 mM TrisHCl, 1 mM EDTA and 0.00002 % Triton-X with agitation at speeds up to about 300rpm (VWR Analog vortex mixer), the beads were washed with three aliquots of fresh binding buffer, followed by 50 microliters of PBS. Freshly prepared beads containing P7 priming sequence(sequencing adaptor)/barcode/ capture sequence oligonucleotides were delivered to the NanoPen chambers which had contained cells prior to lysis. This step was performed using an automated sequence including OEP delivery to the NanoPen chambers, but may also be performed manually if desired. In this experiment, the specific automated process used took 1h to complete. More rapid delivery can be advantageous. Additionally, reduced temperature below 37° C may be advantageous for effective DNA capture.

**Isothermal Amplification.** Isothermal amplification of the captured DNA on the beds was performed using a recombinase polymerase amplification (RPA) reaction, (TwistAMP TABA S03, TwistDX,), also including a single-strand DNA (ss-DNA) binding protein which stabilizes displacement loops (D-loops) formed during the process. Also present in the reaction mixture were P5-Mosaic sequence, P7 and P5 primers (IDT). The following mixture: dry enzyme pellet of the TwistDx kit; 27.1 microliters of resuspension buffer; 2.4 microliters of 10 micromolar P5 primer; 2.4 microliters of 10 micromolar P7 primer; and 2.4 microliters of 10 micromolar P5 end index primer (e.g. S521) was added to 2.5 microliters of 280 millimolar magnesium acetate (MgOAc) and vortexed within a microfuge tube. Fifteen microliters of this solubilized and spun solution were imported into the microfluidic channel of the OptoSelect device at a rate of 1 microliter/second, and permitted to diffuse into the NanoPen chambers and contact the captured DNA on the beads for 40 to 60 minutes.

After completion of the isothermal amplification period, 50 microliters of fluidic medium were exported from the OptoSelect device, using PBS. The exported solution ("Immediate Export") containing amplified DNA that had diffused out of the NanoPen chambers) was cleaned up using 1x AMPure^{®} beads (Agencourt Bioscience), removing primers and other nucleic acid materials of less than 100bp size.

The OptoSelect Device still containing beads and amplified DNA that did not diffuse into the channel was maintained at 4°C overnight, and a second export using 50 microliters of PBS was made, capturing amplification product then present in the microfluidic channel, "2^{nd} Export". The two samples were further separately amplified via PCR for quantitation and size analysis, each using 5 cycles PCT in a 25 microliter reaction with KAPA HiFi Hotstart (KAPA Biosystems), 1 microliter of 10 micromolar P5 primer, and 1 microliter of 10 micromolar P7 primer. Each of the Immediate Export and 2^{nd} Export samples were cleaned up to remove primers by repeating treatment with 1x AMPure^{®} beads. The Immediate Export sample yielded 40 ng total having fragment sizes suitable for sequencing, having an average size of about 312 bp (data not shown). The 2^{nd} Export sample yielded 85 ng total, with an average size of about 760 bp (data not shown), which were not suitable for further sequencing by NGS parallel techniques.

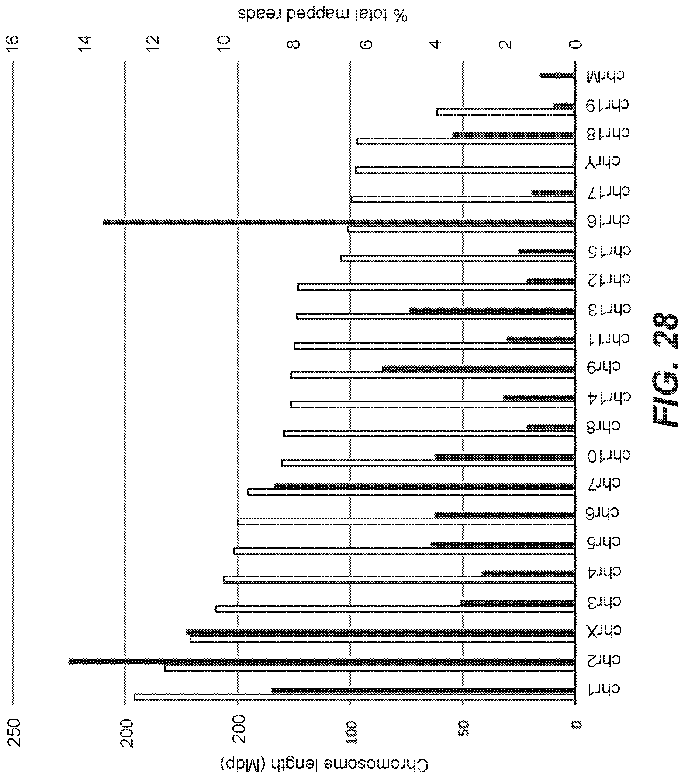

The Immediate Export sample was sequenced within a shared Miseq massively parallel sequencing experiment (Illumina). The coverage was low (mean = 0.002731), but reads mapped throughout the mouse genome, as shown in Figure 28. In Figure 28, each chromosome is displayed along the x axis. The left-hand light colored bar represents the expected length of each chromosome while the right hand dark colored bar represents the percentage of total mapped reads seen in the data from the Immediate Export sample. While some chromosomes were overrepresented in the data (chr 2, chr 16), other chromosomes were underrepresented (chr 8, chr 12, chr 15). Note that no reads were obtained for the Y chromosome, as expected, as the cells originated from a female mouse. Acceptably low level of adaptor contaminants (0.000013%) were identified. Additionally, particular sequences of interest were also found in the data (e.g. CXCR4 sequence, data not shown).

**Example 4. DNA isolation, library preparation and sequencing of a mixture of OKT3 cells and human LCL1 cells from human B-Lymphocyte.** Source of LCL1 cells: Coriell Institute. Catalog number: GM128781C. Media used for culture is RPMI-1640 (Life Technologies, Cat #11875-127), 10% FBS, 1% Pen/Strep (1000 U/ml), 2 mM Glutamax.

Experiments used either 150 OKT3 cells: 150 Hu LCL1 cells or 75 OKT3 cells:75 Hu LCL1 cells. The cells were specifically delivered to individual NanoPen chambers, one cell to a chamber, using OEP forces such that the locations of each OKT3 and each Hu LCL1 cell was known.

The process of lysis and tagmentation, was performed as in Experiment 3, but with Mosaic End plus insert sequences appended to the fragmented DNA by the transposase having one of the following sequences:

Tn5ME-A (Illumina FC-121-1030), 5′-TCGTCGGCAGCGTCAGATGTGTATAAGAGACAG-
3′ (SEQ ID No. 161);

Tn5ME-B (Illumina FC-121-1031), 5′GTCTCGTGGGCTCGGAGATGTGTATAAGAGACAG-
3′ (SEQ ID NO.162)

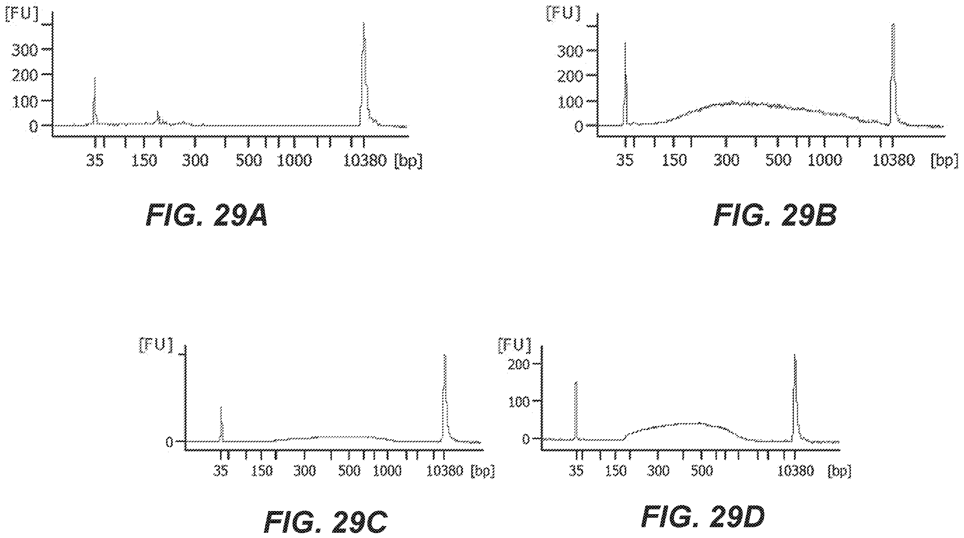

Specific delivery was made of a first set of barcoded beads having a first unique barcode only to the OKT3 cell- containing NanoPens, followed by specific delivery of a second set of barcoded beads having a second unique barcode only to the Hu LCL1 cell- containing NanoPens. This provided specific identifiers for DNA amplified from each set of beads, so that murine DNA reads could be mapped back to murine cells, and Hu LCLlcell DNA reads could be mapped back to human cells. The beads were delivered to the NanoPen chambers after the tagmentation step. Isothermal amplification was performed as in Experiment 3, yielding 5.67ng (from 300 cells total) or 2.62ng (from 150 cells total). Two cycles of PCR, run as described above were performed on each library to ensure the presence of P5, P7 for NGS sequencing, and clean up was similarly performed. Figures 29A (from 150 cells) and 29B (from 300 cells) shows the two (OKT3/hu LCL1) DNA libraries respectively after the subsequent two cycle PCR amplification and clean up. These results can be compared to size distribution traces of control libraries that were generated from OKT3cells and also hu LCL1 cells processed individually in a standard well plate format, as shown in Figure 29C (OKT3) and Figure29D (hu LCL1.) The comparison indicates that further optimization may be desirable to obtain more ideal fragment distribution within the microfluidic protocol. The sequencing results from the mixed OKT3/hu LCL1 DNA libraries showed that reads having each of the two barcodes were obtained (data not shown).

**In-situ barcode detection.** After export of amplified DNA products, sequential flow of fluorescently labeled hybridization probes as described above identified barcode position.

**Example 5. Introduction of the barcoded beads and in-situ detection of the barcoded beads within the DNA isolation, library preparation and amplification workflow sequence**. Without wishing to be bound by theory, the activity of transposon is directed towards double stranded nucleic acids, not single stranded bound oligos. The robustness of the capture beads to these conditions was shown in a corollary experiment. Beads, 20 microliters, as prepared for Experiment 3 were exposed to the tagmentation reaction reagents under the conditions for that same experiment. Both transposon-exposed beads and non- exposed control beads were contacted with 1.4 ng of human standard DNA. 2.4 microliters of each bead set were used in an isothermal amplification, using 2.4 microliters of a paired primer (S521, Illumina) in RPA (S521), and provided substantially similar amounts of amplified DNA. The results show that the capture beads exposed to transposon prior to use in DNA capture yielded reasonably equivalent amounts of amplified product, indicating that transposon did not degrade the capture oligonucleotides on the capture bead.

**Table 5. Comparison of yield between beads exposed to tagmentation reaction conditions and unexposed beads, after isothermal amplification.**

| Condition | Isothermal yield (ng/microliter) |
|---|---|
| Transposon- exposed | 36.4 |
| Non-exposed (control) | 33.4 |
| Transposon-exposed | 24.4 |
| Non-exposed (control) | 31.8 |

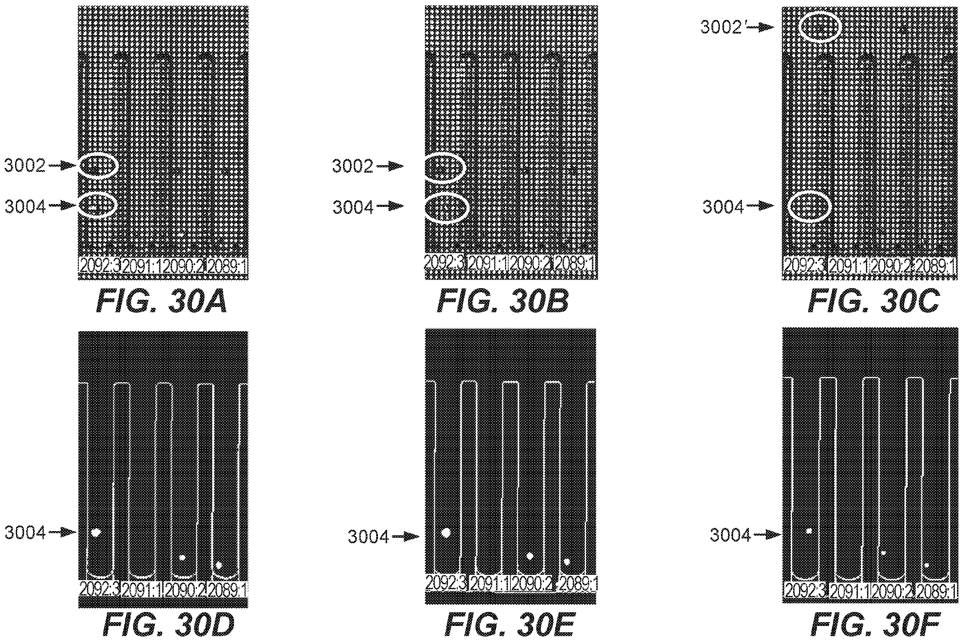

**Example 6. Sequencing nuclear DNA from the same cells for which RNA sequencing has been performed.** Figures 30A-F each show a row of four NanoPen chambers of an OptoSelect device, containing OKT3 cells and capture beads. The series of photographs were taken during the course of a protocol in which RNA capture, tagmentation and export had been performed, as in Experiment 1. NucBlue^{®} LiveReady Probes^{®} Reagent (Molecular Probes, R37605) stain was added to the cells before import (two drops are added to 200 microliters of cell solution just prior to import). No additional stain was added throughout the protocol. Nuclear dsDNA of each cell was stained and the staining was maintained throughout the steps of RNA capture, tagmentation, and reverse transcription. Figures 30A-30C were taken under brightfield conditions, and Figures 30D to 30F were taken under UV excitation illumination (excitation at 360 nm when bound to DNA, with an emission maximum at 460 nm) and visualized through a DAPI filter at 400- 410 nm. Figures 30A and 30D are paired images of the same Nanopen Chamber containing cell at a timepoint prior to lysis, under brightfield and DAPI filter exposure respectively. 3002 is a barcoded bead and 3004 is a biological cell within the same NanoPen chamber. Other beads and other cells in other of the four NanoPen chambers of the figures are also visible, but not labeled. Figures 30B and 30E are paired images of the same NanoPen chamber as shown in Figures 30A and 30C, under brightfield and 400 nm illumination respectively. These photographs were taken after outer membrane lysis as described in Experiment 1 was completed. The nuclear DNA 3004 still is visible under the 400nm excitation illumination (Figure 30E) as well as the shape of the nucleus 3004 still remaining under brightfield along with the bead 3002. Figure 30C and 30F are paired images, brightfield, and 400nm excitation illumination respectively, for the same cells within the same group of four NanoPen chambers as Figures 30A-30D. The photographs of Figures 360C and 30F were taken after reverse transcription was complete and the cDNA decorated barcoded beads 3002'were exported out of each NanoPen chamber (see 3002 within the microfluidic channel at the top of the photograph). The compact nucleus 3006 is still visible under brightfield, and Figure 30F shows that the nucleus 3006 still contained nuclear DNA. Since no additional dye was added, there was no staining of the cDNA produced upon the beads.

Figures 30A- 30F indicate that by using the protocols described herein for RNA capture/library prep, and DNA capture/library prep, the compact nucleus still was a viable source of nuclear dsDNA for DNA library production. Therefore, sequencing results for both RNA and DNA may be obtained from the same single cell, and may be correlated to the location within the OptoSelect device of the specific single cell source of the sequenced RNA and DNA. This ability to correlate the location of the single cell source of RNA/DNA sequencing results may further be correlated to phenotypic observations of the same single cell, such as cells producing antibodies to specific antigens.

The step of introduction of the barcoded priming sequence bearing beds was shown to be suitably performed either prior to the tagmentation step or after tagmentation (as in Experiment 3).

Additionally, the step of reading the barcode(s) on barcoded beads placed within the NanoPen chambers may be performed before tagmentation, before isothermal amplification, prior to export of amplified DNA, or after export of amplified DNA (as shown in Exp. 4). Alternatively, beads may be placed within the NanoPen chambers before importation of biological cells. The barcodes may also be detected before biological cells are brought into the microfluidic environment.

### Example 7. B-cell receptor (BCR) capture, sequencing library preparation and sequencing results as demonstrated for OKT3 cells and OKT8 cells.

**Cells:** OKT3 cells, a murine myeloma hybridoma cell line, were obtained from the ATCC (ATCC^{®} Cat. # CRL-8001^{™}). The cells were provided as a suspension cell line. Cultures were maintained by seeding about 1x10⁵ to about 2x10⁵ viable cells/mL and incubating at 37°C, using 5% carbon dioxide in air as the gaseous environment. Cells were split every 2-3 days. OKT3 cell number and viability were counted and cell density is adjusted to 5x10⁵/ml for loading to the microfluidic device.

OKT8 cells, a murine myeloma hybridoma cell line, were obtained from the ATCC (ATCC^{®} Cat. # CRL-8014^{™}). The cells were provided as a suspension cell line. Cultures were maintained by seeding about 1x10⁵ to about 2x10⁵ viable cells/mL and incubating at 37°C, using 5% carbon dioxide in air as the gaseous environment. Cells were split every 2-3 days. OKT8 cell number and viability were counted and cell density is adjusted to 5x10⁵/ml for loading to the microfluidic device.

**Culture medium:** Iscove's Modified Dulbecco's Medium (For OKT3; ATCC^{®} Catalog No. 30-2005, for OKT8; ATCC^{®} Catalog No. 30-2005), 10 % Fetal Bovine Serum (ATCC^{®} Cat. #30-2020) and 10 ml penicillin- streptomycin (Life Technologies^{®} Cat. # 15140-122) were combined to make the culture medium. The complete medium was filtered through a 0.22µm filter and stored away from light at 4°C until use.

When perfusing during incubation periods, the culture medium was conditioned continuously with 5% carbon dioxide in air before introduction into the OptoSelect device.

**Table 6. Oligonucleotide sequences for use in the experiment.**

| SEQ ID No. | Sequence/s | Name |
|---|---|---|
| 112 | 5'-Me-isodC//Me-isodG//Me- isodC/ACACTCTTTCCCTACACGACGCrGrGrG-3 | |
| 113 | 5'-ACACTCTTTCCCT ACACGACGC-3' | |
| 114 | GTT ATT GCT AGC GGC TCA GCC GGC AAT GGC GGA KGT RMA GCT TCA GGA GTC | YarivH-FOR 1 |
| 115 | GTT ATT GCT AGC GGC TCA GCC GGC AAT GGC GGA GGT BCA GCT BCA GCA GTC | YarivH-FOR 2 |
| 116 | GTT ATT GCT AGC GGC TCA GCC GGC AAT GGC GGA GGT BCA GCT BCA GCA GTC | YarivH-FOR 3 |
| 117 | GTT ATT GCT AGC GGC TCA GCC GGC AAT GGC GGA GGT CCA RCT GCA ACA RTC | YarivH-FOR 4 |
| 118 | GTT ATT GCT AGC GGC TCA GCC GGC AAT GGC GCA GGT YCA GCT BCA GCA RTC | YarivH-FOR 5 |
| 119 | GTT ATT GCT AGC GGC TCA GCC GGC AAT GGC GCA GGT YCA RCT GCA GCA GTC | YarivH-FOR 6 |
| 120 | GTT ATT GCT AGC GGC TCA GCC GGC AAT GGC GCA GGT CCA CGT GAA GCA GTC | YarivH-FOR 7 |
| 121 | GTT ATT GCT AGC GGC TCA GCC GGC AAT GGC GGA GGT GAA SST GGT GGA ATC | YarivH-FOR 8 |
| 122 | GTT ATT GCT AGC GGC TCA GCC GGC AAT GGC GGA VGT GAW GYT GGT GGA GTC | YarivH-FOR 9 |
| 123 | GTT ATT GCT AGC GGC TCA GCC GGC AAT GGC GGA GGT GCA GSK GGT GGA GTC | YarivH-FOR 10 |
| 124 | GTT ATT GCT AGC GGC TCA GCC GGC AAT GGC GGA KGT GCA MCT GGT GGA GTC | YarivH-FOR 11 |
| 125 | GTT ATT GCT AGC GGC TCA GCC GGC AAT GGC GGA GGT GAA GCT GAT GGA RTC | YarivH-FOR 12 |
| 126 | GTT ATT GCT AGC GGC TCA GCC GGC AAT GGC GGA GGT GCA RCT TGT TGA GTC | YarivH-FOR 13 |
| 127 | GTT ATT GCT AGC GGC TCA GCC GGC AAT GGC GGA RGT RAA GCT TCT CGA GTC | YarivH-FOR 14 |
| 128 | GTT ATT GCT AGC GGC TCA GCC GGC AAT GGC GGA AGT GAA RST TGA GGA GTC | YarivH-FOR 15 |
| 129 | GTT ATT GCT AGC GGC TCA GCC GGC AAT GGC GCA GGT TAC TCT RAA AGW GTS TG | YarivH-FOR 16 |
| 130 | GTT ATT GCT AGC GGC TCA GCC GGC AAT GGC GCA GGT CCA ACT VCA GCA RCC | YarivH-FOR 17 |
| 131 | GTT ATT GCT AGC GGC TCA GCC GGC AAT GGC GGA TGT GAA CTT GGA AGT GTC | YarivH-FOR 18 |
| 132 | GTT ATT GCT AGC GGC TCA GCC GGC AAT GGC GGA GGT GAA GGT CAT CGA GTC | YarivH-FOR 19 |
| 133 | AGC CGG CCA TGG CGG AYA TCC AGC TGA CTC AGC C | YarivL-FOR1 |
| 134 | AGC CGG CCA TGG CGG AYA TTG TTC TCW CCC AGT C | YarivL-FOR2 |
| 135 | AGC CGG CCA TGG CGG AYA TTG TGM TMA CTC AGT C | YarivL-FOR3 |
| 136 | AGC CGG CCA TGG CGG AYA TTG TGY TRA CAC AGT C | YarivL-FOR4 |
| 137 | AGC CGG CCA TGG CGG AYA TTG TRA TGA CMC AGT C | YarivL-FOR5 |
| 138 | AGC CGG CCA TGG CGG AYA TTM AGA TRA MCC AGT C | YarivL-FOR6 |
| 139 | AGC CGG CCA TGG CGG AYA TTC AGA TGA YDC AGT C | YarivL-FOR7 |
| 140 | AGC CGG CCA TGG CGG AYA TYC AGA TGA CAC AGA C | YarivL-FOR8 |
| 141 | AGC CGG CCA TGG CGG AYA TTG TTC TCA WCC AGT C | YarivL-FOR9 |
| 142 | AGC CGG CCA TGG CGG AYA TTG WGC TSA CCC AAT C | YarivL-FOR10 |
| 143 | AGC CGG CCA TGG CGG AYA TTS TRA TGA CCC ART C | YarivL-FOR1 1 |
| 144 | AGC CGG CCA TGG CGG AYR TTK TGA TGA CCC ARA C | YarivL-FOR12 |
| 145 | AGC CGG CCA TGG CGG AYA TTG TGA TGA CBC AGK C | YarivL-FOR13 |
| 146 | AGC CGG CCA TGG CGG AYA TTG TGA TAA CYC AGG A | YarivL-FOR14 |
| 147 | AGC CGG CCA TGG CGG AYA TTG TGA TGA CCC AGW T | YarivL-FOR15 |
| 148 | AGC CGG CCA TGG CGG AYA TTG TGA TGA CAC AAC C | YarivL-FOR16 |
| 149 | AGC CGG CCA TGG CGG AYA TTT TGC TGA CTC AGT C | YarivL-FOR17 |
| 150 | AGC CGG CCA TGG CGG ARG CTG TTG TGA CTC AGG AAT C | YarivL-FOR 1 Lambda |
| 151 | R702_Opt3_R2R1_combo AGATCGGAAGAGCACACGTCTGAACTCCAGTCACC GATGTACACTCTTTCCCTACACGACGCTCTTCCGAT CT | |
| 152 | R709_Opt3_R2R1_combo AGATCGGAAGAGCACACGTCTGAACTCCAGTCACG ATCAGACACTCTTTCCCTACACGACGCTCTTCCGAT CT | |
| 153 | 5'-CAAGCAGAAGACGGCATACGAGAT-3' | primer sequence directed against 5' end of 1390 (FIG. 13B) |
| 154 | P5 section in bold: P5_IG_GEN1-3_a_rv AATGATACGGCGACCACCGAGATCTACACGGATA GACHGATGGGGSTGTYGTT | heavy chain |
| 155 | P5 section in bold: P5_IG_KappaCon_rv AATGATACGGCGACCACCGAGATCTACACCTGGA TGGTGGGAAGATGGATACAG | light chain |

**Experiment:** A sample of OKT3 cells were introduced into the OptoSelect device at a density of 2E6 in 200 microliters. Approximately 150 of the cells were moved by optically actuated dielectrophoretic force to load one cell per NanoPen chamber. Each cell was positioned within the section of the chamber furthest from the opening to the microfluidic channel (e.g., isolation region). The OptoSelect device was then flushed once with 50 microliters of priming medium. A brightfield image was taken of the OptoSelect device for the purpose of identifying the locations of penned OKT3 cells (not shown). A sample of OKT8 cells were introduced into the OptoSelect device at a density of 2E6 in 200 microliters. Approximately 150 of the cells were moved by optically actuated dielectrophoretic force to load one cell per NanoPen chamber in fields of view in which OKT3 cells were not penned. The OptoSelect device was then flushed once with 50 microliters of priming medium. A brightfield image was taken of the OptoSelect device for the purpose of identifying the locations of penned OKT8 cells (not shown). A sample of barcoded beads having capture oligos as described herein (two exemplary, but not limiting sequences are SEQ ID NOs. 101 and 102, see Table 2) were introduced into the OptoSelect device at a density of 2E6 in 200 microliters. A single uniquely barcoded bead was subsequently loaded into each of the occupied chambers. The total number of beads loaded to the NanoPen chambers having single biological cells was 126, with 57 beads assigned to OKT3 cells and 69 beads assigned to OKT8 cells, and each bead was also positioned within the portion of each chamber that was not subjected to penetrating fluidic flow. The OptoSelect device was then flushed once with 50 microliters of 1 x DPBS.

Lysis reagent (Single Cell Lysis Kit, Ambion Catalog No. 4458235) was flowed into the microfluidic channel and permitted to diffuse into the NanoPen chambers. The individually penned OKT3 and OKT8 cells were exposed to the lysis buffer for 10 minutes. The OptoSelect device was then flushed once with 30 microliters of 1 x DPBS. Lysis was stopped by flowing in stop lysis buffer (Single Cell Lysis Kit, Ambion Catalog No. 4458235) and incubating for 2 minutes at room temperature while there was no flow in the microfluidic channel. Alternatively, a lysis buffer such as 10x Lysis Buffer, Catalog No. 635013, Clontech/Takara can be used to provide similar results, with the advantage of not requiring the use of a stop lysis buffer. The OptoSelect device was then flushed once with 30 microliters of 1 x DPBS. Under the conditions used, the nuclear membrane was not disrupted. The released mRNA was captured onto the barcoded bead present within the same NanoPen chamber.

The captured RNA was reverse transcribed to cDNA by flowing in a RT reagent mixture (Thermo Scientific^{™} Maxima^{™} H Minus RT (Thermofisher, Catalog No. EP0751)) and template switching oligonucleotide (SEQ ID NO. 112). Diffusion of the enzyme into the NanoPen Chamber was permitted during a 20 minute period at 16°C, followed by a reaction period of 90 minutes at 42°C. After reverse transcription, the OptoSelect device was then flushed once with 30 microliters of 1 x DPBS.

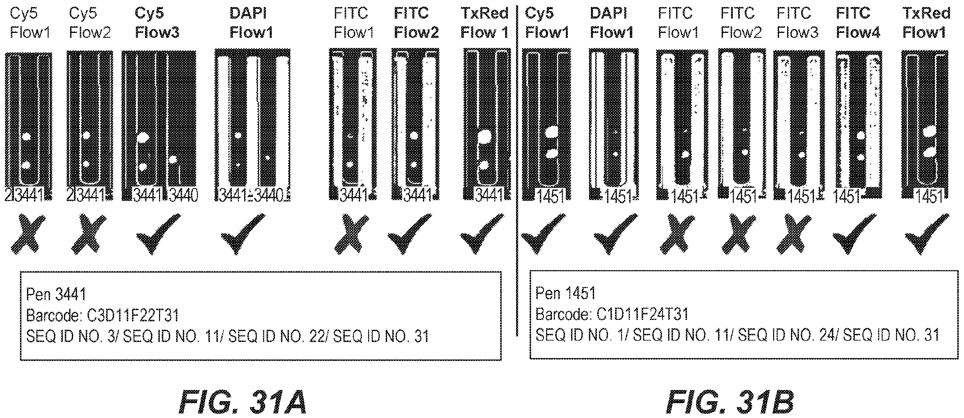

The unique barcode was then identified for each capture bead by multiplexed flows of fluorescently labeled hybridization probes as described herein. Successive reagent flows of each set of fluorescently labeled probes were flowed into the microfluidic channel of the microfluidic device at 1 micromolar diluted in 1 x DPBS (alternatively, IDT Duplex buffer may be used), and permitted to diffuse into the NanoPen chambers. After hybridization background signal was removed by flushing the OptoSelect device with 150 microliters of 1x DPBS. The location of each Cell Barcode so identified (e.g., NanoPen location of the bead labeled with that Cell Barcode) was recorded and was used to identify from which specific cell the BCR sequence was captured to the bead. In Figures 31A and 31B, the images and results are shown for the barcode detection for two individual NanoPen chambers, 3441 and 1451. The barcode for NanoPen chamber 3441 was determined to be C3D11F22T31, where the barcode was formed from four cassetable sequences GAATACGGGG (SEQ ID NO. 3) TTCCTCTCGT (SEQ ID NO. 11) AACATCCCTC (SEQ ID NO. 22) CCGCACTTCT (SEQ ID NO. 31). The barcode for NanoPen chamber 1451 was determined to be C1D11F24T31, where the barcode was formed from four cassetable sequences CAGCCTTCTG (SEQ ID NO. 1) TTCCTCTCGT (SEQ ID NO. 11) TTAGCGCGTC (SEQ ID NO. 24) CCGCACTTCT (SEQ ID NO. 31)

After detection, the chip was washed twice with 10 mM Tris-HCl (200 microliters at 0.5microliters/sec), prior to export of cDNA decorated capture beads.

Optically actuated dielectrophoretic force was used to export selected barcoded cDNA decorated beads from the NanoPen chambers in a displacement buffer, 10 mM Tris-HCl. One export contained 47 beads from OKT3 assigned wells and 69 beads from OKT8 assigned wells. The beads that had been exported from the NanoPen chambers were subsequently exported out of the microfluidic device using flow and pooled.

After treatment with Exonuclease I (NEB, catalog no. M0293L), the export group of beads was subjected to 22 cycles of DNA amplification (Advantage^{®} 2 PCR kit, Clontech, Catalog #. 639206) using as a primer 5'-ACACTCTTTCCCT ACACGACGC-3' (SEQ ID NO. 113). Initial purification of the crude amplification mixture for the export group was performed using 1x SPRI (Solid Phase Reversible Immobilization) beads (Agencourt AMPure XP beads (Beckman Coulter, catalog no. A63881) according to supplier instructions.

The crude amplification mixture was then split in two, where the first of the two portions was subject to 18 cycles of PCR with a mixture of BCR specific forward primers for heavy chain (SEQ ID NOs 114-132, Table 6) and where the second portion was subjected to 18 cycles of PCR with a mixture of BCR specific forward primers for light chain (SEQ ID NOs 133-150, Table 6) (Q5^{®} High-Fidelity DNA polymerase, NEB, catalog no. M0491S). Reverse primers (SEQ ID Nos. 151 and 152) added priming sequences with an index assigned to the export group of beads and heavy or light chain. A touchdown PCR protocol (where the annealing temperature is decreased in successive cycles) was used to increase amplification specificity. Initial purification of the BCR sequence containing amplicons was performed using 1x SPRI (Solid Phase Reversible Immobilization) beads (Agencourt AMPure XP beads (Beckman Coulter, catalog no. A63881) according to supplier instructions and subsequently selected by size on a 2 % Agarose gel (E-gel^{™} EX Agarose Gels 2%, Catalog no. G401002, ThermoFisher Scientific). Gel extraction was performed according to supplier instructions (Zymoclean^{™} Gel DNA Recovery Kit, catalog no. D4001, Zymo Research).

Purified and size-selected BCR sequence containing amplicons were treated with T4 polynucleotide kinase (T4 polynucleotide kinase, NEB, catalog no. M0201) then the reaction was purified with using 1x SPRI (Solid Phase Reversible Immobilization) beads (Agencourt AMPure XP beads (Beckman Coulter, catalog no. A63881) according to supplier instructions. Quantification was performed fluorescently (Qubit^{™}, ThermoFisher Scientific).

Purified T4 polynucleotide kinase-treated BCR sequence containing amplicons using less than or equal to 10 ng of the BCR amplicons were then self-ligated to create circularized DNA molecules (T4 DNA Ligase, Catalog no. EL0011, ThermoFisher Scientific). Any amount of DNA over the limit of detection, roughly about 0.5 ng will be sufficient to the circularization reaction. Not exceeding about 10 ng is useful to drive to self circularization rather than cross-ligating to another molecule of amplicon.

The ligation reaction was purified with using 1x SPRI (Solid Phase Reversible Immobilization) beads (Agencourt AMPure XP beads (Beckman Coulter, catalog no. A63881) according to supplier instructions and the circularized DNA molecules subsequently selected by position on a 2 % Agarose gel (E-gel^{™} EX Agarose Gels 2%, Catalog no. G401002, ThermoFisher Scientific). Gel extraction was performed according to supplier instructions (Zymoclean^{™} Gel DNA Recovery Kit, catalog no. D4001, Zymo Research).

Purified circularized DNA molecules were then re-linearized by performing a Not1 restriction enzyme digest (Not1-HF, NEB, Catalog no. R3189S) according to manufacturer's directions, and subsequently inactivating the reaction. The re-linearized DNA was purified using 1x SPRI (Solid Phase Reversible Immobilization) beads (Agencourt AMPure XP beads (Beckman Coulter, catalog no. A63881) according to supplier instructions.

The re-linearized DNA was subject to PCR 16 cycles with a P7 adaptor sequence forward primer (SEQ ID NO. 153, Table 6) and a BCR constant region primer (SEQ ID NO. 154, Table 6) containing the P5 adaptor sequence (KAPA HiFi HotStart ReadyMix, KK2601, KAPA Biosystems/Roche). The amplified DNA molecule was purified using 1 x SPRI (Solid Phase Reversible Immobilization) beads (Agencourt AMPure XP beads (Beckman Coulter, Catalog # A63881) according to supplier instructions. The amplified DNA product was then subject to PCR, 7 cycles for heavy chain and 6 cycles for light chain, with P7 and P5 adaptor sequence primers (SEQ ID NOs. 153 and 155, Table 6) (KAPA HiFi HotStart ReadyMix, KK2601, KAPA Biosystems/Roche). The resulting sequencing library was purified using 1 x SPRI (Solid Phase Reversible Immobilization) beads (Agencourt AMPure XP beads (Beckman Coulter, catalog no. A63881) according to supplier instructions and subsequently selected by size (550-750 bp) on a 2 % Agarose gel (E-gel^{™} EX Agarose Gels 2%, Catalog no. G401002, ThermoFisher Scientific). Gel extraction was performed according to supplier instructions (Zymoclean^{™} Gel DNA Recovery Kit, catalog no. D4001, Zymo Research).

Quantification of the purified sequencing library was performed fluorescently (Qubit^{™}, ThermoFisher Scientific). Sequencing was performed using a MiSeq Sequencer (Illumina^{®}, Inc.).

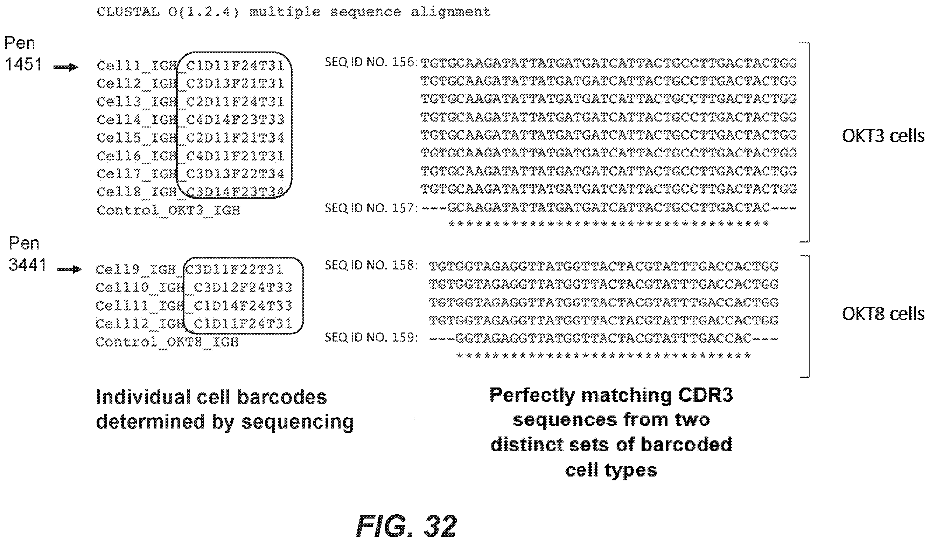

Sequencing results were de-plexed to generate FASTQ files of sequence data separate for each pool (including heavy or light chain) via the index included in the read 1 and read 2 primer, and for each cell as identified by the unique barcode sequence. Known CDR3 BCR sequences, containing a critical sub-region, directed to antigen biding sites, of the variable region for the OKT3 and OKT8 cell lines were aligned to the read data for each cell and used to identify the reads as coming from either OKT3 or OKT8 cells. The right hand column within Figure 32 shows that reads from cells 1-8 matched OKT3 sequence identity (SEQ ID NO. 157, Table 6), having a CDR3 sequence of:
TGTGCAAGATATTATGATGATCATTACTGCCTTGACTACTGG (SEQ ID NO.156).

Reads from cells 9-12 matched OKT8 sequence identity (SEQ ID 159, having a CDR2 sequence of:
TGTGGTAGAGGTTATGGTTACTACGTATTTGACCACTGG (SEQ ID No. 158).

The barcodes for each cell was also determined by sequencing and is shown for each of cells 1-12. Matching the barcodes determined by sequencing to the barcodes determined by the reagent flow methods described above, permitted unequivocal correlation between cell and genome. For example, the barcode determined above for NanoPen chamber 1451 via flow reagent matched to Cell 1, having a CDR3 sequence matching the phenotype for OKT3 cells. The other barcode described above, for NanoPen 3441, matched the barcode for Cell 9, having a CDR3 sequence matching the phenotype for OKT8. As this was a proof of principle experiment, it was known which type of cell was disposed within a specific NanoPen chamber, and the sequencing results showed that the barcode flow reagent detection tied perfectly to the barcode determined by sequencing and with the expected CDR3 sequence. This demonstrated that BCR sequence data was linkable to the physical location of the source cell.

Where reference is made herein to a list of elements (e.g., elements a, b, c), such reference is intended to include any one of the listed elements by itself, any combination of less than all of the listed elements, and/or a combination of all of the listed elements. Also, as used herein, the terms a, an, and one may each be interchangeable with the terms at least one and one or more. It should also be noted, that while the term step is used herein, that term may be used to simply draw attention to different portions of the described methods and is not meant to delineate a starting point or a stopping point for any portion of the methods, or to be limiting in any other way.

**INFORMAL SEQUENCE LISTING**

| **SEQ ID No.** | **Sequence** | **Type** |
|---|---|---|
| 1 | CAGCCTTCTG | Artificial sequence |
| 2 | TGTGAGTTCC | Artificial sequence |
| 3 | GAATACGGGG | Artificial sequence |
| 4 | CTTTGGACCC | Artificial sequence |
| 5 | GCCATACACG | Artificial sequence |
| 6 | AAGCTGAAGC | Artificial sequence |
| 7 | TGTGGCCATT | Artificial sequence |
| 8 | CGCAATCTCA | Artificial sequence |
| 9 | TGCGTTGTTG | Artificial sequence |
| 10 | TACAGTTGGC | Artificial sequence |
| 11 | TTCCTCTCGT | Artificial sequence |
| 12 | GACGTTACGA | Artificial sequence |
| 13 | ACTGACGCGT | Artificial sequence |
| 14 | AGGAGCAGCA | Artificial sequence |
| 15 | TGACGCGCAA | Artificial sequence |
| 16 | TCCTCGCCAT | Artificial sequence |
| 17 | TAGCAGCCCA | Artificial sequence |
| 18 | CAGACGCTGT | Artificial sequence |
| 19 | TGGAAAGCGG | Artificial sequence |
| 20 | GCGACAAGAC | Artificial sequence |
| 21 | TGTCCGAAAG | Artificial sequence |
| 22 | AACATCCCTC | Artificial sequence |
| 23 | AAATGTCCCG | Artificial sequence |
| 24 | TTAGCGCGTC | Artificial sequence |
| 25 | AGTTCAGGCG | Artificial sequence |
| 26 | ACAGGGGAAC | Artificial sequence |
| 27 | ACCGGATTGG | Artificial sequence |
| 28 | TCGTGTGTGA | Artificial sequence |
| 29 | TAGGTCTGCG | Artificial sequence |
| 30 | ACCCATACCC | Artificial sequence |
| 31 | CCGCACTTCT | Artificial sequence |
| 32 | TTGGGTACAG | Artificial sequence |
| 33 | ATTCGTCGGA | Artificial sequence |
| 34 | GCCAGCGTAT | Artificial sequence |
| 35 | GTTGAGCAGG | Artificial sequence |
| 36 | GGTACCTGGT | Artificial sequence |
| 37 | GCATGAACGT | Artificial sequence |
| 38 | TGGCTACGAT | Artificial sequence |
| 39 | CGAAGGTAGG | Artificial sequence |
| 40 | TTCAACCGAG | Artificial sequence |
| 41 | CAGAAGGCTG/3AlexF647N/ | Artificial sequence |
| 42 | GGAACTCACA/3AlexF647N/ | Artificial sequence |
| 43 | CCCCGTATTC/3AlexF647N/ | Artificial sequence |
| 44 | GGGTCCAAAG/3AlexF647N/ | Artificial sequence |
| 45 | CGTGTATGGC/3AlexF647N/ | Artificial sequence |
| 46 | GCTTCAGCTT/3AlexF647N/ | Artificial sequence |
| 47 | AATGGCCACA/3AlexF647N/ | Artificial sequence |
| 48 | TGAGATTGCG/3AlexF647N/ | Artificial sequence |
| 49 | CAACAACGCA/3AlexF647N/ | Artificial sequence |
| 50 | GCCAACTGTA/3AlexF647N/ | Artificial sequence |
| 51 | /5AlexF405N/ACGAGAGGAA | Artificial sequence |
| 52 | /5AlexF405N/TCGTAACGTC | Artificial sequence |
| 53 | /5AlexF405 N/ACGCGTCAGT | Artificial sequence |
| 54 | /5AlexF405N/TGCTGCTCCT | Artificial sequence |
| 55 | /5AlexF405N/TTGCGCGTCA | Artificial sequence |
| 56 | /5AlexF405 N/ATGGCGAG GA | Artificial sequence |
| 57 | /5AlexF405N/TGGGCTGCTA | Artificial sequence |
| 58 | /5AlexF405N/ACAGCGTCTG | Artificial sequence |
| 59 | /5AlexF405 N/CCGCTTTCCA | Artificial sequence |
| 60 | /5AlexF405 N/GTCTTGTCGC | Artificial sequence |
| 61 | CTTTCGGACA/3AlexF488N/ | Artificial sequence |
| 62 | GAGGGATGTT/3AlexF488N/ | Artificial sequence |
| 63 | CGGGACATTT/3AlexF488N/ | Artificial sequence |
| 64 | GACGCGCTAA/3AlexF488N/ | Artificial sequence |
| 65 | CGCCTGAACT/3AlexF488N/ | Artificial sequence |
| 66 | GTTCCCCTGT/3AlexF488N/ | Artificial sequence |
| 67 | CCAATCCGGT/3AlexF488N/ | Artificial sequence |
| 68 | TCACACACGA/3AlexF488N/ | Artificial sequence |
| 69 | CGCAGACCTA/3AlexF488N/ | Artificial sequence |
| 70 | GGGTATGGGT/3AlexF488N/ | Artificial sequence |
| 71 | AGAAGTGCGG/3AlexF594N/ | Artificial sequence |
| 72 | CTGTACCCAA/3AlexF594N/ | Artificial sequence |
| 73 | TCCGACGAAT/3AlexF594N/ | Artificial sequence |
| 74 | ATACGCTGGC/3AlexF594N/ | Artificial sequence |
| 75 | CCTGCTCAAC/3AlexF594N/ | Artificial sequence |
| 76 | ACCAGGTACC/3AlexF594N/ | Artificial sequence |
| 77 | ACGTTCATGC/3AlexF594N/ | Artificial sequence |
| 78 | ATCGTAGCCA/3AlexF594N/ | Artificial sequence |
| 79 | CCTACCTTCG/3AlexF594N/ | Artificial sequence |
| 80 | CTCGGTTGAA/3AlexF594N/ | Artificial sequence |
| 81 | AGTCGACTGA | Artificial sequence |
| 82 | TCAGCTGACT-FITC | Artificial sequence |
| 83 | TCAGCTGACTXXXXXX | Artificial sequence |
| 84 | NNNNNNNNNN | Artificial sequence |
| 85 | TTTTTTTTT | Artificial sequence |
| 86 | AAAAAAAAAA | Artificial sequence |
| 87 | CCCCCCCCCC | Artificial sequence |
| 88 | GGGGGGGGGG | Artificial sequence |
| 89 | GGGGGCCCCCTTTTTTTTTCCGGCCGGCCAAAAATTTTT | Artificial sequence |
| 90 | AAAAAAAAAATTTTTTTTTGGGGGGGGGGCCCCCCCCCC | Artificial sequence |
| 91 | GGGGGCCCCCTTAATTAATTCCGGCCGGCCAAAAATTTTT | Artificial sequence |
| 92 | GGGGGCCCCCTTTTTTTTGGGGGGGGGGCCCCCCCCCC | Artificial sequence |
| 93 | CCCCCGGGGG | Artificial sequence |
| 94 | AATTAATTAA | Artificial sequence |
| 95 | GGCCGGCCGG | Artificial sequence |
| 96 | TTTTTAAAAA | Artificial sequence |

| SEQ ID NO | Sequence | TYPE |
|---|---|---|
| 97 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNNTTTTTTTTTTTTTTTTTTTTVN- 3' | Artificial sequence |
| 98 | Bead-5'-Linker- ACACTCTTTCCCTACACGACGCTCTTCCGATCTCGCAATCTCACAGACGCTGTT CGTGTGTGATGGCTACGATNNNNNNNNNNNNTTTTTTTTTTTTTTTTTTTTVN- 3' | Artificial sequence |
| 99 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNNTTTTTTTTTTTTTTTTTTTTVN- 3' | Artificial sequence |
| 100 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNNTTTTTTTTTTTTTTTTTTTTVN- 3' | Artificial sequence |
| 101 | Bead-5'-Linker- ACACTCTTTCCCTACACGACGCTCTTCCGATCTCAGCCTTCTGTTCCTCTCGTT GTCCGAAAGCCGCACTTCTNNNNNNNNNNATCTCGTATGCCGTCTTCTGCTT GGCGGCCGCTTTTTTTTTTTTTTTTTTTVN | Artificial sequence |
| 102 | Bead-5'-Linker- ACACTCTTTCCCTACACGACGCTCTTCCGATCTTGCGTTGTTGTGGAAAGCGG TAGGTCTGCGCGAAGGTAGGNNNNNNNNNNNNATCTCGTATGCCGTCTTCTGC TTGGCGGCCGCTTTTTTTTTTTTTTTTTTTTVN- 3' | Artificial sequence |

| SEQ ID NO. | Sequence/s | TYPE |
|---|---|---|
| 103 | /5Me-isodC//isodG//iMe- isodC/ACACTCTTTCCCTACACGACGCrGrGrG | Artificial sequence |
| 104 | 5'- ACACTCTTTCCCTACACGACGCTCTTCCGATCT | Artificial sequence |
| 105 | 5'-/5Biosg/ACACTCTT⁻FCCCT ACACGACGC-3' | Artificial sequence |
| 106 | (5'- AATGATACGGCGACCACCGAGATCTACACTCTTTCCCTACACGACGCTCT TC C*G*A*T*C*T-3' | Artificial sequence |
| 107 | 5'-CAAGCAGAAGACGGCATACGAGAT-3' | Artificial sequence |
| 108 | 5'-AATGATACGGCGACCACCGA-3' | Artificial sequence |
| 109 | /5BiotinTEG/CAAGCAGAAGACGGCATACGAGATTCGCCTTAG TCTCGTGGGCTCG*G | Artificial sequence |
| 110 | /5BiotinTEG/CAAGCAGAAGACGGCATACGAGATCTAGTACG GTCTCGTGGGCTCG*G | Artificial sequence |
| 111 | /5BiotinTEG/AATGATACGGCGACCACCGAGATCTACACACTG CATATCGTCGGCAGCGT*C | Artificial sequence |
| 112 | 5'-Me-isodC//Me-isodG//Me- isodC/ACACTCTTTCCCTACACGACGCrGrGrG-3 | Artificial sequence |
| 113 | 5'-ACACTCTTTCCCT ACACGACGC-3' | Artificial sequence |
| 114 | GTT ATT GCT AGC GGC TCA GCC GGC AAT GGC GGA KGT RMA GCT TCA GGA GTC | Artificial sequence |
| 115 | GTT ATT GCT AGC GGC TCA GCC GGC AAT GGC GGA GGT BCA GCT BCA GCA GTC | Artificial sequence |
| 116 | GTT ATT GCT AGC GGC TCA GCC GGC AAT GGC GGA GGT BCA GCT BCA GCA GTC | Artificial sequence |
| 117 | GTT ATT GCT AGC GGC TCA GCC GGC AAT GGC GGA GGT CCA RCT GCA ACA RTC | Artificial sequence |
| 118 | GTT ATT GCT AGC GGC TCA GCC GGC AAT GGC GCA GGT YCA GCT BCA GCA RTC | Artificial sequence |
| 119 | GTT ATT GCT AGC GGC TCA GCC GGC AAT GGC GCA GGT YCA RCT GCA GCA GTC | Artificial sequence |
| 120 | GTT ATT GCT AGC GGC TCA GCC GGC AAT GGC GCA GGT CCA CGT GAA GCA GTC | Artificial sequence |
| 121 | GTT ATT GCT AGC GGC TCA GCC GGC AAT GGC GGA GGT GAA SST GGT GGA ATC | Artificial sequence |
| 122 | GTT ATT GCT AGC GGC TCA GCC GGC AAT GGC GGA VGT GAW GYT GGT GGA GTC | Artificial sequence |
| 123 | GTT ATT GCT AGC GGC TCA GCC GGC AAT GGC GGA GGT GCA GSK GGT GGA GTC | Artificial sequence |
| 124 | GTT ATT GCT AGC GGC TCA GCC GGC AAT GGC GGA KGT GCA MCT GGT GGA GTC | Artificial sequence |
| 125 | GTT ATT GCT AGC GGC TCA GCC GGC AAT GGC GGA GGT GAA GCT GAT GGA RTC | Artificial sequence |
| 126 | GTT ATT GCT AGC GGC TCA GCC GGC AAT GGC GGA GGT GCA RCT TGT TGA GTC | Artificial sequence |
| 127 | GTT ATT GCT AGC GGC TCA GCC GGC AAT GGC GGA RGT RAA GCT TCT CGA GTC | Artificial sequence |
| 128 | GTT ATT GCT AGC GGC TCA GCC GGC AAT GGC GGA AGT GAA RST TGA GGA GTC | Artificial sequence |
| 129 | GTT ATT GCT AGC GGC TCA GCC GGC AAT GGC GCA GGT TAC TCT RAA AGW GTS TG | Artificial sequence |
| 130 | GTT ATT GCT AGC GGC TCA GCC GGC AAT GGC GCA GGT CCA ACT VCA GCA RCC | Artificial sequence |
| 131 | GTT ATT GCT AGC GGC TCA GCC GGC AAT GGC GGA TGT GAA CTT GGA AGT GTC | Artificial sequence |
| 132 | GTT ATT GCT AGC GGC TCA GCC GGC AAT GGC GGA GGT GAA GGT CAT CGA GTC | Artificial sequence |
| 133 | AGC CGG CCA TGG CGG AYA TCC AGC TGA CTC AGC C | Artificial sequence |
| 134 | AGC CGG CCA TGG CGG AYA TTG TTC TCW CCC AGT C | Artificial sequence |
| 135 | AGC CGG CCA TGG CGG AYA TTG TGM TMA CTC AGT C | Artificial sequence |
| 136 | AGC CGG CCA TGG CGG AYA TTG TGY TRA CAC AGT C | Artificial sequence |
| 137 | AGC CGG CCA TGG CGG AYA TTG TRA TGA CMC AGT C | Artificial sequence |
| 138 | AGC CGG CCA TGG CGG AYA TTM AGA TRA MCC AGT C | Artificial sequence |
| 139 | AGC CGG CCA TGG CGG AYA TTC AGA TGA YDC AGT C | Artificial sequence |
| 140 | AGC CGG CCA TGG CGG AYA TYC AGA TGA CAC AGA C | Artificial sequence |
| 141 | AGC CGG CCA TGG CGG AYA TTG TTC TCA WCC AGT C | Artificial sequence |
| 142 | AGC CGG CCA TGG CGG AYA TTG WGC TSA CCC AAT C | Artificial sequence |
| 143 | AGC CGG CCA TGG CGG AYA TTS TRA TGA CCC ART C | Artificial sequence |
| 144 | AGC CGG CCA TGG CGG AYR TTK TGA TGA CCC ARA C | Artificial sequence |
| 145 | AGC CGG CCA TGG CGG AYA TTG TGA TGA CBC AGK C | Artificial sequence |
| 146 | AGC CGG CCA TGG CGG AYA TTG TGA TAA CYC AGG A | Artificial sequence |
| 147 | AGC CGG CCA TGG CGG AYA TTG TGA TGA CCC AGW T | Artificial sequence |
| 148 | AGC CGG CCA TGG CGG AYA TTG TGA TGA CAC AAC C | Artificial sequence |
| 149 | AGC CGG CCA TGG CGG AYA TTT TGC TGA CTC AGT C | Artificial sequence |
| 150 | AGC CGG CCA TGG CGG ARG CTG TTG TGA CTC AGG AAT C | Artificial sequence |
| 151 | AGATCGGAAGAGCACACGTCTGAACTCCAGTCACCGATGTACACTCTTT CCCTACACGACGCTCTTCCGATCT | Artificial sequence |
| 152 | AGATCGGAAGAGCACACGTCTGAACTCCAGTCACGATCAGACACTCTTT CCCTACACGACGCTCTTCCGATCT | Artificial sequence |
| 153 | 5'-CAAGCAGAAGACGGCATACGAGAT-3' | Artificial sequence |
| 154 | AATGATACGGCGACCACCGAGATCTACACGGATAGACHGATGGGGSTG TYGTT | Artificial sequence |
| 155 | AATGATACGGCGACCACCGAGATCTACACCTGGATGGTGGGAAGATGG ATACAG | Artificial sequence |
| 156 | TGTGCAAGATATTATGATGATCATTACTGCCTTGACTACTGG | Artificial sequence |
| 157 | ---GCAAGATATTATGATGATCATTACTGCCTTGACTAC--- | Natural Organism: Human OKT8, CDR3 |
| 158 | TGTGGTAGAGGTTATGGTTACTACGTATTTGACCACTGG | Artificial sequence |
| 159 | ---GGTAGAGGTTATGGTTACTACGTATTTGACCAC--- | Natural Organism: Mouse: OKT3, CDR3 |
| 160 | GCGGCCGC | Artificial sequence |
| 161 | 5'-TCGTCGGCAGCGTCAGATGTGTATAAGAGACAG-3' | Artificial sequence |
| 162 | 5'GTCTCGTGGGCTCGGAGATGTGTATAAGAGACAG-3' | Artificial sequence |

## Claims

1. A plurality of capture objects, wherein each capture object of said plurality is a capture object comprising a plurality of capture oligonucleotides, wherein each capture oligonucleotide of said plurality comprises:
a priming sequence;
a capture sequence; and
a barcode sequence comprising three or more cassetable oligonucleotide sequences, each cassetable oligonucleotide sequence being non-identical to the other cassetable oligonucleotide sequences of said barcode sequence, and wherein a cassetable oligonucleotide sequence is an oligonucleotide sequence that is one of a defined set of oligonucleotide sequences, and,
wherein each capture oligonucleotide of said plurality comprises the same barcode sequence,
wherein said barcode sequence of said capture oligonucleotides of each capture object of said plurality is different from the barcode sequence of the capture oligonucleotides of every other capture object of said plurality.

2. The plurality of capture objects of claim 1, wherein each capture oligonucleotide of said plurality comprises a 5'-most nucleotide and a 3'-most nucleotide,
wherein said priming sequence is adjacent to or comprises said 5'-most nucleotide,
wherein said capture sequence is adjacent to or comprises said 3'-most nucleotide, and
wherein said barcode sequence is located 3' to said priming sequence and 5' to said capture sequence.

3. The plurality of capture objects of any one of claims 1-2, wherein each of said three or more cassetable oligonucleotide sequences comprises 8 to 12 nucleotides.

4. The plurality of capture objects of any one of claims 1-3, wherein said three or more cassetable oligonucleotide sequences of said barcode sequence are linked in tandem without any intervening oligonucleotide sequences.

5. The plurality of capture objects of any one of claims 1-4, wherein each of said three or more cassetable oligonucleotide sequences of said barcode sequence has a sequence of any one of SEQ ID NOs: 1-40.

6. The plurality of capture objects of any one of claims 1-5, wherein said barcode sequence comprises four cassetable oligonucleotide sequences.

7. The plurality of capture objects of any one of claims 1-6, wherein each capture oligonucleotide of said plurality further comprises a unique molecule identifier (UMI) sequence, and, optionally, wherein said UMI is located 3' to said priming sequence and 5' to said capture sequence.

8. The plurality of capture objects of any one of claims 1-7, wherein each capture oligonucleotide further comprises a restriction site comprising a recognition sequence of at least 8 base pairs.

9. The plurality of capture objects of any one of claims 1-7, wherein said capture sequence comprises a poly-dT sequence, a random hexamer sequence, a gene specific sequence, or a mosaic end sequence.

10. A set of spectrally distinguishable hybridization probes, each probe comprising:
a non-identical oligonucleotide sequence comprising a sequence of any one of SEQ ID NOs: 41 to 80 and a fluorescent label.

11. A reagent comprising a plurality of hybridization probes, wherein each hybridization probe of said plurality is a hybridization probe according to claim 10, and wherein each hybridization probe of said plurality (i) comprises an oligonucleotide sequence which is non-identical to the oligonucleotide sequence of every other hybridization probe of the plurality and (ii) comprises a fluorescent label which is spectrally distinguishable from the fluorescent label of every other hybridization probe of said plurality.

12. The reagent of claim 11, wherein:
a first hybridization probe of said plurality comprises a sequence selected from a first subset of SEQ ID NOs: 41-80, and a first fluorescent label;
a second hybridization probe of said plurality comprises a sequence selected from a second subset of SEQ ID NOs: 41-80, and a second fluorescent label which is spectrally distinguishable from said first fluorescent label,
a third hybridization probe of said plurality comprises a sequence selected from a third subset of SEQ ID NOs: 41-80, and a third fluorescent label which is spectrally distinguishable from each of said first and second fluorescent labels,
wherein said first, second, and third subsets of SEQ ID NOs: 41-80 are nonoverlapping subsets.

13. A method of in-situ identification of one or more capture objects from a plurality of capture objects according to claims 1-9 within a microfluidic device, said method comprising:
disposing a single capture object of said one or more capture objects within an isolation region of each of one or more sequestration pens located within an enclosure of said microfluidic device, wherein each capture object comprises a plurality of capture oligonucleotides, and wherein each capture oligonucleotide of said plurality comprises:
a priming sequence;
a capture sequence; and
a barcode sequence, wherein said barcode sequence comprises three or more cassetable oligonucleotide sequences, each cassetable oligonucleotide sequence being non-identical to the other cassetable oligonucleotide sequences of said barcode sequence; and
wherein each capture oligonucleotide of said plurality comprises said same barcode sequence;
flowing a first reagent solution comprising a first set of hybridization probes into a flow region within said enclosure of said microfluidic device, wherein said flow region is fluidically connected to each of said one or more sequestration pens, and wherein each hybridization probe of said first set comprises:
an oligonucleotide sequence complementary to a cassetable oligonucleotide sequence comprised by any of said barcode sequences of any of said capture oligonucleotides of any of said one or more capture objects, wherein said complementary oligonucleotide sequence of each hybridization probe in said first set is non-identical to every other complementary oligonucleotide sequence of said hybridization probes in said first set; and
a fluorescent label selected from a set of spectrally distinguishable fluorescent labels, wherein said fluorescent label of each hybridization probe in said first set is different from the fluorescent label of every other hybridization probe in said first set of hybridization probes;
hybridizing said hybridization probes of said first set to corresponding cassetable oligo-nucleotide sequences in any of said barcode sequences of any of said capture oligonucleotides of any of said one or more capture objects;
detecting, for each hybridization probe of said first set of hybridization probes, a corresponding fluorescent signal associated with any of said one or more capture objects; and
generating a record, for each capture object disposed within one of said one or more sequestration pens, comprising (i) a location of said sequestration pen within said enclosure of said microfluidic device, and (ii) an association or non-association of said corresponding fluorescent signal of each hybridization probe of said first set of hybridization probes with said capture object, wherein said record of associations and non-associations constitute a barcode which links said capture object with said sequestration pen.

14. The method of claim 13, further comprising:
flowing an n^{th} reagent solution comprising an n^{th} set of hybridization probes into said flow region of said microfluidic device, wherein each hybridization probe of said n^{th} set comprises:
an oligonucleotide sequence complementary to a cassetable oligonucleotide sequence comprised by any of said barcode sequences of any of said capture oligonucleotides of any of said one or more capture objects, wherein said complementary oligonucleotide sequence of each hybridization probe in said n^{th} set is non-identical to every other complementary oligonucleotide sequence of said hybridization probes in said n^{th} set and any other set of hybridization probes flowed into said flow region of said microfluidic device; and
a fluorescent label selected from a set of spectrally distinguishable fluorescent labels, wherein said fluorescent label of each hybridization probe in said n^{th} set is different from the fluorescent label of every other hybridization probe in said n^{th} set of hybridization probes;
hybridizing said hybridization probes of said n^{th} set to corresponding cassetable oligo-nucleotide sequences in any of said barcode sequences of any of said capture oligonucleotides of any of said one or more capture objects;
detecting, for each hybridization probe of said n^{th} set of hybridization probes, a corresponding fluorescent signal associated with any of said one or more capture objects; and
supplementing said record, for each capture object disposed within one of said one or more sequestration pens, with an association or non-association of said corresponding fluorescent signal of each hybridization probe of said n^{th} set of hybridization probes with said capture object,
wherein n is a set of positive integers having values of {2,..., m},
wherein m is a positive integer having a value of 2 or greater,
wherein the foregoing steps of flowing said n^{th} reagent, hybridizing said n^{th} set of hybridization probes, detecting said corresponding fluorescent signals, and supplementing said records are repeated for each value of n in said set of positive integers {2, ...., m}, and,
wherein m has a value greater than or equal to 3 and less than or equal to 20.

15. The method of any one of claims 13-14, wherein each barcode sequence of each capture oligonucleotide of each capture object comprises three or four cassetable oligonucleotide sequences.

16. The method of any one of claims 14-15, wherein said first set of hybridization probes and each of said n^{th} sets of hybridization probes comprise three or four hybridization probes.

17. The method of any one of claims 13-16, further comprising disposing one or more biological cells within said one or more sequestration pens of said microfluidic device, wherein each one of said one or more biological cells are disposed in a different one of said one or more sequestration pens.

18. The method of any one of claims 13-17, wherein said enclosure of said microfluidic device further comprises a dielectrophoretic (DEP) configuration, and wherein disposing said one or more capture objects into one or more sequestration pens is performed using dielectrophoretic (DEP) force, and, optionally, wherein
disposing said one or more biological cells within said one or more sequestration pens is performed using dielectrophoretic (DEP) forces.

19. A method of correlating genomic data with a biological cell in a microfluidic device, comprising:
disposing a capture object from a plurality of capture objects according to claims 1-9, into a sequestration pen of a microfluidic device, wherein said capture object comprises a plurality of capture oligonucleotides, wherein each capture oligonucleotide of said plurality comprises:
a priming sequence;
a capture sequence; and
a barcode sequence, wherein said barcode sequence comprises three or more cassetable oligonucleotide sequences, each cassetable oligonucleotide sequence being non-identical to the other cassetable oligonucleotide sequences of said barcode sequence; and
wherein each capture oligonucleotide of said plurality comprises said same barcode sequence;
identifying said barcode sequence of said plurality of capture oligonucleotides in-situ and recording an association between said identified barcode sequence and said sequestration pen;
disposing said biological cell into said sequestration pen;
lysing said biological cell and allowing nucleic acids released from said lysed biological cell to be captured by said plurality of capture oligonucleotides comprised by said capture object;
transcribing said captured nucleic acids, thereby producing a plurality of barcoded cDNAs, each barcoded cDNA comprising a complementary captured nucleic acid sequence covalently linked to one of said capture oligonucleotides;
sequencing said transcribed nucleic acids and said barcode sequence, thereby obtaining read sequences of said plurality of transcribed nucleic acids associated with read sequences of said barcode sequence;
identifying said barcode sequence based upon said read sequences; and
using said read sequence-identified barcode sequence and said in situ-identified barcode sequence to link said read sequences of said plurality of transcribed nucleic acids with said sequestration pen and thereby correlate said read sequences of said plurality of transcribed nucleic acids with said biological cell placed into said sequestration pen.

20. The method of claim 19, further comprising:
observing a phenotype of said biological cell; and
correlating said read sequences of said plurality of transcribed nucleic acids with said phenotype of said biological cell.

21. The method of any one of claims 19-20, wherein identifying said barcode sequence of said plurality of capture oligonucleotide in-situ comprises performing the method of one of claims 13-18.

22. The method of any one of claims 19-21, further comprising:
disposing a plurality of capture objects into a corresponding plurality of sequestration pens of said microfluidic device;
disposing a plurality of biological cells into said corresponding plurality of sequestration pens, and
processing each of said plurality of capture objects and plurality of biological cells according to said additional steps of said method.

## Patentansprüche

1. Vielzahl von Einfangobjekten, wobei jedes Einfangobjekt der Vielzahl ein Einfangobjekt ist, das eine Vielzahl an Einfangoligonukleotiden umfasst, wobei jedes Einfangoligonukleotid aus der Vielzahl:
eine Priming-Sequenz;
eine Einfangsequenz; und
eine Barcode-Sequenz, umfassend drei oder mehr kassettierbare Oligonukleotidsequenzen, umfasst, wobei jede kassettierbare Oligonukleotidsequenz nicht-identisch zu den übrigen kassettierbaren Oligonukleotidsequenzen der Barcode-Sequenz ist, und wobei eine kassettierbare Oligonukleotidsequenz eine Oligonukleotidsequenz ist, die eine einzelne aus einem definierten Satz von Oligonukleotidsequenzen ist, und
wobei jedes Einfangoligonukleotid aus der Vielzahl die gleiche Barcode-Sequenz umfasst,
wobei sich die Barcode-Sequenz der Einfangoligonukleotide jedes Einfangobjekts aus der Vielzahl von der Barcode-Sequenz der Einfangoligonukleotide jedes anderen Einfangobjekts aus der Vielzahl unterscheidet.

2. Vielzahl von Einfangobjekten nach Anspruch 1, wobei jedes Einfangoligonukleotid aus der Vielzahl ein am weitesten 5' gelegenes Nukleotid und ein am weitesten 3' gelegenes Nukleotid umfasst,
wobei die Priming-Sequenz an das am weitesten 5' gelegene Nukleotid angrenzt oder dieses umfasst,
wobei die Einfangsequenz an das am weitesten 3' gelegene Nukleotid angrenzt oder dieses umfasst, und
wobei die Barcode-Sequenz 3' zu der Priming-Sequenz und 5' zu der Einfangsequenz lokalisiert ist.

3. Vielzahl von Einfangobjekten nach einem der Ansprüche 1-2, wobei jede der drei oder mehr kassettierbaren Oligonukleotidsequenzen 8 bis 12 Nukleotide umfasst.

4. Vielzahl von Einfangobjekten nach einem der Ansprüche 1-3, wobei die drei oder mehr kassettierbaren Oligonukleotidsequenzen der Barcode-Sequenz tandemartig hintereinander ohne irgendwelche dazwischenliegenden Oligonukleotidsequenzen verknüpft sind.

5. Vielzahl von Einfangobjekten nach einem der Ansprüche 1-4, wobei jede der drei oder mehr kassettierbaren Oligonukleotidsequenzen der Barcode-Sequenz eine Sequenz von beliebigen der SEQ ID NOs: 1-40 aufweist.

6. Vielzahl von Einfangobjekten nach einem der Ansprüche 1-5, wobei die Barcode-Sequenz vier kassettierbare Oligonukleotidsequenzen umfasst.

7. Vielzahl von Einfangobjekten nach einem der Ansprüche 1-6, wobei jedes Einfangoligonukleotid der Vielzahl ferner eine "Unique-Molekül-Identifizierer" (UMI)-Sequenz umfasst, und wobei optional die UMI 3' zu der Priming-Sequenz und 5' zu der Einfangsequenz lokalisiert ist.

8. Vielzahl von Einfangobjekten nach einem der Ansprüche 1-7, wobei jedes Einfangoligonukleotid ferner eine Restriktionsstelle umfasst, die eine Erkennungssequenz von mindestens 8 Basenpaaren umfasst.

9. Vielzahl von Einfangobjekten nach einem der Ansprüche 1-7, wobei die Einfangsequenz eine Poly-dT-Sequenz, eine Zufallshexamer-Sequenz, eine genspezifische Sequenz oder eine Mosaik-Endsequenz umfasst.

10. Satz von spektral unterscheidbaren Hybridisierungssonden, wobei jede Sonde umfasst:
eine nicht-identische Oligonukleotidsequenz, umfassend eine Sequenz von einer beliebigen der SEQ ID NOs: 41 bis 80 und eine fluoreszierende Markierung.

11. Reagens, umfassend eine Vielzahl von Hybridisierungssonden, wobei jede Hybridisierungssonde der Vielzahl eine Hybridisierungssonde nach Anspruch 10 ist, und wobei jede Hybridisierungssonde der Vielzahl (i) eine Oligonukleotidsequenz umfasst, die nicht-identisch zu der Oligonukleotidsequenz jedweder anderen Hybridisierungssonde der Vielzahl ist, und (ii) eine fluoreszierende Markierung umfasst, die spektral von der fluoreszierenden Markierung jedweder anderen Hybridisierungssonde der Vielzahl unterscheidbar ist.

12. Reagenz nach Anspruch 11, wobei:
eine erste Hybridisierungssonde der Vielzahl eine Sequenz, ausgewählt aus einer ersten Teilmenge der SEQ ID NOs: 41-80, und eine erste fluoreszierende Markierung umfasst;
eine zweite Hybridisierungssonde der Vielzahl eine Sequenz, ausgewählt aus einer zweiten Teilmenge der SEQ ID NOs: 41-80, und eine zweite fluoreszierende Markierung, die spektral von der ersten fluoreszierenden Markierung unterscheidbar ist, umfasst,
eine dritte Hybridisierungssonde der Vielzahl eine Sequenz, ausgewählt aus einer dritten Teilmenge der SEQ ID NOs: 41-80, und eine dritte fluoreszierende Markierung, die spektral von jeder der ersten und zweiten fluoreszierenden Markierungen unterscheidbar ist, umfasst,
wobei die erste, zweite und dritte Teilmenge der SEQ ID NOs: 41-80 nicht-überlappende Teilmengen sind.

13. Verfahren zur In-situ-Identifizierung eines oder mehrerer Einfangobjekte aus einer Vielzahl von Einfangobjekten nach den Ansprüchen 1-9 innerhalb einer mikrofluidischen Vorrichtung, wobei das Verfahren Folgendes umfasst:
Einbringen eines einzelnen Einfangobjekts von dem einen oder den mehreren Einfangobjekte(n) innerhalb eines Isolationsbereichs von jedem von einem oder mehreren Sequestrierungsstiften, die innerhalb einer Umhausung der mikrofluidischen Vorrichtung lokalisiert sind, wobei jedes Einfangobjekt eine Vielzahl von Einfangoligonukleotiden umfasst, und wobei jedes Einfangoligonukleotid aus der Vielzahl:
eine Priming-Sequenz;
eine Einfangsequenz; und
eine Barcode-Sequenz umfasst, wobei die Barcode-Sequenz drei oder mehr kassettierbare Oligonukleotidsequenzen umfasst, wobei jede kassettierbare Oligonukleotidsequenz nicht-identisch zu den übrigen kassettierbaren Oligonukleotidsequenzen der Barcode-Sequenz ist; und
wobei jedes Einfangoligonukleotid aus der Vielzahl die gleiche Barcode-Sequenz umfasst;
Fließenlassen einer ersten Reagenzlösung, die einen ersten Satz von Hybridisierungssonden umfasst, in einen Fließbereich innerhalb der Umhausung der mikrofluidischen Vorrichtung, wobei der Fließbereich mit jedem des einen oder der mehreren Sequestrierungsstifte fluidisch verbunden ist, und wobei jede Hybridisierungssonde des ersten Satzes Folgendes umfasst:
eine Oligonukleotidsequenz, die komplementär zu einer kassettierbaren Oligonukleotidsequenz ist, die von beliebigen der Barcode-Sequenzen beliebiger der Einfangoligonukleotide von beliebigen von dem einen oder den mehreren Einfangobjekte(n) beinhaltet ist, wobei die komplementäre Oligonukleotidsequenz jeder Hybridisierungssonde in dem ersten Satz nicht-identisch zu jedweder anderen
komplementären Oligonukleotidsequenz der Hybridisierungssonden in dem ersten Satz ist; und
eine fluoreszierende Markierung, ausgewählt aus einem Satz von spektral unterscheidbaren fluoreszierenden Markierungen, wobei sich die fluoreszierende Markierung jeder Hybridisierungssonde in dem ersten Satz von der fluoreszierenden Markierung jedweder anderen Hybridisierungssonde in dem ersten Satz von Hybridisierungssonden unterscheidet;
Hybridisieren der Hybridisierungssonden des ersten Satzes an entsprechende kassettierbare Oligonukleotidsequenzen in beliebigen der Barcode-Sequenzen beliebiger der Einfangoligonukleotide von beliebigen von dem einen oder den mehreren Einfangobjekte(n);
Nachweisen, für jede Hybridisierungssonde des ersten Satzes von Hybridisierungssonden, eines entsprechenden Fluoreszenzsignals, das mit beliebigen von dem einen oder den mehreren Einfangobjekt(en) assoziiert ist; und
Generieren einer Aufzeichnung für jedes Einfangobjekt, das innerhalb eines von dem einen oder den mehreren Sequestrierungsstift(en) eingebracht ist, umfassend (i) eine Position des Sequestrierungsstifts innerhalb der Umhausung der mikrofluidischen Vorrichtung und (ii) eine Assoziation oder Nicht-Assoziation des entsprechenden Fluoreszenzsignals jeder Hybridisierungssonde des ersten Satzes von Hybridisierungssonden mit dem Einfangobjekt, wobei die Aufzeichnung von Assoziationen und Nicht-Assoziationen einen Barcode darstellt, der das Einfangobjekt mit dem Sequestrierungsstift in Verbindung bringt.

14. Verfahren nach Anspruch 13, ferner umfassend: Fließenlassen einer n-ten Reagenzlösung, die einen n-ten Satz von Hybridisierungssonden umfasst, in den Fließbereich der mikrofluidischen Vorrichtung, wobei jede Hybridisierungssonde des n-ten Satzes Folgendes umfasst:
eine Oligonukleotidsequenz, die komplementär zu einer kassettierbaren Oligonukleotidsequenz ist, die von beliebigen der Barcode-Sequenzen beliebiger der Einfangoligonukleotide von beliebigen von dem einen oder den mehreren Einfangobjekte(n) beinhaltet ist, wobei die komplementäre Oligonukleotidsequenz jeder Hybridisierungssonde in dem n-ten Satz nicht-identisch ist zu jedweder anderen komplementären Oligonukleotidsequenz der Hybridisierungssonden in dem n-ten Satz und irgendeinem anderen Satz von Hybridisierungssonden, welcher in den Fließbereich der mikrofluidischen Vorrichtung eingeschwemmt wird; und
eine fluoreszierende Markierung, ausgewählt aus einem Satz von spektral unterscheidbaren fluoreszierenden Markierungen, wobei die fluoreszierende Markierung jeder Hybridisierungssonde in dem n-ten Satz sich von der fluoreszierenden Markierung jedweder anderen Hybridisierungssonde in dem n-ten Satz von Hybridisierungssonden unterscheidet;
Hybridisieren der Hybridisierungssonden des n-ten Satzes an entsprechende kassettierbare Oligonukleotidsequenzen in beliebigen der Barcode-Sequenzen beliebiger der Einfangoligonukleotide von beliebigen von dem einen oder den mehreren Einfangobjekte(n);
Nachweisen, für jede Hybridisierungssonde des n-ten Satzes von Hybridisierungssonden, eines entsprechenden Fluoreszenzsignals, das mit beliebigen von dem einen oder den mehreren Einfangobjekte(n) assoziiert ist; und
Ergänzen der Aufzeichnung, für jedes Einfangobjekt, das innerhalb einem von dem einen oder den mehreren Sequestrierungsstifte(n) eingebracht ist, durch eine Assoziation oder Nicht-Assoziation des entsprechenden Fluoreszenzsignals jeder Hybridisierungssonde des n-ten Satzes von Hybridisierungssonden mit dem Einfangobjekt,
wobei n ein Satz positiver ganzer Zahlen mit Werten von {2, ..., m} ist,
wobei m eine positive ganze Zahl mit einem Wert von 2 oder größer ist,
wobei die vorstehenden Schritte des Fließenlassens des n-ten Reagens, Hybridisierens des n-ten Satzes von Hybridisierungssonden, Nachweisens der entsprechenden Fluoreszenzsignale und Ergänzens der Aufzeichnungen für jeden Wert von n in dem Satz von positiven ganzen Zahlen {2, ..., m } wiederholt werden, und
wobei m einen Wert größer oder gleich 3 und kleiner oder gleich 20 hat.

15. Verfahren nach einem der Ansprüche 13-14, wobei jede Barcode-Sequenz jedes Einfangoligonukleotids von jedem Einfangobjekt drei oder vier kassettierbare Oligonukleotidsequenzen umfasst.

16. Verfahren nach einem der Ansprüche 14-15, wobei der erste Satz von Hybridisierungssonden und jeder der n-ten Sätze von Hybridisierungssonden drei oder vier Hybridisierungssonden umfassen.

17. Verfahren nach einem der Ansprüche 13-16, ferner umfassend das Einbringen einer oder mehrerer biologischer Zellen innerhalb des einen oder der mehreren Sequestrierungsstifte der mikrofluidischen Vorrichtung, wobei jede einzelne der ein oder mehreren biologischen Zellen in einem anderen der ein oder mehreren Sequestrierungsstifte eingebracht wird.

18. Verfahren nach einem der Ansprüche 13-17, wobei die Umhausung der mikrofluidischen Vorrichtung ferner eine Dielektrophorese(DEP)-Konfiguration umfasst, und wobei das Einbringen des einen oder der mehreren Einfangobjekte in einen oder mehrere Sequestrierungsstift(e) mithilfe von dielektrophoretischer (DEP) Kraft durchgeführt wird, und wobei optional das Einbringen der einen oder der mehreren biologischen Zellen innerhalb des einen oder der mehreren Sequestrierungsstifte mithilfe von dielektrophoretischen (DEP) Kräften durchgeführt wird.

19. Verfahren zum Korrelieren genomischer Daten mit einer biologischen Zelle in einer mikrofluidischen Vorrichtung, umfassend:
Einbringen eines Einfangobjekts aus einer Vielzahl von Einfangobjekten nach den Ansprüchen 1-9 in einen Sequestrierungsstift einer mikrofluidischen Vorrichtung, wobei das Einfangobjekt eine Vielzahl von Einfangoligonukleotiden umfasst, wobei jedes Einfangoligonukleotid aus der Vielzahl:
eine Priming-Sequenz;
eine Einfangsequenz; und
eine Barcode-Sequenz umfasst, wobei die Barcode-Sequenz drei oder mehr kassettierbare Oligonukleotidsequenzen umfasst, wobei jede kassettierbare Oligonukleotidsequenz nicht-identisch zu den übrigen kassettierbaren Oligonukleotidsequenzen der Barcode-Sequenz ist, und
wobei jedes Einfangoligonukleotid aus der Vielzahl die gleiche Barcode-Sequenz umfasst,
Identifizieren der Barcode-Sequenz der Vielzahl von Einfangoligonukleotiden in-situ und Aufzeichnen einer Assoziation zwischen der identifizierten Barcode-Sequenz und dem Sequestrierungsstift;
Einbringen der biologischen Zelle in den Sequestrierungsstift;
Lysieren der biologischen Zelle und Ermöglichen, dass aus der lysierten biologischen Zelle freigesetzte Nukleinsäuren durch die Vielzahl von Einfangoligonukleotiden, die von dem Einfangobjekt beinhaltet sind, eingefangen werden;
Transkribieren der eingefangenen Nukleinsäuren, wodurch eine Vielzahl von barcodierten cDNAs produziert wird, wobei jede barcodierte cDNA eine komplementäre eingefangene Nukleinsäuresequenz, kovalent gebunden an eines der Einfangoligonukleotide, umfasst;
Sequenzieren der transkribierten Nukleinsäuren und der Barcode-Sequenz, wodurch Ablese-Sequenzen der Vielzahl von transkribierten Nukleinsäuren, die mit AbleseSequenzen der Barcode-Sequenz assoziiert sind, erhalten werden;
Identifizieren der Barcode-Sequenz basierend auf den Ablese-Sequenzen; und
Verwenden der Ablese-Sequenz-identifizierten Barcode-Sequenz und der in-situ-identifizierten Barcode-Sequenz, um die Ablese-Sequenzen der Vielzahl von transkribierten Nukleinsäuren mit dem Sequestrierungsstift in Verbindung zu bringen und dadurch die Ablese-Sequenzen der Vielzahl von transkribierten Nukleinsäuren mit der in den Sequestrierungsstift platzierten biologischen Zelle zu korrelieren.

20. Verfahren nach Anspruch 19, ferner umfassend:
Beobachten eines Phänotyps der biologischen Zelle; und
Korrelieren der Ablese-Sequenzen der Vielzahl von transkribierten Nukleinsäuren mit dem Phänotyp der biologischen Zelle.

21. Verfahren nach einem der Ansprüche 19-20, wobei das In-situ-Identifizieren der Barcode-Sequenz der Vielzahl von Einfangoligonukleotiden das Durchführen des Verfahrens nach einem der Ansprüche 13-18 umfasst.

22. Verfahren nach einem der Ansprüche 19-21, ferner umfassend:
Einbringen einer Vielzahl von Einfangobjekten in eine entsprechende Vielzahl von Sequestrierungsstiften der mikrofluidischen Vorrichtung;
Einbringen einer Vielzahl von biologischen Zellen in die entsprechende Vielzahl von Sequestrierungsstiften, und
Verarbeiten jeder von der Vielzahl von Einfangobjekten und der Vielzahl von biologischen Zellen gemäß der zusätzlichen Schritte des Verfahrens.

## Revendications

1. Pluralité d'objets de capture, chaque objet de capture de ladite pluralité étant un objet de capture comprenant une pluralité d'oligonucléotides de capture, chaque oligonucléotide de capture de ladite pluralité comprenant :
une séquence d'amorçage ;
une séquence de capture ; et
une séquence de code-barres comprenant trois séquences d'oligonucléotides cassettables ou plus, chaque séquence d'oligonucléotides cassettable étant non identique aux autres séquences d'oligonucléotides cassettables de ladite séquence de code-barres, et une séquence d'oligonucléotides cassettable étant une séquence d'oligonucléotides qui est l'une parmi un ensemble défini de séquences d'oligonucléotides, et,
chaque oligonucléotide de capture de ladite pluralité comprenant la même séquence de code-barres,
chaque séquence de code-barres desdits oligonucléotides de capture de chaque objet de capture parmi ladite pluralité étant différente de la séquence de code-barres des oligonucléotides de capture de tout autre objet de capture de ladite pluralité.

2. Pluralité d'objets de capture selon la revendication 1, chaque oligonucléotide de capture de ladite pluralité comprenant un nucléotide le plus en 5' et un nucléotide le plus en 3',
ladite séquence d'amorçage étant adjacente à ou comprenant ledit nucléotide le plus en 5',
ladite séquence de capture étant adjacente à ou comprenant ledit nucléotide le plus en 3', et
ladite séquence de code-barres étant située en 3' par rapport à ladite séquence d'amorçage et en 5' par rapport à ladite séquence de capture.

3. Pluralité d'objets de capture selon l'une quelconque des revendications 1 et 2, chacune desdites trois séquences d'oligonucléotides cassettables ou plus comprenant 8 à 12 nucléotides.

4. Pluralité d'objets de capture selon l'une quelconque des revendications 1 à 3, lesdites trois séquences d'oligonucléotides cassettables ou plus de ladite séquence de code-barres étant liées en tandem sans aucune séquence d'oligonucléotides interposée.

5. Pluralité d'objets de capture selon l'une quelconque des revendications 1 à 4, chacune desdites trois séquences d'oligonucléotides cassettables ou plus de ladite séquence de code-barres possédant une séquence parmi l'une quelconque parmi les SEQ ID NO: 1 à 40.

6. Pluralité d'objets de capture selon l'une quelconque des revendications 1 à 5, ladite séquence de code-barres comprenant quatre séquences d'oligonucléotides cassettables.

7. Pluralité d'objets de capture selon l'une quelconque des revendications 1 à 6, chaque oligonucléotide de capture parmi ladite pluralité comprenant en outre une séquence d'identificateur de molécule unique (UMI), et, éventuellement, ledit UMI étant situé en 3' par rapport à ladite séquence d'amorçage et en 5' par rapport à ladite séquence de capture.

8. Pluralité d'objets de capture selon l'une quelconque des revendications 1 à 7, chaque oligonucléotide de capture comprenant en outre un site de restriction comprenant une séquence de reconnaissance d'au moins 8 paires de bases.

9. Pluralité d'objets de capture selon l'une quelconque des revendications 1 à 7, ladite séquence de capture comprenant une séquence de poly-dT, une séquence d'hexamère aléatoire, une séquence spécifique à un gène, ou une séquence d'extrémité en mosaïque.

10. Ensemble de sondes d'hybridation distinguables spectralement, chaque sonde comprenant :
une séquence d'oligonucléotides non identiques comprenant une séquence parmi l'une quelconque parmi les SEQ ID NO: 41 à 80 et un marqueur fluorescent.

11. Réactif comprenant une pluralité de sondes d'hybridation, chaque sonde d'hybridation de ladite pluralité étant une sonde d'hybridation selon la revendication 10, et chaque sonde d'hybridation de ladite pluralité (i) comprenant une séquence d'oligonucléotides qui est non identique à la séquence d'oligonucléotides de toute autre sonde d'hybridation parmi la pluralité et (ii) comprenant un marqueur fluorescent qui est distinguable spectralement du marqueur fluorescent de toute autre sonde d'hybridation de ladite pluralité.

12. Réactif selon la revendication 11,
une première sonde d'hybridation parmi ladite pluralité comprenant une séquence choisie parmi un premier sous-ensemble parmi les SEQ ID NO: 41 à 80, et un premier marqueur fluorescent ;
une deuxième sonde d'hybridation parmi ladite pluralité comprenant une séquence choisie parmi un deuxième sous-ensemble parmi les SEQ ID NO: 41 à 80, et un deuxième marqueur fluorescent qui est distinguable spectralement dudit premier marqueur fluorescent,
une troisième sonde d'hybridation parmi ladite pluralité comprenant une séquence choisie parmi un troisième sous-ensemble parmi les SEQ ID NO: 41 à 80, et un troisième marqueur fluorescent qui est distinguable spectralement de chacun parmi lesdits premier et deuxième marqueurs fluorescents,
lesdits premier, deuxième et troisième sous-ensembles parmi les SEQ ID NO: 41 à 80 étant des sous-ensembles ne se chevauchant pas.

13. Procédé d'identification *in situ* d'un ou plusieurs objets de capture parmi une pluralité d'objets de capture selon les revendications 1 à 9 dans un dispositif microfluidique, ledit procédé comprenant :
la disposition d'un unique objet de capture parmi ledit ou lesdits objets de capture à l'intérieur d'une région d'isolement de chacune parmi une ou plusieurs aiguilles de séquestration situées à l'intérieur d'une enceinte dudit dispositif microfluidique, chaque objet de capture comprenant une pluralité d'oligonucléotides de capture, et chaque oligonucléotide de capture parmi ladite pluralité comprenant :
une séquence d'amorçage ;
une séquence de capture ; et
une séquence de code-barres, ladite séquence de code-barres comprenant trois séquences d'oligonucléotides cassettables ou plus, chaque séquence d'oligonucléotides cassettable étant non identique aux autres séquences d'oligonucléotides cassettables de ladite séquence de code-barres ; et
chaque oligonucléotide de capture de ladite pluralité comprenant ladite même séquence de code-barres ;
l'écoulement d'une première solution de réactif comprenant un premier ensemble de sondes d'hybridation dans une région d'écoulement à l'intérieur de ladite enceinte dudit dispositif microfluidique, ladite région d'écoulement étant reliée physiquement à chacune parmi ladite ou lesdites aiguilles de séquestration, et chaque sonde d'hybridation parmi ledit premier ensemble comprenant :
une séquence d'oligonucléotides complémentaire à une séquence d'oligonucléotides cassettable comprise dans l'une quelconque parmi lesdites séquences de code-barres d'un quelconque parmi lesdits oligonucléotides de capture de l'un quelconque parmi ledit ou lesdits objets de capture, ladite séquence d'oligonucléotides complémentaire de chaque sonde d'hybridation dans ledit premier ensemble étant non identique à toute autre séquence d'oligonucléotides complémentaire parmi lesdites sondes d'hybridation dans ledit premier ensemble ; et
un marqueur fluorescent choisi parmi un ensemble de marqueurs fluorescents distinguables spectralement, ledit marqueur fluorescent de chaque sonde d'hybridation dans ledit premier ensemble étant différent du marqueur fluorescent de toute autre sonde d'hybridation dans ledit premier ensemble de sondes d'hybridation ;
l'hybridation desdites sondes d'hybridation dudit première ensemble avec des séquences d'oligonucléotides cassettables correspondantes dans l'une quelconque parmi lesdites séquences de code-barres de l'un quelconque parmi lesdits oligonucléotides de capture de l'un quelconque parmi ledit ou lesdits objets de capture ;
la détection, pour chaque sonde d'hybridation parmi ledit premier ensemble de sondes d'hybridation, d'un signal fluorescent correspondant associé à l'un quelconque parmi ledit ou lesdits objets de capture ; et
la génération d'un enregistrement, pour chaque objet de capture disposé à l'intérieur de l'une parmi ladite ou lesdites aiguilles de séquestration, comprenant (i) un emplacement de ladite aiguille de séquestration à l'intérieur de ladite enceinte dudit dispositif microfluidique, et (ii) une association ou une non-association dudit signal fluorescent correspondant de chaque sonde d'hybridation dudit premier ensemble de sondes d'hybridation avec ledit objet de capture, ledit enregistrement d'associations ou de non-associations constituant un code-barres qui lie ledit objet de capture avec ladite aiguille de séquestration.

14. Procédé selon la revendication 13, comprenant en outre :
l'écoulement d'une solution de n^{ème} réactif comprenant un n^{ème} ensemble de sondes d'hybridation dans ladite région d'écoulement dudit dispositif microfluidique, chaque sonde d'hybridation dudit n^{ème} ensemble comprenant :
une séquence d'oligonucléotides complémentaire à une séquence d'oligonucléotides cassettable comprise dans l'une quelconque parmi lesdites séquences de code-barres d'un quelconque parmi lesdits oligonucléotides de capture de l'un quelconque parmi ledit ou lesdits objets de capture, ladite séquence d'oligonucléotides complémentaire de chaque sonde d'hybridation dans ledit n^{ème} ensemble étant non identique à toute autre séquence d'oligonucléotides complémentaire parmi lesdites sondes d'hybridation dans ledit n^{ème} ensemble et tout autre ensemble de sondes d'hybridation s'étant écoulé dans ladite région d'écoulement dudit dispositif microfluidique ; et
un marqueur fluorescent choisi parmi un ensemble de marqueurs fluorescents distinguables spectralement, ledit marqueur fluorescent de chaque sonde d'hybridation dans ledit n^{ème} ensemble étant différent du marqueur fluorescent de toute autre sonde d'hybridation dans ledit n^{ème} ensemble de sondes d'hybridation ;
l'hybridation desdites sondes d'hybridation parmi ledit n^{ème} ensemble avec des séquences d'oligonucléotides cassettables correspondantes dans l'une quelconque parmi lesdites séquences de code-barres de l'un quelconque parmi lesdits oligonucléotides de capture de l'un quelconque parmi ledit ou lesdits objets de capture ;
la détection, pour chaque sonde d'hybridation dudit n^{ème} ensemble de sondes d'hybridation, d'un signal fluorescent correspondant associé à l'un quelconque parmi ledit ou lesdits objets de capture ; et
la complémentation dudit enregistrement, pour chaque objet de capture disposé à l'intérieur de l'une parmi ladite ou lesdites aiguilles de séquestration, avec une association ou une non-association dudit signal fluorescent correspondant de chaque sonde d'hybridation dudit n^{ème} ensemble de sondes d'hybridation avec ledit objet de capture,
n étant un ensemble d'entiers positifs possédant des valeurs de {2,..., m},
m étant un entier positif possédant une valeur de 2 ou plus,
les étapes précédentes d'écoulement dudit n^{ème} réactif, d'hybridation dudit n^{ème} ensemble de sondes d'hybridation, de détection desdits signaux fluorescents correspondants, et de complémentation desdits enregistrement étant répétées pour chaque valeur de n dans ledit ensemble d'entiers positifs {2,..., m}, et,
m possédant une valeur supérieure ou égale à 3 et inférieure ou égale à 20.

15. Procédé selon l'une quelconque des revendications 13 et 14, chaque séquence de code-barres de chaque oligonucléotide de capture de chaque objet de capture comprenant trois ou quatre séquences d'oligonucléotides cassettables.

16. Procédé selon l'une quelconque des revendications 14 et 15, ledit premier ensemble de sondes d'hybridation et chacun parmi lesdits n^{èmes} ensembles de sondes d'hybridation comprenant trois ou quatre sondes d'hybridation.

17. Procédé selon l'une quelconque des revendications 13 à 16, comprenant en outre la disposition d'une ou plusieurs cellules biologiques à l'intérieur de ladite ou desdites aiguilles de séquestration dudit dispositif microfluidique, chacune parmi ladite ou lesdites cellules biologiques étant disposée dans une aiguille différente parmi ladite ou lesdites aiguilles de séquestration.

18. Procédé selon l'une quelconque des revendications 13 à 17, ladite enceinte dudit dispositif microfluidique comprenant en outre une configuration diélectrophorétique (DEP), et la disposition dudit ou desdits objets de capture dans une ou plusieurs aiguilles de séquestration étant réalisée en utilisant une force diélectrophorétique (DEP), et, éventuellement,
la disposition de ladite ou desdites cellules biologiques à l'intérieur de ladite ou desdites aiguilles de séquestration étant réalisée en utilisant des forces diélectrophorétiques (DEP).

19. Procédé de corrélation de données génomiques avec une cellule biologique dans un dispositif microfluidique, comprenant :
la disposition d'un objet de capture d'une pluralité d'objets de capture selon les revendications 1 à 9, dans une aiguille de séquestration d'un dispositif microfluidique, ledit objet de capture comprenant une pluralité d'oligonucléotides de capture, chaque oligonucléotide de capture de ladite pluralité comprenant :
une séquence d'amorçage ;
une séquence de capture ; et
une séquence de code-barres, ladite séquence de code-barres comprenant trois séquences d'oligonucléotides cassettables ou plus, chaque séquence d'oligonucléotides cassettable étant non identique aux autres séquences d'oligonucléotides cassettables de ladite séquence de code-barres ; et
chaque oligonucléotide de capture de ladite pluralité comprenant ladite même
séquence de code-barres ;
l'identification de ladite séquence de code-barres de ladite pluralité d'oligonucléotides de capture *in situ* et l'enregistrement d'une association entre ladite séquence de code-barres identifiée et ladite aiguille de séquestration ;
la disposition de ladite cellule biologique dans ladite aiguille de séquestration ;
la lyse de ladite cellule biologique et le fait de permettre à des acides nucléiques libérés de ladite cellule biologique lysée d'être capturés par ladite pluralité d'oligonucléotides de capture compris dans ledit objet de capture ;
la transcription desdits acides nucléiques capturés, produisant ainsi une pluralité d'ADNc à code-barres, chaque ADNc à code-barres comprenant une séquence d'acides nucléiques capturée complémentaire liée de manière covalente à l'un parmi lesdits oligonucléotides de capture ;
le séquençage desdits acides nucléiques transcrits et de ladite séquence de code-barres, obtenant ainsi des séquences de lecture de ladite pluralité d'acides nucléiques transcrits associées à des séquences de lecture de ladite séquence de code-barres ;
l'identification de ladite séquence de code-barres sur la base desdites séquence de lecture ; et
l'utilisation de ladite séquence de code-barres à séquence de lecture identifiée et de ladite séquence de code-barres identifiée *in situ* pour lier lesdites séquences de lecture de ladite pluralité d'acides nucléiques transcrits avec ladite aiguille de séquestration et ainsi corréler lesdites séquences de lecture de ladite pluralité d'acides nucléiques transcrits avec ladite cellule biologique placée dans ladite aiguille de séquestration.

20. Procédé selon la revendication 19, comprenant en outre :
l'observation d'un phénotype de ladite cellule biologique ; et
la corrélation desdites séquences de lecture de ladite pluralité d'acides nucléiques transcrits avec ledit phénotype de ladite cellule biologique.

21. Procédé selon l'une quelconque des revendications 19 et 20, l'identification de ladite séquence de code-barres de ladite pluralité d'oligonucléotides de capture *in situ* comprenant la réalisation du procédé selon l'une des revendications 13 à 18.

22. Procédé selon l'une quelconque des revendications 19 à 21, comprenant en outre :
la disposition d'une pluralité d'objets de capture dans une pluralité correspondante d'aiguilles de séquestration dudit dispositif microfluidique ;
la disposition d'une pluralité de cellules biologiques dans ladite pluralité correspondante d'aiguilles de séquestration, et
le traitement de chacun parmi ladite pluralité d'objets de capture et ladite pluralité de cellules biologiques selon lesdites étapes supplémentaires dudit procédé.
